# EUROPEAN PATENT APPLICATION

(11) **EP 2 471 536 A1**
(43) Date of publication of application: **04.07.2012**
(21) Application number: 12161344.2
(22) Date of filing: 29.09.2005
(51) Int. Cl.: A61K 31/567, C07J 1/00, A61P 7/00, A61P 29/00, A61P 11/00, A61P 11/06, A61P 19/08, A61P 19/10

(54) **STEROID ANALOGS AND CHARACTERIZATION AND TREATMENT METHODS**

(30) Priority: 29.09.2004 US 614869 P
(62) Divisional of application: 05857726.3
(71) Applicant: Harbor BioSciences, Inc., San Diego, CA 92122 (US)
(72) Inventor: Frincke, James, San Diego, CA 92122 (US); Reading, Christopher, San Diego, CA 92192 (US); Dowding, Charles, San Diego, CA 92124 (US)
(74) Representative: Huenges, Martin

(57) **Abstract**

The invention relates to methods to characterize exemplified compounds such as 3β,17β-dihydroxyandrost-1,5,11-triene and 3β,17β-dihydroxy-17α-ethynylandrost-1,5,11-triene and to the use of described compounds to ameliorate or treat a condition such as thrombocytopenia, inflammation or other exemplified conditions.

## Description

### FIELD OF THE INVENTION

The invention relates to the characterization and use of compounds to treat blood cell deficiencies such as neutropenia, thrombocytopenia, unwanted inflammation conditions such as asthma, cystic fibrosis or obstructive pulmonary disorders, trauma, unwanted bone loss conditions such as osteoporosis or glucocorticoid- or trauma-associated bone loss and other exemplified conditions. Methods to use and characterize the compounds are also provided.

### BACKGROUND

U.S. patents 4908358 and 4902681 describe the capacity of compounds such as 5α-pregnan-3,20-dione, cortexolone, 17-hydroxyprogesterone and 16α-methylprogesterone to inhibit the clearance of antibody-coated cells from circulation in disorders such as immune thrombocytopenic purpura or immune hemolytic anemia.

U.S. patents 5532230, 5686438, 5753640 and 5811418 and J. Bratt and M. Heimburger, Scand. J. Rheumatol. 1999 28:308-313 describe the capacity of compounds such as prednisolone, and 3β-hydroxyandrost-5-ene-17-one to limit tissue damage in ischemic tissues by inhibiting adhesion of cells such as neutrophils to endothelial cells or to treat pulmonary hypertension.

U.S. patent 5859000 describes the capacity compounds such as 3β-hydroxyandrost-5-ene-17-one to reduce mast cell mediated allergic reactions.

U.S. patents 5763433 and 6372732 and PCT publication WO 96/35428 describe the capacity of certain androstane and androstene compounds such as 3β-hydroxyandrost-5-ene-17-one to treat certain immune disorder conditions such as systemic lupus erythematosus.

U.S. patents 5925630, 5939545 and 5962443 describe the capacity of 19-nur-pregnane steroids, 3α-hydroxy-5a-pregnan-20-one and related steroids to modulate certain neurological activities such as hypothalamic function and GABA receptor activity.

### DESCRIPTION OF THE INVENTION

Summary of invention embodiments. In principal embodiments the invention provides steroid compounds, methods to characterize them, formulations that contain the compounds and therapeutic treatment methods using the compounds.

The methods include a method to prevent, treat, ameliorate or slow the progression of one or more of a blood cell deficiency, unwanted inflammation, allergy, immune suppression condition, immunosenescence, autoimmune disorder, infection, cancer or precancer, neurological disorder, cardiovascular disorder, pulmonary disorder, trauma, hemorrhage, bone fracture, unwanted or excess bone loss, androgen deficiency, estrogen deficiency, a congenital or hereditary disorder or a symptom of any of these conditions in a subject who has the condition or who is subject to developing the condition, comprising administering to a subject, or delivering to the subject's tissues, an effective amount of a formula 1 compound

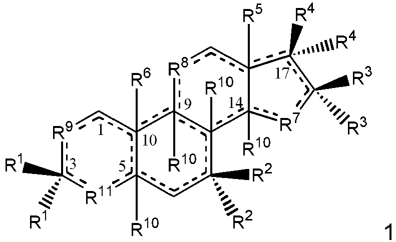

or a metabolic precursor, a metabolite, salt or tautomer thereof, wherein the dotted lines are optional double bonds and 0, 1, 2, 3, 4 or 5 double bonds, some of which may be conjugated, each R¹, R², R³, R⁴, R⁵, R⁶ and R¹⁰ independently or together are -H, -OH, -OR^{PR}, -SR^{PR}, -SH, -N(R^{PR})₂, -NHR^{PR}, -NH₂, -O-Si-(R¹³)₃, -CHO, -CHS, -CN, -SCN, -NO₂, -N₃, -COOH, -COOR^{PR}, -OSO₃H, -OSO₂H, -OPO₃H₂, =O, =S, =N-OH, =N-OCH₃, =CH₂, =CH-CH₃, =CH-optionally substituted alkyl, ester, thioester, thionoester, phosphoester, phosphothioester, phosphonate, phosphonate ester, thiophosphonate, thiophosphonate ester, phosphiniester, sulfite ester, sulfate ester, sulfamate, sulfonate, sulfonamide, amide, amino acid, peptide, ether, thioether, acyl, thioacyl, carbonate, carbamate, halogen, optionally substituted alkyl, optionally substituted alkenyl, optionally substituted alkynyl, optionally substituted aryl, optionally substituted heteroaryl, optionally substituted heterocycle, optionally substituted monosaccharide, optionally substituted oligosaccharide, polymer, spiro ring, epoxide, acetal, thioacetal, ketal, thioketal, -S-S-optionally substituted alkyl, =N-O-optionally substituted alkyl, =N-optionally substituted alkyl, -NH-optionally substituted alkyl, -NH-S(O)(O)-optionally substituted alkyl, -N(optionally substituted alkyl)₂ where each optionally substituted alkyl is independently selected, or, one or more of two adjacent R¹, R², R³, R⁴, R⁵, R⁶ and R¹⁰ comprise an independently selected epoxide or optionally substituted saturated or unsaturated cyclopropyl, cyclobutyl, cyclopentyl, cyclohexyl, cycloheptyl or cyclooxyl ring any of which rings optionally contain a ring heteroatom such as -O-, -S-, -NH- or =N-; R⁷ is -O-, -S-, -S(O)(O)-, -NR^{PR}-, -C(R¹⁰)₂-, -C(R¹⁰)₂-C(R¹⁰)₂-, -C(R¹⁰)₂-C(R¹⁰)₂-C(R¹⁰)₂-, - C(R¹⁰)₂-O-C(R¹⁰)₂-, -C(R¹⁰)₂-S-C(R¹⁰)₂-, -C(R¹⁰)₂-NR^{PR}-C( R¹⁰)₂-, -O-C(R¹⁰)₂-, -S-C(R¹⁰)₂-or-NR^{PR}-C(R¹⁰)₂-, where each R¹⁰ is independently selected; R⁸ and R⁹ independently are -C(R¹⁰)₂-, -C(R¹⁰)₂-C(R¹⁰)₂-, -O-, -O-C(R¹⁰)₂-, -S-, -S(O)(O)-, -S-C(R¹⁰)₂-, -S(O)(O)-C(R¹⁰)₂-, -NR^{PR}- or -NR^{PR}-C(R¹⁰)₂-, or one or both of R⁸ or R⁹ independently are absent, leaving a 5-membered ring, where each R¹⁰ is independently selected; R¹¹ is -O-, -S-, - S(O)(O)-, -NR^{PR}-, -CH₂-, -CHR¹⁰-, -C(R¹⁰)₂-, -C(R¹⁰)₂-O-C(R¹⁰)₂-, -C(R¹⁰)₂-S-C(R¹⁰)₂-, - C(R¹⁰)₂-S(O)(O)-C(R¹⁰)₂-, -C(R¹⁰)₂-NR^{PR}-C(R¹⁰)₂-, -O-C(R¹⁰)₂-, -S-C(R¹⁰)₂-, -S(O)(O)-C(R¹⁰)₂- or-NR^{PR}-C(R¹⁰)₂-, where each R¹⁰ is independently selected; R¹³ independently is C₁₋₆ alkyl; R^{PR} independently are -H or a protecting group; and optionally wherein one, two or three of the 1-, 4-, 6- and/or 12-positions are optionally substituted with (i) an independently selected R¹⁰ moiety when a double bond is present at the corresponding 1-, 4-, 6- or 12-position, or (ii) one or two independently selected R¹⁰ moieties when no double bond is present at the corresponding 1-, 4-, 6- and/or 12-position.

Other embodiments include (1) compositions that comprise a formula 1 compound and one or more other compounds such as an excipient(s) or a reactant or by-product of synthesis of the formula 1 compound, (2) formulations that comprise a formula 1 compound and 1, 2, 3, 4, 5, 6 or more excipients and (3) compositions that comprise partially purified or purified formula 1 compounds, optionally in a composition that comprises 1, 2, 3, 4, 5, 6 or more excipients and/or other compounds. The formulations can be designed for human or pharmaceutical use or they can be suitable for veterinary use. Therapeutic uses include the use of a formula 1 compound for the preparation of a medicament and use of a formula 1 compound for the preparation of a medicament for the prophylaxis, treatment or amelioration of a condition or symptom disclosed herein. Other embodiments are as described elsewhere in the specification or the claims.

Definitions. As used herein and unless otherwise stated or implied by context, terms that are used herein have the meanings defined below. Unless otherwise contraindicated or implied, e.g., by including mutually exclusive elements or options, in these definitions and throughout this specification, the terms "a" and "an" mean one or more and the term "or" means and/or.

Reference to an androstene compound, e.g., 3,16α,17β-trihydroxyandrost-3,6-diene, means that the hydrogen atom or other moiety at the 5-position is in the α-configuration, which is sometimes specified in the compound name, e.g., 3,16α,17β-trihydroxy-5α-androst-3,6-diene. For androstanes with hydrogen at the 5-position in the β-configuration, the compound name will specify this configuration, e.g., 3,16α,17β-trihydroxy-5β-androst-3,6-diene, unless the configuration is otherwise apparent from a chemical structure or from context. For androstanes or androstenes, hydrogen atoms or other R¹⁰ moieties at the 8-, 9- and 14-position, are in the β-, α- and α-configurations respectively, unless otherwise specified, e.g., by chemical structure, or implied by context.

As is apparent from the formula 1 structure, one or more variable groups may be absent when a double bond is present. Thus, when the compound contains an 8(9) double bond, R¹⁰ at the 8- and 9-positions are both absent. Similarly, when a double bond is present at the 3-position one R¹ moiety will be absent and when a double bond is present at the 16-position one R³ moiety and one R⁴ moiety will be absent.

A "formulation", "pharmaceutical formulation" or the like means a composition that one can administer to a subject, e.g., human, mammal or other animal, usually without further manipulations that change the ingredients or the ingredient proportions that are present. Formulations include powders or other preparations that are prepared for use by addition of one or more liquids that act as solvents or suspension vehicles. Formulations will typically comprise a single formula 1 compound and one or more excipients. Formulations are suitable for human or veterinary applications and would typically have expected characteristics for the formulation, e.g., parenteral formulations for human use would usually be sterile and stored in a suitable closed container.

An "excipient", "carrier", "pharmaceutically acceptable excipient", "pharmaceutically acceptable carrier" or similar terms mean one or more component(s) or ingredient(s) that is acceptable in the sense of being compatible with the other ingredients of invention compositions or formulations and not overly deleterious to the patient, animal, tissues or cells to which the F1 C, composition or formulation is to be administered.

The terms "effective amount", "effective dose" or the like with reference to a F1C(s) mean an amount of the F1C(s) that is sufficient to elicit a desired or detectable response, e.g., detectable restoration of normal immune responsiveness in an immunodeficient subject to which it is administered, e.g., a human, or to detectable modulation or amelioration of cellular parameter or a clinical condition or symptom or a detectable amount for analytical or other characterization use.

At various locations in the present disclosure, e.g., in any disclosed embodiments or in the claims, reference is made to compounds, compositions, formulations, or methods that "comprise" one or more specified components, elements or steps. Invention embodiments also specifically include those compounds, compositions, formulations or methods that are or that consist of or that consist essentially of those specified components, elements or steps. The terms "comprising", "consist of" and "consist essentially of " have their normally accepted meanings under U.S. patent law. For example, disclosed compositions or methods that "comprise" a component or step are open and they include or read on those compositions or methods plus an additional component(s) or step(s). Similarly, disclosed compositions or methods that "consist of" a component or step are closed and they would not include or read on those compositions or methods having appreciable amounts of an additional component(s) or an additional step(s).

"Alkyl" as used here means linked normal, secondary, tertiary or cyclic carbon atoms, i.e., linear, branched, cyclic or any combination thereof. Alkyl moieties, as used herein, may be saturated, or unsaturated, i.e., the moiety may comprise one, two, three or more independently selected double bonds or triple bonds. Unsaturated alkyl moieties include moieties as described for alkenyl, alkynyl and aryl moieties described below. The number of carbon atoms in an alkyl group or moiety can vary and typically is 1 to about 50, e.g., about 1-30 or about 1-20, unless otherwise specified, e.g., C₁₋₈ alkyl or C1-C8 alkyl means an alkyl moiety containing 1, 2, 3, 4, 5, 6, 7 or 8 carbon atoms. Unless otherwise specified, alkyl groups will contain 1, 2, 3, 4, 5, 6, 7, 8, 9, 10, 11, 12, 13, 14, 15, 16, 17, 18, 19, 20, 21, 22, 23, 24, 25, 26, 27, 28, 29, 30 or more carbon atoms, typically from 1 to 20 carbon atoms or from 1 to 8 carbon atoms. When an alkyl group is specified, species may include methyl, ethyl, 1-propyl (n-propyl), 2-propyl (i-propyl, -CH(CH₃)₂), 1-butyl (n-butyl), 2-methyl-1-propyl (i-butyl, -CH₂CH(CH₃)₂), 2-butyl (s-butyl, -CH(CH₃)CH₂CH₃), 2-methyl-2-propyl (t-butyl, -C(CH₃)₃), amyl, isoamyl, sec-amyl, 1-pentyl (n-pentyl), 2-pentyl (-CH(CH₃)CH₂CH₂CH₃), 3-pentyl (-CH(CH₂CH₃)₂), 2-methyl-2-butyl (-C(CH₃)₂CH₂CH₃), 3-methyl-2-butyl (-CH(CH₃)CH(CH₃)₂), 3-methyl-1-butyl (-CH₂CH₂CH(CH₃)₂), 2-methyl-1-butyl (-CH₂CH(CH₃)CH₂CH₃), 1-hexyl, 2-hexyl (-CH(CH₃)CH₂CH₂CH₂CH₃), 3-hexyl (-CH(CH₂CH₃)(CH₂CH₂CH₃)), 2-methyl-2-pentyl (-C(CH₃)₂CH₂CH₂CH₃), 3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH₃)₂), 2-methyl-3-pentyl (-CH(CH₂CH₃)CH(CH₃)₂), 2,3-dimethyl-2-butyl (-C(CH₃)₂CH(CH₃)₂), 3,3-dimethyl-2-butyl (-CH(CH₃)C(CH₃)₃), cyclopropyl (-CH<CH₂CH₂), cyclobutyl (-CH<CH₂CH₂CH₂), 1-methylcyclobutyl (-CH<CH(CH₃)CH₂CH₂), 1,2-dimethylpropyl, 1,1-dimethylpropyl, hexyl, 4-methylpentyl, 1-methylpentyl, 2-methylpentyl, 3-methylpentyl, 1,1-dimethylbutyl, 2,2-dimethylbutyl, 3,3-dimethylbutyl, 1,2-dimethylbutyl, 1,3-dimethylbutyl, 1,2,2,-trimethylpropyl, 1,1,2-trimethylpropyl, heptyl, 5-methylhexyl, 1-methylhexyl, 2,2-dimethylpentyl, 3,3-dimethylpentyl, 4,4-dimethylpentyl, 1,2-dimethylpentyl, 1,3-dimethylpentyl, 1,4-dimethylpentyl, 1,2,3,-trimethylbutyl, 1,1,2-trimethylbutyl, 1,1,3-trimethylbutyl, normal or branched octyl, 6-methylheptyl, 1-methylheptyl, 1,1,3,3-tetramethylbutyl, normal or branched nonyl, 1-, 2-, 3-, 4-, 5-, 6- and 7-methyloctyl, 1-, 2-, 3-, 4-, 5-ethylheptyl, 1-, 2- and 3-propylhexyl, decyl, 1-, 2-, 3-, 4-, 5-, 6-, 7- and 8-methylnonyl, 1- 2-, 3-, 4-, 5- and 6-ethyloctyl, 1-, 2-, 3- and 4-propylheptyl, undecyl 1-, 2-, 3-, 4-, 5-, 6-, 7-, 8- and 9-methyldecyl, 1-, 2-, 3-, 4-, 5-, 6- and 7-ethylnonyl, 1-, 2-, 3-, 4- and 5-propyloctyl, 1-, 2- and 3-butyloctyl, 1-pentylhexyl, dodecyl, 1-, 2-, 3-, 4-, 5-, 6-, 7-, 8-, 9- and 10-methylundecyl, 1-, 2-, 3-, 4-, 5-, 6-, 7- and 8-ethyldecyl, 1-, 2-, 3-, 4-, 5- and 6-propylnonyl, 1-, 2-, 3- and 4-butyloctyl, 1-2-pentylheptyl, cyclopropyl, cyclobutyl, cyclopentyl, cyclohexyl, cycloheptyl, cyclooctyl, cyclononyl, cyclodecyl, -(CH₂)ₙ-(CHCH₃)ₘ-(CH₂)ₒ-CH₃, -(CH₂)ₙ-(CHC₂H₅)ₘ-(CH₂)ₒ-CH₃ and positional isomers of any of these moieties that can have one or more positional isomers, where n, m and o independently are 0, 1, 2, 3, 4, 5, 6, 7 or 8. Alkyl also includes species and groups described below for alkenyl, alkynyl groups, aryl groups, arylalkyl groups alkylaryl groups and the like. "Alkyl" thus includes vinyl, ethynyl, 1-propynyl and the like.

"Alkenyl" as used here means a moiety that comprises linked normal, secondary, tertiary or cyclic carbon atoms, i.e., linear, branched, cyclic or any combination thereof, that comprises one or more double bonds (-CH=CH-), e.g., 1, 2, 3, 4, 5, 6 or more, typically 1, 2 or 3, which can include an aryl moiety such as benzene. The number of carbon atoms in an alkenyl group or moiety can vary and typically is 2 to about 50, e.g., about 2-30 or about 2-20, unless otherwise specified, e.g., C₂₋₈ alkenyl or C2-8 alkenyl means an alkenyl moiety containing 2, 3, 4, 5, 6, 7 or 8 carbon atoms. Alkenyl groups will typically have 2, 3, 4, 5, 6, 7, 8, 9, 10, 11, 12, 13, 14, 15, 16, 17, 18, 18 or 20 carbon atoms. When an alkenyl group is specified, species include, e.g., any of the alkyl moieties described above that has one or more double bonds, methylene (=CH₂), methylmethylene (=CH-CH₃), ethylmethylene (=CH-CH₂-CH₃), =CH-CH₂-CH₂-CH₃, vinyl (-CH=CH₂), allyl, 1-methylvinyl, butenyl, iso-butenyl, 3-methyl-2-butenyl, 1-pentenyl, cyclopentenyl, 1-methyl-cyclopentenyl, 1-hexenyl, 3-hexenyl, cyclohexenyl, 1-heptenyl, 3-heptenyl, 1-octenyl, cyclooctenyl, 1-nonenyl, 2-nonenyl, 3-nonenyl, 1-decenyl, 3-decenyl, 1,3-butadienyl, 1,4-pentadienyl, 1,3-cyclopentadienyl, 1,3-hexadienyl, 1,4-hexadienyl, 1,3-cyclohexadienyl, 1,4-cyclohexaidenyl, 1,3-cycloheptadienyl, 1,3,5-cycloheptatrienyl, 1,3,5,7-cyclooctatetraenyl, -(CH₂)ₙ-(CH=CH)-(CH₂)ₘ-CH₃, -(CH₂)ₙ-(CCH₃=CH)-(CH₂)ₘ-CH₃, -(CH₂)ₙ-(CH=CCH₃)-(CH₂)ₘ-CH₃, -(CH₂)ₙ-(CH=CH)₀₋₁-(CH₂)ₘ-CH₂CH=CH₂ and -(CH₂)ₙ-(CH=CH)₀₋₁-(CH₂)ₘ-CH₂(CH=CH)₀₋₁-CH₃, where n and m independently are 0, 1, 2, 3, 4, 5, 6, 7 or 8. Unless otherwise specified, alkenyl groups will contain 2, 3, 4, 5, 6, 7, 8, 9, 10, 11, 12, 13, 14, 15, 16, 17, 18, 19, 20, 21, 22, 23, 24, 25, 26, 27, 28, 29, 30 or more carbon atoms, typically from 2 to 20 carbon atoms or from 2 to 8 carbon atoms.

"Alkynyl" as used here means a moiety that comprises linked normal, secondary, tertiary or cyclic carbon atoms, i.e., linear, branched, cyclic or any combination thereof, that comprises one or more triple bonds (-C≡C-), e.g., 1, 2, 3, 4, 5, 6 or more, typically 1 or 2 triple bonds, optionally comprising 1, 2, 3, 4, 5, 6 or more double bonds, with the remaining bonds being single bonds. The number of carbon atoms in an alkenyl group or moiety can vary and typically is 2 to about 50, e.g., about 2-30 or about 2-20, unless otherwise specified, e.g., C₂₋₈ alkynyl or C2-8 alkynyl means an alkynyl moiety containing 2, 3, 4, 5, 6, 7 or 8 carbon atoms. Alkynyl groups will typically have 2, 3, 4, 5, 6, 7, 8, 9, 10, 11, 12, 13, 14, 15, 16, 17, 18, 18 or 20 carbon atoms. When an alkynyl group is specified, species include, e.g., any of the alkyl moieties described above that has one or more double bonds, butynyl, iso-butynyl, 3-methyl-2-butynyl, 1-pentynyl, cyclopentynyl, 1-methyl-cyclopentynyl, 1-hexynyl, 3-hexynyl, cyclohexynyl, 1-heptynyl, 3-heptynyl, 1-octynyl, cyclooctynyl, 1-nonynyl, 2-nonynyl, 3-nonynyl, 1-decynyl, 3-decynyl, 1,3-butadiynyl, 1,4-pentadynyl, 1,3-pentadynyl, 1,3-hexadynyl, 1,4-hexadynyl, 1,5-hexadynyl, 1,3-heptadynyl, 1,3,5-heptatriynyl, 1,3,5,7-octatetraynyl, -CCH, -CCCH₃, -CCCH₂CH₃, -CCC₃H₇, -CCCH₂C₃H₇, -(CH₂)ₙ-(C≡C)-(CH₂)ₘ-CH₃, -(CH₂)ₙ-(C≡C)₀₋₁-(CH₂)ₘ-CH₂C≡CH, -(CH₂)ₙ-(C≡C)₀₋₁-(CH₂)ₘ-CH₂(C=C)₀₋₁-CH₃, -(CH₂)ₙ-(C≡C)-CH₂(C≡C)-(CH₂)ₘ-CH₃, where each n and m independently are 0, 1, 2, 3, 4, 5, 6, 7 or 8. Unless otherwise specified, alkynyl groups will contain 2, 3, 4, 5, 6, 7, 8, 9, 10, 11, 12, 13, 14, 15, 16, 17, 18, 19, 20, 21, 22, 23, 24, 25, 26, 27, 28, 29, 30 or more carbon atoms, typically from 2 to 20 carbon atoms or from 2 to 8 carbon atoms.

"Aryl" means an aromatic ring or fused ring system with no ring heteroatoms, e.g., phenyl or naphthyl.

"Alkylaryl" means a moiety where an alkyl group is bonded to an aryl group, i.e., -alkyl-aryl, where alkyl and aryl groups are as described above, e.g., -CH₂-C₆H₅ or -CH₂CH(CH₃)-C₆H₅.

"Arylalkyl" means a moiety where an aryl group is bonded to an alkyl group, i.e., -aryl-alkyl, where aryl and alkyl groups are as described above, e.g., -C₂H₄-CH₃ or -C₆H₄-CH₂CH(CH₃).

"Substituted alkyl", "substituted alkenyl", "substituted alkynyl", substituted alkylaryl", "substituted arylalkyl", "substituted heterocycle", "substituted aryl", "substituted monosaccharide" and the like mean an alkyl, alkenyl, alkynyl, alkylaryl, arylalkyl heterocycle, aryl, monosaccharide or other group or moiety as defined or disclosed herein that has a substituent(s) that replaces a hydrogen atom(s) or a substituent(s) that interrupts a carbon atom chain. Substituted heterocycles may thus have a substituent bonded to a ring carbon or a ring heteroatom such as nitrogen. Substituents for any of these moieties include 1, 2, 3, 4, 5, 6, 7, 8, 9, or 10 or more independently selected -O-, -S-, -NH-, -C(O)-, -C(O)OH, -C(O)OR^{15A}, -C(O)OR^{PR}, -C(O)SR^{15A}, -C(O)SR^{PR}, -CHO, - CHS, -CH₂SH, -C=N-, -OH, =O, -OR^{15A}, -OR^{PR}, -C(O)OR^{PR}, -O-C(O)H, -C(O)CH₃, - C(S)CH₃, -C(S)SH, -C(S)SR^{15A}, -C(S)SR^{PR}, -C(O)CH₂OH, -C(O)CH₂F, -C(O)CH₂Cl, - C(O)CH₂Br, -C(O)CH₂I, -C(O)CF₂H, -C(O)CF₃, -C(O)NHCH₃, -C(O)NHC₂H₅, - C(O)NHC(CH₃)₃, -O-CH₂-C(O)-C(CH₃)₃, -C(O)-C(CH₃)₃, -O-CH(CH₃)-O-C(CH₃)₃, - C(O)O-, -C(S)OR^{PR}, -C(S)O-, -OC(O)-, -C(O)H, -OCH₂-, -CH₂-O-CH₂-, -(CH₂)₁₋₂-O-(CH₂)₂, -OCH₂CH₂-, -OCH₂O-, -OCH₂CH₂O-, -CH₂OH, -CH₂F, -CHF₂, -CF₃, -CH₂Cl, - CH₂Br, -CH₂I, -C₂H₄Cl, -C₂H₄Br, -C₂H₄I, -CH₂CH₂F, -CH₂CHF₂, -CH₂CF₃, -NH₂, -NHR^{15A}, -N(R^{15A})₂, -N(R^{PR})₂, -NHR^{PR}, -NHC(O)-, -CH₂-NR^{PR}-, -CH₂-NHR^{PR}, -CH₂-NHC(O)-, - C(O)NH-, -C(O)NHR^{PR}, -OC(O)NR^{PR}-, -OC(O)NHR^{PR}, -C(=NH)-NH₂, -C(=NH)OH, -C(=N-NH₂)OH, -C(O)NHOH, =NOH, =NOCH₃, =NOC₂H₅, =NOC₃H₇, =NOC₄H₉, -NHR^{15A}, =NR^{15A}, =N-, -NR^{PR}C(O)NR^{PR}-, -NR^{PR}C(O)NHR^{PR}, -NR^{PR}CH₂-, -NR^{PR}CH₂CH₂-, -NO₂, ONO₂, -S-, -SH, -SR^{15A}, -SR^{PR}, =S, -S(O)R^{15A}, -S(O)OR^{15A}, -S(O)-, -S(O)(O)-, -O-S(O)(O)-NR^{PR}-, -O-S(O)(O)-NR^{PR}-CH₂-, -CH₂-O-S(O)(O)-NR^{PR}-, -CHR^{15A}-S(O)(O)-NR^{PR}-, -CHR^{15A}-S(O)(O)-NR^{PR}-CHR^{15A}-, -NH-S(O)(O)H, -CH₂-NH-S(O)(O)H, -CHR^{15A}-NH-S(O)(O)H, -O-S(O)(O)-CHR^{15A}-, -CHR^{15A}-O-S(O)(O)-, -CHR^{15A}-O-S(O)(O)-CHR^{15A}-, - S(O)(O)H, -CHR^{15A}-S(O)(O)H, -NH-S(O)(O)-NH-, -CHR^{15A}-NH-S(O)(O)-NH-, -CHR^{15A}-NH-S(O)(O)-NH-CHR^{15A}, -NH-S(O)(O)-NHR^{PR}, -NH-S(O)(O)-NH₂, -NH-S(O)(O)-NHCH₃, -NH-S(O)-NH-, -CHR^{15A}-NH-S(O)-NH-, -CHR^{15A}-NH-S(O)-NH-CHR^{15A}, -NH-S(O)-NHR^{PR}, -NH-S(O)-NH₂, -NH-S(O)-NHCH₃, -NH-S(O)-, -CHR^{15A}-NH-S(O)-, -NH-S(O)-CHR^{15A}, - S(O)-NHR^{PR}, -S(O)-NH₂, -S(O)-NHCH₃, -S(O)(O)-O-, -S(O)OR^{PR}, -S(O)(O)OH, -OSO₃H₂, -S(O)(O)OR^{15A}, -S(O)(O)OR^{PR}, -S(O)OH, -S(O)OR^{15A}, -S(O)OR^{PR}, -S(O)R^{15A}, -S(O)R^{PR}, - CN, -SCN, -C(O)OH, -C(O)OR^{15A}, -C(O)OR^{PR}, -C(O)SH, -C(O)SR^{15A}, -C(O)SR^{PR}, - C(S)OH, -C(S)OR^{15A}, -C(S)OR^{PR}, -O-P(O)(O)OH, -O-P(O)(O)OR^{15A}, -O-P(O)(O)OR^{PR}, - O-P(S)(O)OH, -O-P(S)(O)OR^{15A}, -O-P(S)(O)OR^{PR}, -O-P(O)(O)SH, -O-P(O)(O)SR^{15A}, -OP(O)(O)SR^{PR}, -F, -Cl, -Br, -I, -C=NH, -C=NCH₃, -C=NC₂H₅, -C(=S)-, -C₆H₅, -CH₂C₆H₅, - O-A8, -S-A8, -C(O)-A8, -OC(O)-A8, -C(O)O-A8, -OPO₃(R^{PR})₂, -amino acid-, -O-monosaccharide, -O-disaccharide, -S-monosaccharide, -S-disaccharide, a polymer, e.g., a PEG, and combinations of these moieties and salts on any of these moieties that can form a salt, where each R^{PR} independently is -H, an independently selected protecting group or both R^{PR} together are a protecting group, A8 is C1-C10 optionally substituted alkyl, and R^{15A} independently are -H, -CH₃, -C₂H₅, -C₃H₇, -C₄H₉, -C(CH₃)₃, -CH₂OH,-C₂H₄OH, -C₃H₆OH -C₄H₈OH -C(CH₂OH)(CH₃)₂ -C₃H₅, -C₄H₇, optionally substituted C1-10 alkyl, C1-10 perfluoroalkyl, optionally substituted aryl, optionally substituted C1-12 alkylaryl, optionally substituted C1-12 arylalkyl, optionally substituted allyl, optionally substituted heterocycle, optionally substituted C1-4 alkyl-optionally substituted heterocycle or optionally substituted heterocycle-optionally substituted C1-4 alkyl. Substituents are independently chosen when more than one is present. Alkenyl and alkynyl groups that comprise a substituent(s), are optionally substituted at a carbon that is one or more methylene moiety removed from the double bond, e.g., the substituent is optionally separated by one, two, three or more independently selected -CH₂-, -H(C₁₋₆ optionally substituted alkyl)-, -CH(C₁₋₆ optionally substituted alkenyl)-, -CH(C₁₋₆ optionally substituted alkynyl)-, -CH(optionally substituted heterocycle)-, -CH(optionally substituted aryl-optionally substituted alkyl)- or -CH(optionally substituted alkyl-optionally substituted aryl)- moieties. Other substituted alkenyl and alkynyl moieties include =CHOH, =CH-halogen, =CH-COOR^{PR}, =CH-(CH₂)ₘ-NH₂, =CH-(CH₂)ₘ-NH(C1-C6 alkyl), =CH-N(C1-C6 alkyl)₂, =CH-CH₂OH, =CH-CH₂-halogen, =CH-CH₂-COOR^{PR}, =CH-CH₂-NH₂, =CH-CH₂-NH(C1-C6 alkyl), =CH-CH₂-N(C1-C6 alkyl)₂, =CH-CH₂-CH₂OH, =CH-CH₂-CH₂-halogen, =CH-CHOH-CH₃, =CH-CHOH-CH₂-CH₃,.=CH-CH₂-CH₂-COOR^{PR}, =CH-CH₂-CH₂-NH₂, =CH-CH₂-CH₂-N(C1-C4 alkyl)₂, -CH=CH-(CH₂)ₘ-OH, -CH=CH-halogen, -CH=CH-CH₂OH, -CH=CH-CH₂-halogen, -C≡C-halogen, -C≡C-CH₂-NH₂, -C≡C-CH₂-NH(C1-C6 alkyl), -C=C-CH₂-N(C1-C6 alkyl)₂, -C≡C-OH, -C≡C-COOR^{PR}, -C≡C-CH₂-halogen, -C≡C-CH₂-OH and -C≡C-CH₂-COOR^{PR}, where each alkyl moiety is the same or different, e.g., both are methyl, ethyl or propyl or one is methyl and the other is ethyl, propyl or butyl and m is 1, 2, 3 or 4. The organic moieties and substitutions described here, and for other any other moieties described herein, usually will exclude obviously unstable moieties, e.g., -O-O-, except where such unstable moieties are transient species that one can use to make a compound such as a F1C with sufficient chemical stability for the one or more of the uses described herein.

"Optionally substituted alkyl", "optionally substituted alkenyl", "optionally substituted alkynyl", substituted alkylaryl", "optionally substituted arylalkyl", "optionally substituted heterocycle", "optionally substituted aryl", "optionally substituted monosaccharide" and the like mean an alkyl, alkenyl, alkynyl, alkylaryl, arylalkyl heterocycle, aryl, monosaccharide or other group or moiety as defined or disclosed herein that has a substituent(s) that optionally replaces a hydrogen atom(s) or a substituent(s) that interrupts a carbon atom chain. Such substituents are as described above.

For any group or moiety described by a given range of carbon atoms, the designated range means that any individual number of carbon atoms is described. Thus, reference to, e.g., "C1-C4 optionally substituted alkyl", "C₂₋₆ alkenyl", "C3-C8 optionally substituted heterocycle", or "optionally substituted alkenyl", specifically means that a 1, 2, 3 or 4 carbon optionally substituted alkyl moiety as defined herein is present, or a 2, 3, 4, 5 or 6 carbon alkenyl, or a 3, 4, 5, 6, 7 or 8 carbon moiety comprising a heterocycle or optionally substituted alkenyl moiety as defined herein is present. All such designations are expressly intended to disclose all of the individual carbon atom groups and thus "C1-C4 optionally substituted alkyl" includes, e.g., 3 carbon alkyl, 4 carbon substituted alkyl and 4 carbon alkyl, including all positional isomers and the like are disclosed and can be expressly referred to or named.

The term "O-linked moiety" means a moiety that is bonded through an oxygen atom. Thus, when an R¹ group, is an O-linked moiety, that R¹ is bonded to the steroid at the 3-position through oxygen and it can thus be =O, -O-S(O)(O)-OR^{PR}, ether, ester (e.g., -O-C(O)-optionally substituted alkyl), carbonate or a carbamate (e.g., -O-C(O)-NH₂ or-O-C(O)-NH-optionally substituted alkyl). Similarly, the term "S-linked moiety" means a moiety that is bonded through a sulfur atom. Thus, when an R⁴ group is an S-linked moiety, that R⁴ is bonded to the steroid at the 17-position through sulfur and it can thus be =S, thioether (e.g., -S-optionally substituted alkyl), thioester (-S-C(O)-optionally substituted alkyl) or a disulfide (e.g., -S-S-optionally substituted alkyl). The term "N-linked moiety" means a moiety that is bonded through a nitrogen atom. Thus, when when one or more of R², R³ or R⁴ group is an N-linked moiety, those R², R³ or R⁴ are bonded to the steroid at the 7-, 16- or 17-position respectively through nitrogen and one or more of these can thus be =NOH, =NOCH₃, =N-CH₃, an N-linked amino acid such as -NH-CH₂-COOH, a carbamate such as -NH-C(O)-O-optionally substituted alkyl, an amine such as -NH-optionally substituted alkyl, an amide such as -NH-C(O)-optionally substituted alkyl or -N₃. The term "C-linked moiety" means a moiety that is bonded through a carbon atom. Thus, when when one or more of R², R³ or R⁴ group is a C-linked moiety, those R², R³ or R⁴ are bonded to the steroid at the 7-, 16- or 17-position respectively through carbon and one or more of these can thus be -optionally substituted alkyl such as -CH₂-CH₂-O-CH₃, -C(O)-optionally substituted alkyl hydroxyalkyl, mercaptoalkyl, aminoalkyl or =CH-optionally substituted alkyl.

The term C-linked heterocycle means a heterocycle that is bonded to the steroid ring nucleus through a carbon atom, e.g. steroid-(CH₂)ₙ-heterocycle where n is 1, 2 or 3 or steroid-C<heterocycle where C< represents a carbon atom in a heterocycle ring. Similarly, R¹⁰ moieties that are N-linked heterocycles mean a heterocycle that is bonded to the steroid ring nucleus through a heterocycle ring nitrogen atom, e.g. steroid-N<heterocycle where N< represents a nitrogen atom in a heterocycle ring. A variable group such as R¹, R³, R⁴, R⁶, R^{10H} or other R¹⁰ moieties, e.g., at R⁸ or R⁹, that is bonded to a formula 1 compound can be a C-linked heterocycle or a N-linked heterocycle, These heterocycles include those listed below or described elsewhere herein.

"Halogen" or "halo" means fluorine, chlorine, bromine or iodine.

"Ester" means a moiety that contains a -C(O)-O- structure. Typically, esters as used here comprise an organic moiety containing about 1-50 carbon atoms (e.g., about 2-20 carbon atoms) and 0 to about 10 independently selected heteroatoms (e.g., O, S, N, P, Si), where the organic moiety is bonded to a formula 1 steroid nucleus at, e.g., R¹ or R² through the -C(O)-O- structure, e.g., organic moiety-C(O)-O-steroid organic moiety-O-C(O)-steroid. The organic moiety usually comprises one or more of any of the organic groups described herein, e.g., C₁₋₂₀ alkyl moieties, C₂₋₂₀ alkenyl moieties, C₂₋₂₀ alkynyl moieties, aryl moieties, C₂₋₉ heterocycles or substituted derivatives of any of these, e.g., comprising 1, 2, 3, 4 or more substituents, where each substituent is independently chosen. Exemplary substitutions for hydrogen or carbon atoms in these organic groups are as described above for substituted alkyl and other substituted moieties. Substitutions are independently chosen. The organic moiety includes compounds defined by the R₄ variable. The organic moieties exclude obviously unstable moieties, e.g., -O-O-, except where such unstable moieties are transient species that one can use to make a compound with sufficient chemical stability for one or more of the uses described herein, including for synthesis of the formula 1 or other compounds. The substitutions listed above are typically substituents that one can use to replace one or more carbon atoms, e.g., -O- or -C(O)-, or one or more hydrogen atom, e.g., halogen, - NH₂ or -OH. Exemplary esters include one or more independently selected acetate, enanthate, propionate, isopropionate, isobutyrate, butyrate, valerate, caproate, isocaproate, hexanoate, heptanoate, octanoate, nonanoate, decanoate, undecanoate, phenylacetate or benzoate, which are typically hydroxyl esters.

"Ether" means an organic moiety as described for ester that comprises 1, 2, 3, 4 or more -O- moieties, usually 1 or 2. In some embodiments, the -O- group is linked to the steroid nucleus at a variable group such as R¹, R², R³, R⁴, R⁵, R⁶, R¹⁰, R¹⁵, R¹⁷ or R¹⁸ , e.g., organic moiety-O-steroid. The organic moiety is as described above for esters.

"Acyl group" or "acyl" means an organic moiety as described for ester that comprises 1, 2, 3, 4 or more -C(O)- groups. In some embodiments, the -C(O)- group is linked to the steroid nucleus at a variable group such as R¹, R², R³, R⁴, R⁵, R⁶, R¹⁰, R¹⁵, R¹⁷ or R¹⁸ , e.g., organic moiety-C(O)-steroid. The organic moiety is as described above for esters. Exemplary acyl moieties include moieties such as -C(O)-N(C1-C6 alkyl)₂, - C(O)-NH(C1-C6 alkyl), -C(O)-NH-C(CH₃)₃, -C(O)-NH-CH(CH₃)₂, -C(O)-NH-C(CH₃)₂-CH₃, -C(O)-NH-CH(CH₃)-CH₃, -C(O)-NH-C(CH₃)-CH₂-CH₃, -C(O)NH₂, -C(O)NHR^{PR}, -C(O)-CH₃, -C(O)-CH₂-CH₃, -C(O)-CH₂-CH₂-CH₃, -C(O)-CH₂OH, -C(O)-CH₂OR^{PR}, -C(O)-CH₂-CH₂OH, -C(O)-CH₂-CH₂OR^{PR}, -C(O)-CH₂-halogen, -C(O)-CH₂-CH₂-halogen, -C(O)-CH₂-COOR^{PR}, -C(O)-CH₂-CH₂-COOR^{PR}, -C(O)-CH₂-CH₂-CHOH, -C(O)-CH₂-NH₂, -C(O)-CH₂-NHR^{PR}, -C(O)-CH₂-N(R^{PR})₂, -C(O)-CH₂-NH-(C1-C6 alkyl), -C(O)-CH₂-N(C1-C6 alkyl)₂, - C(O)-NH-CH=CH₂, -C(O)-NH-C≡CH, -C(O)-NH-CH₃, -C(O)-NH-CN, -C(O)-NH-CH₂-CN, where each alkyl is the same or different and is optionally independently substituted and each R^{PR} is -H or an independently selected protecting group for the atom or functional group to which it is attached, or two R^{PR} together are a protecting group for the atom or functional group to which they are attached.

Optionally substituted alkyl group, optionally substituted alkenyl group, optionally substituted alkynyl group, optionally substituted aryl moiety and optionally substituted heterocycle mean an alkyl, alkenyl, alkynyl, aryl or heterocycle moiety that contains an optional substitution(s). Such moieties include C₁₋₂₀ alkyl moieties, C₂₋₂₀ alkenyl moieties, C₂₋₂₀ alkynyl moieties, aryl moieties, C₂₋₉ heterocycles or substituted derivatives of any of these.

Position numbers that are given for the F1 Cs use the numbering convention for cholesterol.

Stereoisomers. The F1Cs include enriched or resolved optical isomers at any or all asymmetric atoms as are apparent from the depictions or are included in the compound structures. Both racemic and diasteromeric mixtures, as well as the individual optical isomers can be isolated or synthesized so as to be substantially free of their enantiomeric or diastereomeric partners, and these are all within the scope of the invention. Chiral centers may be found in F1Cs at, for example, one or more of R¹, R², R³, R⁴ or R¹⁰.

Embodiments of formula 1 compounds. For formula 1 compounds ("F1Cs"), 2, 3 or more of R¹, R², R³ and R⁴ are usually not -H, and typically one or both R¹ and R⁴, R³ and R⁴, R², R³ and R⁴ or R² and R⁴ are not -H, and/or 1 or 2 of R^{10A}, R^{10B}, R^{10C} and R^{10D} are optionally not -H. For any F1C disclosed herein, steroid nucleus carbon atoms that contain two variable groups (e.g., two R¹⁰ at R⁸ or R⁹ or two R³ or R⁴ at the 16- or 17-position), each variable group is independently selected and each can thus be the same or different, e.g., both can be methyl, ethyl, methoxy, ethoxy, -F, -Cl, - Br, -I, or they can be different. Exemplary F1C include compounds where no double bond is present at the 3-poistion, one R¹ is an O-linked, S-linked or N-linked moiety and the other R¹ is -H or a C-linked moiety or both R¹ together are =O or another double bonded moiety, and/or no double bond is present at the 17-poistion, one R⁴, in the α- or β-configuration, is an O-linked, S-linked or N-linked moiety and the other R⁴ is -H or a C-linked moiety and/or no double bond is present at the 16-poistion, one R³ is an O-linked, S-linked or N-linked moiety and the other R³ is -H or a C-linked moiety or both R³ are a halogen or together are =O or another double bonded moiety. Other embodiments are described below.

The formula 1 compounds may contain 0, 1, 2, 3, 4 or 5 carbon-carbon or carbon-nitrogen double bonds within the fused four-ring system, such that the compound is unsaturated.

As is apparent from the F1 C structure, when a double bond is present at a given position in any of these androstenes, one or two variable groups at the steroid ring atoms will be absent. Thus, when a double bond is present at the 16-position, one R³ and one R⁴ will be absent, when a double bond is present at the 5-position, R¹⁰ at the 5-position will be absent, when a double bond is present at the 5(10)-position, R¹⁰ at the 5-position and R⁶ will be absent as shown in the structures or when a double bond is present at the 5(10)- and 6-positions, R¹⁰ at the 5-position, R⁶ and one R² will be absent as shown in the structures 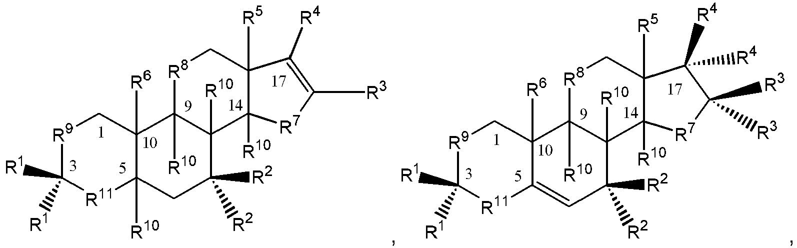 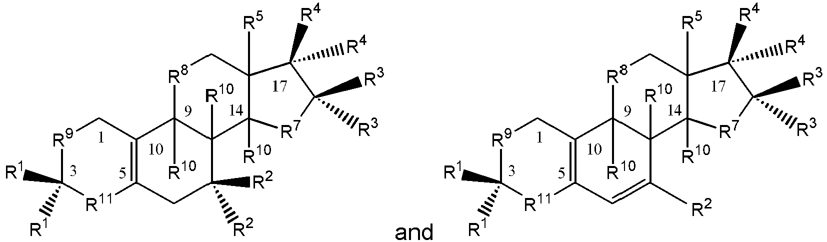 wherein 0, 1, 2, 3 or 4 aditional double bonds are present in the rings.

In some embodiments, the formula 1 compound contains no double bonds and is a 5α- or 5β-androstane compound or an analog thereof. Reference to a 1-ene compound means that a double bond is present at the 1-2 position, reference to a 5-ene or a 5(6)-ene compound means that a double bond is present at the 5-6 position, reference to a 5(10)-ene compound means that a double bond is present at the 5-10 position, while reference to a 5(10),16-diene compound means that a double bond is present at the 5-10 and at the 16-17 positions. Similarly, reference to a 13(17) double bond means a double bond is between the 13- and 17-positions and reference to a 9(11) double bond means a double bond is between the 9- and 11-positions, while a 9 and a 9(10) double bond means a double bond is between the 9- and 10-positions. Other double bond positions in the steroid rings are defined in an analogous manner. When a compound such as an androstane or an androstene without a double bond at the 5-position is described, the hydrogen atom or other substituent at the 5-position will be in the α-configuration, unless specified otherwise explicitly or by context. When the hydrogen atom or other substituent at the 5-position is in the β-configuration, the compound name or description will usually specify this. Thus, for 3α-amino-16α,17β-dihydroxyandrostane, 3β-amino-16α,17β-dihydroxyandrostane or 3α-amino-16α, 17β-dihydroxyandrost-1,9(11)-diene the hydrogen atom at the 5-position is in the α-configuration. Similarly, for 3α-amino-16α,17β-dihydroxy-5β-androstane, 3β-amino-16α, 17β-dihydroxy-5β-androstane or 3α-amino-16α, 17β-dihydroxy-5β-androst-1,9(11)-diene the hydrogen atom at the 5-position is in the β-configuration. Other classes of compounds are defined in an analogous manner.

F1Cs include compounds having the structure 5, 6, 7, 8, 9 and 10,

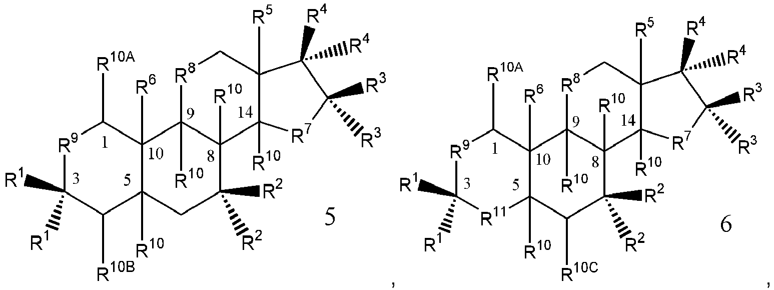

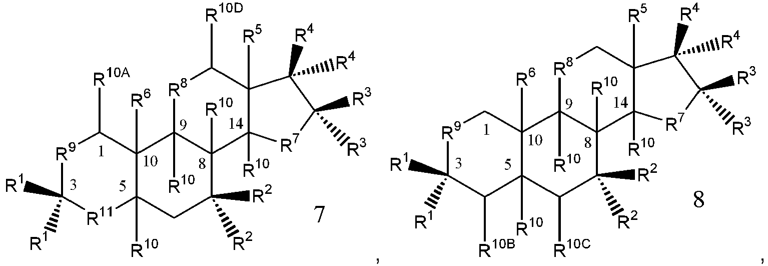

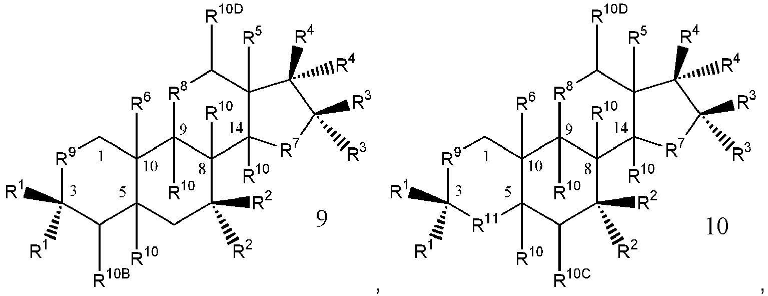

or a metabolic precursor, a metabolite, salt or tautomer thereof, wherein there is 0, 1, 2, 3, 4 or 5 double bonds in the steroid rings at the 1-, 2-, 3-, 4-, 5-, 5(10), 6-, 7-, 8-, 8(14)-, 9-, 9(11)-, 11-, 12-, 13(17)-, 14-, 15- or 16- positions; each R¹, R², R³, R⁴, R¹⁰ at the 2, 11 and 15 positions, R^{10A}, R^{10B}, R^{10C} and R^{10D} independently are -H, -OH, -OR^{PR}, - SR^{PR}, -SH, -N(R^{PR})₂, -NHR^{PR}, -NH₂, -NO₂, -ONO₂, -O-Si-(R¹³)₃, -CHO, -CHS, -CN, -SCN, -NO₂, -COOH, -COOR^{PR}, -OSO₃H, -OPO₃H₂, =O =S, =N-OH, =N-OCH₃, =CH₂, =CH-CH₃, an ester, a thioester, or another R¹⁰ moiety described herein, or, one or more of two adjacent R¹-R⁴, R¹⁰, R^{10A}, R^{10B}, R^{10C} and R^{10D} are an independently selected epoxide or cyclopropyl ring; R⁵, R⁶ and R¹⁰ at the 5 (if present), 8, 9 and 14 positions independently are R¹⁰ moieties described herein, e.g., -H, -CH₃, -C₂H₅, -OH, -OR^{PR}, -SR^{PR}, -N(R^{PR})₂, -O-Si-(R¹³)₃, -CHO, -CHS, -CN, -SCN, -N₃, -COOH, -OS(O)(O)OH, an ester, a thioester, a halogen, optionally substituted alkyl, or, one, two or more of R⁵, R⁶ and R¹⁰ at the 5, 8, 9 and 14 positions, together with a carbon atom that is adjacent to the carbon to which the R⁵, R⁶ or R¹⁰ at the 5-, 8-, 9- or 14-position is bonded are an independently selected epoxide or cyclopropyl ring; R⁷ is -C(R¹⁰)₂-, -C(R¹⁰)₂-C(R¹⁰)₂-, -O-, or another R⁷ moiety described herein; R⁸ and R⁹ independently as previously described; R¹³ independently is C₁₋₆ alkyl; and R^{PR} independently are -H, a protecting group or together are a protecting group, wherein 0, 1, 2, 3 or 4 of R^{10A}, R^{10B}, R^{10C} and R^{10D} are -H, R⁵ and R⁶ respectively are in the β,β, α,β, β,α, or α,α configurations, and wherein, R¹⁰ moieties at the 5 (if present), 8, 9 and 14 positions respectively are in the α,α,α,α, α,α,α,β, α,α,β,α, α,β,α,α, β,α,α,α, α,α,β,β, α,β,α,β,β,α,α,β, β,α,β,α, β,β,α,α, α,β,β,α, α,β,β,β, β,α,β,β, β,β,α,β, β,β,β,α or β,β,β,β configurations. For any of the F1Cs of structure 5, 6, 7, 8, 9 or 10 where two variable groups are bonded to the same carbon, e.g., R¹, R², R³, R⁴ or R¹⁰ at the 11 position, each variable group at that position is independently selected.

Invention embodiments include a composition comprising a F1C and 1, 2, 3, 4 or more nonaqueous liquid excipients. These compositions can contain less than about 3% w/v water, less than about 2% w/v water, less than about 1.5% w/v water, less than about 1 % w/v water, less than about 0.8% w/v water, less than about 0.5% w/v water, less than about 0.3% w/v water or less than about 0.1 % w/v water. Typically, the nonaqueous liquid excipients include propylene glycol and a PEG or a PEG mixture and can optionally include one or both of benzyl alcohol and benzyl benzoate.

Embodiments of F1 Cs include or exclude any subset of compounds within the definition of formula 1, provided that at least one F1 C remains. For example, a subset of F1Cs that are may be included, for example in the invention nonaqueous formulations and in the invention intermittent dosing protocols and immune modulation methods, are (1) F1Cs where R² is hydroxyl, or a group that can hydrolyze or metabolize to hydroxyl or thiol, in either configuration and R⁵and R⁶ are methyl in the β-configuration or (2) any 1, 2, 3, 4, 5, 6 or more of the F1Cs or genera of compounds that are disclosed herein. Another group of compounds that are optionally excluded from F1 Cs comprises one or all compounds that are disclosed in one or more prior art references or publications, e.g., one or more compounds that are disclosed in one or more of the references cited herein.

When R^{10E}, R^{10F}, R^{10G} and R^{10H} respectively are in the β,β,α,α configurations exemplary B, C, D, E, F and G structures with one, two or more optional double bonds at 3, 4, 5(6), 5(10), 6, 7, 8(9), 8(14), 11, 12, 13(17) and/or 14 include

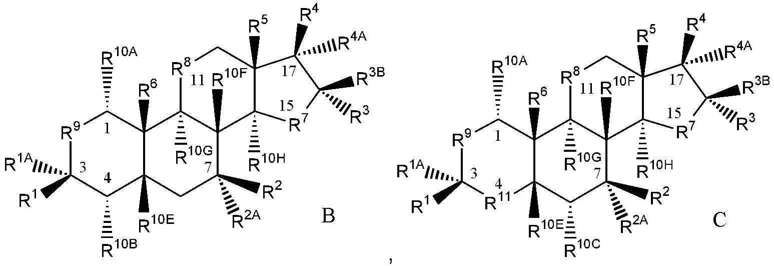

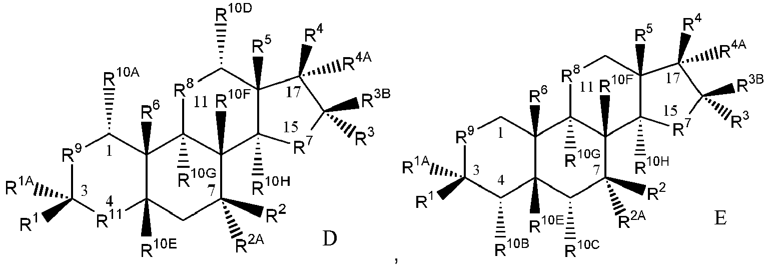

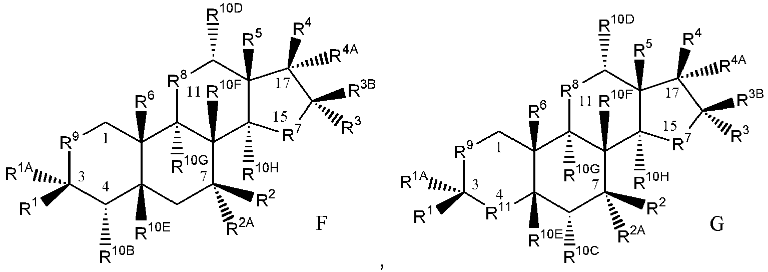

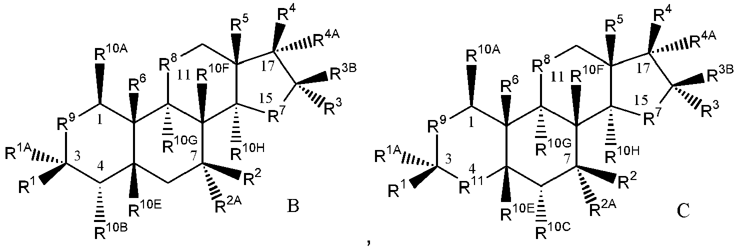

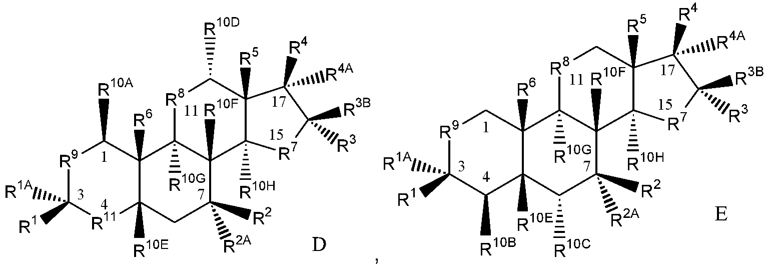

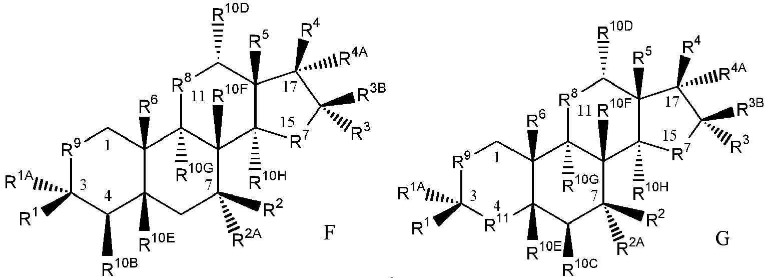

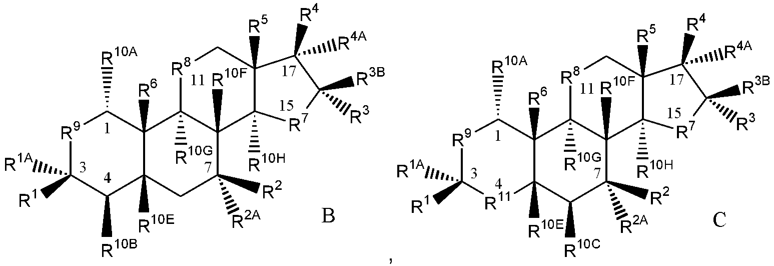

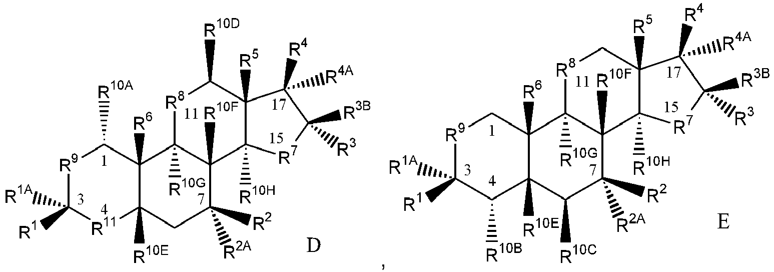

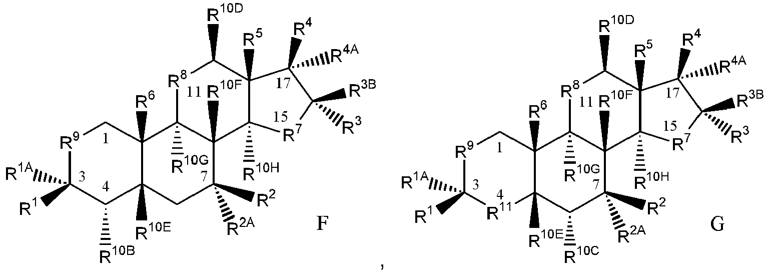

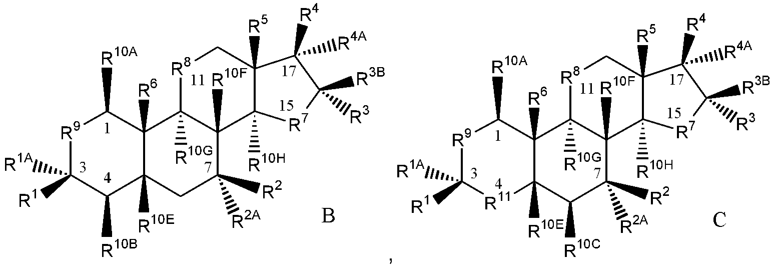

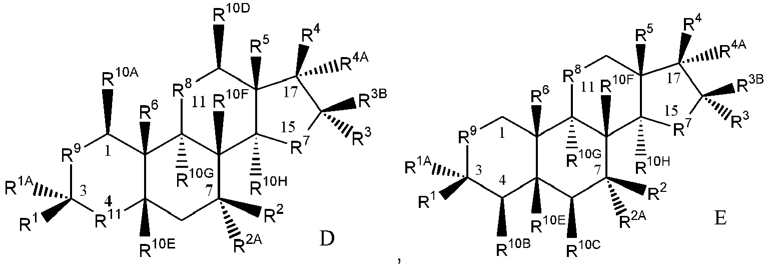

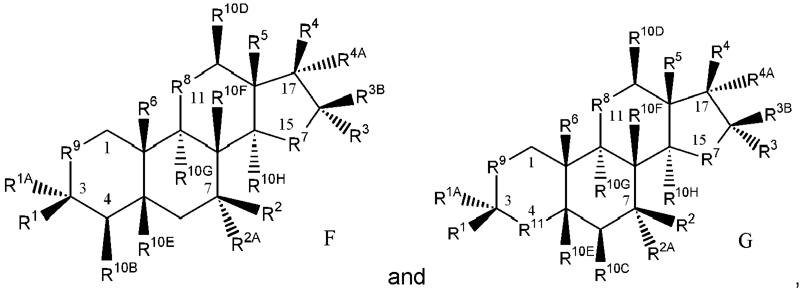

where R¹¹ is a moiety as defined herein such as -O-, -S-, -CH(α-R^{10B})-, -CH(β-R^{10B})-, -NR^{10B}- or, -C(R^{10B})₂- where the R^{10B} are the same or different, when no double bond is present at the 4-position, or =N-, =CH- or =CR^{10B}-, when a double bond is present at the 4-position, and R^{1A}, R^{2A} and R^{4A} respectively are independently selected R¹, R² and R⁴ moieties in the α-configuration and R^{3B} is an R³ moiety in the β-configuration. In these structures, each R¹, R², R³ and R⁴ is the same or different. In any of these structures exemplary R¹¹ moieties include -C(CH₃)₂-, -C(C₂H₅)₂-, -CF₂-, -CH(α-OH)-, -CH(β-OH)-, -C(β-C1-C8 optionally substituted alkyl)(α-OH)-, -C(α-C1-C8 optionally substituted alkyl)(β-OH)-, -CH(α-NO₂)-, -CH(β-NO₂)-, -CH(α-ether)-, -CH(β-ether)-, -CH(α-thioether)-, -CH(β-thioether)-, -CH(α-C1-C8 optionally substituted alkyl)-,-CH(β-C1-C8 optionally substituted alkyl)-, -CH(C1-C8 optionally substituted alkyl)₂where each C1-C8 optionally substituted alkyl is the same or different, -C(O)-, -C(S)-, - C(NOH)-, -CH(α-NH-C1-C8 optionally substituted alkyl)- and -CH(β-NH-C1-C8 optionally substituted alkyl)-.

These embodiments contain F1C, such as the B, C, D, E, F and G structures wherein R⁴ and R^{4A} are present, i.e., no 16-17 double bond is present, and both are the same, such as optionally substituted alkyl, halogen, ether, ester, thioether, thioester, e.g., -OR^{PR}, -SR^{PR}, -F, -Cl, -Br, -I, methyl, ethyl, methoxy, ethoxy acetate or propionate. However, in many embodiments, when they are both present, R⁴ and R^{4A} are two independently selected dissimilar moieties defined herein, e.g., independently selected -H, -OH, -OR^{PR}, an ester (e.g., -OC(O)-CH₃, -OC(O)-C₂H₅, -OC(O)-C3 alkyl, - OC(O)-C4 alkyl,), ether (e.g., -OCH₃, -OC₂H₅, -OCH₂CH₂CH₃, or -OCH(CH₃)CH₃, -O-C4 alkyl, -O-C5 alkyl or -O-C6 alkyl), a thioester, a thioether, an acyl moiety, a carbonate, a carbamate an amide, a monosaccharide, a disaccharide, or an amino acid, optionally substituted alkyl, optionally substituted alkenyl, optionally substituted alkynyl or another moiety described herein.

For any F1C, examples of dissimilar R⁴ and R^{4A} moieties at the 17-position include (α-ester, β-optionally substituted alkynyl), (β-ester, α-optionally substituted alkynyl), (α-thioester, β-optionally substituted alkynyl), (β-thioester, α-optionally substituted alkynyl), (α-ester, β-optionally substituted alkenyl), (β-ester, α-optionally substituted alkenyl), (α-thioester, β-optionally substituted alkenyl), (β-thioester, α-optionally substituted alkenyl), (α-optionally substituted alkyl, β-ester), (β-optionally substituted alkyl, α-ester), (α-optionally substituted alkyl, β-optionally substituted amine), (β-optionally substituted alkyl, α-optionally substituted amine), (α-optionally substituted alkyl, β-halogen)-, (β-optionally substituted alkyl, α-halogen), (α-halogen, β-ether), (β-halogen, α-ether), (α-halogen, β-optionally substituted alkyl), (β-halogen, α-optionally substituted alkyl), (β-ester, α-acyl), (α-ester, β-acyl), (β-ester, α-C(O)-C1-C10 optionally substituted alkyl), (α-ester, β-C(O)-C1-C10 optionally substituted alkyl), (β-thioester, α-C(O)-C1-C10 optionally substituted alkyl), (α-thioester, β-C(O)-C1-C10 optionally substituted alkyl), (β-OH, α-ester), (α-OH, β-ester), (β-OH, α-ether), (α-OH, β-ether), (β-OH, α-acyl), (α-OH, β-acyl), (α-halogen, β-OR^{PR}), (β-halogen, α-OR^{PR}), (α-F, β-ester), (β-F, α-ester), (α-F, β-ether), (β-F, α-ether), (α-Br, β-ether), (β-Br, α-ether), (α-F, β-optionally substituted alkyl), (β-F, α-optionally substituted alkyl), (α-OH, β-optionally, substituted alkynyl), (β-OH, α-optionally substituted alkynyl), (α-OH, β-C≡CCH₂-halogen), (β-OH, α-C≡CCH₂-halogen), (α-OH, β-C≡C-halogen), (β-OH, α- C≡C-halogen), (β-epoxy, α-halogen, where the epoxy is formed with an adjacent steroid nucleus atom), (α-epoxy, β-halogen), (α-cyclopropyl, β-halogen), (β-cyclopropyl, α-halogen), (α-cyclopropyl, β- optionally substituted alkyl), (β-cyclopropyl, α-optionally substituted alkyl), (α-optionally substituted alkyl, β-NH-C1-C8 optionally substituted alkyl), (β-optionally substituted alkyl, α-NH-C1-C8 optionally substituted alkyl), (α-ether, β-NH-C1-C8 optionally substituted alkyl), (β-ether, α-NH-C1-C8 optionally substituted alkyl), (α-thioester, β-NH-C1-C8 optionally substituted alkyl), (β-thioester, α-NH-C1-C8 optionally substituted alkyl), (α-ester, β-NH-C1-C8 optionally substituted alkyl), (β-ester, α-NH-C1-C8 optionally substituted alkyl), (α-C(O)CH₃, β-NH-C1-C8 optionally substituted alkyl), (β-C(O)CH₃, α-NH-C1-C8 optionally substituted alkyl), (α-OH, β-NH-C1-C8 optionally substituted alkyl), (β-OH, α-NH-C1-C8 optionally substituted alkyl) and other combinations of groups that are within the scope of R⁴ and R^{4A}. Such moieties, which are the same or different can also be at 1, 2, 3 or more R¹ and R^{1A}, R² and R^{2A}, R³ and R^{3B} variable groups, and/or the R¹⁰ variable groups at R⁷, R⁸ and R⁹.

Specific dissimilar R⁴ and R^{4A} moieties include, e.g., (α-F, β-CH₃), (β-F, α-CH₃), (α-F, β-C₂H₅), (β-F, α-C₂H₅), (α-Br, β-OCH₃), (β-Br, α-OCH₃), (α-F, β-OCH₃), (β-F, α-OCH₃), (α-F, β-OH), (β-F, α-OH), (α-Br, β-OCH₃), (β-Br, α-OCH₃), (α-F, β-CH₃), (β-F, α-CH₃), (α-Br, β-CH₃), (β-Br, α-CH₃), (α-OH, β-CCCH₃), (β-OH, α-CCCH₃), (α-OH, β-CCCH₂OH), (β-OH, α-CCCH₂OH), (α-OH, β-CCH), (β-OH, α-CCH), (α-CH₃, β-OC(O)CH₃), (β-CH₃, α-OC(O)CH₃), (α-C₂H₅, β-OC(O)CH₃), (β-C₂H₅, α-OC(O)CH₃), (α-C₃H₇, β-OC(O)CH₃), (β-C₃H₇, α-OC(O)CH₃), (α-C₄H₉, β-OC(O)CH₃), (β-C₄H₉, α-OC(O)CH₃), (α-C₂H₃, β-OC(O)CH₃), (β-C₂H₃, α-OC(O)CH₃), (α-C₂H₄OH, β-OC(O)CH₃), (β-C₂H₄OH, α-OC(O)CH₃), (α-C₃H₅, β-OC(O)CH₃), (β-C₃H₅, α-OC(O)CH₃), (α-C₄H₇, β-OC(O)CH₃), (β-C₄H₇, α-OC(O)CH₃), (α-C₃H₃, β-OC(O)CH₃), (β-C₃H₃, α-OC(O)CH₃), (α-C₄H₅, β-OC(O)CH₃), (β-C₄H₅, α-OC(O)CH₃), (α-CH₃, β-OC(O)C₂H₅), (β-CH₃, α-OC(O)C₂H₅), (α-C₂H₅, β-OC(O)C₂H₅), (β-C₂H₅, α-OC(O)C₂H₅), (α-C₃H₇, β-OC(O)C₂H₅), (β-C₃H₇, α-OC(O)C₂H₅), (α-C₄H₉, β-OC(O)C₂H₅), (β-C₄H₉, α-OC(O)C₂H₅), (α-C₂H₃, β-OC(O)C₂H₅), (β-C₂H₃, α-OC(O)C₂H₅), (α-C₂H₄OH, β-OC(O)C₂H₅), (β-C₂H₄OH, α-OC(O)C₂H₅), (α-C₃H₅, β-OC(O)C₂H₅), (β-C₃H₅, α-OC(O)C₂H₅), (α-C₄H₇, β-OC(O)C₂H₅), (β-C₄H₇, α-OC(O)C₂H₅), (α-C₃H₃, β-OC(O)C₂H₅), (β-C₃H₃, α-OC(O)C₂H₅), (α-C₄H₅, β-OC(O)C₂H₅), (β-C₄H₅, α-OC(O)C₂H₅), (α-C(O)CH₃, β-OC(O)CH₃), (β-C(O)CH₃, α-OC(O)CH₃), (α-C(O)C₂H₅, β-OC(O)CH₃), (β-C(O)C₂H₅, α-OC(O)CH₃), (α-CH₃, β-SC(O)CH₃), (β-CH₃, α-SC(O)CH₃), (α-C₂H₅, β-SC(O)CH₃), (β-C₂H₅, α-SC(O)CH₃), (α-C₃H₇, β-SC(O)CH₃), (β-C₃H₇, α-SC(O)CH₃), (α-C₄H₉, β-SC(O)CH₃), (β-C₄H₉, α-SC(O)CH₃), (α-C₂H₃, β-SC(O)CH₃), (β-C₂H₃, α-SC(O)CH₃), (α-C₂H₄OH, β-SC(O)CH₃), (β-C₂H₄OH, α-SC(O)CH₃), (α-C₃H₅, β-SC(O)CH₃), (β-C₃H₅, α-SC(O)CH₃), (α-C₄H₇, β-SC(O)CH₃), (β-C₄H₇, α-SC(O)CH₃), (α-C₃H₃, β-SC(O)CH₃), (β-C₃H₃, α-SC(O)CH₃), (α-C₄H₅, β-SC(O)CH₃), (β-C₄H₅, α-SC(O)CH₃), (α-CH₃, β-SC(O)C₂H₅), (β-CH₃, α-SC(O)C₂H₅), (α-C₂H₅, β-SC(O)C₂H₅), (β-C₂H₅, α-SC(O)C₂H₅), (α-C₃H₇, β-SC(O)C₂H₅), (β-C₃H₇, α-SC(O)C₂H₅), (α-C₄H₉, β-SC(O)C₂H₅), (β-C₄H₉, α-SC(O)C₂H₅), (α-C₂H₃, β-SC(O)C₂H₅), (β-C₂H₃, α-SC(O)C₂H₅), (α-C₂H₄OH, β-SC(O)C₂H₅), (β-C₂H₄OH, α-SC(O)C₂H₅), (α-C₃H₅, β-SC(O)C₂H₅), (β-C₃H₅, α-SC(O)C₂H₅), (α-C₄H₇, β-SC(O)C₂H₅), (β-C₄H₇, α-SC(O)C₂H₅), (α-C₃H₃, β-SC(O)C₂H₅), (β-C₃H₃, α-SC(O)C₂H₅), (α-C₄H₅, β-SC(O)C₂H₅), (β-C₄H₅, α-SC(O)C₂H₅), (α-C(O)CH₃, β-SC(O)CH₃), (β-C(O)CH₃, α-SC(O)CH₃), (α-C(O)C₂H₅, β-SC(O)CH₃), (β-C(O)C₂H₅, α-SC(O)CH₃), (α-C(O)CH₃, β-NH-CH₃), (β-C(O)CH₃, α-NH-CH₃), (α-OH, β-NH-CH₃), (β-OH, α-NH-CH₃), (α-C(O)CH₃, β-N(CH₃)₂), (β-C(O)CH₃, α-N(CH₃)₂), (α-OH, β-N(CH₃)₂), (β-OH, α-N(CH₃)₂), (α-C(O)CH₃, β-N(C₂H₅)₂), (β-C(O)CH₃, α-N(C₂H₅)₂), (α-OH, β-N(C₂H₅)₂), (β-OH, α-N(C₂H₅)₂), (β-epoxy, α-H), (α-epoxy, β-H), (β-epoxy, α-Br), (α-epoxy, β-Br), (β-epoxy, α-F), (α-epoxy, β-F), (β-cyclopropyl, α-H), (α-cyclopropyl, β-H), (β-cyclopropyl, α-F) and (α-cyclopropyl, β-F). For moieties that contain an epoxy, cyclopropyl or other cyclic moiety, the cyclic moiety can be formed with an adjacent variable group, e.g., R³ or R^{3B}. As is apparent from the foregoing disclosure, these or other dissimilar moieties can also be present at one or more of, e.g., the 2-, 3-, 7-, 11-, 15- or 16-positions.

F1 C embodiments include structures where 1, 2, 3 or 4 variable groups such as R¹, R², R³, R⁴, R^{10A}, R^{10B}, R^{10C} or another R¹⁰ moiety are an independently selected oxygen linked moiety and 0, 1, 2 or 3 variable groups such as R², R³, R⁴, R⁵, R⁶, R^{10A}, R^{10B}, R^{10C} or another R¹⁰ moiety are an independently selected carbon linked moiety. Oxygen linked moieties are moieties where oxygen is bonded to the steroid ring, e.g., -OH, =O, -OR^{PR}, carbonate, ester, ether, -O-monosaccharide, -O-polymer, -O-C(O)-NH₂ or-O-C(O)-NH-optionally substituted alkyl. Carbon linked moieties are moieties where carbon is bonded to the steroid ring, e.g., optionally substituted alkyl, -C(O)-optionally substituted alkyl, -C(O)-O-optionally substituted alkyl, optionally substituted alkenyl or optionally substituted alkynyl such as -CH₃, -CF₃, -CH₂OH, -C₂H₅ or -C₂H₄OH. These F1Cs include compounds where (1) one R¹ is an oxygen linked moiety (an 'O-linked' moiety), an S-linked moiety or an N-linked moiety and the other R¹ is -H or a carbon linked moiety (a'C-linked' moiety), (2) one R⁴ is an oxygen linked moiety and the other R⁴ is -H or a C-linked moiety, (3) one R³ is an oxygen linked moiety and the other R³ is -H or a C-linked moiety and/or (4) one R² is an oxygen linked moiety and the other R² is -H or a C-linked moiety, where the oxygen linked moiety or moieties independently are in the α- or β-configuration.

Compound groups. Specific F1Cs or genera of F1Cs that can be used in the assay methods, clinical treatments, e.g., blood cell deficiency treatments, cancer or infection treatment methods, radiation protection treatment methods, autoimmune disease treatment methods or trauma treatment methods, and other methods described herein include the following compound groups.

Group 1. Exemplary embodiments include the formula 1 compounds named according to the compound structure designations given in Tables A and B below. Each compound named in Table B is depicted as a compound having the structure

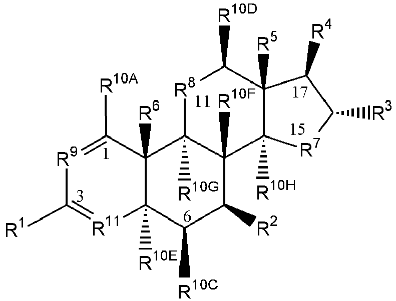

where R⁵ and R⁶ are both -CH₃, there is a double bond at the 1-2- and 3-4 positions, R⁷ R⁸ and R⁹ are all -CH₂- or =CH-, R¹¹ is =CR^{10B}-, R^{10A}, R^{10B} R^{10C} R^{10D}, R^{10E}, R^{10F}, R^{10G} and R^{10H} are all -H and R¹, R², R³ and R⁴ are the substituents designated in Table A. The compounds named according to Tables A and B are referred to as "group 1" compounds.

Compounds named in Table B are named by numbers assigned to R¹, R², R³ and R⁴ according to the following compound naming convention, R¹.R².R³.R⁴, using the numbered chemical substituents in Table A. Each Table A number specifies a different structure for each of R¹, R², R³ and R⁴. When R¹, R², R³ or R⁴ is a divalent moiety, e.g., =O, the hydrogen at the corresponding position is absent. Thus, the group **1** compound named 1.2.4.9 is a group 1 compound with a β-hydroxyl bonded to carbons at the 3- and 7-positions (the variable groups R¹ and R² respectively), an α-fluorine bonded to carbon 16 (the variable group R³) and acetate at carbon 17 (the variable group R⁴), i.e., 1.2.4.9 is 3,7β,17β-trihydroxy-16α-fluoroandrost-1,3-diene, which has the structure

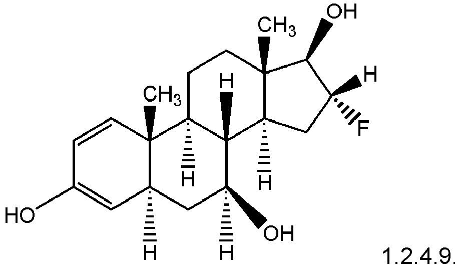

Similarly, group 1 compound 1.2.4.1 is 3,7β-dihydroxy-16α-fluoro-17β-aminoandrost-1,3-diene, group 1 compound 1.1.5.9 is 3,17β-dihydroxyandrost-1,3-diene, 1.1.7.1, which is 3-hydroxy-16α-acetoxy-17β-aminoandrost-1,3-diene and compound 1.1.4.10, which is 3-hydroxy-16α-fluoro-17β-acetoxyandrost-1,3-diene. Other exemplary group 1 compounds include 3,17β-dihydroxy-7β-acetoxyandrost-1,3-diene, 3,17β-dihydroxy-7β-methylandrost-1,3-diene, 3,17β-dihydroxy-7β-methoxyandrost-1,3-diene, 3,7β,17β-trihydroxyandrost-1,3-diene, 3-amino-17β-hydroxyandrost-1,3-diene, 3-amino-7β,17β-dihydroxyandrost-1,3-diene, 3-hydroxy-17β-aminoandrost-1,3-diene, 3,7β-dihydroxy-17β-aminoandrost-1,3-diene, 3,17β-dihydroxy-7β-aminoandrost-1,3-diene, 3-hydroxy-7β,17β-diacetylaminoandrost-1,3-diene, 3-hydroxy-7β,17β-dimethylaminoandrost-1,3-diene and 16α-hydroxy, 16α-methyl, 16α-amino, 16α-aminomethyl, 16α-acetate and 16α-halo analogs of any of these compounds.

**TABLE A**

| **R¹** | **R²** |
|---|---|
| 1 -OH | 1 -H |
| 2 -OCH₃ | 2 -OH |
| 3 -SH | 3 -OCH₃ |
| 4 -O-C(O)-CH₃ | 4 -N(CH₃)₂ |
| 5 -NHCH₃ | 5 -CH₃ |
| 6 -NH₂ | 6 -NH₂ |
| 7 -NH-C(O)-CH₃ | 7 -NH-C(O)-CH₃ |
| 8 -N(CH₃)₂ | 8 -NH-CH₃ |
| 9 -O-D-β-glucoside | 9 -O-C(O)-CH₃ |
| 10 -O-S(O)(OH)-OH | 10 -SH |

| **R³** | **R⁴** |
|---|---|
| 1 -Br | 1 -NH₂ |
| 2 -Cl | 2 -NH-C(O)-CH₃ |
| 3 -I | 3 -NH-C(O)-OCH₃ |
| 4 -F | 4 -NH-CH₃ |
| 5 -H | 5 -N(CH₃)₂ |
| 6 -OH | 6 -OCH₃ |
| 7 -O-C(O)-CH₃ | 7 -O-S(O)(OH)-OH |
| 8 -CH₃ | 8 -O-C(O)-CH₂CH₃ |
| 9 -NH₂ | 9 -OH |
| 10 -NHCH₃ | 10 -O-C(O)-CH₃ |

**TABLE B**

| |
|---|
| 1.1.1.1, 1.1.1.2, 1.1.1.3, 1.1.1.4, 1.1.1.5, 1.1.1.6, 1.1.1.7, 1.1.1.8, 1.1.1.9, 1.1.1.10, 1.1.2.1, 1.1.2.2, 1.1.2.3, 1.1.2.4, 1.1.2.5, 1.1.2.6, 1.1.2.7, 1.1.2.8, 1.1.2.9, 1.1.2.10, 1.1.3.1, 1.1.3.2, 1.1.3.3, 1.1.3.4, 1.1.3.5, 1.1.3.6, 1.1.3.7, 1.1.3.8, 1.1.3.9, 1.1.3.10, 1.1.4.1, 1.1.4.2, 1.1.4.3, 1.1.4.4, 1.1.4.5, 1.1.4.6, 1.1.4.7, 1.1.4.8, 1.1.4.9, 1.1.4.10, 1.1.5.1, 1.1.5.2, 1.1.5.3, 1.1.5.4, 1.1.5.5, 1.1.5.6, 1.1.5.7, 1.1.5.8, 1.1.5.9, 1.1.5.10, 1.1.6.1, 1.1.6.2, 1.1.6.3, 1.1.6.4, 1.1.6.5, 1.1.6.6, 1.1.6.7, 1.1.6.8, 1.1.6.9, 1.1.6.10, 1.1.7.1, 1.1.7.2, 1.1.7.3, 1.1.7.4, 1.1.7.5, 1.1.7.6, 1.1.7.7, 1.1.7.8, 1.1.7.9, 1.1.7.10, 1.1.8.1, 1.1.8.2, 1.1.8.3, 1.1.8.4, 1.1.8.5, 1.1.8.6, 1.1.8.7, 1.1.8.8, 1.1.8.9, 1.1.8.10, 1.1.9.1, 1.1.9.2, 1.1.9.3, 1.1.9.4, 1.1.9.5, 1.1.9.6, 1.1.9.7, 1.1.9.8, 1.1.9.9, 1.1.9.10, 1.1.10.1, 1.1.10.2, 1.1.10.3, 1.1.10.4, 1.1.10.5, 1.1.10.6, 1.1.10.7, 1.1.10.8, 1.1.10.9, 1.1.10.10, 1.2.1.1, 1.2.1.2, 1.2.1.3, 1.2.1.4, 1.2.1.5, 1.2.1.6, 1.2.1.7, 1.2.1.8, 1.2.1.9, 1.2.1.10, 1.2.2.1, 1.2.2.2, 1.2.2.3, 1.2.2.4, 1.2.2.5, 1.2.2.6, 1.2.2.7, 1.2.2.8, 1.2.2.9, 1.2.2.10, 1.2.3.1, 1.2.3.2, 1.2.3.3, 1.2.3.4, 1.2.3.5, 1.2.3.6, 1.2.3.7, 1.2.3.8, 1.2.3.9, 1.2.3.10, 1.2.4.1, 1.2.4.2, 1.2.4.3, 1.2.4.4, 1.2.4.5, 1.2.4.6, 1.2.4.7, 1.2.4.8, 1.2.4.9, 1.2.4.10, 1.2.5.1, 1.2.5.2, 1.2.5.3, 1.2.5.4, 1.2.5.5, 1.2.5.6, 1.2.5.7, 1.2.5.8, 1.2.5.9, 1.2.5.10, 1.2.6.1, 1.2.6.2, 1.2.6.3, 1.2.6.4, 1.2.6.5, 1.2.6.6, 1.2.6.7, 1.2.6.8, 1.2.6.9, 1.2.6.10, 1.2.7.1, 1.2.7.2, 1.2.7.3, 1.2.7.4, 1.2.7.5, 1.2.7.6, 1.2.7.7, 1.2.7.8, 1.2.7.9, 1.2.7.10, 1.2.8.1, 1.2.8.2, 1.2.8.3, 1.2.8.4, 1.2.8.5, 1.2.8.6, 1.2.8.7, 1.2.8.8, 1.2.8.9, 1.2.8.10, 1.2.9.1, 1.2.9.2, 1.2.9.3, 1.2.9.4, 1.2.9.5, 1.2.9.6, 1.2.9.7, 1.2.9.8, 1.2.9.9, 1.2.9.10, 1.2.10.1, 1.2.10.2, 1.2.10.3, 1.2.10.4, 1.2.10.5, 1.2.10.6, 1.2.10.7, 1.2.10.8, 1.2.10.9, 1.2.10.10, 1.3.1.1, 1.3.1.2, 1.3.1.3, 1.3.1.4, 1.3.1.5, 1.3.1.6, 1.3.1.7, 1.3.1.8, 1.3.1.9, 1.3.1.10, 1.3.2.1, 1.3.2.2, 1.3.2.3, 1.3.2.4, 1.3.2.5, 1.3.2.6, 1.3.2.7, 1.3.2.8, 1.3.2.9, 1.3.2.10, 1.3.3.1, 1.3.3.2, 1.3.3.3, 1.3.3.4, 1.3.3.5, 1.3.3.6, 1.3.3.7, 1.3.3.8, 1.3.3.9, 1.3.3.10, 1.3.4.1, 1.3.4.2, 1.3.4.3, 1.3.4.4, 1.3.4.5, 1.3.4.6, 1.3.4.7, 1.3.4.8, 1.3.4.9, 1.3.4.10, 1.3.5.1, 1.3.5.2, 1.3.5.3, 1.3.5.4, 1.3.5.5, 1.3.5.6, 1.3.5.7, 1.3.5.8, 1.3.5.9, 1.3.5.10, 1.3.6.1, 1.3.6.2, 1.3.6.3, 1.3.6.4, 1.3.6.5, 1.3.6.6, 1.3.6.7, 1.3.6.8, 1.3.6.9, 1.3.6.10, 1.3.7.1, 1.3.7.2, 1.3.7.3, 1.3.7.4, 1.3.7.5, 1.3.7.6, 1.3.7.7, 1.3.7.8, 1.3.7.9, 1.3.7.10, 1.3.8.1, 1.3.8.2, 1.3.8.3, 1.3.8.4, 1.3.8.5, 1.3.8.6, 1.3.8.7, 1.3.8.8, 1.3.8.9, 1.3.8.10, 1.3.9.1, 1.3.9.2, 1.3.9.3, 1.3.9.4, 1.3.9.5, 1.3.9.6, 1.3.9.7, 1.3.9.8, 1.3.9.9, 1.3.9.10, 1.3.10.1, 1.3.10.2, 1.3.10.3, 1.3.10.4, 1.3.10.5, 1.3.10.6, 1.3.10.7, 1.3.10.8, 1.3.10.9, 1.3.10.10, 1.4.1.1, 1.4.1.2, 1.4.1.3, 1.4.1.4, 1.4.1.5, 1.4.1.6, 1.4.1.7, 1.4.1.8, 1.4.1.9, 1.4.1.10, 1.4.2.1, 1.4.2.2, 1.4.2.3, 1.4.2.4, 1.4.2.5, 1.4.2.6, 1.4.2.7, 1.4.2.8, 1.4.2.9, 1.4.2.10, 1.4.3.1, 1.4.3.2, 1.4.3.3, 1.4.3.4, 1.4.3.5, 1.4.3.6, 1.4.3.7, 1.4.3.8, 1.4.3.9, 1.4.3.10, 1.4.4.1, 1.4.4.2, 1.4.4.3, 1.4.4.4, 1.4.4.5, 1.4.4.6, 1.4.4.7, 1.4.4.8, 1.4.4.9, 1.4.4.10, 1.4.5.1, 1.4.5.2, 1.4.5.3, 1.4.5.4, 1.4.5.5, 1.4.5.6, 1.4.5.7, 1.4.5.8, 1.4.5.9, 1.4.5.10, 1.4.6.1, 1.4.6.2, 1.4.6.3, 1.4.6.4, 1.4.6.5, 1.4.6.6, 1.4.6.7, 1.4.6.8, 1.4.6.9, 1.4.6.10, 1.4.7.1, 1.4.7.2, 1.4.7.3, 1.4.7.4, 1.4.7.5, 1.4.7.6, 1.4.7.7, 1.4.7.8, 1.4.7.9, 1.4.7.10, 1.4.8.1, 1.4.8.2, 1.4.8.3, 1.4.8.4, 1.4.8.5, 1.4.8.6, 1.4.8.7, 1.4.8.8, 1.4.8.9, 1.4.8.10, 1.4.9.1, 1.4.9.2, 1.4.9.3, 1.4.9.4, 1.4.9.5, 1.4.9.6, 1.4.9.7, 1.4.9.8, 1.4.9.9, 1.4.9.10, 1.4.10.1, 1.4.10.2, 1.4.10.3, 1.4.10.4, 1.4.10.5, 1.4.10.6, 1.4.10.7, 1.4.10.8, 1.4.10.9, 1.4.10.10, 1.5.1.1, 1.5.1.2, 1.5.1.3, 1.5.1.4, 1.5.1.5, 1.5.1.6, 1.5.1.7, 1.5.1.8, 1.5.1.9, 1.5.1.10, 1.5.2.1, 1.5.2.2, 1.5.2.3, 1.5.2.4, 1.5.2.5, 1.5.2.6, 1.5.2.7, 1.5.2.8, 1.5.2.9, 1.5.2.10, 1.5.3.1, 1.5.3.2, 1.5.3.3, 1.5.3.4, 1.5.3.5, 1.5.3.6, 1.5.3.7, 1.5.3.8, 1.5.3.9, 1.5.3.10, 1.5.4.1, 1.5.4.2, 1.5.4.3, 1.5.4.4, 1.5.4.5, 1.5.4.6, 1.5.4.7, 1.5.4.8, 1.5.4.9, 1.5.4.10, 1.5.5.1, 1.5.5.2, 1.5.5.3, 1.5.5.4, 1.5.5.5, 1.5.5.6, 1.5.5.7, 1.5.5.8, 1.5.5.9, 1.5.5.10, 1.5.6.1, 1.5.6.2, 1.5.6.3, 1.5.6.4, 1.5.6.5, 1.5.6.6, 1.5.6.7, 1.5.6.8, 1.5.6.9, 1.5.6.10, 1.5.7.1, 1.5.7.2, 1.5.7.3, 1.5.7.4, 1.5.7.5, 1.5.7.6, 1.5.7.7, 1.5.7.8, 1.5.7.9, 1.5.7.10, 1.5.8.1, 1.5.8.2, 1.5.8.3, 1.5.8.4, 1.5.8.5, 1.5.8.6, 1.5.8.7, 1.5.8.8, 1.5.8.9, 1.5.8.10, 1.5.9.1, 1.5.9.2, 1.5.9.3, 1.5.9.4, 1.5.9.5, 1.5.9.6, 1.5.9.7, 1.5.9.8, 1.5.9.9, 1.5.9.10, 1.5.10.1, 1.5.10.2, 1.5.10.3, 1.5.10.4, 1.5.10.5, 1.5.10.6, 1.5.10.7, 1.5.10.8, 1.5.10.9, 1.5.10.10, 1.6.1.1, 1.6.1.2, 1.6.1.3, 1.6.1.4, 1.6.1.5, 1.6.1.6, 1.6.1.7, 1.6.1.8, 1.6.1.9, 1.6.1.10, 1.6.2.1, 1.6.2.2, 1.6.2.3, 1.6.2.4, 1.6.2.5, 1.6.2.6, 1.6.2.7, 1.6.2.8, 1.6.2.9, 1.6.2.10, 1.6.3.1, 1.6.3.2, 1.6.3.3, 1.6.3.4, 1.6.3.5, 1.6.3.6, 1.6.3.7, 1.6.3.8, 1.6.3.9, 1.6.3.10, 1.6.4.1, 1.6.4.2, 1.6.4.3, 1.6.4.4, 1.6.4.5, |
| 1.6.4.6, 1.6.4.7, 1.6.4.8, 1.6.4.9, 1.6.4.10, 1.6.5.1, 1.6.5.2, 1.6.5.3, 1.6.5.4, 1.6.5.5, 1.6.5.6, 1.6.5.7, 1.6.5.8, 1.6.5.9, 1.6.5.10, 1.6.6.1, 1.6.6.2, 1.6.6.3, 1.6.6.4, 1.6.6.5, 1.6.6.6, 1.6.6.7, 1.6.6.8, 1.6.6.9, 1.6.6.10, 1.6.7.1, 1.6.7.2, 1.6.7.3, 1.6.7.4, 1.6.7.5, 1.6.7.6, 1.6.7.7, 1.6.7.8, 1.6.7.9, 1.6.7.10, 1.6.8.1, 1.6.8.2, 1.6.8.3, 1.6.8.4, 1.6.8.5, 1.6.8.6, 1.6.8.7, 1.6.8.8, 1.6.8.9, 1.6.8.10, 1.6.9.1, 1.6.9.2, 1.6.9.3, 1.6.9.4, 1.6.9.5, 1.6.9.6, 1.6.9.7, 1.6.9.8, 1.6.9.9, 1.6.9.10, 1.6.10.1, 1.6.10.2, 1.6.10.3, 1.6.10.4, 1.6.10.5, 1.6.10.6, 1.6.10.7, 1.6.10.8, 1.6.10.9, 1.6.10.10, 1.7.1.1, 1.7.1.2, 1.7.1.3, 1.7.1.4, 1.7.1.5, 1.7.1.6, 1.7.1.7, 1.7.1.8, 1.7.1.9, 1.7.1.10, 1.7.2.1, 1.7.2.2, 1.7.2.3, 1.7.2.4, 1.7.2.5, 1.7.2.6, 1.7.2.7, 1.7.2.8, 1.7.2.9, 1.7.2.10, 1.7.3.1, 1.7.3.2, 1.7.3.3, 1.7.3.4, 1.7.3.5, 1.7.3.6, 1.7.3.7, 1.7.3.8, 1.7.3.9, 1.7.3.10, 1.7.4.1, 1.7.4.2, 1.7.4.3, 1.7.4.4, 1.7.4.5, 1.7.4.6, 1.7.4.7, 1.7.4.8, 1.7.4.9, 1.7.4.10, 1.7.5.1, 1.7.5.2, 1.7.5.3, 1.7.5.4, 1.7.5.5, 1.7.5.6, 1.7.5.7, 1.7.5.8, 1.7.5.9, 1.7.5.10, 1.7.6.1, 1.7.6.2, 1.7.6.3, 1.7.6.4, 1.7.6.5, 1.7.6.6, 1.7.6.7, 1.7.6.8, 1.7.6.9, 1.7.6.10, 1.7.7.1, 1.7.7.2, 1.7.7.3, 1.7.7.4, 1.7.7.5, 1.7.7.6, 1.7.7.7, 1.7.7.8, 1.7.7.9, 1.7.7.10, 1.7.8.1, 1.7.8.2, 1.7.8.3, 1.7.8.4, 1.7.8.5, 1.7.8.6, 1.7.8.7, 1.7.8.8, 1.7.8.9, 1.7.8.10, 1.7.9.1, 1.7.9.2, 1.7.9.3, 1.7.9.4, 1.7.9.5, 1.7.9.6, 1.7.9.7, 1.7.9.8, 1.7.9.9, 1.7.9.10, 1.7.10.1, 1.7.10.2, 1.7.10.3, 1.7.10.4, 1.7.10.5, 1.7.10.6, 1.7.10.7, 1.7.10.8, 1.7.10.9, 1.7.10.10, 1.8.1.1, 1.8.1.2, 1.8.1.3, 1.8.1.4, 1.8.1.5, 1.8.1.6, 1.8.1.7, 1.8.1.8, 1.8.1.9, 1.8.1.10, 1.8.2.1, 1.8.2.2, 1.8.2.3, 1.8.2.4, 1.8.2.5, 1.8.2.6, 1.8.2.7, 1.8.2.8, 1.8.2.9, 1.8.2.10, 1.8.3.1, 1.8.3.2, 1.8.3.3, 1.8.3.4, 1.8.3.5, 1.8.3.6, 1.8.3.7, 1.8.3.8, 1.8.3.9, 1.8.3.10, 1.8.4.1, 1.8.4.2, 1.8.4.3, 1.8.4.4, 1.8.4.5, 1.8.4.6, 1.8.4.7, 1.8.4.8, 1.8.4.9, 1.8.4.10, 1.8.5.1, 1.8.5.2, 1.8.5.3, 1.8.5.4, 1.8.5.5, 1.8.5.6, 1.8.5.7, 1.8.5.8, 1.8.5.9, 1.8.5.10, 1.8.6.1, 1.8.6.2, 1.8.6.3, 1.8.6.4, 1.8.6.5, 1.8.6.6, 1.8.6.7, 1.8.6.8, 1.8.6.9, 1.8.6.10, 1.8.7.1, 1.8.7.2, 1.8.7.3, 1.8.7.4, 1.8.7.5, 1.8.7.6, 1.8.7.7, 1.8.7.8, 1.8.7.9, 1.8.7.10, 1.8.8.1, 1.8.8.2, 1.8.8.3, 1.8.8.4, 1.8.8.5, 1.8.8.6, 1.8.8.7, 1.8.8.8, 1.8.8.9, 1.8.8.10, 1.8.9.1, 1.8.9.2, 1.8.9.3, 1.8.9.4, 1.8.9.5, 1.8.9.6, 1.8.9.7, 1.8.9.8, 1.8.9.9, 1.8.9.10, 1.8.10.1, 1.8.10.2, 1.8.10.3, 1.8.10.4, 1.8.10.5, 1.8.10.6, 1.8.10.7, 1.8.10.8, 1.8.10.9, 1.8.10.10, 1.9.1.1, 1.9.1.2, 1.9.1.3, 1.9.1.4, 1.9.1.5, 1.9.1.6, 1.9.1.7, 1.9.1.8, 1.9.1.9, 1.9.1.10, 1.9.2.1, 1.9.2.2, 1.9.2.3, 1.9.2.4, 1.9.2.5, 1.9.2.6, 1.9.2.7, 1.9.2.8, 1.9.2.9, 1.9.2.10, 1.9.3.1, 1.9.3.2, 1.9.3.3, 1.9.3.4, 1.9.3.5, 1.9.3.6, 1.9.3.7, 1.9.3.8, 1.9.3.9, 1.9.3.10, 1.9.4.1, 1.9.4.2, 1.9.4.3, 1.9.4.4, 1.9.4.5, 1.9.4.6, 1.9.4.7, 1.9.4.8, 1.9.4.9, 1.9.4.10, 1.9.5.1, 1.9.5.2, 1.9.5.3, 1.9.5.4, 1.9.5.5, 1.9.5.6, 1.9.5.7, 1.9.5.8, 1.9.5.9, 1.9.5.10, 1.9.6.1, 1.9.6.2, 1.9.6.3, 1.9.6.4, 1.9.6.5, 1.9.6.6, 1.9.6.7, 1.9.6.8, 1.9.6.9, 1.9.6.10, 1.9.7.1, 1.9.7.2, 1.9.7.3, 1.9.7.4, 1.9.7.5, 1.9.7.6, 1.9.7.7, 1.9.7.8, 1.9.7.9, 1.9.7.10, 1.9.8.1, 1.9.8.2, 1.9.8.3, 1.9.8.4, 1.9.8.5, 1.9.8.6, 1.9.8.7, 1.9.8.8, 1.9.8.9, 1.9.8.10, 1.9.9.1, 1.9.9.2, 1.9.9.3, 1.9.9.4, 1.9.9.5, 1.9.9.6, 1.9.9.7, 1.9.9.8, 1.9.9.9, 1.9.9.10, 1.9.10.1, 1.9.10.2, 1.9.10.3, 1.9.10.4, 1.9.10.5, 1.9.10.6, 1.9.10.7, 1.9.10.8, 1.9.10.9, 1.9.10.10, 1.10.1.1, 1.10.1.2, 1.10.1.3, 1.10.1.4, 1.10.1.5, 1.10.1.6, 1.10.1.7, 1.10.1.8, 1.10.1.9, 1.10.1.10, 1.10.2.1, 1.10.2.2, 1.10.2.3, 1.10.2.4, 1.10.2.5, 1.10.2.6, 1.10.2.7, 1.10.2.8, 1.10.2.9, 1.10.2.10, 1.10.3.1, 1.10.3.2, 1.10.3.3, 1.10.3.4, 1.10.3.5, 1.10.3.6, 1.10.3.7, 1.10.3.8, 1.10.3.9, 1.10.3.10, 1.10.4.1, 1.10.4.2, 1.10.4.3, 1.10.4.4, 1.10.4.5, 1.10.4.6, 1.10.4.7, 1.10.4.8, 1.10.4.9, 1.10.4.10, 1.10.5.1, 1.10.5.2, 1.10.5.3, 1.10.5.4, 1.10.5.5, 1.10.5.6, 1.10.5.7, 1.10.5.8, 1.10.5.9, 1.10.5.10, 1.10.6.1, 1.10.6.2, 1.10.6.3, 1.10.6.4, 1.10.6.5, 1.10.6.6, 1.10.6.7, 1.10.6.8, 1.10.6.9, 1.10.6.10, 1.10.7.1, 1.10.7.2, 1.10.7.3, 1.10.7.4, 1.10.7.5, 1.10.7.6, 1.10.7.7, 1.10.7.8, 1.10.7.9, 1.10.7.10, 1.10.8.1, 1.10.8.2, 1.10.8.3, 1.10.8.4, 1.10.8.5, 1.10.8.6, 1.10.8.7, 1.10.8.8, 1.10.8.9, 1.10.8.10, 1.10.9.1, 1.10.9.2, 1.10.9.3, 1.10.9.4, 1.10.9.5, 1.10.9.6, 1.10.9.7, 1.10.9.8, 1.10.9.9, 1.10.9.10, 1.10.10.1, 1.10.10.2, 1.10.10.3, 1.10.10.4, 1.10.10.5, 1.10.10.6, 1.10.10.7, 1.10.10.8, 1.10.10.9, 1.10.10.10, 2.1.1.1, 2.1.1.2, 2.1.1.3, 2.1.1.4, 2.1.1.5, 2.1.1.6, 2.1.1.7, 2.1.1.8, 2.1.1.9, 2.1.1.10, 2.1.2.1, 2.1.2.2, 2.1.2.3, 2.1.2.4, 2.1.2.5, 2.1.2.6, 2.1.2.7, 2.1.2.8, 2.1.2.9, 2.1.2.10, 2.1.3.1, 2.1.3.2, 2.1.3.3, 2.1.3.4, 2.1.3.5, 2.1.3.6, 2.1.3.7, 2.1.3.8, 2.1.3.9, 2.1.3.10, 2.1.4.1, 2.1.4.2, 2.1.4.3, 2.1.4.4, 2.1.4.5, 2.1.4.6, 2.1.4.7, 2.1.4.8, 2.1.4.9, 2.1.4.10, 2.1.5.1, 2.1.5.2, 2.1.5.3, 2.1.5.4, 2.1.5.5, 2.1.5.6, 2.1.5.7, 2.1.5.8, 2.1.5.9, 2.1.5.10, 2.1.6.1, 2.1.6.2, 2.1.6.3, 2.1.6.4, 2.1.6.5, 2.1.6.6, 2.1.6.7, 2.1.6.8, 2.1.6.9, 2.1.6.10, 2.1.7.1, 2.1.7.2, 2.1.7.3, 2.1.7.4, 2.1.7.5, 2.1.7.6, 2.1.7.7, 2.1.7.8, 2.1.7.9, 2.1.7.10, 2.1.8.1, 2.1.8.2, 2.1.8.3, 2.1.8.4, 2.1.8.5, 2.1.8.6, 2.1.8.7, 2.1.8.8, 2.1.8.9, 2.1.8.10, 2.1.9.1, 2.1.9.2, 2.1.9.3, 2.1.9.4, 2.1.9.5, 2.1.9.6, 2.1.9.7, 2.1.9.8, 2.1.9.9, 2.1.9.10, 2.1.10.1, 2.1.10.2, 2.1.10.3, 2.1.10.4, 2.1.10.5, 2.1.10.6, 2.1.10.7, 2.1.10.8, 2.1.10.9, 2.1.10.10, 2.2.1.1, 2.2.1.2, 2.2.1.3, 2.2.1.4, 2.2.1.5, 2.2.1.6, 2.2.1.7, 2.2.1.8, 2.2.1.9, 2.2.1.10, 2.2.2.1, 2.2.2.2, 2.2.2.3, 2.2.2.4, 2.2.2.5, 2.2.2.6, 2.2.2.7, 2.2.2.8, 2.2.2.9, 2.2.2.10, 2.2.3.1, 2.2.3.2, 2.2.3.3, 2.2.3.4, 2.2.3.5, 2.2.3.6, 2.2.3.7, 2.2.3.8, 2.2.3.9, 2.2.3.10, 2.2.4.1, 2.2.4.2, 2.2.4.3, 2.2.4.4, 2.2.4.5, 2.2.4.6, 2.2.4.7, 2.2.4.8, 2.2.4.9, 2.2.4.10, 2.2.5.1, 2.2.5.2, 2.2.5.3, 2.2.5.4, 2.2.5.5, 2.2.5.6, 2.2.5.7, 2.2.5.8, 2.2.5.9, 2.2.5.10, 2.2.6.1, 2.2.6.2, 2.2.6.3, 2.2.6.4, 2.2.6.5, 2.2.6.6, 2.2.6.7, 2.2.6.8, 2.2.6.9, 2.2.6.10, 2.2.7.1, 2.2.7.2, 2.2.7.3, 2.2.7.4, 2.2.7.5, 2.2.7.6, 2.2.7.7, 2.2.7.8, 2.2.7.9, 2.2.7.10, 2.2.8.1, 2.2.8.2, 2.2.8.3, 2.2.8.4, 2.2.8.5, 2.2.8.6, 2.2.8.7, 2.2.8.8, 2.2.8.9, |
| 2.2.8.10, 2.2.9.1, 2.2.9.2, 2.2.9.3, 2.2.9.4, 2.2.9.5, 2.2.9.6, 2.2.9.7, 2.2.9.8, 2.2.9.9, 2.2.9.10, 2.2.10.1, 2.2.10.2, 2.2.10.3, 2.2.10.4, 2.2.10.5, 2.2.10.6, 2.2.10.7, 2.2.10.8, 2.2.10.9, 2.2.10.10, 2.3.1.1, 2.3.1.2, 2.3.1.3, 2.3.1.4, 2.3.1.5, 2.3.1.6, 2.3.1.7, 2.3.1.8, 2.3.1.9, 2.3.1.10, 2.3.2.1, 2.3.2.2, 2.3.2.3, 2.3.2.4, 2.3.2.5, 2.3.2.6, 2.3.2.7, 2.3.2.8, 2.3.2.9, 2.3.2.10, 2.3.3.1, 2.3.3.2, 2.3.3.3, 2.3.3.4, 2.3.3.5, 2.3.3.6, 2.3.3.7, 2.3.3.8, 2.3.3.9, 2.3.3.10, 2.3.4.1, 2.3.4.2, 2.3.4.3, 2.3.4.4, 2.3.4.5, 2.3.4.6, 2.3.4.7, 2.3.4.8, 2.3.4.9, 2.3.4.10, 2.3.5.1, 2.3.5.2, 2.3.5.3, 2.3.5.4, 2.3.5.5, 2.3.5.6, 2.3.5.7, 2.3.5.8, 2.3.5.9, 2.3.5.10, 2.3.6.1, 2.3.6.2, 2.3.6.3, 2.3.6.4, 2.3.6.5, 2.3.6.6, 2.3.6.7, 2.3.6.8, 2.3.6.9, 2.3.6.10, 2.3.7.1, 2.3.7.2, 2.3.7.3, 2.3.7.4, 2.3.7.5, 2.3.7.6, 2.3.7.7, 2.3.7.8, 2.3.7.9, 2.3.7.10, 2.3.8.1, 2.3.8.2, 2.3.8.3, 2.3.8.4, 2.3.8.5, 2.3.8.6, 2.3.8.7, 2.3.8.8, 2.3.8.9, 2.3.8.10, 2.3.9.1, 2.3.9.2, 2.3.9.3, 2.3.9.4, 2.3.9.5, 2.3.9.6, 2.3.9.7, 2.3.9.8, 2.3.9.9, 2.3.9.10, 2.3.10.1, 2.3.10.2, 2.3.10.3, 2.3.10.4, 2.3.10.5, 2.3.10.6, 2.3.10.7, 2.3.10.8, 2.3.10.9, 2.3.10.10, 2.4.1.1, 2.4.1.2, 2.4.1.3, 2.4.1.4, 2.4.1.5, 2.4.1.6, 2.4.1.7, 2.4.1.8, 2.4.1.9, 2.4.1.10, 2.4.2.1, 2.4.2.2, 2.4.2.3, 2.4.2.4, 2.4.2.5, 2.4.2.6, 2.4.2.7, 2.4.2.8, 2.4.2.9, 2.4.2.10, 2.4.3.1, 2.4.3.2, 2.4.3.3, 2.4.3.4, 2.4.3.5, 2.4.3.6, 2.4.3.7, 2.4.3.8, 2.4.3.9, 2.4.3.10, 2.4.4.1, 2.4.4.2, 2.4.4.3, 2.4.4.4, 2.4.4.5, 2.4.4.6, 2.4.4.7, 2.4.4.8, 2.4.4.9, 2.4.4.10, 2.4.5.1, 2.4.5.2, 2.4.5.3, 2.4.5.4, 2.4.5.5, 2.4.5.6, 2.4.5.7, 2.4.5.8, 2.4.5.9, 2.4.5.10, 2.4.6.1, 2.4.6.2, 2.4.6.3, 2.4.6.4, 2.4.6.5, 2.4.6.6, 2.4.6.7, 2.4.6.8, 2.4.6.9, 2.4.6.10, 2.4.7.1, 2.4.7.2, 2.4.7.3, 2.4.7.4, 2.4.7.5, 2.4.7.6, 2.4.7.7, 2.4.7.8, 2.4.7.9, 2.4.7.10, 2.4.8.1, 2.4.8.2, 2.4.8.3, 2.4.8.4, 2.4.8.5, 2.4.8.6, 2.4.8.7, 2.4.8.8, 2.4.8.9, 2.4.8.10, 2.4.9.1, 2.4.9.2, 2.4.9.3, 2.4.9.4, 2.4.9.5, 2.4.9.6, 2.4.9.7, 2.4.9.8, 2.4.9.9, 2.4.9.10, 2.4.10.1, 2.4.10.2, 2.4.10.3, 2.4.10.4, 2.4.10.5, 2.4.10.6, 2.4.10.7, 2.4.10.8, 2.4.10.9, 2.4.10.10, 2.5.1.1, 2.5.1.2, 2.5.1.3, 2.5.1.4, 2.5.1.5, 2.5.1.6, 2.5.1.7, 2.5.1.8, 2.5.1.9, 2.5.1.10, 2.5.2.1, 2.5.2.2, 2.5.2.3, 2.5.2.4, 2.5.2.5, 2.5.2.6, 2.5.2.7, 2.5.2.8, 2.5.2.9, 2.5.2.10, 2.5.3.1, 2.5.3.2, 2.5.3.3, 2.5.3.4, 2.5.3.5, 2.5.3.6, 2.5.3.7, 2.5.3.8, 2.5.3.9, 2.5.3.10, 2.5.4.1, 2.5.4.2, 2.5.4.3, 2.5.4.4, 2.5.4.5, 2.5.4.6, 2.5.4.7, 2.5.4.8, 2.5.4.9, 2.5.4.10, 2.5.5.1, 2.5.5.2, 2.5.5.3, 2.5.5.4, 2.5.5.5, 2.5.5.6, 2.5.5.7, 2.5.5.8, 2.5.5.9, 2.5.5.10, 2.5.6.1, 2.5.6.2, 2.5.6.3, 2.5.6.4, 2.5.6.5, 2.5.6.6, 2.5.6.7, 2.5.6.8, 2.5.6.9, 2.5.6.10, 2.5.7.1, 2.5.7.2, 2.5.7.3, 2.5.7.4, 2.5.7.5, 2.5.7.6, 2.5.7.7, 2.5.7.8, 2.5.7.9, 2.5.7.10, 2.5.8.1, 2.5.8.2, 2.5.8.3, 2.5.8.4, 2.5.8.5, 2.5.8.6, 2.5.8.7, 2.5.8.8, 2.5.8.9, 2.5.8.10, 2.5.9.1, 2.5.9.2, 2.5.9.3, 2.5.9.4, 2.5.9.5, 2.5.9.6, 2.5.9.7, 2.5.9.8, 2.5.9.9, 2.5.9.10, 2.5.10.1, 2.5.10.2, 2.5.10.3, 2.5.10.4, 2.5.10.5, 2.5.10.6, 2.5.10.7, 2.5.10.8, 2.5.10.9, 2.5.10.10, 2.6.1.1, 2.6.1.2, 2.6.1.3, 2.6.1.4, 2.6.1.5, 2.6.1.6, 2.6.1.7, 2.6.1.8, 2.6.1.9, 2.6.1.10, 2.6.2.1, 2.6.2.2, 2.6.2.3, 2.6.2.4, 2.6.2.5, 2.6.2.6, 2.6.2.7, 2.6.2.8, 2.6.2.9, 2.6.2.10, 2.6.3.1, 2.6.3.2, 2.6.3.3, 2.6.3.4, 2.6.3.5, 2.6.3.6, 2.6.3.7, 2.6.3.8, 2.6.3.9, 2.6.3.10, 2.6.4.1, 2.6.4.2, 2.6.4.3, 2.6.4.4, 2.6.4.5, 2.6.4.6, 2.6.4.7, 2.6.4.8, 2.6.4.9, 2.6.4.10, 2.6.5.1, 2.6.5.2, 2.6.5.3, 2.6.5.4, 2.6.5.5, 2.6.5.6, 2.6.5.7, 2.6.5.8, 2.6.5.9, 2.6.5.10, 2.6.6.1, 2.6.6.2, 2.6.6.3, 2.6.6.4, 2.6.6.5, 2.6.6.6, 2.6.6.7, 2.6.6.8, 2.6.6.9, 2.6.6.10, 2.6.7.1, 2.6.7.2, 2.6.7.3, 2.6.7.4, 2.6.7.5, 2.6.7.6, 2.6.7.7, 2.6.7.8, 2.6.7.9, 2.6.7.10, 2.6.8.1, 2.6.8.2, 2.6.8.3, 2.6.8.4, 2.6.8.5, 2.6.8.6, 2.6.8.7, 2.6.8.8, 2.6.8.9, 2.6.8.10, 2.6.9.1, 2.6.9.2, 2.6.9.3, 2.6.9.4, 2.6.9.5, 2.6.9.6, 2.6.9.7, 2.6.9.8, 2.6.9.9, 2.6.9.10, 2.6.10.1, 2.6.10.2, 2.6.10.3, 2.6.10.4, 2.6.10.5, 2.6.10.6, 2.6.10.7, 2.6.10.8, 2.6.10.9, 2.6.10.10, 2.7.1.1, 2.7.1.2, 2.7.1.3, 2.7.1.4, 2.7.1.5,2.7.1.6,2.7.1.7,2.7.1.8,2.7.1.9,2.7.1.10,2.7.2.1,2.7.2.2,2.7.2.3,2.7.2.4,2.7.2.5, 2.7.2.6, 2.7.2.7, 2.7.2.8, 2.7.2.9, 2.7.2.10, 2.7.3.1, 2.7.3.2, 2.7.3.3, 2.7.3.4, 2.7.3.5, 2.7.3.6, 2.7.3.7, 2.7.3.8, 2.7.3.9, 2.7.3.10, 2.7.4.1, 2.7.4.2, 2.7.4.3, 2.7.4.4, 2.7.4.5, 2.7.4.6, 2.7.4.7, 2.7.4.8, 2.7.4.9, 2.7.4.10, 2.7.5.1, 2.7.5.2, 2.7.5.3, 2.7.5.4, 2.7.5.5, 2.7.5.6, 2.7.5.7, 2.7.5.8, 2.7.5.9, 2.7.5.10, 2.7.6.1, 2.7.6.2, 2.7.6.3, 2.7.6.4, 2.7.6.5, 2.7.6.6, 2.7.6.7, 2.7.6.8, 2.7.6.9, 2.7.6.10, 2.7.7.1, 2.7.7.2, 2.7.7.3, 2.7.7.4, 2.7.7.5, 2.7.7.6, 2.7.7.7, 2.7.7.8, 2.7.7.9, 2.7.7.10, 2.7.8.1, 2.7.8.2, 2.7.8.3, 2.7.8.4, 2.7.8.5, 2.7.8.6, 2.7.8.7, 2.7.8.8, 2.7.8.9, 2.7.8.10, 2.7.9.1, 2.7.9.2, 2.7.9.3, 2.7.9.4, 2.7.9.5, 2.7.9.6, 2.7.9.7, 2.7.9.8, 2.7.9.9, 2.7.9.10, 2.7.10.1, 2.7.10.2, 2.7.10.3, 2.7.10.4, 2.7.10.5, 2.7.10.6, 2.7.10.7, 2.7.10.8, 2.7.10.9, 2.7.10.10, 2.8.1.1, 2.8.1.2, 2.8.1.3, 2.8.1.4, 2.8.1.5, 2.8.1.6, 2.8.1.7, 2.8.1.8, 2.8.1.9, 2.8.1.10, 2.8.2.1, 2.8.2.2, 2.8.2.3, 2.8.2.4, 2.8.2.5, 2.8.2.6, 2.8.2.7, 2.8.2.8, 2.8.2.9, 2.8.2.10, 2.8.3.1, 2.8.3.2, 2.8.3.3, 2.8.3.4, 2.8.3.5, 2.8.3.6, 2.8.3.7, 2.8.3.8, 2.8.3.9, 2.8.3.10, 2.8.4.1, 2.8.4.2, 2.8.4.3, 2.8.4.4, 2.8.4.5, 2.8.4.6, 2.8.4.7, 2.8.4.8, 2.8.4.9, 2.8.4.10, 2.8.5.1, 2.8.5.2, 2.8.5.3, 2.8.5.4, 2.8.5.5, 2.8.5.6, 2.8.5.7, 2.8.5.8, 2.8.5.9, 2.8.5.10, 2.8.6.1, 2.8.6.2, 2.8.6.3, 2.8.6.4, 2.8.6.5, 2.8.6.6, 2.8.6.7, 2.8.6.8, 2.8.6.9, 2.8.6.10, 2.8.7.1, 2.8.7.2, 2.8.7.3, 2.8.7.4, 2.8.7.5, 2.8.7.6, 2.8.7.7, 2.8.7.8, 2.8.7.9, 2.8.7.10, 2.8.8.1, 2.8.8.2, 2.8.8.3, 2.8.8.4, 2.8.8.5, 2.8.8.6, 2.8.8.7, 2.8.8.8, 2.8.8.9, 2.8.8.10, 2.8.9.1, 2.8.9.2, 2.8.9.3, 2.8.9.4, 2.8.9.5, 2.8.9.6, 2.8.9.7, 2.8.9.8, 2.8.9.9, 2.8.9.10, 2.8.10.1, 2.8.10.2, 2.8.10.3, 2.8.10.4, 2.8.10.5, 2.8.10.6, 2.8.10.7, 2.8.10.8, 2.8.10.9, 2.8.10.10, 2.9.1.1, 2.9.1.2, 2.9.1.3, 2.9.1.4, 2.9.1.5, 2.9.1.6, 2.9.1.7, 2.9.1.8, 2.9.1.9, 2.9.1.10, 2.9.2.1, 2.9.2.2, 2.9.2.3, 2.9.2.4, 2.9.2.5, 2.9.2.6, 2.9.2.7, 2.9.2.8, 2.9.2.9, 2.9.2.10, 2.9.3.1, 2.9.3.2, 2.9.3.3, 2.9.3.4, 2.9.3.5, 2.9.3.6, 2.9.3.7, 2.9.3.8, 2.9.3.9, 2.9.3.10, 2.9.4.1, 2.9.4.2, 2.9.4.3, |
| 2.9.4.4, 2.9.4.5, 2.9.4.6, 2.9.4.7, 2.9.4.8, 2.9.4.9, 2.9.4.10, 2.9.5.1, 2.9.5.2, 2.9.5.3, 2.9.5.4, 2.9.5.5, 2.9.5.6, 2.9.5.7, 2.9.5.8, 2.9.5.9, 2.9.5.10, 2.9.6.1, 2.9.6.2, 2.9.6.3, 2.9.6.4, 2.9.6.5, 2.9.6.6, 2.9.6.7, 2.9.6.8, 2.9.6.9, 2.9.6.10, 2.9.7.1, 2.9.7.2, 2.9.7.3, 2.9.7.4, 2.9.7.5, 2.9.7.6, 2.9.7.7, 2.9.7.8, 2.9.7.9, 2.9.7.10, 2.9.8.1, 2.9.8.2, 2.9.8.3, 2.9.8.4, 2.9.8.5, 2.9.8.6, 2.9.8.7, 2.9.8.8, 2.9.8.9, 2.9.8.10, 2.9.9.1, 2.9.9.2, 2.9.9.3, 2.9.9.4, 2.9.9.5, 2.9.9.6, 2.9.9.7, 2.9.9.8, 2.9.9.9, 2.9.9.10, 2.9.10.1, 2.9.10.2, 2.9.10.3, 2.9.10.4, 2.9.10.5, 2.9.10.6, 2.9.10.7, 2.9.10.8, 2.9.10.9, 2.9.10.10, 2.10.1.1, 2.10.1.2, 2.10.1.3, 2.10.1.4, 2.10.1.5, 2.10.1.6, 2.10.1.7, 2.10.1.8, 2.10.1.9, 2.10.1.10, 2.10.2.1, 2.10.2.2, 2.10.2.3, 2.10.2.4, 2.10.2.5, 2.10.2.6, 2.10.2.7, 2.10.2.8, 2.10.2.9, 2.10.2.10, 2.10.3.1, 2.10.3.2, 2.10.3.3, 2.10.3.4, 2.10.3.5, 2.10.3.6, 2.10.3.7, 2.10.3.8, 2.10.3.9, 2.10.3.10, 2.10.4.1, 2.10.4.2, 2.10.4.3, 2.10.4.4, 2.10.4.5, 2.10.4.6, 2.10.4.7, 2.10.4.8, 2.10.4.9, 2.10.4.10, 2.10.5.1, 2.10.5.2, 2.10.5.3, 2.10.5.4, 2.10.5.5, 2.10.5.6, 2.10.5.7, 2.10.5.8, 2.10.5.9, 2.10.5.10, 2.10.6.1, 2.10.6.2, 2.10.6.3, 2.10.6.4, 2.10.6.5, 2.10.6.6, 2.10.6.7, 2.10.6.8, 2.10.6.9, 2.10.6.10, 2.10.7.1, 2.10.7.2, 2.10.7.3, 2.10.7.4, 2.10.7.5, 2.10.7.6, 2.10.7.7, 2.10.7.8, 2.10.7.9, 2.10.7.10, 2.10.8.1, 2.10.8.2, 2.10.8.3, 2.10.8.4, 2.10.8.5, 2.10.8.6, 2.10.8.7, 2.10.8.8, 2.10.8.9, 2.10.8.10, 2.10.9.1, 2.10.9.2, 2.10.9.3, 2.10.9.4, 2.10.9.5, 2.10.9.6, 2.10.9.7, 2.10.9.8, 2.10.9.9, 2.10.9.10, 2.10.10.1, 2.10.10.2, 2.10.10.3, 2.10.10.4, 2.10.10.5, 2.10.10.6, 2.10.10.7, 2.10.10.8, 2.10.10.9, 2.10.10.10, 3.1.1.1, 3.1.1.2, 3.1.1.3, 3.1.1.4, 3.1.1.5, 3.1.1.6, 3.1.1.7, 3.1.1.8, 3.1.1.9, 3.1.1.10, 3.1.2.1, 3.1.2.2, 3.1.2.3, 3.1.2.4, 3.1.2.5, 3.1.2.6, 3.1.2.7, 3.1.2.8, 3.1.2.9, 3.1.2.10, 3.1.3.1, 3.1.3.2, 3.1.3.3, 3.1.3.4, 3.1.3.5, 3.1.3.6, 3.1.3.7, 3.1.3.8, 3.1.3.9, 3.1.3.10, 3.1.4.1, 3.1.4.2, 3.1.4.3, 3.1.4.4, 3.1.4.5, 3.1.4.6, 3.1.4.7, 3.1.4.8, 3.1.4.9, 3.1.4.10, 3.1.5.1, 3.1.5.2, 3.1.5.3, 3.1.5.4, 3.1.5.5, 3.1.5.6, 3.1.5.7, 3.1.5.8, 3.1.5.9, 3.1.5.10, 3.1.6.1, 3.1.6.2, 3.1.6.3, 3.1.6.4, 3.1.6.5, 3.1.6.6, 3.1.6.7, 3.1.6.8, 3.1.6.9, 3.1.6.10, 3.1.7.1, 3.1.7.2, 3.1.7.3,3.1.7.4,3.1.7.5,3.1.7.6,3.1.7.7,3.1.7.8,3.1.7.9,3.1.7.10,3.1.8.1,3.1.8.2,3.1.8.3, 3.1.8.4, 3.1.8.5, 3.1.8.6, 3.1.8.7, 3.1.8.8, 3.1.8.9, 3.1.8.10, 3.1.9.1, 3.1.9.2, 3.1.9.3, 3.1.9.4, 3.1.9.5, 3.1.9.6, 3.1.9.7, 3.1.9.8, 3.1.9.9, 3.1.9.10, 3.1.10.1, 3.1.10.2, 3.1.10.3, 3.1.10.4, 3.1.10.5, 3.1.10.6, 3.1.10.7, 3.1.10.8, 3.1.10.9, 3.1.10.10, 3.2.1.1, 3.2.1.2, 3.2.1.3, 3.2.1.4, 3.2.1.5, 3.2.1.6, 3.2.1.7, 3.2.1.8, 3.2.1.9, 3.2.1.10, 3.2.2.1, 3.2.2.2, 3.2.2.3, 3.2.2.4, 3.2.2.5, 3.2.2.6, 3.2.2.7, 3.2.2.8, 3.2.2.9, 3.2.2.10, 3.2.3.1, 3.2.3.2, 3.2.3.3, 3.2.3.4, 3.2.3.5, 3.2.3.6, 3.2.3.7, 3.2.3.8, 3.2.3.9, 3.2.3.10, 3.2.4.1, 3.2.4.2, 3.2.4.3, 3.2.4.4, 3.2.4.5, 3.2.4.6, 3.2.4.7, 3.2.4.8, 3.2.4.9, 3.2.4.10, 3.2.5.1, 3.2.5.2, 3.2.5.3, 3.2.5.4, 3.2.5.5, 3.2.5.6, 3.2.5.7, 3.2.5.8, 3.2.5.9, 3.2.5.10, 3.2.6.1, 3.2.6.2, 3.2.6.3, 3.2.6.4, 3.2.6.5, 3.2.6.6, 3.2.6.7, 3.2.6.8, 3.2.6.9, 3.2.6.10, 3.2.7.1, 3.2.7.2, 3.2.7.3, 3.2.7.4, 3.2.7.5, 3.2.7.6, 3.2.7.7, 3.2.7.8, 3.2.7.9, 3.2.7.10, 3.2.8.1, 3.2.8.2, 3.2.8.3, 3.2.8.4, 3.2.8.5, 3.2.8.6, 3.2.8.7, 3.2.8.8, 3.2.8.9, 3.2.8.10, 3.2.9.1, 3.2.9.2, 3.2.9.3, 3.2.9.4, 3.2.9.5, 3.2.9.6, 3.2.9.7, 3.2.9.8, 3.2.9.9, 3.2.9.10, 3.2.10.1, 3.2.10.2, 3.2.10.3, 3.2.10.4, 3.2.10.5, 3.2.10.6, 3.2.10.7, 3.2.10.8, 3.2.10.9, 3.2.10.10, 3.3.1.1, 3.3.1.2, 3.3.1.3, 3.3.1.4, 3.3.1.5, 3.3.1.6, 3.3.1.7, 3.3.1.8, 3.3.1.9, 3.3.1.10, 3.3.2.1, 3.3.2.2, 3.3.2.3, 3.3.2.4, 3.3.2.5, 3.3.2.6, 3.3.2.7, 3.3.2.8, 3.3.2.9, 3.3.2.10, 3.3.3.1, 3.3.3.2, 3.3.3.3, 3.3.3.4, 3.3.3.5, 3.3.3.6, 3.3.3.7, 3.3.3.8, 3.3.3.9, 3.3.3.10, 3.3.4.1, 3.3.4.2, 3.3.4.3, 3.3.4.4, 3.3.4.5, 3.3.4.6, 3.3.4.7, 3.3.4.8, 3.3.4.9, 3.3.4.10, 3.3.5.1, 3.3.5.2, 3.3.5.3, 3.3.5.4, 3.3.5.5, 3.3.5.6, 3.3.5.7, 3.3.5.8, 3.3.5.9, 3.3.5.10, 3.3.6.1, 3.3.6.2, 3.3.6.3, 3.3.6.4, 3.3.6.5, 3.3.6.6, 3.3.6.7, 3.3.6.8, 3.3.6.9, 3.3.6.10, 3.3.7.1, 3.3.7.2, 3.3.7.3, 3.3.7.4, 3.3.7.5, 3.3.7.6, 3.3.7.7, 3.3.7.8, 3.3.7.9, 3.3.7.10, 3.3.8.1, 3.3.8.2, 3.3.8.3, 3.3.8.4, 3.3.8.5, 3.3.8.6, 3.3.8.7, 3.3.8.8, 3.3.8.9, 3.3.8.10, 3.3.9.1, 3.3.9.2, 3.3.9.3, 3.3.9.4, 3.3.9.5, 3.3.9.6, 3.3.9.7, 3.3.9.8, 3.3.9.9, 3.3.9.10, 3.3.10.1, 3.3.10.2, 3.3.10.3, 3.3.10.4, 3.3.10.5, 3.3.10.6, 3.3.10.7, 3.3.10.8, 3.3.10.9, 3.3.10.10, 3.4.1.1, 3.4.1.2, 3.4.1.3, 3.4.1.4, 3.4.1.5, 3.4.1.6, 3.4.1.7, 3.4.1.8, 3.4.1.9, 3.4.1.10, 3.4.2.1, 3.4.2.2, 3.4.2.3, 3.4.2.4, 3.4.2.5, 3.4.2.6, 3.4.2.7, 3.4.2.8, 3.4.2.9, 3.4.2.10, 3.4.3.1, 3.4.3.2, 3.4.3.3, 3.4.3.4, 3.4.3.5, 3.4.3.6, 3.4.3.7, 3.4.3.8, 3.4.3.9, 3.4.3.10, 3.4.4.1, 3.4.4.2, 3.4.4.3, 3.4.4.4, 3.4.4.5, 3.4.4.6, 3.4.4.7, 3.4.4.8, 3.4.4.9, 3.4.4.10, 3.4.5.1, 3.4.5.2, 3.4.5.3, 3.4.5.4, 3.4.5.5, 3.4.5.6, 3.4.5.7, 3.4.5.8, 3.4.5.9, 3.4.5.10, 3.4.6.1, 3.4.6.2, 3.4.6.3, 3.4.6.4, 3.4.6.5, 3.4.6.6, 3.4.6.7, 3.4.6.8, 3.4.6.9, 3.4.6.10, 3.4.7.1, 3.4.7.2, 3.4.7.3, 3.4.7.4, 3.4.7.5, 3.4.7.6, 3.4.7.7, 3.4.7.8, 3.4.7.9, 3.4.7.10, 3.4.8.1, 3.4.8.2, 3.4.8.3, 3.4.8.4, 3.4.8.5, 3.4.8.6, 3.4.8.7, 3.4.8.8, 3.4.8.9, 3.4.8.10, 3.4.9.1, 3.4.9.2, 3.4.9.3, 3.4.9.4, 3.4.9.5, 3.4.9.6, 3.4.9.7, 3.4.9.8, 3.4.9.9, 3.4.9.10, 3.4.10.1, 3.4.10.2, 3.4.10.3, 3.4.10.4, 3.4.10.5, 3.4.10.6, 3.4.10.7, 3.4.10.8, 3.4.10.9, 3.4.10.10, 3.5.1.1, 3.5.1.2, 3.5.1.3, 3.5.1.4, 3.5.1.5, 3.5.1.6, 3.5.1.7, 3.5.1.8, 3.5.1.9, 3.5.1.10, 3.5.2.1, 3.5.2.2, 3.5.2.3, 3.5.2.4, 3.5.2.5, 3.5.2.6, 3.5.2.7, 3.5.2.8, 3.5.2.9, 3.5.2.10, 3.5.3.1, 3.5.3.2, 3.5.3.3, 3.5.3.4, 3.5.3.5, 3.5.3.6, 3.5.3.7, 3.5.3.8, 3.5.3.9, 3.5.3.10, 3.5.4.1, 3.5.4.2, 3.5.4.3, 3.5.4.4, 3.5.4.5, 3.5.4.6, 3.5.4.7, 3.5.4.8, 3.5.4.9, 3.5.4.10, 3.5.5.1, 3.5.5.2, 3.5.5.3, 3.5.5.4, 3.5.5.5, 3.5.5.6, 3.5.5.7, 3.5.5.8, 3.5.5.9, 3.5.5.10, 3.5.6.1, 3.5.6.2, 3.5.6.3, 3.5.6.4, 3.5.6.5, 3.5.6.6, 3.5.6.7, 3.5.6.8, 3.5.6.9, 3.5.6.10, 3.5.7.1, 3.5.7.2, 3.5.7.3, 3.5.7.4, 3.5.7.5, 3.5.7.6, 3.5.7.7, 3.5.7.8, 3.5.7.9, 3.5.7.10, 3.5.8.1, 3.5.8.2, 3.5.8.3, 3.5.8.4, 3.5.8.5, 3.5.8.6, 3.5.8.7, |
| 3.5.8.8, 3.5.8.9, 3.5.8.10, 3.5.9.1, 3.5.9.2, 3.5.9.3, 3.5.9.4, 3.5.9.5, 3.5.9.6, 3.5.9.7, 3.5.9.8, 3.5.9.9, 3.5.9.10, 3.5.10.1, 3.5.10.2, 3.5.10.3, 3.5.10.4, 3.5.10.5, 3.5.10.6, 3.5.10.7, 3.5.10.8, 3.5.10.9, 3.5.10.10, 3.6.1.1, 3.6.1.2, 3.6.1.3, 3.6.1.4, 3.6.1.5, 3.6.1.6, 3.6.1.7, 3.6.1.8, 3.6.1.9, 3.6.1.10, 3.6.2.1, 3.6.2.2, 3.6.2.3, 3.6.2.4, 3.6.2.5, 3.6.2.6, 3.6.2.7, 3.6.2.8, 3.6.2.9, 3.6.2.10, 3.6.3.1, 3.6.3.2, 3.6.3.3, 3.6.3.4, 3.6.3.5, 3.6.3.6, 3.6.3.7, 3.6.3.8, 3.6.3.9, 3.6.3.10, 3.6.4.1, 3.6.4.2, 3.6.4.3, 3.6.4.4, 3.6.4.5, 3.6.4.6, 3.6.4.7, 3.6.4.8, 3.6.4.9, 3.6.4.10, 3.6.5.1, 3.6.5.2, 3.6.5.3, 3.6.5.4, 3.6.5.5, 3.6.5.6, 3.6.5.7, 3.6.5.8, 3.6.5.9, 3.6.5.10, 3.6.6.1, 3.6.6.2, 3.6.6.3, 3.6.6.4, 3.6.6.5, 3.6.6.6, 3.6.6.7, 3.6.6.8, 3.6.6.9, 3.6.6.10, 3.6.7.1, 3.6.7.2, 3.6.7.3, 3.6.7.4, 3.6.7.5, 3.6.7.6, 3.6.7.7, 3.6.7.8, 3.6.7.9, 3.6.7.10, 3.6.8.1, 3.6.8.2, 3.6.8.3, 3.6.8.4, 3.6.8.5, 3.6.8.6, 3.6.8.7, 3.6.8.8, 3.6.8.9, 3.6.8.10, 3.6.9.1, 3.6.9.2, 3.6.9.3, 3.6.9.4, 3.6.9.5, 3.6.9.6, 3.6.9.7, 3.6.9.8, 3.6.9.9, 3.6.9.10, 3.6.10.1, 3.6.10.2, 3.6.10.3, 3.6.10.4, 3.6.10.5, 3.6.10.6, 3.6.10.7, 3.6.10.8, 3.6.10.9, 3.6.10.10, 3.7.1.1, 3.7.1.2, 3.7.1.3, 3.7.1.4, 3.7.1.5, 3.7.1.6, 3.7.1.7, 3.7.1.8, 3.7.1.9, 3.7.1.10, 3.7.2.1, 3.7.2.2, 3.7.2.3, 3.7.2.4, 3.7.2.5, 3.7.2.6, 3.7.2.7, 3.7.2.8, 3.7.2.9, 3.7.2.10, 3.7.3.1, 3.7.3.2, 3.7.3.3, 3.7.3.4, 3.7.3.5, 3.7.3.6, 3.7.3.7, 3.7.3.8, 3.7.3.9, 3.7.3.10, 3.7.4.1, 3.7.4.2, 3.7.4.3, 3.7.4.4, 3.7.4.5, 3.7.4.6, 3.7.4.7, 3.7.4.8, 3.7.4.9, 3.7.4.10, 3.7.5.1, 3.7.5.2, 3.7.5.3, 3.7.5.4, 3.7.5.5, 3.7.5.6, 3.7.5.7, 3.7.5.8, 3.7.5.9, 3.7.5.10, 3.7.6.1, 3.7.6.2, 3.7.6.3, 3.7.6.4, 3.7.6.5, 3.7.6.6, 3.7.6.7, 3.7.6.8, 3.7.6.9, 3.7.6.10, 3.7.7.1, 3.7.7.2, 3.7.7.3, 3.7.7.4, 3.7.7.5, 3.7.7.6, 3.7.7.7, 3.7.7.8, 3.7.7.9, 3.7.7.10, 3.7.8.1, 3.7.8.2, 3.7.8.3, 3.7.8.4, 3.7.8.5, 3.7.8.6, 3.7.8.7, 3.7.8.8, 3.7.8.9, 3.7.8.10, 3.7.9.1, 3.7.9.2, 3.7.9.3, 3.7.9.4, 3.7.9.5, 3.7.9.6, 3.7.9.7, 3.7.9.8, 3.7.9.9, 3.7.9.10, 3.7.10.1, 3.7.10.2, 3.7.10.3, 3.7.10.4, 3.7.10.5, 3.7.10.6, 3.7.10.7, 3.7.10.8, 3.7.10.9, 3.7.10.10, 3.8.1.1, 3.8.1.2, 3.8.1.3, 3.8.1.4, 3.8.1.5, 3.8.1.6, 3.8.1.7, 3.8.1.8, 3.8.1.9, 3.8.1.10, 3.8.2.1, 3.8.2.2, 3.8.2.3, 3.8.2.4, 3.8.2.5, 3.8.2.6, 3.8.2.7, 3.8.2.8, 3.8.2.9, 3.8.2.10, 3.8.3.1, 3.8.3.2, 3.8.3.3, 3.8.3.4, 3.8.3.5, 3.8.3.6, 3.8.3.7, 3.8.3.8, 3.8.3.9, 3.8.3.10, 3.8.4.1, 3.8.4.2, 3.8.4.3, 3.8.4.4, 3.8.4.5, 3.8.4.6, 3.8.4.7, 3.8.4.8, 3.8.4.9, 3.8.4.10, 3.8.5.1, 3.8.5.2, 3.8.5.3, 3.8.5.4, 3.8.5.5, 3.8.5.6, 3.8.5.7, 3.8.5.8, 3.8.5.9, 3.8.5.10, 3.8.6.1, 3.8.6.2, 3.8.6.3, 3.8.6.4, 3.8.6.5, 3.8.6.6, 3.8.6.7, 3.8.6.8, 3.8.6.9, 3.8.6.10, 3.8.7.1, 3.8.7.2, 3.8.7.3, 3.8.7.4, 3.8.7.5, 3.8.7.6, 3.8.7.7, 3.8.7.8, 3.8.7.9, 3.8.7.10, 3.8.8.1, 3.8.8.2, 3.8.8.3, 3.8.8.4, 3.8.8.5, 3.8.8.6, 3.8.8.7, 3.8.8.8, 3.8.8.9, 3.8.8.10, 3.8.9.1, 3.8.9.2, 3.8.9.3, 3.8.9.4, 3.8.9.5, 3.8.9.6, 3.8.9.7, 3.8.9.8, 3.8.9.9, 3.8.9.10, 3.8.10.1, 3.8.10.2, 3.8.10.3, 3.8.10.4, 3.8.10.5, 3.8.10.6, 3.8.10.7, 3.8.10.8, 3.8.10.9, 3.8.10.10, 3.9.1.1, 3.9.1.2, 3.9.1.3, 3.9.1.4, 3.9.1.5, 3.9.1.6, 3.9.1.7, 3.9.1.8, 3.9.1.9, 3.9.1.10, 3.9.2.1, 3.9.2.2, 3.9.2.3, 3.9.2.4, 3.9.2.5, 3.9.2.6, 3.9.2.7, 3.9.2.8, 3.9.2.9, 3.9.2.10, 3.9.3.1, 3.9.3.2, 3.9.3.3, 3.9.3.4, 3.9.3.5, 3.9.3.6, 3.9.3.7, 3.9.3.8, 3.9.3.9, 3.9.3.10, 3.9.4.1, 3.9.4.2, 3.9.4.3, 3.9.4.4, 3.9.4.5, 3.9.4.6, 3.9.4.7, 3.9.4.8, 3.9.4.9, 3.9.4.10, 3.9.5.1, 3.9.5.2, 3.9.5.3, 3.9.5.4, 3.9.5.5, 3.9.5.6, 3.9.5.7, 3.9.5.8, 3.9.5.9, 3.9.5.10, 3.9.6.1, 3.9.6.2, 3.9.6.3, 3.9.6.4, 3.9.6.5, 3.9.6.6, 3.9.6.7, 3.9.6.8, 3.9.6.9, 3.9.6.10, 3.9.7.1, 3.9.7.2, 3.9.7.3, 3.9.7.4, 3.9.7.5, 3.9.7.6, 3.9.7.7, 3.9.7.8, 3.9.7.9, 3.9.7.10, 3.9.8.1, 3.9.8.2, 3.9.8.3, 3.9.8.4, 3.9.8.5, 3.9.8.6, 3.9.8.7, 3.9.8.8, 3.9.8.9, 3.9.8.10, 3.9.9.1, 3.9.9.2, 3.9.9.3, 3.9.9.4, 3.9.9.5, 3.9.9.6, 3.9.9.7, 3.9.9.8, 3.9.9.9, 3.9.9.10, 3.9.10.1, 3.9.10.2, 3.9.10.3, 3.9.10.4, 3.9.10.5, 3.9.10.6, 3.9.10.7, 3.9.10.8, 3.9.10.9, 3.9.10.10, 3.10.1.1, 3.10.1.2, 3.10.1.3, 3.10.1.4, 3.10.1.5, 3.10.1.6, 3.10.1.7, 3.10.1.8, 3.10.1.9, 3.10.1.10, 3.10.2.1, 3.10.2.2, 3.10.2.3, 3.10.2.4, 3.10.2.5, 3.10.2.6, 3.10.2.7, 3.10.2.8, 3.10.2.9, 3.10.2.10, 3.10.3.1, 3.10.3.2, 3.10.3.3, 3.10.3.4, 3.10.3.5, 3.10.3.6, 3.10.3.7, 3.10.3.8, 3.10.3.9, 3.10.3.10, 3.10.4.1, 3.10.4.2, 3.10.4.3, 3.10.4.4, 3.10.4.5, 3.10.4.6, 3.10.4.7, 3.10.4.8, 3.10.4.9, 3.10.4.10, 3.10.5.1, 3.10.5.2, 3.10.5.3, 3.10.5.4, 3.10.5.5, 3.10.5.6, 3.10.5.7, 3.10.5.8, 3.10.5.9, 3.10.5.10, 3.10.6.1, 3.10.6.2, 3.10.6.3, 3.10.6.4, 3.10.6.5, 3.10.6.6, 3.10.6.7, 3.10.6.8, 3.10.6.9, 3.10.6.10, 3.10.7.1, 3.10.7.2, 3.10.7.3, 3.10.7.4, 3.10.7.5, 3.10.7.6, 3.10.7.7, 3.10.7.8, 3.10.7.9, 3.10.7.10, 3.10.8.1, 3.10.8.2, 3.10.8.3, 3.10.8.4, 3.10.8.5, 3.10.8.6, 3.10.8.7, 3.10.8.8, 3.10.8.9, 3.10.8.10, 3.10.9.1, 3.10.9.2, 3.10.9.3, 3.10.9.4, 3.10.9.5, 3.10.9.6, 3.10.9.7, 3.10.9.8, 3.10.9.9, 3.10.9.10, 3.10.10.1, 3.10.10.2, 3.10.10.3, 3.10.10.4, 3.10.10.5, 3.10.10.6, 3.10.10.7, 3.10.10.8, 3.10.10.9, 3.10.10.10, 4.1.1.1, 4.1.1.2, 4.1.1.3, 4.1.1.4, 4.1.1.5, 4.1.1.6, 4.1.1.7, 4.1.1.8, 4.1.1.9, 4.1.1.10, 4.1.2.1, 4.1.2.2, 4.1.2.3, 4.1.2.4, 4.1.2.5, 4.1.2.6, 4.1.2.7, 4.1.2.8, 4.1.2.9, 4.1.2.10, 4.1.3.1, 4.1.3.2, 4.1.3.3, 4.1.3.4, 4.1.3.5, 4.1.3.6, 4.1.3.7, 4.1.3.8, 4.1.3.9, 4.1.3.10, 4.1.4.1, 4.1.4.2, 4.1.4.3, 4.1.4.4, 4.1.4.5, 4.1.4.6, 4.1.4.7, 4.1.4.8, 4.1.4.9, 4.1.4.10, 4.1.5.1, 4.1.5.2, 4.1.5.3, 4.1.5.4, 4.1.5.5, 4.1.5.6, 4.1.5.7, 4.1.5.8, 4.1.5.9, 4.1.5.10, 4.1.6.1, 4.1.6.2, 4.1.6.3, 4.1.6.4, 4.1.6.5, 4.1.6.6, 4.1.6.7,4.1.6.8,4.1.6.9,4.1.6.10,4.1.7.1,4.1.7.2,4.1.7.3,4.1.7.4,4.1.7.5,4.1.7.6,4.1.7.7, 4.1.7.8, 4.1.7.9, 4.1.7.10, 4.1.8.1, 4.1.8.2, 4.1.8.3, 4.1.8.4, 4.1.8.5, 4.1.8.6, 4.1.8.7, 4.1.8.8, 4.1.8.9, 4.1.8.10, 4.1.9.1, 4.1.9.2, 4.1.9.3, 4.1.9.4, 4.1.9.5, 4.1.9.6, 4.1.9.7, 4.1.9.8, 4.1.9.9, 4.1.9.10, 4.1.10.1, 4.1.10.2, 4.1.10.3, 4.1.10.4, 4.1.10.5, 4.1.10.6, 4.1.10.7, 4.1.10.8, 4.1.10.9, 4.1.10.10, 4.2.1.1, 4.2.1.2, 4.2.1.3, 4.2.1.4, 4.2.1.5, 4.2.1.6, 4.2.1.7, 4.2.1.8, 4.2.1.9, 4.2.1.10, 4.2.2.1, 4.2.2.2, 4.2.2.3, 4.2.2.4, 4.2.2.5, 4.2.2.6, 4.2.2.7, 4.2.2.8, 4.2.2.9, 4.2.2.10, 4.2.3.1, |
| 4.2.3.2, 4.2.3.3, 4.2.3.4, 4.2.3.5, 4.2.3.6, 4.2.3.7, 4.2.3.8, 4.2.3.9, 4.2.3.10, 4.2.4.1, 4.2.4.2, 4.2.4.3, 4.2.4.4, 4.2.4.5, 4.2.4.6, 4.2.4.7, 4.2.4.8, 4.2.4.9, 4.2.4.10, 4.2.5.1, 4.2.5.2, 4.2.5.3, 4.2.5.4, 4.2.5.5, 4.2.5.6, 4.2.5.7, 4.2.5.8, 4.2.5.9, 4.2.5.10, 4.2.6.1, 4.2.6.2, 4.2.6.3, 4.2.6.4, 4.2.6.5, 4.2.6.6, 4.2.6.7, 4.2.6.8, 4.2.6.9, 4.2.6.10, 4.2.7.1, 4.2.7.2, 4.2.7.3, 4.2.7.4, 4.2.7.5, 4.2.7.6, 4.2.7.7, 4.2.7.8, 4.2.7.9, 4.2.7.10, 4.2.8.1, 4.2.8.2, 4.2.8.3, 4.2.8.4, 4.2.8.5, 4.2.8.6, 4.2.8.7, 4.2.8.8, 4.2.8.9, 4.2.8.10, 4.2.9.1, 4.2.9.2, 4.2.9.3, 4.2.9.4, 4.2.9.5, 4.2.9.6, 4.2.9.7, 4.2.9.8, 4.2.9.9, 4.2.9.10, 4.2.10.1, 4.2.10.2, 4.2.10.3, 4.2.10.4, 4.2.10.5, 4.2.10.6, 4.2.10.7, 4.2.10.8, 4.2.10.9, 4.2.10.10, 4.3.1.1, 4.3.1.2, 4.3.1.3, 4.3.1.4, 4.3.1.5, 4.3.1.6, 4.3.1.7, 4.3.1.8, 4.3.1.9, 4.3.1.10, 4.3.2.1, 4.3.2.2, 4.3.2.3, 4.3.2.4, 4.3.2.5, 4.3.2.6, 4.3.2.7, 4.3.2.8, 4.3.2.9, 4.3.2.10, 4.3.3.1, 4.3.3.2, 4.3.3.3, 4.3.3.4, 4.3.3.5, 4.3.3.6, 4.3.3.7, 4.3.3.8, 4.3.3.9, 4.3.3.10, 4.3.4.1, 4.3.4.2, 4.3.4.3, 4.3.4.4, 4.3.4.5, 4.3.4.6, 4.3.4.7, 4.3.4.8, 4.3.4.9, 4.3.4.10, 4.3.5.1, 4.3.5.2, 4.3.5.3, 4.3.5.4, 4.3.5.5, 4.3.5.6, 4.3.5.7, 4.3.5.8, 4.3.5.9, 4.3.5.10, 4.3.6.1, 4.3.6.2, 4.3.6.3, 4.3.6.4, 4.3.6.5, 4.3.6.6, 4.3.6.7, 4.3.6.8, 4.3.6.9, 4.3.6.10, 4.3.7.1, 4.3.7.2, 4.3.7.3, 4.3.7.4, 4.3.7.5, 4.3.7.6, 4.3.7.7, 4.3.7.8, 4.3.7.9, 4.3.7.10, 4.3.8.1, 4.3.8.2, 4.3.8.3, 4.3.8.4, 4.3.8.5, 4.3.8.6, 4.3.8.7, 4.3.8.8, 4.3.8.9, 4.3.8.10, 4.3.9.1, 4.3.9.2, 4.3.9.3, 4.3.9.4, 4.3.9.5, 4.3.9.6, 4.3.9.7, 4.3.9.8, 4.3.9.9, 4.3.9.10, 4.3.10.1, 4.3.10.2, 4.3.10.3, 4.3.10.4, 4.3.10.5, 4.3.10.6, 4.3.10.7, 4.3.10.8, 4.3.10.9, 4.3.10.10, 4.4.1.1, 4.4.1.2, 4.4.1.3, 4.4.1.4, 4.4.1.5, 4.4.1.6, 4.4.1.7, 4.4.1.8, 4.4.1.9, 4.4.1.10, 4.4.2.1, 4.4.2.2, 4.4.2.3, 4.4.2.4, 4.4.2.5, 4.4.2.6, 4.4.2.7, 4.4.2.8, 4.4.2.9, 4.4.2.10, 4.4.3.1, 4.4.3.2, 4.4.3.3, 4.4.3.4, 4.4.3.5, 4.4.3.6, 4.4.3.7, 4.4.3.8, 4.4.3.9, 4.4.3.10, 4.4.4.1, 4.4.4.2, 4.4.4.3, 4.4.4.4, 4.4.4.5, 4.4.4.6, 4.4.4.7, 4.4.4.8, 4.4.4.9, 4.4.4.10, 4.4.5.1, 4.4.5.2, 4.4.5.3, 4.4.5.4, 4.4.5.5, 4.4.5.6, 4.4.5.7, 4.4.5.8, 4.4.5.9, 4.4.5.10, 4.4.6.1, 4.4.6.2, 4.4.6.3, 4.4.6.4, 4.4.6.5, 4.4.6.6, 4.4.6.7, 4.4.6.8, 4.4.6.9, 4.4.6.10, 4.4.7.1, 4.4.7.2, 4.4.7.3, 4.4.7.4, 4.4.7.5, 4.4.7.6, 4.4.7.7, 4.4.7.8, 4.4.7.9, 4.4.7.10, 4.4.8.1, 4.4.8.2, 4.4.8.3, 4.4.8.4, 4.4.8.5, 4.4.8.6, 4.4.8.7, 4.4.8.8, 4.4.8.9, 4.4.8.10, 4.4.9.1, 4.4.9.2, 4.4.9.3, 4.4.9.4, 4.4.9.5, 4.4.9.6, 4.4.9.7, 4.4.9.8, 4.4.9.9, 4.4.9.10, 4.4.10.1, 4.4.10.2, 4.4.10.3, 4.4.10.4, 4.4.10.5, 4.4.10.6, 4.4.10.7, 4.4.10.8, 4.4.10.9, 4.4.10.10, 4.5.1.1, 4.5.1.2, 4.5.1.3, 4.5.1.4, 4.5.1.5, 4.5.1.6, 4.5.1.7, 4.5.1.8, 4.5.1.9, 4.5.1.10, 4.5.2.1, 4.5.2.2, 4.5.2.3, 4.5.2.4, 4.5.2.5, 4.5.2.6, 4.5.2.7, 4.5.2.8, 4.5.2.9, 4.5.2.10, 4.5.3.1, 4.5.3.2, 4.5.3.3, 4.5.3.4, 4.5.3.5, 4.5.3.6, 4.5.3.7, 4.5.3.8, 4.5.3.9, 4.5.3.10, 4.5.4.1, 4.5.4.2, 4.5.4.3, 4.5.4.4, 4.5.4.5, 4.5.4.6, 4.5.4.7, 4.5.4.8, 4.5.4.9, 4.5.4.10, 4.5.5.1, 4.5.5.2, 4.5.5.3, 4.5.5.4, 4.5.5.5, 4.5.5.6, 4.5.5.7, 4.5.5.8, 4.5.5.9, 4.5.5.10, 4.5.6.1, 4.5.6.2, 4.5.6.3, 4.5.6.4, 4.5.6.5, 4.5.6.6, 4.5.6.7, 4.5.6.8, 4.5.6.9, 4.5.6.10, 4.5.7.1, 4.5.7.2, 4.5.7.3, 4.5.7.4, 4.5.7.5, 4.5.7.6, 4.5.7.7, 4.5.7.8, 4.5.7.9, 4.5.7.10, 4.5.8.1, 4.5.8.2, 4.5.8.3, 4.5.8.4, 4.5.8.5, 4.5.8.6, 4.5.8.7, 4.5.8.8, 4.5.8.9, 4.5.8.10, 4.5.9.1, 4.5.9.2, 4.5.9.3, 4.5.9.4, 4.5.9.5, 4.5.9.6, 4.5.9.7, 4.5.9.8, 4.5.9.9, 4.5.9.10, 4.5.10.1, 4.5.10.2, 4.5.10.3, 4.5.10.4, 4.5.10.5, 4.5.10.6, 4.5.10.7, 4.5.10.8, 4.5.10.9, 4.5.10.10, 4.6.1.1, 4.6.1.2, 4.6.1.3, 4.6.1.4, 4.6.1.5, 4.6.1.6, 4.6.1.7, 4.6.1.8, 4.6.1.9, 4.6.1.10, 4.6.2.1, 4.6.2.2, 4.6.2.3, 4.6.2.4, 4.6.2.5, 4.6.2.6, 4.6.2.7, 4.6.2.8, 4.6.2.9, 4.6.2.10, 4.6.3.1, 4.6.3.2, 4.6.3.3, 4.6.3.4, 4.6.3.5, 4.6.3.6, 4.6.3.7, 4.6.3.8, 4.6.3.9, 4.6.3.10, 4.6.4.1, 4.6.4.2, 4.6.4.3, 4.6.4.4, 4.6.4.5, 4.6.4.6, 4.6.4.7, 4.6.4.8, 4.6.4.9, 4.6.4.10, 4.6.5.1, 4.6.5.2, 4.6.5.3, 4.6.5.4, 4.6.5.5, 4.6.5.6, 4.6.5.7, 4.6.5.8, 4.6.5.9, 4.6.5.10, 4.6.6.1, 4.6.6.2, 4.6.6.3, 4.6.6.4, 4.6.6.5, 4.6.6.6, 4.6.6.7, 4.6.6.8, 4.6.6.9, 4.6.6.10, 4.6.7.1, 4.6.7.2, 4.6.7.3, 4.6.7.4, 4.6.7.5, 4.6.7.6, 4.6.7.7, 4.6.7.8, 4.6.7.9, 4.6.7.10, 4.6.8.1, 4.6.8.2, 4.6.8.3, 4.6.8.4, 4.6.8.5, 4.6.8.6, 4.6.8.7, 4.6.8.8, 4.6.8.9, 4.6.8.10, 4.6.9.1, 4.6.9.2, 4.6.9.3, 4.6.9.4, 4.6.9.5, 4.6.9.6, 4.6.9.7, 4.6.9.8, 4.6.9.9, 4.6.9.10, 4.6.10.1, 4.6.10.2, 4.6.10.3, 4.6.10.4, 4.6.10.5, 4.6.10.6, 4.6.10.7, 4.6.10.8, 4.6.10.9, 4.6.10.10, 4.7.1.1, 4.7.1.2, 4.7.1.3, 4.7.1.4, 4.7.1.5, 4.7.1.6, 4.7.1.7, 4.7.1.8, 4.7.1.9, 4.7.1.10, 4.7.2.1, 4.7.2.2, 4.7.2.3, 4.7.2.4, 4.7.2.5, 4.7.2.6, 4.7.2.7, 4.7.2.8, 4.7.2.9, 4.7.2.10, 4.7.3.1, 4.7.3.2, 4.7.3.3, 4.7.3.4, 4.7.3.5, 4.7.3.6, 4.7.3.7, 4.7.3.8, 4.7.3.9, 4.7.3.10, 4.7.4.1, 4.7.4.2, 4.7.4.3, 4.7.4.4, 4.7.4.5, 4.7.4.6, 4.7.4.7, 4.7.4.8, 4.7.4.9, 4.7.4.10, 4.7.5.1, 4.7.5.2, 4.7.5.3, 4.7.5.4, 4.7.5.5, 4.7.5.6, 4.7.5.7, 4.7.5.8, 4.7.5.9, 4.7.5.10, 4.7.6.1, 4.7.6.2, 4.7.6.3, 4.7.6.4, 4.7.6.5, 4.7.6.6, 4.7.6.7, 4.7.6.8, 4.7.6.9, 4.7.6.10, 4.7.7.1, 4.7.7.2, 4.7.7.3, 4.7.7.4, 4.7.7.5, 4.7.7.6, 4.7.7.7, 4.7.7.8, 4.7.7.9, 4.7.7.10, 4.7.8.1, 4.7.8.2, 4.7.8.3, 4.7.8.4, 4.7.8.5, 4.7.8.6, 4.7.8.7, 4.7.8.8, 4.7.8.9, 4.7.8.10, 4.7.9.1, 4.7.9.2, 4.7.9.3, 4.7.9.4, 4.7.9.5, 4.7.9.6, 4.7.9.7, 4.7.9.8, 4.7.9.9, 4.7.9.10, 4.7.10.1, 4.7.10.2, 4.7.10.3, 4.7.10.4, 4.7.10.5, 4.7.10.6, 4.7.10.7, 4.7.10.8, 4.7.10.9, 4.7.10.10, 4.8.1.1, 4.8.1.2, 4.8.1.3, 4.8.1.4, 4.8.1.5, 4.8.1.6, 4.8.1.7, 4.8.1.8, 4.8.1.9, 4.8.1.10, 4.8.2.1, 4.8.2.2, 4.8.2.3, 4.8.2.4, 4.8.2.5, 4.8.2.6, 4.8.2.7, 4.8.2.8, 4.8.2.9, 4.8.2.10, 4.8.3.1, 4.8.3.2, 4.8.3.3, 4.8.3.4, 4.8.3.5, 4.8.3.6, 4.8.3.7, 4.8.3.8, 4.8.3.9, 4.8.3.10, 4.8.4.1, 4.8.4.2, 4.8.4.3, 4.8.4.4, 4.8.4.5, 4.8.4.6, 4.8.4.7, 4.8.4.8, 4.8.4.9, 4.8.4.10, 4.8.5.1, 4.8.5.2, 4.8.5.3, 4.8.5.4, 4.8.5.5, 4.8.5.6, 4.8.5.7, 4.8.5.8, 4.8.5.9, 4.8.5.10, 4.8.6.1, 4.8.6.2, 4.8.6.3, 4.8.6.4, 4.8.6.5, 4.8.6.6, 4.8.6.7, 4.8.6.8, 4.8.6.9, 4.8.6.10, 4.8.7.1, 4.8.7.2, 4.8.7.3, 4.8.7.4, 4.8.7.5, 4.8.7.6, 4.8.7.7, 4.8.7.8, 4.8.7.9, 4.8.7.10, 4.8.8.1, 4.8.8.2, 4.8.8.3, 4.8.8.4, 4.8.8.5, |
| 4.8.8.6, 4.8.8.7, 4.8.8.8, 4.8.8.9, 4.8.8.10, 4.8.9.1, 4.8.9.2, 4.8.9.3, 4.8.9.4, 4.8.9.5, 4.8.9.6, 4.8.9.7, 4.8.9.8, 4.8.9.9, 4.8.9.10, 4.8.10.1, 4.8.10.2, 4.8.10.3, 4.8.10.4, 4.8.10.5, 4.8.10.6, 4.8.10.7, 4.8.10.8, 4.8.10.9, 4.8.10.10, 4.9.1.1, 4.9.1.2, 4.9.1.3, 4.9.1.4, 4.9.1.5, 4.9.1.6, 4.9.1.7, 4.9.1.8, 4.9.1.9, 4.9.1.10, 4.9.2.1, 4.9.2.2, 4.9.2.3, 4.9.2.4, 4.9.2.5, 4.9.2.6, 4.9.2.7, 4.9.2.8, 4.9.2.9, 4.9.2.10, 4.9.3.1, 4.9.3.2, 4.9.3.3, 4.9.3.4, 4.9.3.5, 4.9.3.6, 4.9.3.7, 4.9.3.8, 4.9.3.9, 4.9.3.10, 4.9.4.1, 4.9.4.2, 4.9.4.3, 4.9.4.4, 4.9.4.5, 4.9.4.6, 4.9.4.7, 4.9.4.8, 4.9.4.9, 4.9.4.10, 4.9.5.1, 4.9.5.2, 4.9.5.3, 4.9.5.4, 4.9.5.5, 4.9.5.6, 4.9.5.7, 4.9.5.8, 4.9.5.9, 4.9.5.10, 4.9.6.1, 4.9.6.2, 4.9.6.3, 4.9.6.4, 4.9.6.5, 4.9.6.6, 4.9.6.7, 4.9.6.8, 4.9.6.9, 4.9.6.10, 4.9.7.1, 4.9.7.2, 4.9.7.3, 4.9.7.4, 4.9.7.5, 4.9.7.6, 4.9.7.7, 4.9.7.8, 4.9.7.9, 4.9.7.10, 4.9.8.1, 4.9.8.2, 4.9.8.3, 4.9.8.4, 4.9.8.5, 4.9.8.6, 4.9.8.7, 4.9.8.8, 4.9.8.9, 4.9.8.10, 4.9.9.1, 4.9.9.2, 4.9.9.3, 4.9.9.4, 4.9.9.5, 4.9.9.6, 4.9.9.7, 4.9.9.8, 4.9.9.9, 4.9.9.10, 4.9.10.1, 4.9.10.2, 4.9.10.3, 4.9.10.4, 4.9.10.5, 4.9.10.6, 4.9.10.7, 4.9.10.8, 4.9.10.9, 4.9.10.10, 4.10.1.1, 4.10.1.2, 4.10.1.3, 4.10.1.4, 4.10.1.5, 4.10.1.6, 4.10.1.7, 4.10.1.8, 4.10.1.9, 4.10.1.10, 4.10.2.1, 4.10.2.2, 4.10.2.3, 4.10.2.4, 4.10.2.5, 4.10.2.6, 4.10.2.7, 4.10.2.8, 4.10.2.9, 4.10.2.10, 4.10.3.1, 4.10.3.2, 4.10.3.3, 4.10.3.4, 4.10.3.5, 4.10.3.6, 4.10.3.7, 4.10.3.8, 4.10.3.9, 4.10.3.10, 4.10.4.1, 4.10.4.2, 4.10.4.3, 4.10.4.4, 4.10.4.5, 4.10.4.6, 4.10.4.7, 4.10.4.8, 4.10.4.9, 4.10.4.10, 4.10.5.1, 4.10.5.2, 4.10.5.3, 4.10.5.4, 4.10.5.5, 4.10.5.6, 4.10.5.7, 4.10.5.8, 4.10.5.9, 4.10.5.10, 4.10.6.1, 4.10.6.2, 4.10.6.3, 4.10.6.4, 4.10.6.5, 4.10.6.6, 4.10.6.7, 4.10.6.8, 4.10.6.9, 4.10.6.10, 4.10.7.1, 4.10.7.2, 4.10.7.3, 4.10.7.4, 4.10.7.5, 4.10.7.6, 4.10.7.7, 4.10.7.8, 4.10.7.9, 4.10.7.10, 4.10.8.1, 4.10.8.2, 4.10.8.3, 4.10.8.4, 4.10.8.5, 4.10.8.6, 4.10.8.7, 4.10.8.8, 4.10.8.9, 4.10.8.10, 4.10.9.1, 4.10.9.2, 4.10.9.3, 4.10.9.4, 4.10.9.5, 4.10.9.6, 4.10.9.7, 4.10.9.8, 4.10.9.9, 4.10.9.10, 4.10.10.1, 4.10.10.2, 4.10.10.3, 4.10.10.4, 4.10.10.5, 4.10.10.6, 4.10.10.7, 4.10.10.8, 4.10.10.9, 4.10.10.10, 5.1.1.1, 5.1.1.2, 5.1.1.3, 5.1.1.4, 5.1.1.5, 5.1.1.6, 5.1.1.7, 5.1.1.8, 5.1.1.9, 5.1.1.10, 5.1.2.1, 5.1.2.2, 5.1.2.3, 5.1.2.4, 5.1.2.5, 5.1.2.6, 5.1.2.7, 5.1.2.8, 5.1.2.9, 5.1.2.10, 5.1.3.1, 5.1.3.2, 5.1.3.3, 5.1.3.4, 5.1.3.5, 5.1.3.6, 5.1.3.7, 5.1.3.8, 5.1.3.9, 5.1.3.10, 5.1.4.1, 5.1.4.2, 5.1.4.3, 5.1.4.4, 5.1.4.5, 5.1.4.6, 5.1.4.7, 5.1.4.8, 5.1.4.9, 5.1.4.10, 5.1.5.1, 5.1.5.2, 5.1.5.3, 5.1.5.4, 5.1.5.5, 5.1.5.6, 5.1.5.7, 5.1.5.8, 5.1.5.9, 5.1.5.10, 5.1.6.1, 5.1.6.2, 5.1.6.3, 5.1.6.4, 5.1.6.5, 5.1.6.6, 5.1.6.7, 5.1.6.8, 5.1.6.9, 5.1.6.10, 5.1.7.1, 5.1.7.2, 5.1.7.3, 5.1.7.4, 5.1.7.5, 5.1.7.6, 5.1.7.7, 5.1.7.8, 5.1.7.9, 5.1.7.10, 5.1.8.1, 5.1.8.2, 5.1.8.3, 5.1.8.4, 5.1.8.5, 5.1.8.6, 5.1.8.7, 5.1.8.8, 5.1.8.9, 5.1.8.10, 5.1.9.1, 5.1.9.2, 5.1.9.3, 5.1.9.4, 5.1.9.5, 5.1.9.6, 5.1.9.7, 5.1.9.8, 5.1.9.9, 5.1.9.10, 5.1.10.1, 5.1.10.2, 5.1.10.3, 5.1.10.4, 5.1.10.5, 5.1.10.6, 5.1.10.7, 5.1.10.8, 5.1.10.9, 5.1.10.10, 5.2.1.1, 5.2.1.2, 5.2.1.3, 5.2.1.4, 5.2.1.5, 5.2.1.6, 5.2.1.7, 5.2.1.8, 5.2.1.9, 5.2.1.10, 5.2.2.1, 5.2.2.2, 5.2.2.3, 5.2.2.4, 5.2.2.5, 5.2.2.6, 5.2.2.7, 5.2.2.8, 5.2.2.9, 5.2.2.10, 5.2.3.1, 5.2.3.2, 5.2.3.3, 5.2.3.4, 5.2.3.5, 5.2.3.6, 5.2.3.7, 5.2.3.8, 5.2.3.9, 5.2.3.10, 5.2.4.1, 5.2.4.2, 5.2.4.3, 5.2.4.4, 5.2.4.5, 5.2.4.6, 5.2.4.7, 5.2.4.8, 5.2.4.9, 5.2.4.10, 5.2.5.1, 5.2.5.2, 5.2.5.3, 5.2.5.4, 5.2.5.5, 5.2.5.6, 5.2.5.7, 5.2.5.8, 5.2.5.9, 5.2.5.10, 5.2.6.1, 5.2.6.2, 5.2.6.3, 5.2.6.4, 5.2.6.5, 5.2.6.6, 5.2.6.7, 5.2.6.8, 5.2.6.9, 5.2.6.10, 5.2.7.1, 5.2.7.2, 5.2.7.3, 5.2.7.4, 5.2.7.5, 5.2.7.6, 5.2.7.7, 5.2.7.8, 5.2.7.9, 5.2.7.10, 5.2.8.1, 5.2.8.2, 5.2.8.3, 5.2.8.4, 5.2.8.5, 5.2.8.6, 5.2.8.7, 5.2.8.8, 5.2.8.9, 5.2.8.10, 5.2.9.1, 5.2.9.2, 5.2.9.3, 5.2.9.4, 5.2.9.5, 5.2.9.6, 5.2.9.7, 5.2.9.8, 5.2.9.9, 5.2.9.10, 5.2.10.1, 5.2.10.2, 5.2.10.3, 5.2.10.4, 5.2.10.5, 5.2.10.6, 5.2.10.7, 5.2.10.8, 5.2.10.9, 5.2.10.10, 5.3.1.1, 5.3.1.2, 5.3.1.3, 5.3.1.4, 5.3.1.5, 5.3.1.6, 5.3.1.7, 5.3.1.8, 5.3.1.9, 5.3.1.10, 5.3.2.1, 5.3.2.2, 5.3.2.3, 5.3.2.4, 5.3.2.5, 5.3.2.6, 5.3.2.7, 5.3.2.8, 5.3.2.9, 5.3.2.10, 5.3.3.1, 5.3.3.2, 5.3.3.3, 5.3.3.4, 5.3.3.5, 5.3.3.6, 5.3.3.7, 5.3.3.8, 5.3.3.9, 5.3.3.10, 5.3.4.1, 5.3.4.2, 5.3.4.3, 5.3.4.4, 5.3.4.5, 5.3.4.6, 5.3.4.7, 5.3.4.8, 5.3.4.9, 5.3.4.10, 5.3.5.1, 5.3.5.2, 5.3.5.3, 5.3.5.4, 5.3.5.5, 5.3.5.6, 5.3.5.7, 5.3.5.8, 5.3.5.9, 5.3.5.10, 5.3.6.1, 5.3.6.2, 5.3.6.3, 5.3.6.4, 5.3.6.5, 5.3.6.6, 5.3.6.7, 5.3.6.8, 5.3.6.9, 5.3.6.10, 5.3.7.1, 5.3.7.2, 5.3.7.3, 5.3.7.4, 5.3.7.5, 5.3.7.6, 5.3.7.7, 5.3.7.8, 5.3.7.9, 5.3.7.10, 5.3.8.1, 5.3.8.2, 5.3.8.3, 5.3.8.4, 5.3.8.5, 5.3.8.6, 5.3.8.7, 5.3.8.8, 5.3.8.9, 5.3.8.10, 5.3.9.1, 5.3.9.2, 5.3.9.3, 5.3.9.4, 5.3.9.5, 5.3.9.6, 5.3.9.7, 5.3.9.8, 5.3.9.9, 5.3.9.10, 5.3.10.1, 5.3.10.2, 5.3.10.3, 5.3.10.4, 5.3.10.5, 5.3.10.6, 5.3.10.7, 5.3.10.8, 5.3.10.9, 5.3.10.10, 5.4.1.1, 5.4.1.2, 5.4.1.3, 5.4.1.4, 5.4.1.5, 5.4.1.6, 5.4.1.7, 5.4.1.8, 5.4.1.9, 5.4.1.10, 5.4.2.1, 5.4.2.2, 5.4.2.3, 5.4.2.4, 5.4.2.5, 5.4.2.6, 5.4.2.7, 5.4.2.8, 5.4.2.9, 5.4.2.10, 5.4.3.1, 5.4.3.2, 5.4.3.3, 5.4.3.4, 5.4.3.5, 5.4.3.6, 5.4.3.7, 5.4.3.8, 5.4.3.9, 5.4.3.10, 5.4.4.1, 5.4.4.2, 5.4.4.3, 5.4.4.4, 5.4.4.5, 5.4.4.6, 5.4.4.7, 5.4.4.8, 5.4.4.9, 5.4.4.10, 5.4.5.1, 5.4.5.2, 5.4.5.3, 5.4.5.4, 5.4.5.5, 5.4.5.6, 5.4.5.7, 5.4.5.8, 5.4.5.9, 5.4.5.10, 5.4.6.1, 5.4.6.2, 5.4.6.3, 5.4.6.4, 5.4.6.5, 5.4.6.6, 5.4.6.7, 5.4.6.8, 5.4.6.9, 5.4.6.10, 5.4.7.1, 5.4.7.2, 5.4.7.3, 5.4.7.4, 5.4.7.5, 5.4.7.6, 5.4.7.7, 5.4.7.8, 5.4.7.9, 5.4.7.10, 5.4.8.1, 5.4.8.2, 5.4.8.3, 5.4.8.4, 5.4.8.5, 5.4.8.6, 5.4.8.7, 5.4.8.8, 5.4.8.9, 5.4.8.10, 5.4.9.1, 5.4.9.2, 5.4.9.3, 5.4.9.4, 5.4.9.5, 5.4.9.6, 5.4.9.7, 5.4.9.8, 5.4.9.9, 5.4.9.10, 5.4.10.1, 5.4.10.2, 5.4.10.3, 5.4.10.4, 5.4.10.5, 5.4.10.6, 5.4.10.7, 5.4.10.8, 5.4.10.9, 5.4.10.10, 5.5.1.1, 5.5.1.2, 5.5.1.3, 5.5.1.4, 5.5.1.5, 5.5.1.6, 5.5.1.7, 5.5.1.8, 5.5.1.9, 5.5.1.10, 5.5.2.1, 5.5.2.2, 5.5.2.3, 5.5.2.4, 5.5.2.5, 5.5.2.6, 5.5.2.7, 5.5.2.8, |
| 5.5.2.9, 5.5.2.10, 5.5.3.1, 5.5.3.2, 5.5.3.3, 5.5.3.4, 5.5.3.5, 5.5.3.6, 5.5.3.7, 5.5.3.8, 5.5.3.9, 5.5.3.10, 5.5.4.1, 5.5.4.2, 5.5.4.3, 5.5.4.4, 5.5.4.5, 5.5.4.6, 5.5.4.7, 5.5.4.8, 5.5.4.9, 5.5.4.10, 5.5.5.1, 5.5.5.2, 5.5.5.3, 5.5.5.4, 5.5.5.5, 5.5.5.6, 5.5.5.7, 5.5.5.8, 5.5.5.9, 5.5.5.10, 5.5.6.1, 5.5.6.2, 5.5.6.3, 5.5.6.4, 5.5.6.5, 5.5.6.6, 5.5.6.7, 5.5.6.8, 5.5.6.9, 5.5.6.10, 5.5.7.1, 5.5.7.2, 5.5.7.3, 5.5.7.4, 5.5.7.5, 5.5.7.6, 5.5.7.7, 5.5.7.8, 5.5.7.9, 5.5.7.10, 5.5.8.1, 5.5.8.2, 5.5.8.3, 5.5.8.4, 5.5.8.5, 5.5.8.6, 5.5.8.7, 5.5.8.8, 5.5.8.9, 5.5.8.10, 5.5.9.1, 5.5.9.2, 5.5.9.3, 5.5.9.4, 5.5.9.5, 5.5.9.6, 5.5.9.7, 5.5.9.8, 5.5.9.9, 5.5.9.10, 5.5.10.1, 5.5.10.2, 5.5.10.3, 5.5.10.4, 5.5.10.5, 5.5.10.6, 5.5.10.7, 5.5.10.8, 5.5.10.9, 5.5.10.10, 5.6.1.1, 5.6.1.2, 5.6.1.3, 5.6.1.4, 5.6.1.5, 5.6.1.6, 5.6.1.7, 5.6.1.8, 5.6.1.9, 5.6.1.10, 5.6.2.1, 5.6.2.2, 5.6.2.3, 5.6.2.4, 5.6.2.5, 5.6.2.6, 5.6.2.7, 5.6.2.8, 5.6.2.9, 5.6.2.10, 5.6.3.1, 5.6.3.2, 5.6.3.3, 5.6.3.4, 5.6.3.5, 5.6.3.6, 5.6.3.7, 5.6.3.8, 5.6.3.9, 5.6.3.10, 5.6.4.1, 5.6.4.2, 5.6.4.3, 5.6.4.4, 5.6.4.5, 5.6.4.6, 5.6.4.7, 5.6.4.8, 5.6.4.9, 5.6.4.10, 5.6.5.1, 5.6.5.2, 5.6.5.3, 5.6.5.4, 5.6.5.5, 5.6.5.6, 5.6.5.7, 5.6.5.8, 5.6.5.9, 5.6.5.10, 5.6.6.1, 5.6.6.2, 5.6.6.3, 5.6.6.4, 5.6.6.5, 5.6.6.6, 5.6.6.7, 5.6.6.8, 5.6.6.9, 5.6.6.10, 5.6.7.1, 5.6.7.2, 5.6.7.3, 5.6.7.4, 5.6.7.5, 5.6.7.6, 5.6.7.7, 5.6.7.8, 5.6.7.9, 5.6.7.10, 5.6.8.1, 5.6.8.2, 5.6.8.3, 5.6.8.4, 5.6.8.5, 5.6.8.6, 5.6.8.7, 5.6.8.8, 5.6.8.9, 5.6.8.10, 5.6.9.1, 5.6.9.2, 5.6.9.3, 5.6.9.4, 5.6.9.5, 5.6.9.6, 5.6.9.7, 5.6.9.8, 5.6.9.9, 5.6.9.10, 5.6.10.1, 5.6.10.2, 5.6.10.3, 5.6.10.4, 5.6.10.5, 5.6.10.6, 5.6.10.7, 5.6.10.8, 5.6.10.9, 5.6.10.10, 5.7.1.1, 5.7.1.2, 5.7.1.3, 5.7.1.4, 5.7.1.5, 5.7.1.6, 5.7.1.7, 5.7.1.8, 5.7.1.9, 5.7.1.10, 5.7.2.1, 5.7.2.2, 5.7.2.3, 5.7.2.4, 5.7.2.5, 5.7.2.6, 5.7.2.7, 5.7.2.8, 5.7.2.9, 5.7.2.10, 5.7.3.1, 5.7.3.2, 5.7.3.3, 5.7.3.4, 5.7.3.5, 5.7.3.6, 5.7.3.7, 5.7.3.8, 5.7.3.9, 5.7.3.10, 5.7.4.1, 5.7.4.2, 5.7.4.3, 5.7.4.4, 5.7.4.5, 5.7.4.6, 5.7.4.7, 5.7.4.8, 5.7.4.9, 5.7.4.10, 5.7.5.1, 5.7.5.2, 5.7.5.3, 5.7.5.4, 5.7.5.5, 5.7.5.6, 5.7.5.7, 5.7.5.8, 5.7.5.9, 5.7.5.10, 5.7.6.1, 5.7.6.2, 5.7.6.3, 5.7.6.4, 5.7.6.5, 5.7.6.6, 5.7.6.7, 5.7.6.8, 5.7.6.9, 5.7.6.10, 5.7.7.1, 5.7.7.2, 5.7.7.3, 5.7.7.4, 5.7.7.5, 5.7.7.6, 5.7.7.7, 5.7.7.8, 5.7.7.9, 5.7.7.10, 5.7.8.1, 5.7.8.2, 5.7.8.3, 5.7.8.4, 5.7.8.5, 5.7.8.6, 5.7.8.7, 5.7.8.8, 5.7.8.9, 5.7.8.10, 5.7.9.1, 5.7.9.2, 5.7.9.3, 5.7.9.4, 5.7.9.5, 5.7.9.6, 5.7.9.7, 5.7.9.8, 5.7.9.9, 5.7.9.10, 5.7.10.1, 5.7.10.2, 5.7.10.3, 5.7.10.4, 5.7.10.5, 5.7.10.6, 5.7.10.7, 5.7.10.8, 5.7.10.9, 5.7.10.10, 5.8.1.1, 5.8.1.2, 5.8.1.3, 5.8.1.4, 5.8.1.5, 5.8.1.6, 5.8.1.7, 5.8.1.8, 5.8.1.9, 5.8.1.10, 5.8.2.1, 5.8.2.2, 5.8.2.3, 5.8.2.4, 5.8.2.5, 5.8.2.6, 5.8.2.7, 5.8.2.8, 5.8.2.9, 5.8.2.10, 5.8.3.1, 5.8.3.2, 5.8.3.3, 5.8.3.4, 5.8.3.5, 5.8.3.6, 5.8.3.7, 5.8.3.8, 5.8.3.9, 5.8.3.10, 5.8.4.1, 5.8.4.2, 5.8.4.3, 5.8.4.4, 5.8.4.5, 5.8.4.6, 5.8.4.7, 5.8.4.8, 5.8.4.9, 5.8.4.10, 5.8.5.1, 5.8.5.2, 5.8.5.3, 5.8.5.4, 5.8.5.5, 5.8.5.6, 5.8.5.7, 5.8.5.8, 5.8.5.9, 5.8.5.10, 5.8.6.1, 5.8.6.2, 5.8.6.3, 5.8.6.4, 5.8.6.5, 5.8.6.6, 5.8.6.7, 5.8.6.8, 5.8.6.9, 5.8.6.10, 5.8.7.1, 5.8.7.2, 5.8.7.3, 5.8.7.4, 5.8.7.5, 5.8.7.6, 5.8.7.7, 5.8.7.8, 5.8.7.9, 5.8.7.10, 5.8.8.1, 5.8.8.2, 5.8.8.3, 5.8.8.4, 5.8.8.5, 5.8.8.6, 5.8.8.7, 5.8.8.8, 5.8.8.9, 5.8.8.10, 5.8.9.1, 5.8.9.2, 5.8.9.3, 5.8.9.4, 5.8.9.5, 5.8.9.6, 5.8.9.7, 5.8.9.8, 5.8.9.9, 5.8.9.10, 5.8.10.1, 5.8.10.2, 5.8.10.3, 5.8.10.4, 5.8.10.5, 5.8.10.6, 5.8.10.7, 5.8.10.8, 5.8.10.9, 5.8.10.10, 5.9.1.1, 5.9.1.2, 5.9.1.3, 5.9.1.4, 5.9.1.5, 5.9.1.6, 5.9.1.7, 5.9.1.8, 5.9.1.9, 5.9.1.10, 5.9.2.1, 5.9.2.2, 5.9.2.3, 5.9.2.4, 5.9.2.5, 5.9.2.6, 5.9.2.7, 5.9.2.8, 5.9.2.9, 5.9.2.10, 5.9.3.1, 5.9.3.2, 5.9.3.3, 5.9.3.4, 5.9.3.5, 5.9.3.6, 5.9.3.7, 5.9.3.8, 5.9.3.9, 5.9.3.10, 5.9.4.1, 5.9.4.2, 5.9.4.3, 5.9.4.4, 5.9.4.5, 5.9.4.6, 5.9.4.7, 5.9.4.8, 5.9.4.9, 5.9.4.10, 5.9.5.1, 5.9.5.2, 5.9.5.3, 5.9.5.4, 5.9.5.5, 5.9.5.6, 5.9.5.7, 5.9.5.8, 5.9.5.9, 5.9.5.10, 5.9.6.1, 5.9.6.2, 5.9.6.3, 5.9.6.4, 5.9.6.5, 5.9.6.6, 5.9.6.7, 5.9.6.8, 5.9.6.9, 5.9.6.10, 5.9.7.1, 5.9.7.2, 5.9.7.3, 5.9.7.4, 5.9.7.5, 5.9.7.6, 5.9.7.7, 5.9.7.8, 5.9.7.9, 5.9.7.10, 5.9.8.1, 5.9.8.2, 5.9.8.3, 5.9.8.4, 5.9.8.5, 5.9.8.6, 5.9.8.7, 5.9.8.8, 5.9.8.9, 5.9.8.10, 5.9.9.1, 5.9.9.2, 5.9.9.3, 5.9.9.4, 5.9.9.5, 5.9.9.6, 5.9.9.7, 5.9.9.8, 5.9.9.9, 5.9.9.10, 5.9.10.1, 5.9.10.2, 5.9.10.3, 5.9.10.4, 5.9.10.5, 5.9.10.6, 5.9.10.7, 5.9.10.8, 5.9.10.9, 5.9.10.10, 5.10.1.1, 5.10.1.2, 5.10.1.3, 5.10.1.4, 5.10.1.5, 5.10.1.6, 5.10.1.7, 5.10.1.8, 5.10.1.9, 5.10.1.10, 5.10.2.1, 5.10.2.2, 5.10.2.3, 5.10.2.4, 5.10.2.5, 5.10.2.6, 5.10.2.7, 5.10.2.8, 5.10.2.9, 5.10.2.10, 5.10.3.1, 5.10.3.2, 5.10.3.3, 5.10.3.4, 5.10.3.5, 5.10.3.6, 5.10.3.7, 5.10.3.8, 5.10.3.9, 5.10.3.10, 5.10.4.1, 5.10.4.2, 5.10.4.3, 5.10.4.4, 5.10.4.5, 5.10.4.6, 5.10.4.7, 5.10.4.8, 5.10.4.9, 5.10.4.10, 5.10.5.1, 5.10.5.2, 5.10.5.3, 5.10.5.4, 5.10.5.5, 5.10.5.6, 5.10.5.7, 5.10.5.8, 5.10.5.9, 5.10.5.10, 5.10.6.1, 5.10.6.2, 5.10.6.3, 5.10.6.4, 5.10.6.5, 5.10.6.6, 5.10.6.7, 5.10.6.8, 5.10.6.9, 5.10.6.10, 5.10.7.1, 5.10.7.2, 5.10.7.3, 5.10.7.4, 5.10.7.5, 5.10.7.6, 5.10.7.7, 5.10.7.8, 5.10.7.9, 5.10.7.10, 5.10.8.1, 5.10.8.2, 5.10.8.3, 5.10.8.4, 5.10.8.5, 5.10.8.6, 5.10.8.7, 5.10.8.8, 5.10.8.9, 5.10.8.10, 5.10.9.1, 5.10.9.2, 5.10.9.3, 5.10.9.4, 5.10.9.5, 5.10.9.6, 5.10.9.7, 5.10.9.8, 5.10.9.9, 5.10.9.10, 5.10.10.1, 5.10.10.2, 5.10.10.3, 5.10.10.4, 5.10.10.5, 5.10.10.6, 5.10.10.7, 5.10.10.8, 5.10.10.9, 5.10.10.10, 6.1.1.1, 6.1.1.2, 6.1.1.3, 6.1.1.4, 6.1.1.5, 6.1.1.6, 6.1.1.7, 6.1.1.8, 6.1.1.9, 6.1.1.10, 6.1.2.1, 6.1.2.2, 6.1.2.3, 6.1.2.4, 6.1.2.5, 6.1.2.6, 6.1.2.7, 6.1.2.8, 6.1.2.9, 6.1.2.10, 6.1.3.1, 6.1.3.2, 6.1.3.3, 6.1.3.4, 6.1.3.5, 6.1.3.6, 6.1.3.7, 6.1.3.8, 6.1.3.9, 6.1.3.10, 6.1.4.1, 6.1.4.2, 6.1.4.3, 6.1.4.4, 6.1.4.5, 6.1.4.6, 6.1.4.7, 6.1.4.8, 6.1.4.9, 6.1.4.10, 6.1.5.1, 6.1.5.2, 6.1.5.3, 6.1.5.4, 6.1.5.5, 6.1.5.6, 6.1.5.7, 6.1.5.8, 6.1.5.9, 6.1.5.10, 6.1.6.1, 6.1.6.2, 6.1.6.3, 6.1.6.4, 6.1.6.5, 6.1.6.6, 6.1.6.7, 6.1.6.8, 6.1.6.9, 6.1.6.10, 6.1.7.1, 6.1.7.2, |
| 6.1.7.3, 6.1.7.4, 6.1.7.5, 6.1.7.6, 6.1.7.7, 6.1.7.8, 6.1.7.9, 6.1.7.10, 6.1.8.1, 6.1.8.2, 6.1.8.3, 6.1.8.4, 6.1.8.5, 6.1.8.6, 6.1.8.7, 6.1.8.8, 6.1.8.9, 6.1.8.10, 6.1.9.1, 6.1.9.2, 6.1.9.3, 6.1.9.4, 6.1.9.5, 6.1.9.6, 6.1.9.7, 6.1.9.8, 6.1.9.9, 6.1.9.10, 6.1.10.1, 6.1.10.2, 6.1.10.3, 6.1.10.4, 6.1.10.5, 6.1.10.6, 6.1.10.7, 6.1.10.8, 6.1.10.9, 6.1.10.10, 6.2.1.1, 6.2.1.2, 6.2.1.3, 6.2.1.4, 6.2.1.5, 6.2.1.6, 6.2.1.7, 6.2.1.8, 6.2.1.9, 6.2.1.10, 6.2.2.1, 6.2.2.2, 6.2.2.3, 6.2.2.4, 6.2.2.5, 6.2.2.6, 6.2.2.7, 6.2.2.8, 6.2.2.9, 6.2.2.10, 6.2.3.1, 6.2.3.2, 6.2.3.3, 6.2.3.4, 6.2.3.5, 6.2.3.6, 6.2.3.7, 6.2.3.8, 6.2.3.9, 6.2.3.10, 6.2.4.1, 6.2.4.2, 6.2.4.3, 6.2.4.4, 6.2.4.5, 6.2.4.6, 6.2.4.7, 6.2.4.8, 6.2.4.9, 6.2.4.10, 6.2.5.1, 6.2.5.2, 6.2.5.3, 6.2.5.4, 6.2.5.5, 6.2.5.6, 6.2.5.7, 6.2.5.8, 6.2.5.9, 6.2.5.10, 6.2.6.1, 6.2.6.2, 6.2.6.3, 6.2.6.4, 6.2.6.5, 6.2.6.6, 6.2.6.7, 6.2.6.8, 6.2.6.9, 6.2.6.10, 6.2.7.1, 6.2.7.2, 6.2.7.3, 6.2.7.4, 6.2.7.5, 6.2.7.6, 6.2.7.7, 6.2.7.8, 6.2.7.9, 6.2.7.10, 6.2.8.1, 6.2.8.2, 6.2.8.3, 6.2.8.4, 6.2.8.5, 6.2.8.6, 6.2.8.7, 6.2.8.8, 6.2.8.9, 6.2.8.10, 6.2.9.1, 6.2.9.2, 6.2.9.3, 6.2.9.4, 6.2.9.5, 6.2.9.6, 6.2.9.7, 6.2.9.8, 6.2.9.9, 6.2.9.10, 6.2.10.1, 6.2.10.2, 6.2.10.3, 6.2.10.4, 6.2.10.5, 6.2.10.6, 6.2.10.7, 6.2.10.8, 6.2.10.9, 6.2.10.10, 6.3.1.1, 6.3.1.2, 6.3.1.3, 6.3.1.4, 6.3.1.5, 6.3.1.6, 6.3.1.7, 6.3.1.8, 6.3.1.9, 6.3.1.10, 6.3.2.1, 6.3.2.2, 6.3.2.3, 6.3.2.4, 6.3.2.5, 6.3.2.6, 6.3.2.7, 6.3.2.8, 6.3.2.9, 6.3.2.10, 6.3.3.1, 6.3.3.2, 6.3.3.3, 6.3.3.4, 6.3.3.5, 6.3.3.6, 6.3.3.7, 6.3.3.8, 6.3.3.9, 6.3.3.10, 6.3.4.1, 6.3.4.2, 6.3.4.3, 6.3.4.4, 6.3.4.5, 6.3.4.6, 6.3.4.7, 6.3.4.8, 6.3.4.9, 6.3.4.10, 6.3.5.1, 6.3.5.2, 6.3.5.3, 6.3.5.4, 6.3.5.5, 6.3.5.6, 6.3.5.7, 6.3.5.8, 6.3.5.9, 6.3.5.10, 6.3.6.1, 6.3.6.2, 6.3.6.3, 6.3.6.4, 6.3.6.5, 6.3.6.6, 6.3.6.7, 6.3.6.8, 6.3.6.9, 6.3.6.10, 6.3.7.1, 6.3.7.2, 6.3.7.3, 6.3.7.4, 6.3.7.5, 6.3.7.6, 6.3.7.7, 6.3.7.8, 6.3.7.9, 6.3.7.10, 6.3.8.1, 6.3.8.2, 6.3.8.3, 6.3.8.4, 6.3.8.5, 6.3.8.6, 6.3.8.7, 6.3.8.8, 6.3.8.9, 6.3.8.10, 6.3.9.1, 6.3.9.2, 6.3.9.3, 6.3.9.4, 6.3.9.5, 6.3.9.6, 6.3.9.7, 6.3.9.8, 6.3.9.9, 6.3.9.10, 6.3.10.1, 6.3.10.2, 6.3.10.3, 6.3.10.4, 6.3.10.5, 6.3.10.6, 6.3.10.7, 6.3.10.8, 6.3.10.9, 6.3.10.10, 6.4.1.1, 6.4.1.2, 6.4.1.3, 6.4.1.4, 6.4.1.5, 6.4.1.6, 6.4.1.7, 6.4.1.8, 6.4.1.9, 6.4.1.10, 6.4.2.1, 6.4.2.2, 6.4.2.3, 6.4.2.4, 6.4.2.5, 6.4.2.6, 6.4.2.7, 6.4.2.8, 6.4.2.9, 6.4.2.10, 6.4.3.1, 6.4.3.2, 6.4.3.3, 6.4.3.4, 6.4.3.5, 6.4.3.6, 6.4.3.7, 6.4.3.8, 6.4.3.9, 6.4.3.10, 6.4.4.1, 6.4.4.2, 6.4.4.3, 6.4.4.4, 6.4.4.5, 6.4.4.6, 6.4.4.7, 6.4.4.8, 6.4.4.9, 6.4.4.10, 6.4.5.1, 6.4.5.2, 6.4.5.3, 6.4.5.4, 6.4.5.5, 6.4.5.6, 6.4.5.7, 6.4.5.8, 6.4.5.9, 6.4.5.10, 6.4.6.1, 6.4.6.2, 6.4.6.3, 6.4.6.4, 6.4.6.5, 6.4.6.6, 6.4.6.7, 6.4.6.8, 6.4.6.9, 6.4.6.10, 6.4.7.1, 6.4.7.2, 6.4.7.3, 6.4.7.4, 6.4.7.5, 6.4.7.6, 6.4.7.7, 6.4.7.8, 6.4.7.9, 6.4.7.10, 6.4.8.1, 6.4.8.2, 6.4.8.3, 6.4.8.4, 6.4.8.5, 6.4.8.6, 6.4.8.7, 6.4.8.8, 6.4.8.9, 6.4.8.10, 6.4.9.1, 6.4.9.2, 6.4.9.3, 6.4.9.4, 6.4.9.5, 6.4.9.6, 6.4.9.7, 6.4.9.8, 6.4.9.9, 6.4.9.10, 6.4.10.1, 6.4.10.2, 6.4.10.3, 6.4.10.4, 6.4.10.5, 6.4.10.6, 6.4.10.7, 6.4.10.8, 6.4.10.9, 6.4.10.10, 6.5.1.1, 6.5.1.2, 6.5.1.3, 6.5.1.4, 6.5.1.5, 6.5.1.6, 6.5.1.7, 6.5.1.8, 6.5.1.9, 6.5.1.10, 6.5.2.1, 6.5.2.2, 6.5.2.3, 6.5.2.4, 6.5.2.5, 6.5.2.6, 6.5.2.7, 6.5.2.8, 6.5.2.9, 6.5.2.10, 6.5.3.1, 6.5.3.2, 6.5.3.3, 6.5.3.4, 6.5.3.5, 6.5.3.6, 6.5.3.7, 6.5.3.8, 6.5.3.9, 6.5.3.10, 6.5.4.1, 6.5.4.2, 6.5.4.3, 6.5.4.4, 6.5.4.5, 6.5.4.6, 6.5.4.7, 6.5.4.8, 6.5.4.9, 6.5.4.10, 6.5.5.1, 6.5.5.2, 6.5.5.3, 6.5.5.4, 6.5.5.5, 6.5.5.6, 6.5.5.7, 6.5.5.8, 6.5.5.9, 6.5.5.10, 6.5.6.1, 6.5.6.2, 6.5.6.3, 6.5.6.4, 6.5.6.5, 6.5.6.6, 6.5.6.7, 6.5.6.8, 6.5.6.9, 6.5.6.10, 6.5.7.1, 6.5.7.2, 6.5.7.3, 6.5.7.4, 6.5.7.5, 6.5.7.6, 6.5.7.7, 6.5.7.8, 6.5.7.9, 6.5.7.10, 6.5.8.1, 6.5.8.2, 6.5.8.3, 6.5.8.4, 6.5.8.5, 6.5.8.6, 6.5.8.7, 6.5.8.8, 6.5.8.9, 6.5.8.10, 6.5.9.1, 6.5.9.2, 6.5.9.3, 6.5.9.4, 6.5.9.5, 6.5.9.6, 6.5.9.7, 6.5.9.8, 6.5.9.9, 6.5.9.10, 6.5.10.1, 6.5.10.2, 6.5.10.3, 6.5.10.4, 6.5.10.5, 6.5.10.6, 6.5.10.7, 6.5.10.8, 6.5.10.9, 6.5.10.10, 6.6.1.1, 6.6.1.2, 6.6.1.3, 6.6.1.4, 6.6.1.5, 6.6.1.6, 6.6.1.7, 6.6.1.8, 6.6.1.9, 6.6.1.10, 6.6.2.1, 6.6.2.2, 6.6.2.3, 6.6.2.4, 6.6.2.5, 6.6.2.6, 6.6.2.7, 6.6.2.8, 6.6.2.9, 6.6.2.10, 6.6.3.1, 6.6.3.2, 6.6.3.3, 6.6.3.4, 6.6.3.5, 6.6.3.6, 6.6.3.7, 6.6.3.8, 6.6.3.9, 6.6.3.10, 6.6.4.1, 6.6.4.2, 6.6.4.3, 6.6.4.4, 6.6.4.5, 6.6.4.6, 6.6.4.7, 6.6.4.8, 6.6.4.9, 6.6.4.10, 6.6.5.1, 6.6.5.2, 6.6.5.3, 6.6.5.4, 6.6.5.5, 6.6.5.6, 6.6.5.7, 6.6.5.8, 6.6.5.9, 6.6.5.10, 6.6.6.1, 6.6.6.2, 6.6.6.3, 6.6.6.4, 6.6.6.5, 6.6.6.6, 6.6.6.7, 6.6.6.8, 6.6.6.9, 6.6.6.10, 6.6.7.1, 6.6.7.2, 6.6.7.3, 6.6.7.4, 6.6.7.5, 6.6.7.6, 6.6.7.7, 6.6.7.8, 6.6.7.9, 6.6.7.10, 6.6.8.1, 6.6.8.2, 6.6.8.3, 6.6.8.4, 6.6.8.5, 6.6.8.6, 6.6.8.7, 6.6.8.8, 6.6.8.9, 6.6.8.1 0, 6.6.9.1, 6.6.9.2, 6.6.9.3, 6.6.9.4, 6.6.9.5, 6.6.9.6, 6.6.9.7, 6.6.9.8, 6.6.9.9, 6.6.9.10, 6.6.10.1, 6.6.10.2, 6.6.10.3, 6.6.10.4, 6.6.10.5, 6.6.10.6, 6.6.10.7, 6.6.10.8, 6.6.10.9, 6.6.10.10, 6.7.1.1, 6.7.1.2, 6.7.1.3, 6.7.1.4, 6.7.1.5, 6.7.1.6, 6.7.1.7, 6.7.1.8, 6.7.1.9, 6.7.1.10, 6.7.2.1, 6.7.2.2, 6.7.2.3, 6.7.2.4, 6.7.2.5, 6.7.2.6, 6.7.2.7, 6.7.2.8, 6.7.2.9, 6.7.2.10, 6.7.3.1, 6.7.3.2, 6.7.3.3, 6.7.3.4, 6.7.3.5, 6.7.3.6, 6.7.3.7, 6.7.3.8, 6.7.3.9, 6.7.3.10, 6.7.4.1, 6.7.4.2, 6.7.4.3, 6.7.4.4, 6.7.4.5, 6.7.4.6, 6.7.4.7, 6.7.4.8, 6.7.4.9, 6.7.4.10, 6.7.5.1, 6.7.5.2, 6.7.5.3, 6.7.5.4, 6.7.5.5, 6.7.5.6, 6.7.5.7, 6.7.5.8, 6.7.5.9, 6.7.5.10, 6.7.6.1, 6.7.6.2, 6.7.6.3, 6.7.6.4, 6.7.6.5, 6.7.6.6, 6.7.6.7, 6.7.6.8, 6.7.6.9, 6.7.6.10, 6.7.7.1, 6.7.7.2, 6.7.7.3, 6.7.7.4, 6.7.7.5, 6.7.7.6, 6.7.7.7, 6.7.7.8, 6.7.7.9, 6.7.7.10, 6.7.8.1, 6.7.8.2, 6.7.8.3, 6.7.8.4, 6.7.8.5, 6.7.8.6, 6.7.8.7, 6.7.8.8, 6.7.8.9, 6.7.8.10, 6.7.9.1, 6.7.9.2, 6.7.9.3, 6.7.9.4, 6.7.9.5, 6.7.9.6, 6.7.9.7, 6.7.9.8, 6.7.9.9, 6.7.9.10, 6.7.10.1, 6.7.10.2, 6.7.10.3, 6.7.10.4, 6.7.10.5, 6.7.10.6, 6.7.10.7, 6.7.10.8, 6.7.10.9, 6.7.10.10, 6.8.1.1, 6.8.1.2, 6.8.1.3, 6.8.1.4, 6.8.1.5, 6.8.1.6, 6.8.1.7, 6.8.1.8, 6.8.1.9, 6.8.1.10, 6.8.2.1, 6.8.2.2, 6.8.2.3, 6.8.2.4, 6.8.2.5, 6.8.2.6, 6.8.2.7, |
| 6.8.2.8, 6.8.2.9, 6.8.2.10, 6.8.3.1, 6.8.3.2, 6.8.3.3, 6.8.3.4, 6.8.3.5, 6.8.3.6, 6.8.3.7, 6.8.3.8, 6.8.3.9, 6.8.3.10, 6.8.4.1, 6.8.4.2, 6.8.4.3, 6.8.4.4, 6.8.4.5, 6.8.4.6, 6.8.4.7, 6.8.4.8, 6.8.4.9, 6.8.4.10, 6.8.5.1, 6.8.5.2, 6.8.5.3, 6.8.5.4, 6.8.5.5, 6.8.5.6, 6.8.5.7, 6.8.5.8, 6.8.5.9, 6.8.5.10, 6.8.6.1, 6.8.6.2, 6.8.6.3, 6.8.6.4, 6.8.6.5, 6.8.6.6, 6.8.6.7, 6.8.6.8, 6.8.6.9, 6.8.6.10, 6.8.7.1, 6.8.7.2, 6.8.7.3, 6.8.7.4, 6.8.7.5, 6.8.7.6, 6.8.7.7, 6.8.7.8, 6.8.7.9, 6.8.7.10, 6.8.8.1, 6.8.8.2, 6.8.8.3, 6.8.8.4, 6.8.8.5, 6.8.8.6, 6.8.8.7, 6.8.8.8, 6.8.8.9, 6.8.8.10, 6.8.9.1, 6.8.9.2, 6.8.9.3, 6.8.9.4, 6.8.9.5, 6.8.9.6, 6.8.9.7, 6.8.9.8, 6.8.9.9, 6.8.9.10, 6.8.10.1, 6.8.10.2, 6.8.10.3, 6.8.10.4, 6.8.10.5, 6.8.10.6, 6.8.10.7, 6.8.10.8, 6.8.10.9, 6.8.10.10, 6.9.1.1, 6.9.1.2, 6.9.1.3, 6.9.1.4, 6.9.1.5, 6.9.1.6, 6.9.1.7, 6.9.1.8, 6.9.1.9, 6.9.1.10, 6.9.2.1, 6.9.2.2, 6.9.2.3, 6.9.2.4, 6.9.2.5, 6.9.2.6, 6.9.2.7, 6.9.2.8, 6.9.2.9, 6.9.2.10, 6.9.3.1, 6.9.3.2, 6.9.3.3, 6.9.3.4, 6.9.3.5, 6.9.3.6, 6.9.3.7, 6.9.3.8, 6.9.3.9, 6.9.3.10, 6.9.4.1, 6.9.4.2, 6.9.4.3, 6.9.4.4, 6.9.4.5, 6.9.4.6, 6.9.4.7, 6.9.4.8, 6.9.4.9, 6.9.4.10, 6.9.5.1, 6.9.5.2, 6.9.5.3, 6.9.5.4, 6.9.5.5, 6.9.5.6, 6.9.5.7, 6.9.5.8, 6.9.5.9, 6.9.5.10, 6.9.6.1, 6.9.6.2, 6.9.6.3, 6.9.6.4, 6.9.6.5, 6.9.6.6, 6.9.6.7, 6.9.6.8, 6.9.6.9, 6.9.6.10, 6.9.7.1, 6.9.7.2, 6.9.7.3, 6.9.7.4, 6.9.7.5, 6.9.7.6, 6.9.7.7, 6.9.7.8, 6.9.7.9, 6.9.7.10, 6.9.8.1, 6.9.8.2, 6.9.8.3, 6.9.8.4, 6.9.8.5, 6.9.8.6, 6.9.8.7, 6.9.8.8, 6.9.8.9, 6.9.8.10, 6.9.9.1, 6.9.9.2, 6.9.9.3, 6.9.9.4, 6.9.9.5, 6.9.9.6, 6.9.9.7, 6.9.9.8, 6.9.9.9, 6.9.9.10, 6.9.10.1, 6.9.10.2, 6.9.10.3, 6.9.10.4, 6.9.10.5, 6.9.10.6, 6.9.10.7, 6.9.10.8, 6.9.10.9, 6.9.10.10, 6.10.1.1, 6.10.1.2, 6.10.1.3, 6.10.1.4, 6.10.1.5, 6.10.1.6, 6.10.1.7, 6.10.1.8, 6.10.1.9, 6.10.1.10, 6.10.2.1, 6.10.2.2, 6.10.2.3, 6.10.2.4, 6.10.2.5, 6.10.2.6, 6.10.2.7, 6.10.2.8, 6.10.2.9, 6.10.2.10, 6.10.3.1, 6.10.3.2, 6.10.3.3, 6.10.3.4, 6.10.3.5, 6.10.3.6, 6.10.3.7, 6.10.3.8, 6.10.3.9, 6.10.3.10, 6.10.4.1, 6.10.4.2, 6.10.4.3, 6.10.4.4, 6.10.4.5, 6.10.4.6, 6.10.4.7, 6.10.4.8, 6.10.4.9, 6.10.4.10, 6.10.5.1, 6.10.5.2, 6.10.5.3, 6.10.5.4, 6.10.5.5, 6.10.5.6, 6.10.5.7, 6.10.5.8, 6.10.5.9, 6.10.5.10, 6.10.6.1, 6.10.6.2, 6.10.6.3, 6.10.6.4, 6.10.6.5, 6.10.6.6, 6.10.6.7, 6.10.6.8, 6.10.6.9, 6.10.6.10, 6.10.7.1, 6.10.7.2, 6.10.7.3, 6.10.7.4, 6.10.7.5, 6.10.7.6, 6.10.7.7, 6.10.7.8, 6.10.7.9, 6.10.7.10, 6.10.8.1, 6.10.8.2, 6.10.8.3, 6.10.8.4, 6.10.8.5, 6.10.8.6, 6.10.8.7, 6.10.8.8, 6.10.8.9, 6.10.8.10, 6.10.9.1, 6.10.9.2, 6.10.9.3, 6.10.9.4, 6.10.9.5, 6.10.9.6, 6.10.9.7, 6.10.9.8, 6.10.9.9, 6.10.9.10, 6.10.10.1, 6.10.10.2, 6.10.10.3, 6.10.10.4, 6.10.10.5, 6.10.10.6, 6.10.10.7, 6.10.10.8, 6.10.10.9, 6.10.10.10, 7.1.1.1, 7.1.1.2, 7.1.1.3, 7.1.1.4, 7.1.1.5, 7.1.1.6, 7.1.1.7, 7.1.1.8, 7.1.1.9, 7.1.1.10, 7.1.2.1, 7.1.2.2, 7.1.2.3, 7.1.2.4, 7.1.2.5, 7.1.2.6, 7.1.2.7, 7.1.2.8, 7.1.2.9, 7.1.2.10, 7.1.3.1, 7.1.3.2, 7.1.3.3, 7.1.3.4, 7.1.3.5, 7.1.3.6, 7.1.3.7, 7.1.3.8, 7.1.3.9, 7.1.3.10, 7.1.4.1, 7.1.4.2, 7.1.4.3, 7.1.4.4, 7.1.4.5,7.1.4.6,7.1.4.7,7.1.4.8,7.1.4.9,7.1.4.10,7.1.5.1,7.1.5.2,7.1.5.3,7.1.5.4,7.1.5.5, 7.1.5.6, 7.1.5.7, 7.1.5.8, 7.1.5.9, 7.1.5.10, 7.1.6.1, 7.1.6.2, 7.1.6.3, 7.1.6.4, 7.1.6.5, 7.1.6.6, 7.1.6.7, 7.1.6.8, 7.1.6.9, 7.1.6.10, 7.1.7.1, 7.1.7.2, 7.1.7.3, 7.1.7.4, 7.1.7.5, 7.1.7.6, 7.1.7.7, 7.1.7.8, 7.1.7.9, 7.1.7.10, 7.1.8.1, 7.1.8.2, 7.1.8.3, 7.1.8.4, 7.1.8.5, 7.1.8.6, 7.1.8.7, 7.1.8.8, 7.1.8.9,7.1.8.10,7.1.9.1,7.1.9.2,7.1.9.3,7.1.9.4,7.1.9.5,7.1.9.6,7.1.9.7,7.1.9.8,7.1.9.9, 7.1.9.10, 7.1.10.1, 7.1.10.2, 7.1.10.3, 7.1.10.4, 7.1.10.5, 7.1.10.6, 7.1.10.7, 7.1.10.8, 7.1.10.9, 7.1.10.10, 7.2.1.1, 7.2.1.2, 7.2.1.3, 7.2.1.4, 7.2.1.5, 7.2.1.6, 7.2.1.7, 7.2.1.8, 7.2.1.9, 7.2.1.10, 7.2.2.1, 7.2.2.2, 7.2.2.3, 7.2.2.4, 7.2.2.5, 7.2.2.6, 7.2.2.7, 7.2.2.8, 7.2.2.9, 7.2.2.10, 7.2.3.1, 7.2.3.2, 7.2.3.3, 7.2.3.4, 7.2.3.5, 7.2.3.6, 7.2.3.7, 7.2.3.8, 7.2.3.9, 7.2.3.10, 7.2.4.1, 7.2.4.2, 7.2.4.3, 7.2.4.4, 7.2.4.5, 7.2.4.6, 7.2.4.7, 7.2.4.8, 7.2.4.9, 7.2.4.10, 7.2.5.1, 7.2.5.2, 7.2.5.3, 7.2.5.4, 7.2.5.5, 7.2.5.6, 7.2.5.7, 7.2.5.8, 7.2.5.9, 7.2.5.10, 7.2.6.1, 7.2.6.2, 7.2.6.3, 7.2.6.4, 7.2.6.5, 7.2.6.6, 7.2.6.7, 7.2.6.8, 7.2.6.9, 7.2.6.10, 7.2.7.1, 7.2.7.2, 7.2.7.3, 7.2.7.4, 7.2.7.5, 7.2.7.6, 7.2.7.7, 7.2.7.8, 7.2.7.9, 7.2.7.10, 7.2.8.1, 7.2.8.2, 7.2.8.3, 7.2.8.4, 7.2.8.5, 7.2.8.6, 7.2.8.7, 7.2.8.8, 7.2.8.9, 7.2.8.10, 7.2.9.1, 7.2.9.2, 7.2.9.3, 7.2.9.4, 7.2.9.5, 7.2.9.6, 7.2.9.7, 7.2.9.8, 7.2.9.9, 7.2.9.10, 7.2.10.1, 7.2.10.2, 7.2.10.3, 7.2.10.4, 7.2.10.5, 7.2.10.6, 7.2.10.7, 7.2.10.8, 7.2.10.9, 7.2.10.10, 7.3.1.1, 7.3.1.2, 7.3.1.3, 7.3.1.4, 7.3.1.5, 7.3.1.6, 7.3.1.7, 7.3.1.8, 7.3.1.9, 7.3.1.10, 7.3.2.1, 7.3.2.2, 7.3.2.3, 7.3.2.4, 7.3.2.5, 7.3.2.6, 7.3.2.7, 7.3.2.8, 7.3.2.9, 7.3.2.10, 7.3.3.1, 7.3.3.2, 7.3.3.3, 7.3.3.4, 7.3.3.5, 7.3.3.6, 7.3.3.7, 7.3.3.8, 7.3.3.9, 7.3.3.10, 7.3.4.1, 7.3.4.2, 7.3.4.3, 7.3.4.4, 7.3.4.5, 7.3.4.6, 7.3.4.7, 7.3.4.8, 7.3.4.9, 7.3.4.10, 7.3.5.1, 7.3.5.2, 7.3.5.3, 7.3.5.4, 7.3.5.5, 7.3.5.6, 7.3.5.7, 7.3.5.8, 7.3.5.9, 7.3.5.10, 7.3.6.1, 7.3.6.2, 7.3.6.3, 7.3.6.4, 7.3.6.5, 7.3.6.6, 7.3.6.7, 7.3.6.8, 7.3.6.9, 7.3.6.10, 7.3.7.1, 7.3.7.2, 7.3.7.3, 7.3.7.4, 7.3.7.5, 7.3.7.6, 7.3.7.7, 7.3.7.8, 7.3.7.9, 7.3.7.10, 7.3.8.1, 7.3.8.2, 7.3.8.3, 7.3.8.4, 7.3.8.5, 7.3.8.6, 7.3.8.7, 7.3.8.8, 7.3.8.9, 7.3.8.10, 7.3.9.1, 7.3.9.2, 7.3.9.3, 7.3.9.4, 7.3.9.5, 7.3.9.6, 7.3.9.7, 7.3.9.8, 7.3.9.9, 7.3.9.10, 7.3.10.1, 7.3.10.2, 7.3.10.3, 7.3.10.4, 7.3.10.5, 7.3.10.6, 7.3.10.7, 7.3.10.8, 7.3.10.9, 7.3.10.10, 7.4.1.1, 7.4.1.2, 7.4.1.3, 7.4.1.4, 7.4.1.5, 7.4.1.6, 7.4.1.7, 7.4.1.8, 7.4.1.9, 7.4.1.10, 7.4.2.1, 7.4.2.2, 7.4.2.3, 7.4.2.4, 7.4.2.5, 7.4.2.6, 7.4.2.7, 7.4.2.8, 7.4.2.9, 7.4.2.10, 7.4.3.1, 7.4.3.2, 7.4.3.3, 7.4.3.4, 7.4.3.5, 7.4.3.6, 7.4.3.7, 7.4.3.8, 7.4.3.9, 7.4.3.10, 7.4.4.1, 7.4.4.2, 7.4.4.3, 7.4.4.4, 7.4.4.5, 7.4.4.6, 7.4.4.7, 7.4.4.8, 7.4.4.9, 7.4.4.10, 7.4.5.1, 7.4.5.2, 7.4.5.3, 7.4.5.4, 7.4.5.5, 7.4.5.6, 7.4.5.7, 7.4.5.8, 7.4.5.9, 7.4.5.10, 7.4.6.1, 7.4.6.2, 7.4.6.3, 7.4.6.4, 7.4.6.5, 7.4.6.6, 7.4.6.7, 7.4.6.8, 7.4.6.9, 7.4.6.10, 7.4.7.1, |
| 7.4.7.2, 7.4.7.3, 7.4.7.4, 7.4.7.5, 7.4.7.6, 7.4.7.7, 7.4.7.8, 7.4.7.9, 7.4.7.10, 7.4.8.1, 7.4.8.2, 7.4.8.3, 7.4.8.4, 7.4.8.5, 7.4.8.6, 7.4.8.7, 7.4.8.8, 7.4.8.9, 7.4.8.10, 7.4.9.1, 7.4.9.2, 7.4.9.3, 7.4.9.4, 7.4.9.5, 7.4.9.6, 7.4.9.7, 7.4.9.8, 7.4.9.9, 7.4.9.10, 7.4.10.1, 7.4.10.2, 7.4.10.3, 7.4.10.4, 7.4.10.5, 7.4.10.6, 7.4.10.7, 7.4.10.8, 7.4.10.9, 7.4.10.10, 7.5.1.1, 7.5.1.2, 7.5.1.3, 7.5.1.4, 7.5.1.5, 7.5.1.6, 7.5.1.7, 7.5.1.8, 7.5.1.9, 7.5.1.10, 7.5.2.1, 7.5.2.2, 7.5.2.3, 7.5.2.4, 7.5.2.5, 7.5.2.6, 7.5.2.7, 7.5.2.8, 7.5.2.9, 7.5.2.10, 7.5.3.1, 7.5.3.2, 7.5.3.3, 7.5.3.4, 7.5.3.5, 7.5.3.6, 7.5.3.7, 7.5.3.8, 7.5.3.9, 7.5.3.10, 7.5.4.1, 7.5.4.2, 7.5.4.3, 7.5.4.4, 7.5.4.5, 7.5.4.6, 7.5.4.7, 7.5.4.8, 7.5.4.9, 7.5.4.10, 7.5.5.1, 7.5.5.2, 7.5.5.3, 7.5.5.4, 7.5.5.5, 7.5.5.6, 7.5.5.7, 7.5.5.8, 7.5.5.9, 7.5.5.10, 7.5.6.1, 7.5.6.2, 7.5.6.3, 7.5.6.4, 7.5.6.5, 7.5.6.6, 7.5.6.7, 7.5.6.8, 7.5.6.9, 7.5.6.10, 7.5.7.1, 7.5.7.2, 7.5.7.3, 7.5.7.4, 7.5.7.5, 7.5.7.6, 7.5.7.7, 7.5.7.8, 7.5.7.9, 7.5.7.10, 7.5.8.1, 7.5.8.2, 7.5.8.3, 7.5.8.4, 7.5.8.5, 7.5.8.6, 7.5.8.7, 7.5.8.8, 7.5.8.9, 7.5.8.10, 7.5.9.1, 7.5.9.2, 7.5.9.3, 7.5.9.4, 7.5.9.5, 7.5.9.6, 7.5.9.7, 7.5.9.8, 7.5.9.9, 7.5.9.10, 7.5.10.1, 7.5.10.2, 7.5.10.3, 7.5.10.4, 7.5.10.5, 7.5.10.6, 7.5.10.7, 7.5.10.8, 7.5.10.9, 7.5.10.10, 7.6.1.1, 7.6.1.2, 7.6.1.3, 7.6.1.4, 7.6.1.5, 7.6.1.6, 7.6.1.7, 7.6.1.8, 7.6.1.9, 7.6.1.10, 7.6.2.1, 7.6.2.2, 7.6.2.3, 7.6.2.4, 7.6.2.5, 7.6.2.6, 7.6.2.7, 7.6.2.8, 7.6.2.9, 7.6.2.10, 7.6.3.1, 7.6.3.2, 7.6.3.3, 7.6.3.4, 7.6.3.5, 7.6.3.6, 7.6.3.7, 7.6.3.8, 7.6.3.9, 7.6.3.10, 7.6.4.1, 7.6.4.2, 7.6.4.3, 7.6.4.4, 7.6.4.5, 7.6.4.6, 7.6.4.7, 7.6.4.8, 7.6.4.9, 7.6.4.10, 7.6.5.1, 7.6.5.2, 7.6.5.3, 7.6.5.4, 7.6.5.5, 7.6.5.6, 7.6.5.7, 7.6.5.8, 7.6.5.9, 7.6.5.10, 7.6.6.1, 7.6.6.2, 7.6.6.3, 7.6.6.4, 7.6.6.5, 7.6.6.6, 7.6.6.7, 7.6.6.8, 7.6.6.9, 7.6.6.10, 7.6.7.1, 7.6.7.2, 7.6.7.3, 7.6.7.4, 7.6.7.5, 7.6.7.6, 7.6.7.7, 7.6.7.8, 7.6.7.9, 7.6.7.10, 7.6.8.1, 7.6.8.2, 7.6.8.3, 7.6.8.4, 7.6.8.5, 7.6.8.6, 7.6.8.7, 7.6.8.8, 7.6.8.9, 7.6.8.10, 7.6.9.1, 7.6.9.2, 7.6.9.3, 7.6.9.4, 7.6.9.5, 7.6.9.6, 7.6.9.7, 7.6.9.8, 7.6.9.9, 7.6.9.10, 7.6.10.1, 7.6.10.2, 7.6.10.3, 7.6.10.4, 7.6.10.5, 7.6.10.6, 7.6.10.7, 7.6.10.8, 7.6.10.9, 7.6.10.10, 7.7.1.1, 7.7.1.2, 7.7.1.3, 7.7.1.4, 7.7.1.5, 7.7.1.6, 7.7.1.7, 7.7.1.8, 7.7.1.9, 7.7.1.10, 7.7.2.1, 7.7.2.2, 7.7.2.3, 7.7.2.4, 7.7.2.5, 7.7.2.6, 7.7.2.7, 7.7.2.8, 7.7.2.9, 7.7.2.10, 7.7.3.1, 7.7.3.2, 7.7.3.3, 7.7.3.4, 7.7.3.5, 7.7.3.6, 7.7.3.7, 7.7.3.8, 7.7.3.9, 7.7.3.10, 7.7.4.1, 7.7.4.2, 7.7.4.3, 7.7.4.4, 7.7.4.5, 7.7.4.6, 7.7.4.7, 7.7.4.8, 7.7.4.9, 7.7.4.10, 7.7.5.1, 7.7.5.2, 7.7.5.3, 7.7.5.4, 7.7.5.5, 7.7.5.6, 7.7.5.7, 7.7.5.8, 7.7.5.9, 7.7.5.10, 7.7.6.1, 7.7.6.2, 7.7.6.3, 7.7.6.4, 7.7.6.5, 7.7.6.6, 7.7.6.7, 7.7.6.8, 7.7.6.9, 7.7.6.10, 7.7.7.1, 7.7.7.2, 7.7.7.3, 7.7.7.4, 7.7.7.5, 7.7.7.6, 7.7.7.7, 7.7.7.8, 7.7.7.9, 7.7.7.10, 7.7.8.1, 7.7.8.2, 7.7.8.3, 7.7.8.4, 7.7.8.5, 7.7.8.6, 7.7.8.7, 7.7.8.8, 7.7.8.9, 7.7.8.10, 7.7.9.1, 7.7.9.2, 7.7.9.3, 7.7.9.4, 7.7.9.5, 7.7.9.6, 7.7.9.7, 7.7.9.8, 7.7.9.9, 7.7.9.10, 7.7.10.1, 7.7.10.2, 7.7.10.3, 7.7.10.4, 7.7.10.5, 7.7.10.6, 7.7.10.7, 7.7.10.8, 7.7.10.9, 7.7.10.10, 7.8.1.1, 7.8.1.2, 7.8.1.3, 7.8.1.4, 7.8.1.5, 7.8.1.6, 7.8.1.7, 7.8.1.8, 7.8.1.9, 7.8.1.10, 7.8.2.1, 7.8.2.2, 7.8.2.3, 7.8.2.4, 7.8.2.5, 7.8.2.6, 7.8.2.7, 7.8.2.8, 7.8.2.9, 7.8.2.10, 7.8.3.1, 7.8.3.2, 7.8.3.3, 7.8.3.4, 7.8.3.5, 7.8.3.6, 7.8.3.7, 7.8.3.8, 7.8.3.9, 7.8.3.10, 7.8.4.1, 7.8.4.2, 7.8.4.3, 7.8.4.4, 7.8.4.5, 7.8.4.6, 7.8.4.7, 7.8.4.8, 7.8.4.9, 7.8.4.10, 7.8.5.1, 7.8.5.2, 7.8.5.3, 7.8.5.4, 7.8.5.5, 7.8.5.6, 7.8.5.7, 7.8.5.8, 7.8.5.9, 7.8.5.10, 7.8.6.1, 7.8.6.2, 7.8.6.3, 7.8.6.4, 7.8.6.5, 7.8.6.6, 7.8.6.7, 7.8.6.8, 7.8.6.9, 7.8.6.10, 7.8.7.1, 7.8.7.2, 7.8.7.3, 7.8.7.4, 7.8.7.5, 7.8.7.6, 7.8.7.7, 7.8.7.8, 7.8.7.9, 7.8.7.10, 7.8.8.1, 7.8.8.2, 7.8.8.3, 7.8.8.4, 7.8.8.5, 7.8.8.6, 7.8.8.7, 7.8.8.8, 7.8.8.9, 7.8.8.10, 7.8.9.1, 7.8.9.2, 7.8.9.3, 7.8.9.4, 7.8.9.5, 7.8.9.6, 7.8.9.7, 7.8.9.8, 7.8.9.9, 7.8.9.10, 7.8.10.1, 7.8.10.2, 7.8.10.3, 7.8.10.4, 7.8.10.5, 7.8.10.6, 7.8.10.7, 7.8.10.8, 7.8.10.9, 7.8.10.10, 7.9.1.1, 7.9.1.2, 7.9.1.3, 7.9.1.4, 7.9.1.5, 7.9.1.6, 7.9.1.7, 7.9.1.8, 7.9.1.9, 7.9.1.10, 7.9.2.1, 7.9.2.2, 7.9.2.3, 7.9.2.4, 7.9.2.5, 7.9.2.6, 7.9.2.7, 7.9.2.8, 7.9.2.9, 7.9.2.10, 7.9.3.1, 7.9.3.2, 7.9.3.3, 7.9.3.4, 7.9.3.5, 7.9.3.6, 7.9.3.7, 7.9.3.8, 7.9.3.9, 7.9.3.10, 7.9.4.1, 7.9.4.2, 7.9.4.3, 7.9.4.4, 7.9.4.5, 7.9.4.6, 7.9.4.7, 7.9.4.8, 7.9.4.9, 7.9.4.10, 7.9.5.1, 7.9.5.2, 7.9.5.3, 7.9.5.4, 7.9.5.5, 7.9.5.6, 7.9.5.7, 7.9.5.8, 7.9.5.9, 7.9.5.10, 7.9.6.1, 7.9.6.2, 7.9.6.3, 7.9.6.4, 7.9.6.5, 7.9.6.6, 7.9.6.7, 7.9.6.8, 7.9.6.9, 7.9.6.10, 7.9.7.1, 7.9.7.2, 7.9.7.3, 7.9.7.4, 7.9.7.5, 7.9.7.6, 7.9.7.7, 7.9.7.8, 7.9.7.9, 7.9.7.10, 7.9.8.1, 7.9.8.2, 7.9.8.3, 7.9.8.4, 7.9.8.5, 7.9.8.6, 7.9.8.7, 7.9.8.8, 7.9.8.9, 7.9.8.10, 7.9.9.1, 7.9.9.2, 7.9.9.3, 7.9.9.4, 7.9.9.5, 7.9.9.6, 7.9.9.7, 7.9.9.8, 7.9.9.9, 7.9.9.10, 7.9.10.1, 7.9.10.2, 7.9.10.3, 7.9.10.4, 7.9.10.5, 7.9.10.6, 7.9.10.7, 7.9.10.8, 7.9.10.9, 7.9.10.10, 7.10.1.1, 7.10.1.2, 7.10.1.3, 7.10.1.4, 7.10.1.5, 7.10.1.6, 7.10.1.7, 7.10.1.8, 7.10.1.9, 7.10.1.10, 7.10.2.1, 7.10.2.2, 7.10.2.3, 7.10.2.4, 7.10.2.5, 7.10.2.6, 7.10.2.7, 7.10.2.8, 7.10.2.9, 7.10.2.10, 7.10.3.1, 7.10.3.2, 7.10.3.3, 7.10.3.4, 7.10.3.5, 7.10.3.6, 7.10.3.7, 7.10.3.8, 7.10.3.9, 7.10.3.10, 7.10.4.1, 7.10.4.2, 7.10.4.3, 7.10.4.4, 7.10.4.5, 7.10.4.6, 7.10.4.7, 7.10.4.8, 7.10.4.9, 7.10.4.10, 7.10.5.1, 7.10.5.2, 7.10.5.3, 7.10.5.4, 7.10.5.5, 7.10.5.6, 7.10.5.7, 7.10.5.8, 7.10.5.9, 7.10.5.10, 7.10.6.1, 7.10.6.2, 7.10.6.3, 7.10.6.4, 7.10.6.5, 7.10.6.6, 7.10.6.7, 7.10.6.8, 7.10.6.9, 7.10.6.10, 7.10.7.1, 7.10.7.2, 7.10.7.3, 7.10.7.4, 7.10.7.5, 7.10.7.6, 7.10.7.7, 7.10.7.8, 7.10.7.9, 7.10.7.10, 7.10.8.1, 7.10.8.2, 7.10.8.3, 7.10.8.4, 7.10.8.5, 7.10.8.6, 7.10.8.7, 7.10.8.8, 7.10.8.9, 7.10.8.10, 7.10.9.1, 7.10.9.2, 7.10.9.3, 7.10.9.4, 7.10.9.5, 7.10.9.6, 7.10.9.7, 7.10.9.8, 7.10.9.9, 7.10.9.10, 7.10.10.1, 7.10.10.2, 7.10.10.3, 7.10.10.4, 7.10.10.5, 7.10.10.6, 7.10.10.7, 7.10.10.8, 7.10.10.9, 7.10.10.10, 8.1.1.1, 8.1.1.2, 8.1.1.3, 8.1.1.4, |
| 8.1.1.5, 8.1.1.6, 8.1.1.7, 8.1.1.8, 8.1.1.9, 8.1.1.10, 8.1.2.1, 8.1.2.2, 8.1.2.3, 8.1.2.4, 8.1.2.5, 8.1.2.6, 8.1.2.7, 8.1.2.8, 8.1.2.9, 8.1.2.10, 8.1.3.1, 8.1.3.2, 8.1.3.3, 8.1.3.4, 8.1.3.5, 8.1.3.6, 8.1.3.7, 8.1.3.8, 8.1.3.9, 8.1.3.10, 8.1.4.1, 8.1.4.2, 8.1.4.3, 8.1.4.4, 8.1.4.5, 8.1.4.6, 8.1.4.7, 8.1.4.8, 8.1.4.9, 8.1.4.10, 8.1.5.1, 8.1.5.2, 8.1.5.3, 8.1.5.4, 8.1.5.5, 8.1.5.6, 8.1.5.7, 8.1.5.8, 8.1.5.9, 8.1.5.10, 8.1.6.1, 8.1.6.2, 8.1.6.3, 8.1.6.4, 8.1.6.5, 8.1.6.6, 8.1.6.7, 8.1.6.8, 8.1.6.9, 8.1.6.10, 8.1.7.1, 8.1.7.2, 8.1.7.3, 8.1.7.4, 8.1.7.5, 8.1.7.6, 8.1.7.7, 8.1.7.8, 8.1.7.9, 8.1.7.10, 8.1.8.1, 8.1.8.2, 8.1.8.3, 8.1.8.4, 8.1.8.5, 8.1.8.6, 8.1.8.7, 8.1.8.8, 8.1.8.9, 8.1.8.10, 8.1.9.1, 8.1.9.2, 8.1.9.3, 8.1.9.4, 8.1.9.5, 8.1.9.6, 8.1.9.7, 8.1.9.8, 8.1.9.9, 8.1.9.10, 8.1.10.1, 8.1.10.2, 8.1.10.3, 8.1.10.4, 8.1.10.5, 8.1.10.6, 8.1.10.7, 8.1.10.8, 8.1.10.9, 8.1.10.10, 8.2.1.1, 8.2.1.2, 8.2.1.3, 8.2.1.4, 8.2.1.5, 8.2.1.6, 8.2.1.7, 8.2.1.8, 8.2.1.9, 8.2.1.10, 8.2.2.1, 8.2.2.2, 8.2.2.3, 8.2.2.4, 8.2.2.5, 8.2.2.6, 8.2.2.7, 8.2.2.8, 8.2.2.9, 8.2.2.10, 8.2.3.1, 8.2.3.2, 8.2.3.3, 8.2.3.4, 8.2.3.5, 8.2.3.6, 8.2.3.7, 8.2.3.8, 8.2.3.9, 8.2.3.10, 8.2.4.1, 8.2.4.2, 8.2.4.3, 8.2.4.4, 8.2.4.5, 8.2.4.6, 8.2.4.7, 8.2.4.8, 8.2.4.9, 8.2.4.10, 8.2.5.1, 8.2.5.2, 8.2.5.3, 8.2.5.4, 8.2.5.5, 8.2.5.6, 8.2.5.7, 8.2.5.8, 8.2.5.9, 8.2.5.10, 8.2.6.1, 8.2.6.2, 8.2.6.3, 8.2.6.4, 8.2.6.5, 8.2.6.6, 8.2.6.7, 8.2.6.8, 8.2.6.9, 8.2.6.10, 8.2.7.1, 8.2.7.2, 8.2.7.3, 8.2.7.4, 8.2.7.5, 8.2.7.6, 8.2.7.7, 8.2.7.8, 8.2.7.9, 8.2.7.10, 8.2.8.1, 8.2.8.2, 8.2.8.3, 8.2.8.4, 8.2.8.5, 8.2.8.6, 8.2.8.7, 8.2.8.8, 8.2.8.9, 8.2.8.10, 8.2.9.1, 8.2.9.2, 8.2.9.3, 8.2.9.4, 8.2.9.5, 8.2.9.6, 8.2.9.7, 8.2.9.8, 8.2.9.9, 8.2.9.10, 8.2.10.1, 8.2.10.2, 8.2.10.3, 8.2.10.4, 8.2.10.5, 8.2.10.6, 8.2.10.7, 8.2.10.8, 8.2.10.9, 8.2.10.10, 8.3.1.1, 8.3.1.2, 8.3.1.3, 8.3.1.4, 8.3.1.5, 8.3.1.6, 8.3.1.7, 8.3.1.8, 8.3.1.9, 8.3.1.10, 8.3.2.1, 8.3.2.2, 8.3.2.3, 8.3.2.4, 8.3.2.5, 8.3.2.6, 8.3.2.7, 8.3.2.8, 8.3.2.9, 8.3.2.10, 8.3.3.1, 8.3.3.2, 8.3.3.3, 8.3.3.4, 8.3.3.5, 8.3.3.6, 8.3.3.7, 8.3.3.8, 8.3.3.9, 8.3.3.10, 8.3.4.1, 8.3.4.2, 8.3.4.3, 8.3.4.4, 8.3.4.5, 8.3.4.6, 8.3.4.7, 8.3.4.8, 8.3.4.9, 8.3.4.10, 8.3.5.1, 8.3.5.2, 8.3.5.3, 8.3.5.4, 8.3.5.5, 8.3.5.6, 8.3.5.7, 8.3.5.8, 8.3.5.9, 8.3.5.10, 8.3.6.1, 8.3.6.2, 8.3.6.3, 8.3.6.4, 8.3.6.5, 8.3.6.6, 8.3.6.7, 8.3.6.8, 8.3.6.9, 8.3.6.10, 8.3.7.1, 8.3.7.2, 8.3.7.3, 8.3.7.4, 8.3.7.5, 8.3.7.6, 8.3.7.7, 8.3.7.8, 8.3.7.9, 8.3.7.10, 8.3.8.1, 8.3.8.2, 8.3.8.3, 8.3.8.4, 8.3.8.5, 8.3.8.6, 8.3.8.7, 8.3.8.8, 8.3.8.9, 8.3.8.10, 8.3.9.1, 8.3.9.2, 8.3.9.3, 8.3.9.4, 8.3.9.5, 8.3.9.6, 8.3.9.7, 8.3.9.8, 8.3.9.9, 8.3.9.10, 8.3.10.1, 8.3.10.2, 8.3.10.3, 8.3.10.4, 8.3.10.5, 8.3.10.6, 8.3.10.7, 8.3.10.8, 8.3.10.9, 8.3.10.10, 8.4.1.1, 8.4.1.2, 8.4.1.3, 8.4.1.4, 8.4.1.5, 8.4.1.6, 8.4.1.7, 8.4.1.8, 8.4.1.9, 8.4.1.10, 8.4.2.1, 8.4.2.2, 8.4.2.3, 8.4.2.4, 8.4.2.5, 8.4.2.6, 8.4.2.7, 8.4.2.8, 8.4.2.9, 8.4.2.10, 8.4.3.1, 8.4.3.2, 8.4.3.3, 8.4.3.4, 8.4.3.5, 8.4.3.6, 8.4.3.7, 8.4.3.8, 8.4.3.9, 8.4.3.10, 8.4.4.1, 8.4.4.2, 8.4.4.3, 8.4.4.4, 8.4.4.5, 8.4.4.6, 8.4.4.7, 8.4.4.8, 8.4.4.9, 8.4.4.10, 8.4.5.1, 8.4.5.2, 8.4.5.3, 8.4.5.4, 8.4.5.5, 8.4.5.6, 8.4.5.7, 8.4.5.8, 8.4.5.9, 8.4.5.10, 8.4.6.1, 8.4.6.2, 8.4.6.3, 8.4.6.4, 8.4.6.5, 8.4.6.6, 8.4.6.7, 8.4.6.8, 8.4.6.9, 8.4.6.10, 8.4.7.1, 8.4.7.2, 8.4.7.3, 8.4.7.4, 8.4.7.5, 8.4.7.6, 8.4.7.7, 8.4.7.8, 8.4.7.9, 8.4.7.10, 8.4.8.1, 8.4.8.2, 8.4.8.3, 8.4.8.4, 8.4.8.5, 8.4.8.6, 8.4.8.7, 8.4.8.8, 8.4.8.9, 8.4.8.10, 8.4.9.1, 8.4.9.2, 8.4.9.3, 8.4.9.4, 8.4.9.5, 8.4.9.6, 8.4.9.7, 8.4.9.8, 8.4.9.9, 8.4.9.10, 8.4.10.1, 8.4.10.2, 8.4.10.3, 8.4.10.4, 8.4.10.5, 8.4.10.6, 8.4.10.7, 8.4.10.8, 8.4.10.9, 8.4.10.10, 8.5.1.1, 8.5.1.2, 8.5.1.3, 8.5.1.4, 8.5.1.5, 8.5.1.6, 8.5.1.7, 8.5.1.8, 8.5.1.9, 8.5.1.10, 8.5.2.1, 8.5.2.2, 8.5.2.3, 8.5.2.4, 8.5.2.5, 8.5.2.6, 8.5.2.7, 8.5.2.8, 8.5.2.9, 8.5.2.10, 8.5.3.1, 8.5.3.2, 8.5.3.3, 8.5.3.4, 8.5.3.5, 8.5.3.6, 8.5.3.7, 8.5.3.8, 8.5.3.9, 8.5.3.10, 8.5.4.1, 8.5.4.2, 8.5.4.3, 8.5.4.4, 8.5.4.5, 8.5.4.6, 8.5.4.7, 8.5.4.8, 8.5.4.9, 8.5.4.10, 8.5.5.1, 8.5.5.2, 8.5.5.3, 8.5.5.4, 8.5.5.5, 8.5.5.6, 8.5.5.7, 8.5.5.8, 8.5.5.9, 8.5.5.10, 8.5.6.1, 8.5.6.2, 8.5.6.3, 8.5.6.4, 8.5.6.5, 8.5.6.6, 8.5.6.7, 8.5.6.8, 8.5.6.9, 8.5.6.10, 8.5.7.1, 8.5.7.2, 8.5.7.3, 8.5.7.4, 8.5.7.5, 8.5.7.6, 8.5.7.7, 8.5.7.8, 8.5.7.9, 8.5.7.10, 8.5.8.1, 8.5.8.2, 8.5.8.3, 8.5.8.4, 8.5.8.5, 8.5.8.6, 8.5.8.7, 8.5.8.8, 8.5.8.9, 8.5.8.10, 8.5.9.1, 8.5.9.2, 8.5.9.3, 8.5.9.4, 8.5.9.5, 8.5.9.6, 8.5.9.7, 8.5.9.8, 8.5.9.9, 8.5.9.10, 8.5.10.1, 8.5.10.2, 8.5.10.3, 8.5.10.4, 8.5.10.5, 8.5.10.6, 8.5.10.7, 8.5.10.8, 8.5.10.9, 8.5.10.10, 8.6.1.1, 8.6.1.2, 8.6.1.3, 8.6.1.4, 8.6.1.5, 8.6.1.6, 8.6.1.7, 8.6.1.8, 8.6.1.9, 8.6.1.10, 8.6.2.1, 8.6.2.2, 8.6.2.3, 8.6.2.4, 8.6.2.5, 8.6.2.6, 8.6.2.7, 8.6.2.8, 8.6.2.9, 8.6.2.10, 8.6.3.1, 8.6.3.2, 8.6.3.3, 8.6.3.4, 8.6.3.5, 8.6.3.6, 8.6.3.7, 8.6.3.8, 8.6.3.9, 8.6.3.10, 8.6.4.1, 8.6.4.2, 8.6.4.3, 8.6.4.4, 8.6.4.5, 8.6.4.6, 8.6.4.7, 8.6.4.8, 8.6.4.9, 8.6.4.10, 8.6.5.1, 8.6.5.2, 8.6.5.3, 8.6.5.4, 8.6.5.5, 8.6.5.6, 8.6.5.7, 8.6.5.8, 8.6.5.9, 8.6.5.10, 8.6.6.1, 8.6.6.2, 8.6.6.3, 8.6.6.4, 8.6.6.5, 8.6.6.6, 8.6.6.7, 8.6.6.8, 8.6.6.9, 8.6.6.10, 8.6.7.1, 8.6.7.2, 8.6.7.3, 8.6.7.4, 8.6.7.5, 8.6.7.6, 8.6.7.7, 8.6.7.8, 8.6.7.9, 8.6.7.10, 8.6.8.1, 8.6.8.2, 8.6.8.3, 8.6.8.4, 8.6.8.5, 8.6.8.6, 8.6.8.7, 8.6.8.8, 8.6.8.9, 8.6.8.10, 8.6.9.1, 8.6.9.2, 8.6.9.3, 8.6.9.4, 8.6.9.5, 8.6.9.6, 8.6.9.7, 8.6.9.8, 8.6.9.9, 8.6.9.10, 8.6.10.1, 8.6.10.2, 8.6.10.3, 8.6.10.4, 8.6.10.5, 8.6.10.6, 8.6.10.7, 8.6.10.8, 8.6.10.9, 8.6.10.10, 8.7.1.1, 8.7.1.2, 8.7.1.3, 8.7.1.4, 8.7.1.5, 8.7.1.6, 8.7.1.7, 8.7.1.8, 8.7.1.9, 8.7.1.10, 8.7.2.1, 8.7.2.2, 8.7.2.3, 8.7.2.4, 8.7.2.5, 8.7.2.6, 8.7.2.7, 8.7.2.8, 8.7.2.9, 8.7.2.10, 8.7.3.1, 8.7.3.2, 8.7.3.3, 8.7.3.4, 8.7.3.5, 8.7.3.6, 8.7.3.7, 8.7.3.8, 8.7.3.9, 8.7.3.10, 8.7.4.1, 8.7.4.2, 8.7.4.3, 8.7.4.4, 8.7.4.5, 8.7.4.6, 8.7.4.7, 8.7.4.8, 8.7.4.9, 8.7.4.10, 8.7.5.1, 8.7.5.2, 8.7.5.3, 8.7.5.4, 8.7.5.5, 8.7.5.6, 8.7.5.7, 8.7.5.8, 8.7.5.9, 8.7.5.10, 8.7.6.1, 8.7.6.2, 8.7.6.3, 8.7.6.4, 8.7.6.5, 8.7.6.6, 8.7.6.7, 8.7.6.8, 8.7.6.9, |
| 8.7.6.10, 8.7.7.1, 8.7.7.2, 8.7.7.3, 8.7.7.4, 8.7.7.5, 8.7.7.6, 8.7.7.7, 8.7.7.8, 8.7.7.9, 8.7.7.10, 8.7.8.1, 8.7.8.2, 8.7.8.3, 8.7.8.4, 8.7.8.5, 8.7.8.6, 8.7.8.7, 8.7.8.8, 8.7.8.9, 8.7.8.10, 8.7.9.1, 8.7.9.2, 8.7.9.3, 8.7.9.4, 8.7.9.5, 8.7.9.6, 8.7.9.7, 8.7.9.8, 8.7.9.9, 8.7.9.10, 8.7.10.1, 8.7.10.2, 8.7.10.3, 8.7.10.4, 8.7.10.5, 8.7.10.6, 8.7.10.7, 8.7.10.8, 8.7.10.9, 8.7.10.10, 8.8.1.1, 8.8.1.2, 8.8.1.3, 8.8.1.4, 8.8.1.5, 8.8.1.6, 8.8.1.7, 8.8.1.8, 8.8.1.9, 8.8.1.10, 8.8.2.1, 8.8.2.2, 8.8.2.3, 8.8.2.4, 8.8.2.5, 8.8.2.6, 8.8.2.7, 8.8.2.8, 8.8.2.9, 8.8.2.10, 8.8.3.1, 8.8.3.2, 8.8.3.3, 8.8.3.4, 8.8.3.5, 8.8.3.6, 8.8.3.7, 8.8.3.8, 8.8.3.9, 8.8.3.10, 8.8.4.1, 8.8.4.2, 8.8.4.3, 8.8.4.4, 8.8.4.5, 8.8.4.6, 8.8.4.7, 8.8.4.8, 8.8.4.9, 8.8.4.10, 8.8.5.1, 8.8.5.2, 8.8.5.3, 8.8.5.4, 8.8.5.5, 8.8.5.6, 8.8.5.7, 8.8.5.8, 8.8.5.9, 8.8.5.10, 8.8.6.1, 8.8.6.2, 8.8.6.3, 8.8.6.4, 8.8.6.5, 8.8.6.6, 8.8.6.7, 8.8.6.8, 8.8.6.9, 8.8.6.10, 8.8.7.1, 8.8.7.2, 8.8.7.3, 8.8.7.4, 8.8.7.5, 8.8.7.6, 8.8.7.7, 8.8.7.8, 8.8.7.9, 8.8.7.10, 8.8.8.1, 8.8.8.2, 8.8.8.3, 8.8.8.4, 8.8.8.5, 8.8.8.6, 8.8.8.7, 8.8.8.8, 8.8.8.9, 8.8.8.10, 8.8.9.1, 8.8.9.2, 8.8.9.3, 8.8.9.4, 8.8.9.5, 8.8.9.6, 8.8.9.7, 8.8.9.8, 8.8.9.9, 8.8.9.10, 8.8.10.1, 8.8.10.2, 8.8.10.3, 8.8.10.4, 8.8.10.5, 8.8.10.6, 8.8.10.7, 8.8.10.8, 8.8.10.9, 8.8.10.10, 8.9.1.1, 8.9.1.2, 8.9.1.3, 8.9.1.4, 8.9.1.5, 8.9.1.6, 8.9.1.7, 8.9.1.8, 8.9.1.9, 8.9.1.10, 8.9.2.1, 8.9.2.2, 8.9.2.3, 8.9.2.4, 8.9.2.5, 8.9.2.6, 8.9.2.7, 8.9.2.8, 8.9.2.9, 8.9.2.10, 8.9.3.1, 8.9.3.2, 8.9.3.3, 8.9.3.4, 8.9.3.5, 8.9.3.6, 8.9.3.7, 8.9.3.8, 8.9.3.9, 8.9.3.10, 8.9.4.1, 8.9.4.2, 8.9.4.3, 8.9.4.4, 8.9.4.5, 8.9.4.6, 8.9.4.7, 8.9.4.8, 8.9.4.9, 8.9.4.10, 8.9.5.1, 8.9.5.2, 8.9.5.3, 8.9.5.4, 8.9.5.5, 8.9.5.6, 8.9.5.7, 8.9.5.8, 8.9.5.9, 8.9.5.10, 8.9.6.1, 8.9.6.2, 8.9.6.3, 8.9.6.4, 8.9.6.5, 8.9.6.6, 8.9.6.7, 8.9.6.8, 8.9.6.9, 8.9.6.10, 8.9.7.1, 8.9.7.2, 8.9.7.3, 8.9.7.4, 8.9.7.5, 8.9.7.6, 8.9.7.7, 8.9.7.8, 8.9.7.9, 8.9.7.10, 8.9.8.1, 8.9.8.2, 8.9.8.3, 8.9.8.4, 8.9.8.5, 8.9.8.6, 8.9.8.7, 8.9.8.8, 8.9.8.9, 8.9.8.10, 8.9.9.1, 8.9.9.2, 8.9.9.3, 8.9.9.4, 8.9.9.5, 8.9.9.6, 8.9.9.7, 8.9.9.8, 8.9.9.9, 8.9.9.10, 8.9.10.1, 8.9.10.2, 8.9.10.3, 8.9.10.4, 8.9.10.5, 8.9.10.6, 8.9.10.7, 8.9.10.8, 8.9.10.9, 8.9.10.10, 8.10.1.1, 8.10.1.2, 8.10.1.3, 8.10.1.4, 8.10.1.5, 8.10.1.6, 8.10.1.7, 8.10.1.8, 8.10.1.9, 8.10.1.10, 8.10.2.1, 8.10.2.2, 8.10.2.3, 8.10.2.4, 8.10.2.5, 8.10.2.6, 8.10.2.7, 8.10.2.8, 8.10.2.9, 8.10.2.10, 8.10.3.1, 8.10.3.2, 8.10.3.3, 8.10.3.4, 8.10.3.5, 8.10.3.6, 8.10.3.7, 8.10.3.8, 8.10.3.9, 8.10.3.10, 8.10.4.1, 8.10.4.2, 8.10.4.3, 8.10.4.4, 8.10.4.5, 8.10.4.6, 8.10.4.7, 8.10.4.8, 8.10.4.9, 8.10.4.10, 8.10.5.1, 8.10.5.2, 8.10.5.3, 8.10.5.4, 8.10.5.5, 8.10.5.6, 8.10.5.7, 8.10.5.8, 8.10.5.9, 8.10.5.10, 8.10.6.1, 8.10.6.2, 8.10.6.3, 8.10.6.4, 8.10.6.5, 8.10.6.6, 8.10.6.7, 8.10.6.8, 8.10.6.9, 8.10.6.10, 8.10.7.1, 8.10.7.2, 8.10.7.3, 8.10.7.4, 8.10.7.5, 8.10.7.6, 8.10.7.7, 8.10.7.8, 8.10.7.9, 8.10.7.10, 8.10.8.1, 8.10.8.2, 8.10.8.3, 8.10.8.4, 8.10.8.5, 8.10.8.6, 8.10.8.7, 8.10.8.8, 8.10.8.9, 8.10.8.10, 8.10.9.1, 8.10.9.2, 8.10.9.3, 8.10.9.4, 8.10.9.5, 8.10.9.6, 8.10.9.7, 8.10.9.8, 8.10.9.9, 8.10.9.10, 8.10.10.1, 8.10.10.2, 8.10.10.3, 8.10.10.4, 8.10.10.5, 8.10.10.6, 8.10.10.7, 8.10.10.8, 8.10.10.9, 8.10.10.10, 9.1.1.1, 9.1.1.2, 9.1.1.3, 9.1.1.4, 9.1.1.5, 9.1.1.6, 9.1.1.7, 9.1.1.8, 9.1.1.9, 9.1.1.10, 9.1.2.1, 9.1.2.2, 9.1.2.3, 9.1.2.4, 9.1.2.5, 9.1.2.6, 9.1.2.7, 9.1.2.8, 9.1.2.9, 9.1.2.10, 9.1.3.1, 9.1.3.2, 9.1.3.3, 9.1.3.4, 9.1.3.5, 9.1.3.6, 9.1.3.7, 9.1.3.8, 9.1.3.9, 9.1.3.10, 9.1.4.1, 9.1.4.2, 9.1.4.3, 9.1.4.4, 9.1.4.5, 9.1.4.6, 9.1.4.7, 9.1.4.8, 9.1.4.9, 9.1.4.10, 9.1.5.1, 9.1.5.2, 9.1.5.3, 9.1.5.4, 9.1.5.5, 9.1.5.6, 9.1.5.7, 9.1.5.8, 9.1.5.9, 9.1.5.10, 9.1.6.1, 9.1.6.2, 9.1.6.3, 9.1.6.4, 9.1.6.5, 9.1.6.6, 9.1.6.7, 9.1.6.8, 9.1.6.9, 9.1.6.10, 9.1.7.1, 9.1.7.2, 9.1.7.3, 9.1.7.4, 9.1.7.5, 9.1.7.6, 9.1.7.7, 9.1.7.8, 9.1.7.9, 9.1.7.10, 9.1.8.1, 9.1.8.2, 9.1.8.3, 9.1.8.4, 9.1.8.5, 9.1.8.6, 9.1.8.7, 9.1.8.8, 9.1.8.9, 9.1.8.10, 9.1.9.1, 9.1.9.2, 9.1.9.3, 9.1.9.4, 9.1.9.5, 9.1.9.6, 9.1.9.7, 9.1.9.8, 9.1.9.9, 9.1.9.10, 9.1.10.1, 9.1.10.2, 9.1.10.3, 9.1.10.4, 9.1.10.5, 9.1.10.6, 9.1.10.7, 9.1.10.8, 9.1.10.9, 9.1.10.10, 9.2.1.1, 9.2.1.2, 9.2.1.3, 9.2.1.4, 9.2.1.5, 9.2.1.6, 9.2.1.7, 9.2.1.8, 9.2.1.9, 9.2.1.10, 9.2.2.1, 9.2.2.2, 9.2.2.3, 9.2.2.4, 9.2.2.5, 9.2.2.6, 9.2.2.7, 9.2.2.8, 9.2.2.9, 9.2.2.10, 9.2.3.1, 9.2.3.2, 9.2.3.3, 9.2.3.4, 9.2.3.5, 9.2.3.6, 9.2.3.7, 9.2.3.8, 9.2.3.9, 9.2.3.10, 9.2.4.1, 9.2.4.2, 9.2.4.3, 9.2.4.4, 9.2.4.5, 9.2.4.6, 9.2.4.7, 9.2.4.8, 9.2.4.9, 9.2.4.10, 9.2.5.1, 9.2.5.2, 9.2.5.3, 9.2.5.4, 9.2.5.5, 9.2.5.6, 9.2.5.7, 9.2.5.8, 9.2.5.9, 9.2.5.10, 9.2.6.1, 9.2.6.2, 9.2.6.3, 9.2.6.4, 9.2.6.5, 9.2.6.6, 9.2.6.7, 9.2.6.8, 9.2.6.9, 9.2.6.10, 9.2.7.1, 9.2.7.2, 9.2.7.3, 9.2.7.4, 9.2.7.5, 9.2.7.6, 9.2.7.7, 9.2.7.8, 9.2.7.9, 9.2.7.10, 9.2.8.1, 9.2.8.2, 9.2.8.3, 9.2.8.4, 9.2.8.5, 9.2.8.6, 9.2.8.7, 9.2.8.8, 9.2.8.9, 9.2.8.10, 9.2.9.1, 9.2.9.2, 9.2.9.3, 9.2.9.4, 9.2.9.5, 9.2.9.6, 9.2.9.7, 9.2.9.8, 9.2.9.9, 9.2.9.10, 9.2.10.1, 9.2.10.2, 9.2.10.3, 9.2.10.4, 9.2.10.5, 9.2.10.6, 9.2.10.7, 9.2.10.8, 9.2.10.9, 9.2.10.10, 9.3.1.1, 9.3.1.2, 9.3.1.3, 9.3.1.4, 9.3.1.5, 9.3.1.6, 9.3.1.7, 9.3.1.8, 9.3.1.9, 9.3.1.10, 9.3.2.1, 9.3.2.2, 9.3.2.3, 9.3.2.4, 9.3.2.5, 9.3.2.6, 9.3.2.7, 9.3.2.8, 9.3.2.9, 9.3.2.10, 9.3.3.1, 9.3.3.2, 9.3.3.3, 9.3.3.4, 9.3.3.5, 9.3.3.6, 9.3.3.7, 9.3.3.8, 9.3.3.9, 9.3.3.10, 9.3.4.1, 9.3.4.2, 9.3.4.3, 9.3.4.4, 9.3.4.5, 9.3.4.6, 9.3.4.7, 9.3.4.8, 9.3.4.9, 9.3.4.10, 9.3.5.1, 9.3.5.2, 9.3.5.3, 9.3.5.4, 9.3.5.5, 9.3.5.6, 9.3.5.7, 9.3.5.8, 9.3.5.9, 9.3.5.10, 9.3.6.1, 9.3.6.2, 9.3.6.3, 9.3.6.4, 9.3.6.5, 9.3.6.6, 9.3.6.7, 9.3.6.8, 9.3.6.9, 9.3.6.10, 9.3.7.1, 9.3.7.2, 9.3.7.3, 9.3.7.4, 9.3.7.5, 9.3.7.6, 9.3.7.7, 9.3.7.8, 9.3.7.9, 9.3.7.10, 9.3.8.1, 9.3.8.2, 9.3.8.3, 9.3.8.4, 9.3.8.5, 9.3.8.6, 9.3.8.7, 9.3.8.8, 9.3.8.9, 9.3.8.10, 9.3.9.1, 9.3.9.2, 9.3.9.3, 9.3.9.4, 9.3.9.5, 9.3.9.6, 9.3.9.7, 9.3.9.8, 9.3.9.9, 9.3.9.10, 9.3.10.1, 9.3.10.2, 9.3.10.3, 9.3.10.4, 9.3.10.5, 9.3.10.6, 9.3.10.7, 9.3.10.8, 9.3.10.9, 9.3.10.10, 9.4.1.1, 9.4.1.2, |
| 9.4.1.3, 9.4.1.4, 9.4.1.5, 9.4.1.6, 9.4.1.7, 9.4.1.8, 9.4.1.9, 9.4.1.10, 9.4.2.1, 9.4.2.2, 9.4.2.3, 9.4.2.4, 9.4.2.5, 9.4.2.6, 9.4.2.7, 9.4.2.8, 9.4.2.9, 9.4.2.10, 9.4.3.1, 9.4.3.2, 9.4.3.3, 9.4.3.4, 9.4.3.5, 9.4.3.6, 9.4.3.7, 9.4.3.8, 9.4.3.9, 9.4.3.10, 9.4.4.1, 9.4.4.2, 9.4.4.3, 9.4.4.4, 9.4.4.5, 9.4.4.6, 9.4.4.7, 9.4.4.8, 9.4.4.9, 9.4.4.10, 9.4.5.1, 9.4.5.2, 9.4.5.3, 9.4.5.4, 9.4.5.5, 9.4.5.6, 9.4.5.7, 9.4.5.8, 9.4.5.9, 9.4.5.10, 9.4.6.1, 9.4.6.2, 9.4.6.3, 9.4.6.4, 9.4.6.5, 9.4.6.6, 9.4.6.7, 9.4.6.8, 9.4.6.9, 9.4.6.10, 9.4.7.1, 9.4.7.2, 9.4.7.3, 9.4.7.4, 9.4.7.5, 9.4.7.6, 9.4.7.7, 9.4.7.8, 9.4.7.9, 9.4.7.10, 9.4.8.1, 9.4.8.2, 9.4.8.3, 9.4.8.4, 9.4.8.5, 9.4.8.6, 9.4.8.7, 9.4.8.8, 9.4.8.9, 9.4.8.10, 9.4.9.1, 9.4.9.2, 9.4.9.3, 9.4.9.4, 9.4.9.5, 9.4.9.6, 9.4.9.7, 9.4.9.8, 9.4.9.9, 9.4.9.10, 9.4.10.1, 9.4.10.2, 9.4.10.3, 9.4.10.4, 9.4.10.5, 9.4.10.6, 9.4.10.7, 9.4.10.8, 9.4.10.9, 9.4.10.10, 9.5.1.1, 9.5.1.2, 9.5.1.3, 9.5.1.4, 9.5.1.5, 9.5.1.6, 9.5.1.7, 9.5.1.8, 9.5.1.9, 9.5.1.10, 9.5.2.1, 9.5.2.2, 9.5.2.3, 9.5.2.4, 9.5.2.5, 9.5.2.6, 9.5.2.7, 9.5.2.8, 9.5.2.9, 9.5.2.10, 9.5.3.1, 9.5.3.2, 9.5.3.3, 9.5.3.4, 9.5.3.5, 9.5.3.6, 9.5.3.7, 9.5.3.8, 9.5.3.9, 9.5.3.10, 9.5.4.1, 9.5.4.2, 9.5.4.3, 9.5.4.4, 9.5.4.5, 9.5.4.6, 9.5.4.7, 9.5.4.8, 9.5.4.9, 9.5.4.10, 9.5.5.1, 9.5.5.2, 9.5.5.3, 9.5.5.4, 9.5.5.5, 9.5.5.6, 9.5.5.7, 9.5.5.8, 9.5.5.9, 9.5.5.10, 9.5.6.1, 9.5.6.2, 9.5.6.3, 9.5.6.4, 9.5.6.5, 9.5.6.6, 9.5.6.7, 9.5.6.8, 9.5.6.9, 9.5.6.10, 9.5.7.1, 9.5.7.2, 9.5.7.3, 9.5.7.4, 9.5.7.5, 9.5.7.6, 9.5.7.7, 9.5.7.8, 9.5.7.9, 9.5.7.10, 9.5.8.1, 9.5.8.2, 9.5.8.3, 9.5.8.4, 9.5.8.5, 9.5.8.6, 9.5.8.7, 9.5.8.8, 9.5.8.9, 9.5.8.10, 9.5.9.1, 9.5.9.2, 9.5.9.3, 9.5.9.4, 9.5.9.5, 9.5.9.6, 9.5.9.7, 9.5.9.8, 9.5.9.9, 9.5.9.10, 9.5.10.1, 9.5.10.2, 9.5.10.3, 9.5.10.4, 9.5.10.5, 9.5.10.6, 9.5.10.7, 9.5.10.8, 9.5.10.9, 9.5.10.10, 9.6.1.1, 9.6.1.2, 9.6.1.3, 9.6.1.4, 9.6.1.5, 9.6.1.6, 9.6.1.7, 9.6.1.8, 9.6.1.9, 9.6.1.10, 9.6.2.1, 9.6.2.2, 9.6.2.3, 9.6.2.4, 9.6.2.5, 9.6.2.6, 9.6.2.7, 9.6.2.8, 9.6.2.9, 9.6.2.10, 9.6.3.1, 9.6.3.2, 9.6.3.3, 9.6.3.4, 9.6.3.5, 9.6.3.6, 9.6.3.7, 9.6.3.8, 9.6.3.9, 9.6.3.10, 9.6.4.1, 9.6.4.2, 9.6.4.3, 9.6.4.4, 9.6.4.5, 9.6.4.6, 9.6.4.7, 9.6.4.8, 9.6.4.9, 9.6.4.10, 9.6.5.1, 9.6.5.2, 9.6.5.3, 9.6.5.4, 9.6.5.5, 9.6.5.6, 9.6.5.7, 9.6.5.8, 9.6.5.9, 9.6.5.10, 9.6.6.1, 9.6.6.2, 9.6.6.3, 9.6.6.4, 9.6.6.5, 9.6.6.6, 9.6.6.7, 9.6.6.8, 9.6.6.9, 9.6.6.10, 9.6.7.1, 9.6.7.2, 9.6.7.3, 9.6.7.4, 9.6.7.5, 9.6.7.6, 9.6.7.7, 9.6.7.8, 9.6.7.9, 9.6.7.10, 9.6.8.1, 9.6.8.2, 9.6.8.3, 9.6.8.4, 9.6.8.5, 9.6.8.6, 9.6.8.7, 9.6.8.8, 9.6.8.9, 9.6.8.10, 9.6.9.1, 9.6.9.2, 9.6.9.3, 9.6.9.4, 9.6.9.5, 9.6.9.6, 9.6.9.7, 9.6.9.8, 9.6.9.9, 9.6.9.10, 9.6.10.1, 9.6.10.2, 9.6.10.3, 9.6.10.4, 9.6.10.5, 9.6.10.6, 9.6.10.7, 9.6.10.8, 9.6.10.9, 9.6.10.10, 9.7.1.1, 9.7.1.2, 9.7.1.3, 9.7.1.4, 9.7.1.5, 9.7.1.6, 9.7.1.7, 9.7.1.8, 9.7.1.9, 9.7.1.1 0, 9.7.2.1, 9.7.2.2, 9.7.2.3, 9.7.2.4, 9.7.2.5, 9.7.2.6, 9.7.2.7, 9.7.2.8, 9.7.2.9, 9.7.2.1 0, 9.7.3.1, 9.7.3.2, 9.7.3.3, 9.7.3.4, 9.7.3.5, 9.7.3.6, 9.7.3.7, 9.7.3.8, 9.7.3.9, 9.7.3.10, 9.7.4.1, 9.7.4.2, 9.7.4.3, 9.7.4.4, 9.7.4.5, 9.7.4.6, 9.7.4.7, 9.7.4.8, 9.7.4.9, 9.7.4.10, 9.7.5.1, 9.7.5.2, 9.7.5.3, 9.7.5.4, 9.7.5.5, 9.7.5.6, 9.7.5.7, 9.7.5.8, 9.7.5.9, 9.7.5.10, 9.7.6.1, 9.7.6.2, 9.7.6.3, 9.7.6.4, 9.7.6.5, 9.7.6.6, 9.7.6.7, 9.7.6.8, 9.7.6.9, 9.7.6.10, 9.7.7.1, 9.7.7.2, 9.7.7.3, 9.7.7.4, 9.7.7.5, 9.7.7.6, 9.7.7.7, 9.7.7.8, 9.7.7.9, 9.7.7.10, 9.7.8.1, 9.7.8.2, 9.7.8.3, 9.7.8.4, 9.7.8.5, 9.7.8.6, 9.7.8.7, 9.7.8.8, 9.7.8.9, 9.7.8.10, 9.7.9.1, 9.7.9.2, 9.7.9.3, 9.7.9.4, 9.7.9.5, 9.7.9.6, 9.7.9.7, 9.7.9.8, 9.7.9.9, 9.7.9.10, 9.7.10.1, 9.7.10.2, 9.7.10.3, 9.7.10.4, 9.7.10.5, 9.7.10.6, 9.7.10.7, 9.7.10.8, 9.7.10.9, 9.7.10.10, 9.8.1.1, 9.8.1.2, 9.8.1.3, 9.8.1.4, 9.8.1.5, 9.8.1.6, 9.8.1.7, 9.8.1.8, 9.8.1.9, 9.8.1.10, 9.8.2.1, 9.8.2.2, 9.8.2.3, 9.8.2.4, 9.8.2.5, 9.8.2.6, 9.8.2.7, 9.8.2.8, 9.8.2.9, 9.8.2.10, 9.8.3.1, 9.8.3.2, 9.8.3.3, 9.8.3.4, 9.8.3.5, 9.8.3.6, 9.8.3.7, 9.8.3.8, 9.8.3.9, 9.8.3.10, 9.8.4.1, 9.8.4.2, 9.8.4.3, 9.8.4.4, 9.8.4.5, 9.8.4.6, 9.8.4.7, 9.8.4.8, 9.8.4.9, 9.8.4.10, 9.8.5.1, 9.8.5.2, 9.8.5.3, 9.8.5.4, 9.8.5.5, 9.8.5.6, 9.8.5.7, 9.8.5.8, 9.8.5.9, 9.8.5.10, 9.8.6.1, 9.8.6.2, 9.8.6.3, 9.8.6.4, 9.8.6.5, 9.8.6.6, 9.8.6.7, 9.8.6.8, 9.8.6.9, 9.8.6.10, 9.8.7.1, 9.8.7.2, 9.8.7.3, 9.8.7.4, 9.8.7.5, 9.8.7.6, 9.8.7.7, 9.8.7.8, 9.8.7.9, 9.8.7.10, 9.8.8.1, 9.8.8.2, 9.8.8.3, 9.8.8.4, 9.8.8.5, 9.8.8.6, 9.8.8.7, 9.8.8.8, 9.8.8.9, 9.8.8.10, 9.8.9.1, 9.8.9.2, 9.8.9.3, 9.8.9.4, 9.8.9.5, 9.8.9.6, 9.8.9.7, 9.8.9.8, 9.8.9.9, 9.8.9.10, 9.8.10.1, 9.8.10.2, 9.8.10.3, 9.8.10.4, 9.8.10.5, 9.8.10.6, 9.8.10.7, 9.8.10.8, 9.8.10.9, 9.8.10.10, 9.9.1.1, 9.9.1.2, 9.9.1.3, 9.9.1.4, 9.9.1.5, 9.9.1.6, 9.9.1.7, 9.9.1.8, 9.9.1.9, 9.9.1.10, 9.9.2.1, 9.9.2.2, 9.9.2.3, 9.9.2.4, 9.9.2.5, 9.9.2.6, 9.9.2.7, 9.9.2.8, 9.9.2.9, 9.9.2.10, 9.9.3.1, 9.9.3.2, 9.9.3.3, 9.9.3.4, 9.9.3.5, 9.9.3.6, 9.9.3.7, 9.9.3.8, 9.9.3.9, 9.9.3.10, 9.9.4.1, 9.9.4.2, 9.9.4.3, 9.9.4.4, 9.9.4.5, 9.9.4.6, 9.9.4.7, 9.9.4.8, 9.9.4.9, 9.9.4.10, 9.9.5.1, 9.9.5.2, 9.9.5.3, 9.9.5.4, 9.9.5.5, 9.9.5.6, 9.9.5.7, 9.9.5.8, 9.9.5.9, 9.9.5.10, 9.9.6.1, 9.9.6.2, 9.9.6.3, 9.9.6.4, 9.9.6.5, 9.9.6.6, 9.9.6.7, 9.9.6.8, 9.9.6.9, 9.9.6.10, 9.9.7.1, 9.9.7.2, 9.9.7.3, 9.9.7.4, 9.9.7.5, 9.9.7.6, 9.9.7.7, 9.9.7.8, 9.9.7.9, 9.9.7.10, 9.9.8.1, 9.9.8.2, 9.9.8.3, 9.9.8.4, 9.9.8.5, 9.9.8.6, 9.9.8.7, 9.9.8.8, 9.9.8.9, 9.9.8.10, 9.9.9.1, 9.9.9.2, 9.9.9.3, 9.9.9.4, 9.9.9.5, 9.9.9.6, 9.9.9.7, 9.9.9.8, 9.9.9.9, 9.9.9.10, 9.9.10.1, 9.9.10.2, 9.9.10.3, 9.9.10.4, 9.9.10.5, 9.9.10.6, 9.9.10.7, 9.9.10.8, 9.9.10.9, 9.9.10.10, 9.10.1.1, 9.10.1.2, 9.10.1.3, 9.10.1.4, 9.10.1.5, 9.10.1.6, 9.10.1.7, 9.10.1.8, 9.10.1.9, 9.10.1.10, 9.10.2.1, 9.10.2.2, 9.10.2.3, 9.10.2.4, 9.10.2.5, 9.10.2.6, 9.10.2.7, 9.10.2.8, 9.10.2.9, 9.10.2.10, 9.10.3.1, 9.10.3.2, 9.10.3.3, 9.10.3.4, 9.10.3.5, 9.10.3.6, 9.10.3.7, 9.10.3.8, 9.10.3.9, 9.10.3.10, 9.10.4.1, 9.10.4.2, 9.10.4.3, 9.10.4.4, 9.10.4.5, 9.10.4.6, 9.10.4.7, 9.10.4.8, 9.10.4.9, 9.10.4.10, 9.10.5.1, 9.10.5.2, 9.10.5.3, 9.10.5.4, 9.10.5.5, 9.10.5.6, 9.10.5.7, 9.10.5.8, 9.10.5.9, 9.10.5.10, 9.10.6.1, 9.10.6.2, |
| 9.10.6.3, 9.10.6.4, 9.10.6.5, 9.10.6.6, 9.10.6.7, 9.10.6.8, 9.10.6.9, 9.10.6.10, 9.10.7.1, 9.10.7.2, 9.10.7.3, 9.10.7.4, 9.10.7.5, 9.10.7.6, 9.10.7.7, 9.10.7.8, 9.10.7.9, 9.10.7.10, 9.10.8.1, 9.10.8.2, 9.10.8.3, 9.10.8.4, 9.10.8.5, 9.10.8.6, 9.10.8.7, 9.10.8.8, 9.10.8.9, 9.10.8.10, 9.10.9.1, 9.10.9.2, 9.10.9.3, 9.10.9.4, 9.10.9.5, 9.10.9.6, 9.10.9.7, 9.10.9.8, 9.10.9.9, 9.10.9.10, 9.10.10.1, 9.10.10.2, 9.10.10.3, 9.10.10.4, 9.10.10.5, 9.10.10.6, 9.10.10.7, 9.10.10.8, 9.10.10.9, 9.10.10.10, 10.1.1.1, 10.1.1.2, 10.1.1.3, 10.1.1.4, 10.1.1.5, 10.1.1.6, 10.1.1.7, 10.1.1.8, 10.1.1.9, 10.1.1.10, 10.1.2.1, 10.1.2.2, 10.1.2.3, 10.1.2.4, 10.1.2.5, 10.1.2.6, 10.1.2.7, 10.1.2.8, 10.1.2.9, 10.1.2.10, 10.1.3.1, 10.1.3.2, 10.1.3.3, 10.1.3.4, 10.1.3.5, 10.1.3.6, 10.1.3.7, 10.1.3.8, 10.1.3.9, 10.1.3.10, 10.1.4.1, 10.1.4.2, 10.1.4.3, 10.1.4.4, 10.1.4.5, 10.1.4.6, 10.1.4.7, 10.1.4.8, 10.1.4.9, 10.1.4.10, 10.1.5.1, 10.1.5.2, 10.1.5.3, 10.1.5.4, 10.1.5.5, 10.1.5.6, 10.1.5.7, 10.1.5.8, 10.1.5.9, 10.1.5.10, 10.1.6.1, 10.1.6.2, 10.1.6.3, 10.1.6.4, 10.1.6.5, 10.1.6.6, 10.1.6.7, 10.1.6.8, 10.1.6.9, 10.1.6.10, 10.1.7.1, 10.1.7.2, 10.1.7.3, 10.1.7.4, 10.1.7.5, 10.1.7.6, 10.1.7.7, 10.1.7.8, 10.1.7.9, 10.1.7.10, 10.1.8.1, 10.1.8.2, 10.1.8.3, 10.1.8.4, 10.1.8.5, 10.1.8.6, 10.1.8.7, 10.1.8.8, 10.1.8.9, 10.1.8.10, 10.1.9.1, 10.1.9.2, 10.1.9.3, 10.1.9.4, 10.1.9.5, 10.1.9.6, 10.1.9.7, 10.1.9.8, 10.1.9.9, 10.1.9.10, 10.1.10.1, 10.1.10.2, 10.1.10.3, 10.1.10.4, 10.1.10.5, 10.1.10.6, 10.1.10.7, 10.1.10.8, 10.1.10.9, 10.1.10.10, 10.2.1.1, 10.2.1.2, 10.2.1.3, 10.2.1.4, 10.2.1.5, 10.2.1.6, 10.2.1.7, 10.2.1.8, 10.2.1.9, 10.2.1.10, 10.2.2.1, 10.2.2.2, 10.2.2.3, 10.2.2.4, 10.2.2.5, 10.2.2.6, 10.2.2.7, 10.2.2.8, 10.2.2.9, 10.2.2.10, 10.2.3.1, 10.2.3.2, 10.2.3.3, 10.2.3.4, 10.2.3.5, 10.2.3.6, 10.2.3.7, 10.2.3.8, 10.2.3.9, 10.2.3.10, 10.2.4.1, 10.2.4.2, 10.2.4.3, 10.2.4.4, 10.2.4.5, 10.2.4.6, 10.2.4.7, 10.2.4.8, 10.2.4.9, 10.2.4.10, 10.2.5.1, 10.2.5.2, 10.2.5.3, 10.2.5.4, 10.2.5.5, 10.2.5.6, 10.2.5.7, 10.2.5.8, 10.2.5.9, 10.2.5.10, 10.2.6.1, 10.2.6.2, 10.2.6.3, 10.2.6.4, 10.2.6.5, 10.2.6.6, 10.2.6.7, 10.2.6.8, 10.2.6.9, 10.2.6.10, 10.2.7.1, 10.2.7.2, 10.2.7.3, 10.2.7.4, 10.2.7.5, 10.2.7.6, 10.2.7.7, 10.2.7.8, 10.2.7.9, 10.2.7.10, 10.2.8.1, 10.2.8.2, 10.2.8.3, 10.2.8.4, 10.2.8.5, 10.2.8.6, 10.2.8.7, 10.2.8.8, 10.2.8.9, 10.2.8.10, 10.2.9.1, 10.2.9.2, 10.2.9.3, 10.2.9.4, 10.2.9.5, 10.2.9.6, 10.2.9.7, 10.2.9.8, 10.2.9.9, 10.2.9.10, 10.2.10.1, 10.2.10.2, 10.2.10.3, 10.2.10.4, 10.2.10.5, 10.2.10.6, 10.2.10.7, 10.2.10.8, 10.2.10.9, 10.2.10.10, 10.3.1.1, 10.3.1.2, 10.3.1.3, 10.3.1.4, 10.3.1.5, 10.3.1.6, 10.3.1.7, 10.3.1.8, 10.3.1.9, 10.3.1.10, 10.3.2.1, 10.3.2.2, 10.3.2.3, 10.3.2.4, 10.3.2.5, 10.3.2.6, 10.3.2.7, 10.3.2.8, 10.3.2.9, 10.3.2.10, 10.3.3.1, 10.3.3.2, 10.3.3.3, 10.3.3.4, 10.3.3.5, 10.3.3.6, 10.3.3.7, 10.3.3.8, 10.3.3.9, 10.3.3.10, 10.3.4.1, 10.3.4.2, 10.3.4.3, 10.3.4.4, 10.3.4.5, 10.3.4.6, 10.3.4.7, 10.3.4.8, 10.3.4.9, 10.3.4.10, 10.3.5.1, 10.3.5.2, 10.3.5.3, 10.3.5.4, 10.3.5.5, 10.3.5.6, 10.3.5.7, 10.3.5.8, 10.3.5.9, 10.3.5.10, 10.3.6.1, 10.3.6.2, 10.3.6.3, 10.3.6.4, 10.3.6.5, 10.3.6.6, 10.3.6.7, 10.3.6.8, 10.3.6.9, 10.3.6.10, 10.3.7.1, 10.3.7.2, 10.3.7.3, 10.3.7.4, 10.3.7.5, 10.3.7.6, 10.3.7.7, 10.3.7.8, 10.3.7.9, 10.3.7.10, 10.3.8.1, 10.3.8.2, 10.3.8.3, 10.3.8.4, 10.3.8.5, 10.3.8.6, 10.3.8.7, 10.3.8.8, 10.3.8.9, 10.3.8.10, 10.3.9.1, 10.3.9.2, 10.3.9.3, 10.3.9.4, 10.3.9.5, 10.3.9.6, 10.3.9.7, 10.3.9.8, 10.3.9.9, 10.3.9.10, 10.3.10.1, 10.3.10.2, 10.3.10.3, 10.3.10.4, 10.3.10.5, 10.3.10.6, 10.3.10.7, 10.3.10.8, 10.3.10.9, 10.3.10.10, 10.4.1.1, 10.4.1.2, 10.4.1.3, 10.4.1.4, 10.4.1.5, 10.4.1.6, 10.4.1.7, 10.4.1.8, 10.4.1.9, 10.4.1.10, 10.4.2.1, 10.4.2.2, 10.4.2.3, 10.4.2.4, 10.4.2.5, 10.4.2.6, 10.4.2.7, 10.4.2.8, 10.4.2.9, 10.4.2.10, 10.4.3.1, 10.4.3.2, 10.4.3.3, 10.4.3.4, 10.4.3.5, 10.4.3.6, 10.4.3.7, 10.4.3.8, 10.4.3.9, 10.4.3.10, 10.4.4.1, 10.4.4.2, 10.4.4.3, 10.4.4.4, 10.4.4.5, 10.4.4.6, 10.4.4.7, 10.4.4.8, 10.4.4.9, 10.4.4.10, 10.4.5.1, 10.4.5.2, 10.4.5.3, 10.4.5.4, 10.4.5.5, 10.4.5.6, 10.4.5.7, 10.4.5.8, 10.4.5.9, 10.4.5.10, 10.4.6.1, 10.4.6.2, 10.4.6.3, 10.4.6.4, 10.4.6.5, 10.4.6.6, 10.4.6.7, 10.4.6.8, 10.4.6.9, 10.4.6.10, 10.4.7.1, 10.4.7.2, 10.4.7.3, 10.4.7.4, 10.4.7.5, 10.4.7.6, 10.4.7.7, 10.4.7.8, 10.4.7.9, 10.4.7.10, 10.4.8.1, 10.4.8.2, 10.4.8.3, 10.4.8.4, 10.4.8.5, 10.4.8.6, 10.4.8.7, 10.4.8.8, 10.4.8.9, 10.4.8.10, 10.4.9.1, 10.4.9.2, 10.4.9.3, 10.4.9.4, 10.4.9.5, 10.4.9.6, 10.4.9.7, 10.4.9.8, 10.4.9.9, 10.4.9.10, 10.4.10.1, 10.4.10.2, 10.4.10.3, 10.4.10.4, 10.4.10.5, 10.4.10.6, 10.4.10.7, 10.4.10.8, 10.4.10.9, 10.4.10.10, 10.5.1.1, 10.5.1.2, 10.5.1.3, 10.5.1.4, 10.5.1.5, 10.5.1.6, 10.5.1.7, 10.5.1.8, 10.5.1.9, 10.5.1.10, 10.5.2.1, 10.5.2.2, 10.5.2.3, 10.5.2.4, 10.5.2.5, 10.5.2.6, 10.5.2.7, 10.5.2.8, 10.5.2.9, 10.5.2.10, 10.5.3.1, 10.5.3.2, 10.5.3.3, 10.5.3.4, 10.5.3.5, 10.5.3.6, 10.5.3.7, 10.5.3.8, 10.5.3.9, 10.5.3.10, 10.5.4.1, 10.5.4.2, 10.5.4.3, 10.5.4.4, 10.5.4.5, 10.5.4.6, 10.5.4.7, 10.5.4.8, 10.5.4.9, 10.5.4.10, 10.5.5.1, 10.5.5.2, 10.5.5.3, 10.5.5.4, 10.5.5.5, 10.5.5.6, 10.5.5.7, 10.5.5.8, 10.5.5.9, 10.5.5.10, 10.5.6.1, 10.5.6.2, 10.5.6.3, 10.5.6.4, 10.5.6.5, 10.5.6.6, 10.5.6.7, 10.5.6.8, 10.5.6.9, 10.5.6.10, 10.5.7.1, 10.5.7.2, 10.5.7.3, 10.5.7.4, 10.5.7.5, 10.5.7.6, 10.5.7.7, 10.5.7.8, 10.5.7.9, 10.5.7.10, 10.5.8.1, 10.5.8.2, 10.5.8.3, 10.5.8.4, 10.5.8.5, 10.5.8.6, 10.5.8.7, 10.5.8.8, 10.5.8.9, 10.5.8.10, 10.5.9.1, 10.5.9.2, 10.5.9.3, 10.5.9.4, 10.5.9.5, 10.5.9.6, 10.5.9.7, 10.5.9.8, 10.5.9.9, 10.5.9.10, 10.5.10.1, 10.5.10.2, 10.5.10.3, 10.5.10.4, 10.5.10.5, 10.5.10.6, 10.5.10.7, 10.5.10.8, 10.5.10.9, 10.5.10.10, 10.6.1.1, 10.6.1.2, 10.6.1.3, 10.6.1.4, 10.6.1.5, 10.6.1.6, 10.6.1.7, 10.6.1.8, 10.6.1.9, 10.6.1.10, 10.6.2.1, 10.6.2.2, 10.6.2.3, 10.6.2.4, 10.6.2.5, 10.6.2.6, 10.6.2.7, 10.6.2.8, 10.6.2.9, 10.6.2.10, 10.6.3.1, 10.6.3.2, 10.6.3.3, 10.6.3.4, 10.6.3.5, 10.6.3.6, 10.6.3.7, 10.6.3.8, 10.6.3.9, 10.6.3.10, 10.6.4.1, 10.6.4.2, 10.6.4.3, 10.6.4.4, 10.6.4.5, 10.6.4.6, 10.6.4.7, 10.6.4.8, 10.6.4.9, 10.6.4.10, 10.6.5.1, 10.6.5.2, 10.6.5.3, 10.6.5.4, |
| 10.6.5.5, 10.6.5.6, 10.6.5.7, 10.6.5.8, 10.6.5.9, 10.6.5.10, 10.6.6.1, 10.6.6.2, 10.6.6.3, 10.6.6.4, 10.6.6.5, 10.6.6.6, 10.6.6.7, 10.6.6.8, 10.6.6.9, 10.6.6.10, 10.6.7.1, 10.6.7.2, 10.6.7.3, 10.6.7.4, 10.6.7.5, 10.6.7.6, 10.6.7.7, 10.6.7.8, 10.6.7.9, 10.6.7.10, 10.6.8.1, 10.6.8.2, 10.6.8.3, 10.6.8.4, 10.6.8.5, 10.6.8.6, 10.6.8.7, 10.6.8.8, 10.6.8.9, 10.6.8.10, 10.6.9.1, 10.6.9.2, 10.6.9.3, 10.6.9.4, 10.6.9.5, 10.6.9.6, 10.6.9.7, 10.6.9.8, 10.6.9.9, 10.6.9.10, 10.6.10.1, 10.6.10.2, 10.6.10.3, 10.6.10.4, 10.6.10.5, 10.6.10.6, 10.6.10.7, 10.6.10.8, 10.6.10.9, 10.6.10.10, 10.7.1.1, 10.7.1.2, 10.7.1.3, 10.7.1.4, 10.7.1.5, 10.7.1.6, 10.7.1.7, 10.7.1.8, 10.7.1.9, 10.7.1.10, 10.7.2.1, 10.7.2.2, 10.7.2.3, 10.7.2.4, 10.7.2.5, 10.7.2.6, 10.7.2.7, 10.7.2.8, 10.7.2.9, 10.7.2.10, 10.7.3.1, 10.7.3.2, 10.7.3.3, 10.7.3.4, 10.7.3.5, 10.7.3.6, 10.7.3.7, 10.7.3.8, 10.7.3.9, 10.7.3.10, 10.7.4.1, 10.7.4.2, 10.7.4.3, 10.7.4.4, 10.7.4.5, 10.7.4.6, 10.7.4.7, 10.7.4.8, 10.7.4.9, 10.7.4.10, 10.7.5.1, 10.7.5.2, 10.7.5.3, 10.7.5.4, 10.7.5.5, 10.7.5.6, 10.7.5.7, 10.7.5.8, 10.7.5.9, 10.7.5.10, 10.7.6.1, 10.7.6.2, 10.7.6.3, 10.7.6.4, 10.7.6.5, 10.7.6.6, 10.7.6.7, 10.7.6.8, 10.7.6.9, 10.7.6.10, 10.7.7.1, 10.7.7.2, 10.7.7.3, 10.7.7.4, 10.7.7.5, 10.7.7.6, 10.7.7.7, 10.7.7.8, 10.7.7.9, 10.7.7.10, 10.7.8.1, 10.7.8.2, 10.7.8.3, 10.7.8.4, 10.7.8.5, 10.7.8.6, 10.7.8.7, 10.7.8.8, 10.7.8.9, 10.7.8.10, 10.7.9.1, 10.7.9.2, 10.7.9.3, 10.7.9.4, 10.7.9.5, 10.7.9.6, 10.7.9.7, 10.7.9.8, 10.7.9.9, 10.7.9.10, 10.7.10.1, 10.7.10.2, 10.7.10.3, 10.7.10.4, 10.7.10.5, 10.7.10.6, 10.7.10.7, 10.7.10.8, 10.7.10.9, 10.7.10.10, 10.8.1.1, 10.8.1.2, 10.8.1.3, 10.8.1.4, 10.8.1.5, 10.8.1.6, 10.8.1.7, 10.8.1.8, 10.8.1.9, 10.8.1.10, 10.8.2.1, 10.8.2.2, 10.8.2.3, 10.8.2.4, 10.8.2.5, 10.8.2.6, 10.8.2.7, 10.8.2.8, 10.8.2.9, 10.8.2.10, 10.8.3.1, 10.8.3.2, 10.8.3.3, 10.8.3.4, 10.8.3.5, 10.8.3.6, 10.8.3.7, 10.8.3.8, 10.8.3.9, 10.8.3.10, 10.8.4.1, 10.8.4.2, 10.8.4.3, 10.8.4.4, 10.8.4.5, 10.8.4.6, 10.8.4.7, 10.8.4.8, 10.8.4.9, 10.8.4.10, 10.8.5.1, 10.8.5.2, 10.8.5.3, 10.8.5.4, 10.8.5.5, 10.8.5.6, 10.8.5.7, 10.8.5.8, 10.8.5.9, 10.8.5.10, 10.8.6.1, 10.8.6.2, 10.8.6.3, 10.8.6.4, 10.8.6.5, 10.8.6.6, 10.8.6.7, 10.8.6.8, 10.8.6.9, 10.8.6.10, 10.8.7.1, 10.8.7.2, 10.8.7.3, 10.8.7.4, 10.8.7.5, 10.8.7.6, 10.8.7.7, 10.8.7.8, 10.8.7.9, 10.8.7.10, 10.8.8.1, 10.8.8.2, 10.8.8.3, 10.8.8.4, 10.8.8.5, 10.8.8.6, 10.8.8.7, 10.8.8.8, 10.8.8.9, 10.8.8.10, 10.8.9.1, 10.8.9.2, 10.8.9.3, 10.8.9.4, 10.8.9.5, 10.8.9.6, 10.8.9.7, 10.8.9.8, 10.8.9.9, 10.8.9.10, 10.8.10.1, 10.8.10.2, 10.8.10.3, 10.8.10.4, 10.8.10.5, 10.8.10.6, 10.8.10.7, 10.8.10.8, 10.8.10.9, 10.8.10.10, 10.9.1.1, 10.9.1.2, 10.9.1.3, 10.9.1.4, 10.9.1.5, 10.9.1.6, 10.9.1.7, 10.9.1.8, 10.9.1.9, 10.9.1.10, 10.9.2.1, 10.9.2.2, 10.9.2.3, 10.9.2.4, 10.9.2.5, 10.9.2.6, 10.9.2.7, 10.9.2.8, 10.9.2.9, 10.9.2.10, 10.9.3.1, 10.9.3.2, 10.9.3.3, 10.9.3.4, 10.9.3.5, 10.9.3.6, 10.9.3.7, 10.9.3.8, 10.9.3.9, 10.9.3.10, 10.9.4.1, 10.9.4.2, 10.9.4.3, 10.9.4.4, 10.9.4.5, 10.9.4.6, 10.9.4.7, 10.9.4.8, 10.9.4.9, 10.9.4.10, 10.9.5.1, 10.9.5.2, 10.9.5.3, 10.9.5.4, 10.9.5.5, 10.9.5.6, 10.9.5.7, 10.9.5.8, 10.9.5.9, 10.9.5.10, 10.9.6.1, 10.9.6.2, 10.9.6.3, 10.9.6.4, 10.9.6.5, 10.9.6.6, 10.9.6.7, 10.9.6.8, 10.9.6.9, 10.9.6.10, 10.9.7.1, 10.9.7.2, 10.9.7.3, 10.9.7.4, 10.9.7.5, 10.9.7.6, 10.9.7.7, 10.9.7.8, 10.9.7.9, 10.9.7.10, 10.9.8.1, 10.9.8.2, 10.9.8.3, 10.9.8.4, 10.9.8.5, 10.9.8.6, 10.9.8.7, 10.9.8.8, 10.9.8.9, 10.9.8.10, 10.9.9.1, 10.9.9.2, 10.9.9.3, 10.9.9.4, 10.9.9.5, 10.9.9.6, 10.9.9.7, 10.9.9.8, 10.9.9.9, 10.9.9.10, 10.9.10.1, 10.9.10.2, 10.9.10.3, 10.9.10.4, 10.9.10.5, 10.9.10.6, 10.9.10.7, 10.9.10.8, 10.9.10.9, 10.9.10.10, 10.10.1.1, 10.10.1.2, 10.10.1.3, 10.10.1.4, 10.10.1.5, 10.10.1.6, 10.10.1.7, 10.10.1.8, 10.10.1.9, 10.10.1.10, 10.10.2.1, 10.10.2.2, 10.10.2.3, 10.10.2.4, 10.10.2.5, 10.10.2.6, 10.10.2.7, 10.10.2.8, 10.10.2.9, 10.10.2.10, 10.10.3.1, 10.10.3.2, 10.10.3.3, 10.10.3.4, 10.10.3.5, 10.10.3.6, 10.10.3.7, 10.10.3.8, 10.10.3.9, 10.10.3.10, 10.10.4.1, 10.10.4.2, 10.10.4.3, 10.10.4.4, 10.10.4.5, 10.10.4.6, 10.10.4.7, 10.10.4.8, 10.10.4.9, 10.10.4.10, 10.10.5.1, 10.10.5.2, 10.10.5.3, 10.10.5.4, 10.10.5.5, 10.10.5.6, 10.10.5.7, 10.10.5.8, 10.10.5.9, 10.10.5.10, 10.10.6.1, 10.10.6.2, 10.10.6.3, 10.10.6.4, 10.10.6.5, 10.10.6.6, 10.10.6.7, 10.10.6.8, 10.10.6.9, 10.10.6.10, 10.10.7.1, 10.10.7.2, 10.10.7.3, 10.10.7.4, 10.10.7.5, 10.10.7.6, 10.10.7.7, 10.10.7.8, 10.10.7.9, 10.10.7.10, 10.10.8.1, 10.10.8.2, 10.10.8.3, 10.10.8.4, 10.10.8.5, 10.10.8.6, 10.10.8.7, 10.10.8.8, 10.10.8.9, 10.10.8.10, 10.10.9.1, 10.10.9.2, 10.10.9.3, 10.10.9.4, 10.10.9.5, 10.10.9.6, 10.10.9.7, 10.10.9.8, 10.10.9.9, 10.10.9.10, 10.10.10.1, 10.10.10.2, 10.10.10.3, 10.10.10.4, 10.10.10.5, 10.10.10.6, 10.10.10.7, 10.10.10.8. 10.10.10.9. 10.10.10.10 |

Additional exemplary compound groups include the following compound groups disclosed below. Unless otherwise specified, the configurations of all hydrogen atoms and R groups for the following compound groups are as defined for the group 1 compounds above. As is apparent from the description, each of the compound groups disclose a number of unique compounds or generic structures. The compounds or generic structures specifically described in any of the compound groups are thus exemplary only and the remaining compounds or structures in each group are described by Tables A and B.

As used in the description of compounds in the compound groups, the definitive structure of compounds in the various compound groups is specified only by the structure defining portion of the compound group and in Tables A and B, which together definitively name or specify individual compound or genus structures. The structure-defining portion of the compound groups is generally contained in the first sentence of the compound groups below and in the following paragraph. This applies regardless of any name or structure, including chemical names in the exemplary compounds that are named in some of the compound groups. Thus, any name or structure for any compound or compound genus that refers to a compound or genus in a compound group and is given anywhere in the disclosure is intended only to refer to the compound or genus that is definitively specified by the compound groups together with Tables A and B.

For the following compound groups, reference to an androstene or a 5α-androstene with no double bond at the 4-5 or 5-6 position means that the hydrogen atom or other moiety at the 5-position is in the α-configuration. For androstenes with no double bond at the 4-5 or 5-6 position and a hydrogen atom or other moiety at the 5-position in the β-configuration will usually be referred to as a 5β-androstene. For compound groups where a double bond is present at the 1-2 or 2-3 position and/or when R⁹ is substituted, R⁹ will be =CH-, =CR¹⁰-, -CHR¹⁰-, -C(R¹⁰)₂- or another moiety defined for R⁹ herein, instead of -CH₂-. For compound groups where a double bond is present at the 9-11 position and/or when R⁸ is substituted, R⁸ will be =CH-, =CR¹⁰-, -CHR¹⁰-, - C(R¹⁰)₂- or another moiety defined for R⁸ herein, instead of -CH₂-. 9-11 and/or 15-16 positions. For compound groups where a double bond is present at the 15-16 position and/or when R⁷ is substituted, R⁷ will be =CH-, =CR¹⁰-, -CHR¹⁰-, -C(R¹⁰)₂- or another moiety defined for R⁷ herein, instead of -CH₂-.

**Group 2**. This group comprises compounds named in Table B having R¹, R², R³ and R⁴ substituents defined in Table A wherein the R¹, R², R³ and R⁴ substituents are bonded to the steroid nucleus described for group 1 compounds, except that R^{10E} is hydrogen in the β-configuration. Exemplary group 2 compounds include 1.2.4.1, which is 3,7β-dihydroxy-16α-fluoro-17β-amino-5β-androst-1,3-diene, 1.1.5.9, which is 3,17β-dihydroxy-5β-androst-1,3-diene, 1.1.7.1, which is 3-hydroxy-16α-acetoxy-17β-amino-5β-androst-1,3-diene and compound 1.1.4.10, which is 3-hydroxy-16α-fluoro-17β-acetoxy-5β-androst-1,3-diene. Other exemplary group 2 compounds include 3,17β-dihydroxy-7β-acetoxy-5β-androst-1,3-diene, 3,17β-dihydroxy-7β-methyl-5β-androst-1,3-diene, 3,17β-dihydroxy-7β-methoxy-5β-androst-1,3-diene, 3,7β,17β-trihydroxy-5β-androst-1,3-diene, 3-amino-17β-hydroxy-5β-androst-1,3-diene, 3-amino-7β,17β-dihydroxy-5β-androst-1,3-diene, 3-hydroxy-17β-amino-5β-androst-1,3-diene, 3,7β-dihydroxy-17β-amino-5β-androst-1,3-diene, 3,17β-dihydroxy-7β-amino-5β-androst-1,3-diene, 3-hydroxy-7β,17β-diacetylamino-5β-androst-1,3-diene, 3-hydroxy-7β,17β-dimethylamino-5β-androst-1,3-diene and 16α-hydroxy, 16α-methyl, 16α-amino, 16α-aminomethyl, 16α-acetate and 16α-halo analogs of any of these compounds.

**Group 3**. This group comprises compounds named in Table B having R¹, R², R³ and R⁴ substituents defined in Table A wherein the R¹, R², R³ and R⁴ substituents are bonded to the steroid nucleus described for group 1 compounds, except that R^{10E} is absent and double bonds are present at the 1-2, 3-4 and 5-6 positions. Exemplary group 3 compounds include 1.2.4.1, which is 3,7β-dihydroxy-16α-fluoro-17β-aminoandrost-1,3,5-triene, 1.1.5.9, which is 3,17β-dihydroxyandrost-1,3,5-triene, 1.1.7.1, which is 3-hydroxy-16α-acetoxy-17β-aminoandrost-1,3,5-triene and compound 1.1.4.10, which is 3-hydroxy-16α-fluoro-17β-acetoxyandrost-1,3,5-triene. Other exemplary group 3 compounds include 3,17β-dihydroxy-7β-acetoxyandrost-1,3,5-triene, 3,17β-dihydroxy-7β-methylandrost-1,3,5-triene, 3,17β-dihydroxy-7β-methoxyandrost-1,3,5-triene, 3,7β,17β-trihydroxyandrost-1,3,5-triene, 3-amino-17β-hydroxyandrost-1,3,5-triene, 3-amino-7β,17β-dihydroxyandrost-1,3,5-triene, 3-hydroxy-17β-aminoandrost-1,3,5-triene, 3,7β-dihydroxy-17β-aminoandrost-1,3,5-triene, 3,17β-dihydroxy-7β-aminoandrost-1,3,5-triene, 3-hydroxy-7β,17β-diacetylaminoandrost-1,3,5-triene, 3-hydroxy-7β,17β-dimethylaminoandrost-1,3,5-triene and 16α-hydroxy, 16α-methyl, 16α-amino, 16α-aminomethyl, 16α-acetate and 16α-halo analogs of any of these compounds.

**Group 29**. This group comprises compounds named in Table B having R¹, R², R³ and R⁴ substituents defined in Table A wherein the R¹, R², R³ and R⁴ substituents are bonded to the steroid nucleus described for group 1 compounds, except that R^{10E} and R⁶ are absent and double bonds are present at the 1-2, 3-4 and 5-10 positions, i.e., the A ring is aromatic. Exemplary group 29 compounds include 1.2.4.1, which is 3,7β-dihydroxy-16α-fluoro-17β-aminoandrost-1,3,5(10)-triene, 1.1.5.9, which is 3,17β-dihydroxyandrost-1,3,5(10)-triene, 1.1.7.1, which is 3-hydroxy-16α-acetoxy-17β-aminoandrost-1,3,5(10)-triene and compound 1.1.4.10, which is 3-hydroxy-16α-fluoro-17β-acetoxyandrost-1,3,5(10)-triene. Other exemplary group 29 compounds include 3,17β-dihydroxy-7β-acetoxyandrost-1,3,5(10)-triene, 3,17β-dihydroxy-7β-methylandrost-1,3,5(10)-triene, 3,17β-dihydroxy-7β-methoxyandrost-1,3,5(10)-triene, 3,7p,17p-trihydroxyandrost-1,3,5(10)-triene, 3-amino-17β-hydroxyandrost-1,3,5(10)-triene, 3-amino-7β,17β-dihydroxyandrost-1,3,5(10)-triene, 3-hydroxy-17β-aminoandrost-1,3,5(10)-triene, 3,7β-dihydroxy-17β-aminoandrost-1,3,5(10)-triene, 3,17β-dihydroxy-7β-aminoandrost-1,3,5(10)-triene, 3-hydroxy-7β,17β-diacetylaminoandrost-1,3,5(10)-triene, 3-hydroxy-7β,17β-dimethylaminoandrost-1,3,5(10)-triene and 16α-hydroxy, 16α-methyl, 16α-amino, 16α-aminomethyl, 16α-acetate and 16α-halo analogs of any of these compounds.

**Group 34**. This group comprises compounds named in Table B having R¹, R², R³ and R⁴ substituents defined in Table A wherein the R¹, R², R³ and R⁴ substituents are bonded to the steroid nucleus described for group 1 compounds, except that R^{10E} and R⁶ are absent and double bonds are present at the 1-2, 3-4, 5-10 and 6-7 positions. Thus, for this group, the A ring is aromatic and a double bond is present at the 6-7 position. Exemplary group 34 compounds include 1.2.4.1, which is 3,7-dihydroxy-16α-fluoro-17β-aminoandrost-1,3,5(10),6-tetraene, 1.1.5.9, which is 3,17β-dihydroxyandrost-1,3,5(10),6-tetraene, 1.1.7.1, which is 3-hydroxy-16α-acetoxy-17β-aminoandrost-1,3,5(10),6-tetraene and compound 1.1.4.10, which is 3-hydroxy-16α-fluoro-17β-acetoxyandrost-1,3,5(10),6-tetraene. Other exemplary group 34 compounds include 3,17β-dihydroxy-7-acetoxyandrost-1,3,5(10),6-tetraene, 3,17β-dihydroxy-7-methylandrost-1,3,5(10),6-tetraene, 3,17β-dihydroxy-7-methoxyandrost-1,3,5(10),6-tetraene, 3,7,17β-trihydroxyandrost-1,3,5(10),6-tetraene, 3-amino-17β-hydroxyandrost-1,3,5(10),6-tetraene, 3-amino-7,17β-dihydroxyandrost-1,3,5(10),6-tetraene, 3-hydroxy-17β-aminoandrost-1,3,5(10),6-tetraene, 3,7β-dihydroxy-17β-aminoandrost-1,3,5(10),6-tetraene, 3,17β-dihydroxy-7β-aminoandrost-1,3,5(10),6-tetraene, 3-hydroxy-7,17β-diacetylaminoandrost-1,3,5(10),6-tetraene, 3-hydroxy-7,17β-dimethylaminoandrost-1,3,5(10),6-tetraene and 16α-hydroxy, 16α-methyl, 16α-amino, 16α-aminomethyl, 16α-acetate and 16α-halo analogs of any of these compounds.

**Group 35**. This group comprises compounds named in Table B having R¹, R², R³ and R⁴ substituents defined in Table A wherein the R¹, R², R³ and R⁴ substituents are bonded to the steroid nucleus described for group 1 compounds, except that R^{10E}, R^{10F} and R⁶ are absent and double bonds are present at the 1-2, 3-4, 5-10 and 7-8 positions. Thus, for this group, the A ring is aromatic and a double bond is present at the 7-8 position. Exemplary group 35 compounds include 1.2.4.1, which is 3,7-dihydroxy-16α-fluoro-17β-aminoandrost-1,3,5(10),7-tetraene, 1.1.5.9, which is 3,17β-dihydroxyandrost-1,3,5(10),7-tetraene, 1.1.7.1, which is 3-hydroxy-16α-acetoxy-17β-aminoandrost-1,3,5(10),7-tetraene and compound 1.1.4.10, which is 3-hydroxy-16α-fluoro-17β-acetoxyandrost-1,3,5(10),7-tetraene. Other exemplary group 35 compounds include 3,17β-dihydroxy-7-acetoxyandrost-1,3,5(10),7-tetraene, 3,17β-dihydroxy-7-methylandrost-1,3,5(10),7-tetraene, 3,17β-dihydroxy-7-methoxyandrost-1,3,5(10),7-tetraene, 3,7,17β-trihydroxyandrost-1,3,5(10),7-tetraene, 3-amino-17β-hydroxyandrost-1,3,5(10),7-tetraene, 3-amino-7,17β-dihydroxyandrost-1,3,5(10),7-tetraene, 3-hydroxy-17β-aminoandrost-1,3,5(10),7-tetraene, 3,7β-dihydroxy-17β-aminoandrost-1,3,5(10),7-tetraene, 3,17β-dihydroxy-7β-aminoandrost-1,3,5(10),7-tetraene, 3-hydroxy-7,17β-diacetylaminoandrost-1,3,5(10),7-tetraene, 3-hydroxy-7,17β-dimethylaminoandrost-1,3,5(10),7-tetraene and 16α-hydroxy, 16α-methyl, 16α-amino, 16α-aminomethyl, 16α-acetate and 16α-halo analogs of any of these compounds.

**Group 36.** This group comprises compounds named in Table B having R¹, R², R³ and R⁴ substituents defined in Table A wherein the R¹, R², R³ and R⁴ substituents are bonded to the steroid nucleus described for group 1 compounds, except that R^{10E}, R^{10F}, R^{10G} and R⁶ are absent and double bonds are present at the 1-2, 3-4, 5-10 and 8-9 positions. Thus, for this group, the A ring is aromatic and a double bond is present at the 8-9 position. Exemplary group 36 compounds include 1.2.4.1, which is 3,7β-dihydroxy-16α-fluoro-17β-aminoandrost-1,3,5(10),8(9)-tetraene, 1.1.5.9, which is 3,17β-dihydroxyandrost-1,3,5(10),8(9)-tetraene, 1.1.7.1, which is 3-hydroxy-16α-acetoxy-17β-aminoandrost-1,3,5(10),8(9)-tetraene and compound 1.1.4.10, which is 3-hydroxy-16α-fluoro-17β-acetoxyandrost-1,3,5(10),8(9)-tetraene. Other exemplary group 36 compounds include 3,17β-dihydroxy-7β-acetoxyandrost-1,3,5(10),8(9)-tetraene, 3,17β-dihydroxy-7β-methylandrost-1,3,5(10),8(9)-tetraene, 3,17β-dihydroxy-7β-methoxyandrost-1,3,5(10),8(9)-tetraene, 3,7β,17β-trihydroxyandrost-1,3,5(10),8(9)-tetraene, 3-amino-17β-hydroxyandrost-1,3,5(10),8(9)-tetraene, 3-amino-7β,17β-dihydroxyandrost-1,3,5(10),8(9)-tetraene, 3-hydroxy-17β-aminoandrost-1,3,5(10),8(9)-tetraene, 3,7β-dihydroxy-17β-aminoandrost-1,3,5(10),8(9)-tetraene, 3,17β-dihydroxy-7β-aminoandrost-1,3,5(10),8(9)-tetraene, 3-hydroxy-7β,17β-diacetylaminoandrost-1,3,5(10),8(9)-tetraene, 3-hydroxy-7β,17β-dimethylaminoandrost-1,3,5(10),8(9)-tetraene and 16α-hydroxy, 16α-methyl, 16α-amino, 16α-aminomethyl, 16α-acetate and 16α-halo analogs of any of these compounds.

**Group 37.** This group comprises compounds named in Table B having R¹, R², R³ and R⁴ substituents defined in Table A wherein the R¹, R², R³ and R⁴ substituents are bonded to the steroid nucleus described for group 1 compounds, except that R^{10E}, R^{10F}, R^{10H} and R⁶ are absent and double bonds are present at the 1-2, 3-4, 5-10 and 8-14 positions. Thus, for this group, the A ring is aromatic and a double bond is present at the 8-14 position. Exemplary group 37 compounds include 1.2.4.1, which is 3,7β-dihydroxy-16α-fluoro-17β-aminoandrost-1,3,5(10),8(14)-tetraene, 1.1.5.9, which is 3,17β-dihydroxyandrost-1,3,5(10),8(14)-tetraene, 1.1.7.1, which is 3-hydroxy-16α-acetoxy-17β-aminoandrost-1,3,5(10),8(14)-tetraene and compound 1.1.4.10, which is 3-hydroxy-16α-fluoro-17β-acetoxyandrost-1,3,5(10),8(14)-tetraene. Other exemplary group 37 compounds include 3,17β-dihydroxy-7β-acetoxyandrost-1,3,5(10),8(14)-tetraene, 3,17β-dihydroxy-7β-methylandrost-1,3,5(10),8(14)-tetraene, 3,17β-dihydroxy-7β-methoxyandrost-1,3,5(10),8(14)-tetraene, 3,7β,17β-trihydroxyandrost-1,3,5(10),8(14)-tetraene, 3-amino-17β-hydroxyandrost-1,3,5(10),8(14)-tetraene, 3-amino-7β,17β-dihydroxyandrost-1,3,5(10),8(14)-tetraene, 3-hydroxy-17β-aminoandrost-1,3,5(10),8(14)-tetraene, 3,7β-dihydroxy-17β-aminoandrost-1,3,5(10),8(14)-tetraene, 3,17β-dihydroxy-7β-aminoandrost-1,3,5(10),8(14)-tetraene, 3-hydroxy-7β,17β-diacetylaminoandrost-1,3,5(10),8(14)-tetraene, 3-hydroxy-7β,17β-dimethylaminoandrost-1,3,5(10),8(14)-tetraene and 16α-hydroxy, 16α-methyl, 16α-amino, 16α-aminomethyl, 16α-acetate and 16α-halo analogs of any of these compounds.

**Group 38.** This group comprises compounds named in Table B having R¹, R², R³ and R⁴ substituents defined in Table A wherein the R¹, R², R³ and R⁴ substituents are bonded to the steroid nucleus described for group 1 compounds, except that R^{10E} and R⁶ are absent and double bonds are present at the 1-2, 3-4, 5-10 and 15-16 positions. Thus, for this group, the A ring is aromatic and a double bond is present at the 15-16 position. Exemplary group 38 compounds include 1.2.4.1, which is 3,7β-dihydroxy-16-fluoro-17β-aminoandrost-1,3,5(10),15-tetraene, 1.1.5.9, which is 3,17β-dihydroxyandrost-1,3,5(10),15-tetraene, 1.1.7.1, which is 3-hydroxy-16-acetoxy-17β-aminoandrost-1,3,5(10),15-tetraene and compound 1.1.4.10, which is 3-hydroxy-16-fluoro-17β-acetoxyandrost-1,3,5(10),15-tetraene. Other exemplary group 38 compounds include 3,17β-dihydroxy-7β-acetoxyandrost-1,3,5(10),15-tetraene, 3,17β-dihydroxy-7β-methylandrost-1,3,5(10),15-tetraene, 3,17β-dihydroxy-7β-methoxyandrost-1,3,5(10),15-tetraene, 3,7β,17β-trihydroxyandrost-1,3,5(10),15-tetraene, 3-amino-17β-hydroxyandrost-1,3,5(10),15-tetraene, 3-amino-7β,17β-dihydroxyandrost-1,3,5(10),15-tetraene, 3-hydroxy-17β-aminoandrost-1,3,5(10),15-tetraene, 3,7β-dihydroxy-17β-aminoandrost-1,3,5(10),15-tetraene, 3,17β-dihydroxy-7β-aminoandrost-1,3,5(10),15-tetraene, 3-hydroxy-7β,17β-diacetylaminoandrost-1,3,5(10),15-tetraene, 3-hydroxy-7β,17β-dimethylaminoandrost-1,3,5(10),15-tetraene and 16-hydroxy, 16-methyl, 16-amino, 16-aminomethyl, 16-acetate and 16-halo analogs of any of these compounds.

**Group 39**. This group comprises compounds named in Table B having R¹, R², R³ and R⁴ substituents defined in Table A wherein the R¹, R², R³ and R⁴ substituents are bonded to the steroid nucleus described for group 1 compounds, except that R^{10E} and R⁶ are absent and double bonds are present at the 1-2, 3-4, 5-10 and 16-17 positions. Thus, for this group, the A ring is aromatic and a double bond is present at the 15-16 position. Exemplary group 39 compounds include 1.2.4.1, which is 3,7β-dihydroxy-16-fluoro-17-aminoandrost-1,3,5(10),16-tetraene, 1.1.5.9, which is 3,17-dihydroxyandrost-1,3,5(10),16-tetraene, 1.1.7.1, which is 3-hydroxy-16-acetoxy-17-aminoandrost-1,3,5(10),16-tetraene and compound 1.1.4.10, which is 3-hydroxy-16-fluoro-17-acetoxyandrost-1,3,5(10),16-tetraene. Other exemplary group 39 compounds include 3,17-dihydroxy-7β-acetoxyandrost-1,3,5(10),16-tetraene, 3,17-dihydroxy-7β-methylandrost-1,3,5(10),16-tetraene, 3,17-dihydroxy-7β-methoxyandrost-1,3,5(10),16-tetraene, 3,7β,17-trihydroxyandrost-1,3,5(10),16-tetraene, 3-amino-17-hydroxyandrost-1,3,5(10),16-tetraene, 3-amino-7β,17-dihydroxyandrost-1,3,5(10),16-tetraene, 3-hydroxy-17-aminoandrost-1,3,5(10),16-tetraene, 3,7β-dihydroxy-17-aminoandrost-1,3,5(10),16-tetraene, 3-hydroxy-7β,17-diacetylaminoandrost-1,3,5(10),16-tetraene, 3-hydroxy-7β,17-dimethylaminoandrost-1,3,5(10),16-tetraene and 16-hydroxy, 16-methyl, 16-amino, 16-aminomethyl, 16-acetate and 16-halo analogs of any of these compounds.

**Group 47.** This group comprises compounds named in Table B having R¹, R², R³ and R⁴ substituents defined in Table A wherein the R¹, R², R³ and R⁴ substituents are bonded to the steroid nucleus described for group 1 compounds, except that R¹ is in the β-configuration, R^{10E} and R⁶ are absent and a double bond is present at the 5-10 position. Exemplary group 47 compounds include 1.2.4.1, which is 3,7β-dihydroxy-16α-fluoro-17β-aminoandrost-5(10)-ene, 1.1.5.9, which is 3,17β-dihydroxyandrost-5(10)-ene, 1.1.7.1, which is 3-hydroxy-16α-acetoxy-17β-aminoandrost-5(10)-ene and compound 1.1.4.10, which is 3-hydroxy-16α-fluoro-17β-acetoxyandrost-5(10)-ene. Other exemplary group 47 compounds include 3,17β-dihydroxy-7β-acetoxyandrost-5(10)-ene, 3,17β-dihydroxy-7β-methylandrost-5(10)-ene, 3,17β-dihydroxy-7β-methoxyandrost-5(10)-ene, 3,7β,17β-trihydroxyandrost-5(10)-ene, 3-amino-17β-hydroxyandrost-5(10)-ene, 3-amino-7β,17β-dihydroxyandrost-5(10)-ene, 3-hydroxy-17β-aminoandrost-5(10)-ene, 3,7β-dihydroxy-17β-aminoandrost-5(10)-ene, 3,17β-dihydroxy-7β-aminoandrost-5(10)-ene, 3-hydroxy-7β,17β-diacetylaminoandrost-5(10)-ene, 3-hydroxy-7β,17β-dimethylaminoandrost-5(10)-ene and 16α-hydroxy, 16α-methyl, 16α-amino, 16α-aminomethyl, 16α-acetate and 16α-halo analogs of any of these compounds.

**Group 48**. This group comprises compounds named in Table B having R¹, R², R³ and R⁴ substituents defined in Table A wherein the R¹, R², R³ and R⁴ substituents are bonded to the steroid nucleus described for group 1 compounds, except that R¹ is in the β-configuration, R^{10E} and R⁶ are absent and double bonds are present at the 5-10 and 15-16 positions. Exemplary group 48 compounds include 1.2.4.1, which is 3,7β-dihydroxy-16-fluoro-17β-aminoandrost-5(10),15-diene, 1.1.5.9, which is 3,17β-dihydroxyandrost-5(10),15-diene, 1.1.7.1, which is 3-hydroxy-16-acetoxy-17β-aminoandrost-5(10),15-diene and compound 1.1.4.10, which is 3-hydroxy-16-fluoro-17β-acetoxyandrost-5(10),15-diene. Other exemplary group 48 compounds include 3,17p-dihydroxy-7β-acetoxyandrost-5(10),15-diene, 3,17β-dihydroxy-7β-methylandrost-5(10),15-diene, 3,17β-dihydroxy-7β-methoxyandrost-5(10),15-diene, 3,7β, 17β-trihydroxyandrost-5(10),15-diene, 3-amino-17β-hydroxyandrost-5(10),15-diene, 3-amino-7β,17β-dihydroxyandrost-5(10),15-diene, 3-hydroxy-17β-aminoandrost-5(10),15-diene, 3,7β-dihydroxy-17β-aminoandrost-5(10),15-diene, 3,17β-dihydroxy-7β-aminoandrost-5(10),15-diene, 3-hydroxy-7β,17β-diacetylaminoandrost-5(10),15-diene, 3-hydroxy-7β,17β-dimethylaminoandrost-5(10),15-diene and 16-hydroxy, 16-methyl, 16-amino, 16-aminomethyl, 16-acetate and 16-halo analogs of any of these compounds.

**Group 49**. This group comprises compounds named in Table B having R¹, R², R³ and R⁴ substituents defined in Table A wherein the R¹, R², R³ and R⁴ substituents are bonded to the steroid nucleus described for group 1 compounds, except that R¹ is in the β-configuration, R^{10E} and R⁶ are absent and double bonds are present at the 5-10 and 16-17 positions. Exemplary group 49 compounds include 1.2.4.1, which is 3β,7β-dihydroxy-16-fluoro-17-aminoandrost-5(10),16-diene, 1.1.5.9, which is 3β,17-dihydroxyandrost-5(10),16-diene, 1.1.7.1, which is 3β-hydroxy-16-acetoxy-17-aminoandrost-5(10),16-diene and compound 1.1.4.10, which is 3β-hydroxy-16-fluoro-17-acetoxyandrost-5(10),16-diene. Other exemplary group 49 compounds include 3β,17-dihydroxy-7β-acetoxyandrost-5(10),16-diene, 3β,17-dihydroxy-7β-methylandrost-5(10),16-diene, 3β,17-dihydroxy-7β-methoxyandrost-5(10),16-diene, 3β,7β,17-trihydroxyandrost-5(10),16-diene, 3β-amino-17-hydroxyandrost-5(10),16-diene, 3β-amino-7β,17-dihydroxyandrost-5(10),16-diene, 3β-hydroxy-17-aminoandrost-5(10),16-diene, 3β,7β-dihydroxy-17-aminoandrost-5(10),16-diene, 3β,17-dihydroxy-7β-aminoandrost-5(10),16-diene, 3β-hydroxy-7β,17-diacetylaminoandrost-5(10),16-diene, 3β-hydroxy-7β,17-dimethylaminoandrost-5(10),16-diene and 16-hydroxy, 16-methyl, 16-amino, 16-aminomethyl, 16-acetate and 16-halo analogs of any of these compounds.

**Group 50.** This group comprises compounds in compound groups 1-49 described above where no double bond is present at the 16-17 position, i.e., groups 1-3, 6-16, 19-24, 27-32, 34-38 and 40-48, and R⁴ is in the α-configuration instead of in the β-configuration. These compound groups are specified by adding group number 50- to the included group numbers. Thus, for example, compounds in group 50-1 are compounds in group 1 where R⁴ is in the α-configuration. Similarly, compounds in group 50-2 are compounds in group 2 where R⁴ is in the α-configuration and compounds in group 50-3 are compounds in group 3 where R⁴ is in the α-configuration. Other group 50 compound groups where R⁴ is in the α-configuration are defined in a similar manner and therefore are 50-6, 50-7, 50-8, 50-9, 50-10, 50-11, 50-12, 50-13, 50-14, 50-15, 50-16, 50-19, 50-20, 50-21, 50-22, 50-23, 50-24, 50-27, 50-28, 50-29, 50-30, 50-31, 50-32, 50-34, 50-35, 50-36, 50-37, 50-38, 50-40, 50-41, 50-42, 50-43, 50-44, 50-45, 50-46, 50-47 and 50-48. For each of these compound groups, compounds 1.1.1.1 through 10.10.10.10 in Table B specifies a compound as defined by the Table A substituents and the R⁴ a-configuration as specified in this group.

Exemplary group 50-1 compounds include 1.2.4.1, which is 3,7β-dihydroxy-16α-fluoro-17α-aminoandrost-1,3-diene, 1.1.5.9, which is 3,17α-dihydroxyandrost-1,3-diene, 1.1.6.1, which is 3,16α-dihydroxy-17α-aminoandrost-1,3-diene and 1.1.4.9, which is 3,17α-dihydroxy-16α-fluoroandrost-1,3-diene. Exemplary group 50-2 compounds include 1.2.4.1, which is 3,7β-dihydroxy-16α-fluoro-17α-amino-5β-androst-1,3-diene, 1.1.5.9, which is 3,17α-dihydroxy-5β-androst-1,3-diene, 1.1.6.1, which is 3,16α-dihydroxy-17α-amino-5β-androst-1,3-diene and 1.1.4.9, which is 3,17α-dihydroxy-16α-fluoro-5β-androst-1,3-diene. Exemplary group 50-3 compounds include 1.2.4.1, which is 3,7β-dihydroxy-16α-fluoro-17α-aminoandrost-1,3,5-triene, 1.1.5.9, which is 3,17α-dihydroxyandrost-1,3,5-triene, 1.1.6.1, which is 3,16α-dihydroxy-17α-aminoandrost-1,3,5-triene and 1.1.4.9, which is 3,17α-dihydroxy-16α-fluoroandrost-1,3,5-triene. Exemplary group 50-48 compounds include 1.2.4.1, which is 3β,7β-dihydroxy-16α-fluoro-17α-aminoandrost-5(10),15-diene, 1.1.5.9, which is 3β,17α-dihydroxyandrost-5(10),15-diene, 1.1.6.1, which is 3β,16α-dihydroxy-17α-aminoandrost-5(10),15-diene and 1.1.4.9, which is 3β,17α-dihydroxy-16α-fluoroandrost-5(10),15-diene. Compounds in the other group 50 compound groups are specified or defined in an analogous manner.

**Group 51.** This group comprises compounds in compound groups 1-50 described above, wherein no double bond is present at the 2-3 or 3-4 positions and R¹ is in the α-configuration instead of in the β-configuration, i.e., groups 6 through 24, 30 through 33, 40 through 45, 47 through 49, 50-6 through 50-16, 50-19 through 50-24, 50-30 through 50-32, 50-40 through 50-45, 50-47 and 50-48. These compound groups are specified in a manner that is similar to that described for group 50, i.e., by adding group number 51- to the included group numbers. Thus, compounds in group 51-6 are compounds in group 6 where R¹ is in the α-configuration, compounds in group 51-7 are compounds in group 7 where R¹ is in the α-configuration, compounds in group 51-47 are compounds in group 47 where R¹ is in the α-configuration are compounds in group where R¹ is in the α-configuration, group 51-50-6 are compounds in group 50-6 where R¹ is in the α-configuration, group 51-50-7 are compounds in group 50-7 where R¹ is in the α-configuration, group 51-50-47 are compounds in group 50-47 where R¹ is in the α-configuration and group 51-50-48 are compounds in group 50-48 where R¹ is in the α-configuration. Other group 51 compound groups where R¹ is in the α-configuration are defined in a similar manner and therefore are 51-8, 51-9, 51-10, 51-11, 51-12, 51-13, 51-14, 51-15, 51-16, 51-17, 51-18, 51-19, 51-20, 51-21, 51-22, 51-23, 51-24, 51-30, 51-31, 51-32, 51-33, 51-40, 51-41, 51-42, 51-43, 51-44, 51-45, 51-47, 51-48, 51-49, 51-50-6, 51-50-7, 51-50-8, 51-50-9, 51-50-10, 51-50-11, 51-50-12, 51-50-13, 51-50-14, 51-50-15, 51-50-16, 51-50-19, 51-50-20, 51-50-21, 51-50-22, 51-50-23, 51-50-24, 51-50-30, 51-50-31, 51-50-32, 51-50-40, 51-50-41, 51-50-42, 51-50-43, 51-50-44, 51-50-45, 51-50-47 and 51-50-48. For each of these compound groups, compounds 1.1.1.1 through 10.10.10.10 in Table B specifies a compound as defined by the Table A substituents and the R¹ α-configuration as specified in this group.

Exemplary group 51-6 compounds include 1.2.4.1, which is 3α,7β-dihydroxy-16α-fluoro-17β-aminoandrost-1,5-diene, 1.1.5.9, which is 3α,17β-dihydroxyandrost-1,5-diene, 1.1.6.1, which is 3α,16α-dihydroxy-17β-aminoandrost-1,5-diene and 1.1.4.9, which is 3α,17β-dihydroxy-16α-fluoroandrost-1,5-diene. Exemplary group 51-7 compounds include 1.2.4.1, which is 3α,7-dihydroxy-16α-fluoro-17β-aminoandrost-1,6-diene, 1.1.5.9, which is 3α,17β-dihydroxyandrost-1,6-diene, 1.1.6.1, which is 3α,16α-dihydroxy-17β-aminoandrost-1,6-diene and 1.1.4.9, which is 3α,17β-dihydroxy-16α-fluoroandrost-1,6-diene. Exemplary group 51-50-47 compounds include 1.2.4.1, which is 3α,7β-dihydroxy-16α-fluoro-17a-aminoandrost-5(10)-ene, 1.1.5.9, which is 3α,17α-dihydroxyandrost-5(10)-ene, 1.1.6.1, which is 3α,16α-dihydroxy-17α-aminoandrost-5(10)-ene and 1.1.4.9, which is 3α,17α-dihydroxy-16α-fluoroandrost-5(10)-ene. Exemplary group 51-50-48 compounds include 1.2.4.1, which is 3α,7β-dihydroxy-16α-fluoro-17α-aminoandrost-5(10),15-diene, 1.1.5.9, which is 3α,17α-dihydroxyandrost-5(10),15-diene, 1.1.6.1, which is 3α,16α-dihydroxy-17α-aminoandrost-5(10),15-diene and 1.1.4.9, which is 3α,17α-dihydroxy-16α-fluoroandrost-5(10),15-diene. Compounds in the other group 51 compound groups are defined in an analogous manner.

**Group 52.** This group comprises compounds in compound groups 1-51 described above, wherein no double bond is present at the 15-16 or 16-17 positions and R³ is in the β-configuration instead of in the α-configuration, i.e., groups 1 through 3, 6 through 14, 23, 24, 29 through 37, 41 through 47, 50-1, 50-2, 50-3, 50-6 through 50-14, 50-23, 50-24, 50-29, 50-30, 50-31, 50-34 through 50-37, 50-41 through 50-47, 51-6 through 51-14, 51-23, 51-24, 51-30, 51-31, 51-41 through 51-45 and 51-47. Compound groups in group 52 where R³ is in the β-configuration are 52-1, 52-2, 52-3, 52-6, 52-7, 52-8, 52-9, 52-10, 52-11, 52-12, 52-13, 52-14, 52-23, 52-24, 52-29, 52-30, 52-31, 52-32, 52-33, 52-34, 52-35, 52-36, 52-37, 52-41, 52-42, 52-43, 52-44, 52-45, 52-46, 52-47, 52-50-1, 52-50-2, 52-50-3, 52-50-6, 52-50-7, 52-50-8, 52-50-9, 52-50-10, 52-50-11, 52-50-12, 52-50-13, 52-50-14, 52-50-23, 52-50-24, 52-50-29, 52-50-30, 52-50-31, 52-50-34, 52-50-35, 52-50-36, 52-50-37, 52-50-41, 52-50-42, 52-50-43, 52-50-44, 52-50-45, 52-50-46, 52-50-47, 52-51-6, 52-51-7, 52-51-8, 52-51-9, 52-51-10, 52-51-11, 52-51-12, 52-51-13, 52-51-14, 52-51-23, 52-51-24, 52-51-30, 52-51-31, 52-51-41, 52-51-42, 52-51-43, 52-51-44, 52-51-45, 52-51-47, 52-51-50-6, 52-51-50-7, 52-51-50-8, 52-51-50-9, 52-51-50-10, 52-51-50-11, 52-51-50-12, 52-51-50-13, 52-51-50-14, 52-51-50-23, 52-51-50-24, 52-51-50-30, 52-51-50-31, 52-51-50-41, 52-51-50-42, 52-51-50-43, 52-51-50-44, 52-51-50-45 and 52-51-50-47. For each of these compound groups, compounds 1.1.1.1 through 10.10.10.10 in Table B specifies a compound as defined by the Table A substituents and the R³ β-configuration as specified in this group.

These compound groups are specified in a manner that is similar to that described for groups 50 and 51, i.e., by adding group number 52- to the included group numbers. Thus, for example, compounds in group 52-1 are compounds in group 1 where R³ is in the β-configuration, compounds in group 52-6 are compounds in group 6 where R³ is in the β-configuration, compounds in group 52-7 are compounds in group 7 where R³ is in the β-configuration compounds in group 52-50-1 are compounds in group 50-1 where R³ is in the β-configuration, compounds in group 52-51-50-6 are compounds in group 51-50-6 where R³ is in the β-configuration and group 52-51-50-47 are compounds in group 51-50-47 where R³ is in the β-configuration.

Exemplary group 52-6 compounds include 1.2.4.1, which is 3β,7β-dihydroxy-16β-fluoro-17β-aminoandrost-1,5-diene, 1.1.6.9, which is 3β,16β,17β-trihydroxyandrost-1,5-diene, 1.1.6.1, which is 3β,16β-dihydroxy-17β-aminoandrost-1,5-diene and 1.1.4.9, which is 3β,17β-dihydroxy-16β-fluoroandrost-1,5-diene. Exemplary group 52-50-7 compounds include 1.2.4.1, which is 3β,7-dihydroxy-16β-fluoro-17α-aminoandrost-1,6-diene, 1.1.6.9, which is 3β,16β,17α-dihydroxyandrost-1,6-diene, 1.1.6.1, which is 3β,16β-dihydroxy-17α-aminoandrost-1,6-diene and 1.1.4.9, which is 3β,17α-dihydroxy-16β-fluoroandrost-1,6-diene. Exemplary group 52-50-8 compounds include 1.2.4.1, which is 3β,7-dihydroxy-16β-fluoro-17α-amino-5β-androst-1,6-diene, 1.1.6.9, which is 3β,16β,17α-dihydroxy-5β-androst-1,6-diene, 1.1.6.1, which is 3β,16β-dihydroxy-17α-amino-5β-androst-1,6-diene and 1.1.4.9, which is 3β,17α-dihydroxy-16β-fluoro-5β-androst-1,6-diene. Exemplary group 52-51-7 compounds include 1.2.4.1, which is 3α,7-dihydroxy-16β-fluoro-17β-aminoandrost-1,6-diene, 1.1.6.9, which is 3α,16β,17β-dihydroxyandrost-1,6-diene, 1.1.6.1, which is 3α,16β-dihydroxy-17β-aminoandrost-1,6-diene and 1.1.4.9, which is 3α,17β-dihydroxy-16β-fluoroandrost-1,6-diene. Exemplary group 52-51-50-7 compounds include 1.2.4.1, which is 3α,7-dihydroxy-16β-fluoro-17α-aminoandrost-1,6-diene, 1.1.6.9, which is 3α,16β,17α-dihydroxyandrost-1,6-diene, 1.1.6.1, which is 3α,16β-dihydroxy-17α-aminoandrost-1,6-diene and 1.1.4.9, which is 3α,17α-dihydroxy-16β-fluoroandrost-1,6-diene. Exemplary group 52-51-47 compounds include 1.2.4.1, which is 3α,7β-dihydroxy-16β-fluoro-17β-aminoandrost-5(10)-ene, 1.1.6.9, which is 3α,16β,17β-dihydroxyandrost-5(10)-ene, 1.1.6.1, which is 3α,16β-dihydroxy-17β-aminoandrost-5(10)-ene and 1.1.4.9, which is 3α,17β-dihydroxy-16β-fluoroandrost-5(10)-ene. Exemplary group 52-51-50-47 compounds include 1.2.4.1, which is 3α,7β-dihydroxy-16β-fluoro-17a-aminoandrost-5(10)-ene, 1.1.6.9, which is 3α,16β,17α-dihydroxyandrost-5(10)-ene, 1.1.6.1, which is 3α,16β-dihydroxy-17α-aminoandrost-5(10)-ene and 1.1.4.9, which is 3α,17α-dihydroxy-16β-fluoroandrost-5(10)-ene. Compounds in the other group 52 compound groups are defined in an analogous manner.

**Group 57**. This group comprises compounds in the compound groups 1 through 56-55-54-53-52-51-50-47 described above, wherein 1, 2, 3 or 4 of R¹, R², R³ and R⁴ are a moiety defined herein other than one of the moieties listed in Table A, with exemplary moieties as described in the following paragraphs (1) through (15). Moieties or groups listed in paragraphs (1) through (15) such as optionally substituted alkyl, optionally substituted alkylamine, O-linked carbamate, N-linked carbamate and N-linked amino acid ester include the exemplary groups described (a) in the following paragraphs and (b) elsewhere herein. Optionally substituted alkyl groups for any of the moieties described in paragraphs (1) through (12) will typically be a C1-20, a C1-12 or a C1-6 optionally substituted alkyl group that is (i) optionally substituted with 1, 2, 3, 4, 5, 6 or more independently selected substitutions as described herein and (ii) saturated or unsaturated with 1, 2, 3 or more independently selected -CH₂=C_{H2}-, -CHR^{10A}=CHR^{10B}-, - CH₂≡CH₂-, -CHR^{10A}≡CHR^{10B}-, where R^{10K} and R^{10L} independently are an R¹⁰ moiety as defined for F1Cs, e.g., they can be independently selected -H, C1-C6 optionally substituted alkyl, C1-6 ether, C1-6 thioether, -NH-C1-6 optionally substituted alkyl, halogen or another R¹⁰ moiety described elsewhere herein. Similarly, other organic moieties, e.g., carbamates, esters, thioesters or carbonates, will typically be a C1-20, a C1-12 or a C1-6 organic moiety that is optionally substituted with 1, 2, 3, 4, 5, 6 or more independently selected substitutions as described herein, e.g., for substituted alkyl groups.

(1) Compounds in any of the foregoing groups 1 through 56-55-54- 53-52-51-50-47 where R¹ moieties 1 through 10 in Table A are replaced with the following moieties: 1 is -Z-optionally substituted alkyl, 2 is an ester (e.g., -O-C(O)-(CH₂)ₙ-CH₃, -O-C(O)-(CH₂)ₙ-NH₂, -O-C(O)-(CH₂)ₙ-N(R^{PR})₂, -O-C(O)-(CH₂)ₙ- CH₂ZR^{PR}, -O-C(O)-CH(ZR^{PR})-(CH₂)ₙ-CH₃ or another ester described herein, where n is 0, 1 , 2, 3, 4, 5, 6, 7 or 8, Z independently are -NH-, oxygen or sulfur and R^{PR} independently or together are - H, a protecting group or a counterion, e.g., methoxymethyl, -CH₃ Or -C₂H₅), 3 is a thioester (e.g., -S-C(O)-(CH₂)ₙ-CH₃, -S-C(O)-(CH₂)ₙ-NH₂, -S-C(O)-(CH₂)ₙ-NHR^{PR}, -S-C(O)-(CH₂)ₙ-CH₂ZR^{PR}, -S-C(O)-CH(ZR^{PR})-(CH₂)ₙ-CH₃ or another thioester described herein, where n is 0, 1 , 2, 3, 4, 5, 6, 7 or 8, Z independently are -NH-, oxygen (-O-) or sulfur (-S-) and R^{PR} is -H or a protecting group, e.g., -CH₃ or -C₂H₅), 4 is a carbonate (e.g., -O-C(O)-O-optionally substituted alkyl), 5 is optionally substituted alkylamine (e.g., -NH-optionally substituted alkyl), 6 is optionally substituted dialkylamine (e.g., - N(optionally substituted alkyl)₂, where each optionally substituted alkyl is independently chosen), 7 is an N linked carbamate (e.g., -NH-C(O)-O-optionally substituted alkyl or-NH-C(O)-OH), 8 is an O linked carbamate (e.g., -O-C(O)-NH₂ or-O-C(O)-NH-optionally substituted alkyl), 9 is -O-optionally substituted monosaccharide and 10 is -H. Exemplary optionally substituted alkyl groups for any of these moieties include -CH₂CH₂-O-CH₃, - CH₂CH₂-S-CH₃, -CH(ZR^{PR})-(CH₂)ₙ-CH₂ZR^{PR}, -CH(ZR^{PR})-(CH₂)ₙ-CH₂NHR^{PR}, -CH₂-(CH₂)ₙ-C(O)OR^{PR}, -CH₂-(CH₂)ₙ-C(O)SR^{PR}, -C H₂-(CH₂)ₙ-C(O)NHR^{PR}, or any alkyl, alkenyl or alkynyl moiety described herein, e.g., any of which optionally having 1, 2, 3, 4, 5, 6, 7, 8, 9, 10, 11, 12 or more carbon atoms, with any of these being optionally substituted with 1 , 2, 3, 4, 5, 6 or more independently selected substitutions, where n and Z are as described above.

(3) Compounds in any of the foregoing groups 1 through 56-55-54-53-52-51-50-47 where there is no double bond at the 2-3 or 3-4 position and R¹ moieties 1 through 10 in Table A are replaced with the following moieties: 1 is =O, 2 is =S, 3 is =NOH, 4 is =NOCH₃, 5 is =NOC₂H₅, 6 is =N- optionally substituted alkyl, 7 is =NO-optionally substituted alkyl, 8 is =NH, 9 is =CH₂ and 10 is =C-optionally substituted alkyl. Exemplary group 57(3)-6 (i.e., group 57 paragraph 3 compounds from group 6, which described 1,5-dienes) compounds include compound 1.2.4.1, which is 3-oxo-7β-hydroxy-16α-fluoro-17β-aminoandrost-1,5-diene, 1.1.4.1, which is 3-oxo-16α-fluoro-17β-aminoandrost-1,5-diene, 1.1.5.9, which is 3-oxo-17β-hydroxyandrost-1,5-diene, 1.1.7.1, which is 3-oxo-16α-acetoxy-17β-aminoandrost-1,5-diene and compound 1.1.1.10, which is 3β-hydroxy-16α-bromo-17β-acetoxyandrost-1,5-diene and 16α-hydroxy, 16α-methyl, 16α-amino, 16α-aminomethyl, 16α-acetate and 16α-halo analogs of any of these compounds. Exemplary group 57-7 compounds include compound 1.2.4.1, which is 3-oxo-7-hydroxy-16α-fluoro-17β-aminoandrost-1,6-diene, 1.1.4.1, which is 3-oxo-16α-fluoro-17β-aminoandrost-1,6-diene, 1.1.5.9, which is 3-oxo-17β-dihydroxyandrost-1,6-diene, 1.1.7.1, which is 3-oxo-16α-acetoxy-17β-aminoandrost-1,6-diene and compound 1.1.1.10, which is 3β-hydroxy-16α-bromo-17β-acetoxyandrost-1,6-diene and 16α-hydroxy, 16α-methyl, 16α-amino, 16α-aminomethyl, 16α-acetate and 16α-halo analogs of any of these compounds. Exemplary group 57-8 compounds include compound 1.2.4.1, which is 3-oxo-7-hydroxy-16α-fluoro-17β-amino-5β-androst-1,6-diene, 1.1.4.1, which is 3-oxo-16α-fluoro-17β-amino-5β-androst-1,6-diene, 1.1.5.9, which is 3-oxo-17β-dihydroxy-5β-androst-1,6-diene, 1.1.7.1, which is 3-oxo-16α-acetoxy-17β-amino-5β-androst-1,6-diene and compound 1.1.1.10, which is 3β-hydroxy-16α-bromo-17β-acetoxy-5β-androst-1,6-diene and 16α-hydroxy, 16α-methyl, 16α-amino, 16α-aminomethyl, 16α-acetate and 16α-halo analogs of any of these compounds. Other group 57 compounds include of any of these compounds where R¹ is in the α-configuration, and/or R³ is in the β-configuration and/or R⁴ is in the α-configuration and/or R⁵ is a moiety other than methyl, e.g., -CH₂OH, -CHO, -C₂H₅, -C₃H₇ or another R⁵ described herein and/or R⁶ is a moiety other than methyl, e.g., -H, -F, -Cl, -OH, -SH, - NH₂, -NHR^{PR}, an ester or ether, -CH₂OH, -C-C≡CH, -C₂H₅, -C₃H₇ or another R⁶ described herein and/or R^{10G} is a moiety other than -H, e.g., -F, -Cl, -Br, -CH₃, -OH, -SH, -NHR^{PR} or another R^{10G} moiety described herein and/or R⁸ is a moiety other than methylene, e.g., -O-, -S-, -NH-, =N-, -N(CH₃)-, -N(C₂H₅)-, -CH(α-optionally substituted C1-C6 alkyl)-, -CH(β-optionally substituted C1-C6 alkyl)-, -CH(α-OH)-, -CH(β-OH)-, - C(O)-, -CH(α-SH)-, -CH(β-SH)-, -CH(α-F)-, -CH(β-F)-, -CH(α-I)-, -CH(β-I)- or another R⁸ moiety described herein or R⁸ is absent, leaving a 5-membered ring and/or R⁹ is a moiety other than methylene, e.g., -O-, -S-, -NH-, -N(CH₃)-, -N(C₂H₅)-, -CH(α-optionally substituted C1-C6 alkyl)-, -CH(β-optionally substituted C1-C6 alkyl)-, -CH(α-OH)-, - CH(β-OH)-, -C(O)-, -CH(α-SH)-, -CH(β-SH)-, -CH(α-F)-, -CH(β-F)-, -CH(α-I)-, -CH(β-I)- or another R⁹ moiety described herein or R⁹ is absent, leaving a 5-membered ring. Other exemplary compounds include analogs of any of these compounds where (i) R⁷ is another R⁷ moiety described herein such as -O-, -NH-, =N-, -NCH₃-, -NC₂H₅-, -CH=CH-, -CR¹⁰=CR¹⁰-, -CH₂-CH(α-R¹⁰)-, -CH₂CH(β-R¹⁰)-, -O-, -CH₂-C(β-R¹⁰)(α-R¹⁰)-, -C(β-R¹⁰)(α-R¹⁰)-, where R¹⁰ independently or together are -OH, =O, -NH₂, -NHR^{PR}, -SH, halogen, - C(O)-OR^{PR}, an ester, an ether, C1-C8 optionally substituted alkyl, a heterocycle, a monosaccharide, a polymer or another R¹⁰ moiety described herein or (ii) R^{10H} is a moiety other than -H such as -OH, -OR^{PR}, -SH, -SR^{PR}, -NH₂, -NHR^{PR}, -NHCH₃, -N(CH₃)₂, -CH₃ or C1-C6 optionally substituted alkyl. Other groups and analogous compounds include those in group 57-9, 57-10, 57-11, 57-12, 57-13, 57-14, 57-15, 57-16, 57-17, 57-18, 57-19, 57-20, 57-21, 57-22, 57-23, 57-24, 57-30, 57-31, 57-32, 57-33, 57-40, 57-41, 57-42, 57-43, 57-44, 57-45, 57-46, 57-47, 57-48, 57-49 and analogs or epimers where R¹ is =O, =S or =NOH, and/or R² is in the α-configuration, and/or R³ is in the β-configuration and/or R⁴ is in the α-configuration and/or R⁵ is a moiety other than methyl such as -H, ethyl, ethynyl, 1-propynyl or C2-C6 optionally substituted alkyl and/or R⁶ is a moiety other than methyl such as -H, -F, -Cl, -Br, -OH, -SH, -NH₂, -NHR^{PR}, ethyl, ethynyl, 1-propynyl or C2-C6 optionally substituted alkyl and/or R^{10G} is a moiety other than -H such as -F or -Cl, and/or R⁸ is a moiety other than methylene or R⁸ is absent, leaving a 5-membered ring and/or R⁹ is a moiety other than methylene or R⁹ is absent, leaving a 5-membered ring. In any of these compounds, R^{PR} in dependently or together are -H or a protecting group.

(5) Compounds in any of the foregoing groups 1 through 56-55-54-53-52-51-50-47 and in paragraphs (1), (2), (3) and (4) in this group 57 where R⁴ moieties 1 through 10 in Table A are replaced with the following moieties: 1 is -O-optionally substituted alkyl, 2 is an ester (e.g., -O-C(O)-CH₃, -O-C(O)-CH₂CH₃, -O-C(O)-(CH₂)ₙ-CH₃, -O-C(O)-CF₃, -O-C(O)-(CH₂)ₙ-CF₃, -O-C(O)-(CH₂)ₙ-C(O)OR^{PR}, -O-C(O)-CH₂-C(O)OR^{PR}, -O-C(O)-(CH₂)₂-C(O)OR^{PR}, -O-C(O)-(CH₂)₃-C(O)OR^{PR}, -O-C(O)-(CH₂)₄-C(O)OR^{PR}, -O-C(O)-(CH₂)ₙ-NH₂, -O-C(O)-(CH₂)ₙ-N(R^{PR})₂, -O-C(O)-(CH₂)ₙ-CH₂ZR^{PR}, -O-C(O)-CH(ZR^{PR})-(CH₂)ₙ-CH₃ or another ester described herein, where n is 0, 1 , 2, 3, 4, 5, 6, 7 or 8, Z is - O-, -NH- or -S- and R^{PR} independently or together are -H, a protecting group or a counter ion, e.g., methoxymethyl, Na⁺, K⁺, -CH₃ or -C₂H₅), 3 is a thioester (e.g., -S-C(O)-(CH₂)ₙ-CH₃, -S-C(O)-(CH₂)ₙ-NH₂, -S-C(O)-(CH₂)ₙ-NHR^{PR}, -S-C(O)-(CH₂)ₙ-CH₂ZR^{PR}, -S-C(O)-CH(ZR^{PR})-(CH₂)ₙ-CH₃ or another thioester described herein, where n is 0, 1 , 2, 3, 4, 5, 6, 7 or 8, Z is oxygen or sulfur and R^{PR} is -H or a protecting group, e.g., -CH₃ or - C₂H₅), 4 is a carbonate (e.g., -O-C(O)-O-Optionally substituted alkyl), 5 is optionally substituted alkylamine (e.g., -NH-Optionally substituted alkyl), 6 is optionally substituted dialkylamine (e.g., -N(Optionally substituted alkyl)₂, where each optionally substituted alkyl is independently chosen), 7 is an N linked carbamate (e.g., -NH-C(O)-O-Optionally substituted alkyl or-NH-C(O)-OH), 8 is an O linked carbamate (e.g., -O-C(O)-NH₂ or-O-C(O)-NH-Optionally substituted alkyl), 9 is -O-optionally substituted monosaccharide and 10 is -H. Exemplary optionally substituted alkyl moieties include any such moiety described herein for any variable group and moieties such as -CF₃, -CF₂CF₃, -CH₂CF₃, - CF₂CF₂CF₃, -CH₂CH₂CF₃, -CF₂CF₂CF₂CF₃, -C(O)-CH₃, -C(O)-C₂H₅, -C(O)-C₃H₇, -C(O)-C₄H₉, -C(O)-C₆H₁₃, -CH(OH)-CH₃, -CH(OH)-C₂H₅, -CH(OH)-C₃H₇, -CH(OH)-C₄H₉, - CH(OH)-C₆H₁₃, -C(O)-C₂H₄OR^{PR}, -C(O)-C₃H₆OR^{PR}, -C(O)-C₄H₈OR^{PR}, -C(O)-C₆H₁₃OR^{PR}, -C(O)-C₂H₄SR^{PR}, -C(O)-C₃H₆SR^{PR}, -C(O)-C₄H₈SR^{PR}, -C(O)-C₆H₁₃SR^{PR}, -C(O)-C₂H₄NH^{PR}, -C(O)-C₃H₆NHR^{PR}, -C(O)-C₄H₈NHR^{PR}, -C(O)-C₆H₁₃NHR^{PR}, -C(O)OR^{PR}, - CH₂C(O)OR^{PR}, -CH₂CH₂C(O)OR^{PR}, -C(O)-O-CH₂C(O)OR^{PR} -C(O)-O-CH₂CH₂C(O)OR^{PR}, -C(O)-O- CH₂CH₂CH₂C(O)OR^{PR}, where R^{PR} is -H, a protecting group or a counter ion such as Cl", Na⁺ or K⁺.

(9) Compounds in any of the foregoing groups 1 through 56-55-54- 53-52-51-50-47 and in paragraphs (1), (2), (3), (4), (5), (6), (7) and (8) in this group 57 where R³ moieties 1 through 10 in Table A are replaced with the following moieties: 1 is -O-optionally substituted alkyl, 2 is an ester (e.g., -O-C(O)-(CH₂)ₙ-CH₃, -O-C(O)-(CH₂)ₙ-NH₂, -O-C(O)-(CH₂)ₙ-NHR^{PR}, -O-C(O)-(CH₂)ₙ-CH₂ZR^{PR}, -O-C(O)-CH(ZR^{PR})-(CH₂)ₙ-CH₃ or another ester described herein, where n is 0, 1 , 2, 3, 4, 5, 6, 7 or 8, Z is -NH-, -O- or -S- and R^{PR} independently or together are -H, a protecting group or a counterion, e.g., methoxymethyl, -CH₃ or -C₂H₅), 3 is a thioester (e.g., -S-C(O)-(CH₂)ₙ-CH₃, -S-C(O)-(CH₂)ₙ-NH₂, -S-C(O)-(CH₂)ₙ-N(R^{PR})₂, -S-C(O)-(CH₂)ₙ-CH₂ZR^{PR}, -S-C(O)-CH(ZR^{PR})-(CH₂)ₙ-CH₃ or another thioester described herein, where n is 0, 1 , 2, 3, 4, 5, 6, 7 or 8, Z is -NH-, -O- or - S- and R^{PR} is -H or a protecting group, e.g., -CH₃ or -C₂H₅), 4 is a carbonate (e.g., -O-C(O)-O-Optionally substituted alky]), 5 is optionally substituted alkylamine (e.g., -NH-Optionally substituted alkyl), 6 is optionally substituted dialkylamine (e.g., -N(Optionally substituted alkyl)₂, where each optionally substituted alkyl is independently chosen), 7 is an N linked carbamate (e.g., -NH-C(O)-O-Optionally substituted alkyl or -NH-C(O)-OH), 8 is an O linked carbamate (e.g., -O-C(O)-NH₂ or -O-C(O)-NH-Optionally substituted alkyl), 9 is -O-optionally substituted monosaccharide and 10 is -H.

(12) Compounds in any of the foregoing groups 1 through 56-55-54- 53-52-51-50-47 and in paragraphs (1), (2), (3), (4), (5), (6), (7), (8), (9), (10) and (11) in this group 57 where R² moieties 1 through 10 in Table A are replaced with the following moieties: 1 is -O-optionally substituted alkyl, 2 is an ester (e.g., -O-C(O)-(CH₂)ₙ-CH₃, -O-C(O)-(CH₂)ₙ-NH₂, -O-C(O)-(CH₂)ₙ-NHR^{PR}, -O-C(O)-(CH₂)ₙ, -CH₂ZR^{PR}, -O-C(O)-CH(ZR^{PR})-(CH₂)ₙ-CH₃ or another ester described herein, where n is 0, 1 , 2, 3, 4, 5, 6, 7 or 8, Z is -NH-, -O- or -S- and R^{PR} is -H or a protecting group, e.g., methoxymethyl, -CH₃ or - C₂H₅), 3 is a thioester (e.g., -S-C(O)-(CH₂)ₙ-CH₃, -S-C(O)-(CH₂)ₙ-NH₂, -S-C(O)-(CH₂)ₙ-N(R^{PR})₂, -S-C(O)-(CH₂)ₙ-CH₂ZR^{PR}, -S-C(O)-CH(ZR^{PR})-(CH₂)ₙ-CH₃ or another thioester described herein, where n is 0, 1 , 2, 3, 4, 5, 6, 7 or 8, Z is -NH-, -O- or -S- and R^{PR} independently or together are -H, a protecting group or a counterion, e.g., -CH₃ or - C₂H₅), 4 is a carbonate (e.g., -O-C(O)-O-Optionally substituted alkyl), 5 is optionally substituted alkylamine (e.g., -NH-Optionally substituted alkyl), 6 is optionally substituted dialkylamine (e.g., -N(Optionally substituted alkyl)₂, where each optionally substituted alkyl is independently chosen), 7 is an N linked carbamate (e.g., -NH-C(O)-O-Optionally substituted alkyl or-NH-C(O)-OH), 8 is an O linked carbamate (e.g., -O-C(O)-NH₂ or-O-C(O)-NH-Optionally substituted alkyl), 9 is -O-optionally substituted monosaccharide and 10 is -H.

As is apparent from the F1Cs described in groups 1 through 57, compound groups 14 through 57 contain a number of defined subgroups, e.g., group 14-3 is a subgroup as described for group 14 compounds where R¹, R², R³ and R⁴ can be in the configurations described in group 14, e.g., α,β,α,β, α,α,α,β, β,β,β,β, β,β,β,α or β,β,α,α respectively. Similarly, group 49 includes subgroups such as 49-18-17-14-3, 49-18-17-14-4, 49-18-17-14-5, 49-18-17-14-5A, 49-18-17-14-6, 49-18-17-14-7 and 49-18-17-14-9, which are subgroups where R⁹ is substituted, e.g., R⁹ is -O- or a moiety described in group 18, and such subgroups, although not specifically named or described, are expressly included in group 49. The F1C therefore include all possible subgroups in each group, regardless of whether each subgroup is specifically named or described in a given group or not. For example, groups such as 22, 23, 26, 26B, 26C, 26D and 26E, all include subgroups analogous to those described in group 26A and additional subgroups that are not expressly described, e.g., subgroups such as 26-18-1, 26-18-2, 26-18-3, 26-18-4, 26-18-5, 26-18-5A, 26-18-6, 26-18-14-1, 26-18-14-2, 26-18-14-3, 26-18-14-4, 26-18-14-5, 26-18-14-5A and 26-18-14-6 are not described expressly in group 26 above, but are included in group 26. Similarly, groups 29, 30, 33, 33B, 33C, 33D and 33E, all include subgroups analogous to those described in group 33A, while groups 36, 37, 40B, 40C, 40D, 40E and 41 all include subgroups analogous to those described in group 40A and groups 47B, 47C, 47D, 47E and 48 all include subgroups analogous to those described in group 47A. Thus, subgroups such as 33-18-3 and 33-18-14-3, which are not described expressly in group 33 above, are included in group 33.

The F1Cs include compounds in groups 1 through 57 where R^{10F} and/or R^{10H} is a moiety other than hydrogen, e.g., a halogen, an ether, a thioether, a polymer or optionally substituted alkyl such as -F, -Cl, -Br, -I, -CH₃, -OCH₃, -SCH₃, -OH, -OR^{PR}, -SH, -SR^{PR}, -NH₂ or -NHR^{PR} where R^{PR} independently are -H or a protecting group. Thus, for any of the compounds or genera of compounds in groups 1 through 57, R^{10F} can be -F, - Cl, -CH₃ or -OH in the α- or β-configuration. Similarly, in groups 1 through 57, R^{10H} can be -F, -NH₂, -OH, -SH, -CH₃, -C₂H₅ or -CH₂OH in the α- or β-configuration or an epoxide or cyclopropyl ring with R⁷ where the ring bonds are in the α- or β-configuration.

The F1Cs include analogs of compounds in groups 1 through 57 where R¹¹ is a moiety such as -O-, =N-, -NH-, -NCH₃-, -NC₂H₅-, -S-, -S(O)(O)- or another moiety disclosed herein within the scope of the R¹¹ definition. As is apparent from the F1C structures, when R¹¹ is a moiety such as -O- or -S-, a double bond at the 3-4 or 4-5 position will not be present. Exemplary F1Cs where R¹¹ is one of these moieties includes 3β,17β-dihydroxy-3α-C1-8 optionally substituted alkyl-4-aza-androst-1,5-diene, 3β,17β-dihydroxy-4-aza-androst-1,5-diene, 3α,17β-dihydroxy-3β-C1-8 optionally substituted alkyl-4-aza-androst-1,5-diene, 3α,17β-dihydroxy-4-aza-androst-1,5-diene, 3β-hydroxy-3α-C1-8 optionally substituted alkyl-4-aza-17-thioxoandrost-1,5-diene, 3β-hydroxy-4-aza-17-thioxoandrost-1,5-diene, 3α-hydroxy-3β-C1-8 optionally substituted alkyl-4-aza-17-thioxoandrost-1,5-diene, 3α-hydroxy-4-aza-17-thioxoandrost-1,5-diene, 3β,17β-dihydroxy-3α-C1-8 optionally substituted alkyl-2,4-dioxa-androst-1,5-diene, 3β,17β-dihydroxy-2,4-dioxa-androst-1,5-diene, 3α,17β-dihydroxy-3β-C1-8 optionally substituted alkyl-2,4-dioxa-androst-1,5-diene, 3α,17β-dihydroxy-2,4-dioxa-androst-1,5-diene, 3β,17β-dihydroxy-3α-C1-8 optionally substituted alkyl-4-thia-androst-1,5-diene, 3β,17β-dihydroxy-4-thia-androst-1,5-diene, 3α,17β-dihydroxy-3β-C1-8 optionally substituted alkyl-4-thia-androst-1,5-diene, 3α,17β-dihydroxy-4-thia-androst-1,5-diene, 3β,17β-dihydroxy-3α-C1-8 optionally substituted alkyl-4-oxa-androst-1,5-diene, 3β,17β-dihydroxy-4-oxa-androst-1,5-diene, 3α,17β-dihydroxy-3β-C1-8 optionally substituted alkyl-4-oxa-androst-1,5-diene, 3α,17β-dihydroxy-4-oxa-androst-1,5-diene, 3β,17β-dihydroxy-3α-C1-8 optionally substituted alkyl-4-aza-androstane, 3β,17β-dihydroxy-4-aza-androstane, 3α,17β-dihydroxy-3β-C1-8 optionally substituted alkyl-4-aza-androstane, 3α,17β-dihydroxy-4-aza-androstane, 3β,17β-dihydroxy-3α-C1-8 optionally substituted alkyl-4-aza-5β-androstane, 3β,17β-dihydroxy-4-aza-5β-androstane, 3α,17β-dihydroxy-3β-C1-8 optionally substituted alkyl-4-aza-5β-androstane, 3α,17β-dihydroxy-4-aza-5β-androstane and analogs of any of these compounds where independently selected -OH, -NH₂, -NHCH₃, -SH, -F, -Cl, -Br, -I, C1-8 optionally substituted alkyl or another oxygen-, nitrogen- or sulfur-linked moiety is present at 1, 2 or 3 of the 2-position, the 6-position, the 7-position, the 12-position and/or the 16-position, any of which are in the α- or β-configuration when no double bond is present at the substituted position, or analogs wherein one or more of these positions is substituted with a double bonded moiety such as =O, =S, =NOH, =N-C1-8 optionally substituted alkyl, or =CH-C1-8 optionally substituted alkyl, or a 19-nor, D ring homo, 1-ene, 2-ene, 3-ene, 4-ene, 5-ene (i.e., 5(6)-ene), 5(10)-ene, 9(11)-ene, 11-ene, 12-ene, 15-ene, 16-ene 1,4-diene, 1,15-diene, 1,16-diene, 3,5-diene, 5,7-diene or aromatic A ring analog of any of these compounds or analogs. Other exemplary analogs include compounds and genera of compounds of any of these compounds where the moiety at the 3- and/or 17-position is replaced with independently selected moieties as described herein such as =O, =S, =NOH, -SH, -NH₂, -NHCH₃, -NHC₂H₅, -N(CH₃)₂, -N(C₂H₅)₂, -NH(C1-8 optionally substituted alkyl), -N(C1-8 optionally substituted alkyl)₂, -C(O)-CH₃, -O-C(O)-CH₃, -O-C(O)-CF₃, -C(S)-CH₃, -S-C(O)-CH₃, -C(O)-CH₂Cl, -C(O)-CH₂OH, ester such as a C2-8 ester, thioester such as a C2-8 thioester, ether such as a C1-8 ether, thioether such as C1-8 thioether, a carbamate such as a C1-8 carbamate, a carbonate such as a C1-8 carbonate, an optionally substituted monosaccharide or a polymer.

Analytical characterization and reference standards. Individual F1Cs described or disclosed herein are suitable for use as standards for determining chemical or physical properties using one, two or more analytical methods, e.g., for use in HPLC, reverse phase HPLC, MS (mass spectrometry), quadrupole MS, GC-MS, LC-MS, NMR (nuclear magnetic resonance spectrometry), ²H-NMR , ³H-NMR, ¹³C-NMR, ¹⁴C-NMR, infrared spectrometry (IR), Fourier transform-IR, optical rotary dispersion, loss on drying for water and solvent measurement, Karl Fisher titration for water determination, differential scanning calorimetry, melting point, density, refractive index, solubility characteristics in organic solvents, aqueous systems or aqueous-organic solvent mixtures, the partition coefficient in immiscible solvent systems, e.g., octanol:water partition coefficient, heat stability or epimerization rate or characteristics of a given enantiomer. These analytical or chemical properties of each F1C are collectively referred to as analytical characteristics. For general methods, see, e.g., H.L.J. Makin et al., eds. Steroid Analysis 1995, Chapman & Hall, ISBN 0751401285. Thus, to aid in the determination of, e.g., the structure of a metabolite of a F1C or a structurally related compound, the parent compound or another structurally related F1 C could be used as a standard. Metabolism of F1 Cs will often include one or more of oxidation, reduction, hydroxylation or conjugation, e.g., oxidation or reduction to a -OH or =O moiety, or conjugation with a moiety such as sulfate, phosphate, amino acid, dipeptide or a monosaccharide such as glucuronic acid at, e.g., the 2, 3, 6, 7, 11, 15, 16, 17 or other positions on the steroid nucleus. In these embodiments, the appropriate use of a F1C of known structure as a standard can aid in or verify the identification of metabolites that are projected to have closely related structures. Information regarding the identification can be useful or sometimes is necessary for, e.g., obtaining regulatory approval to market a therapeutic agent such as a F1 C or understanding the potential biological role that a F1C or its metabolite can play in one of the applications disclosed herein or in a cited reference. To facilitate such uses, the F1C may be labeled as appropriate, e.g., using a F1C with, e.g., one or more ²H, ³H, ¹³C, ¹⁴C, ¹⁵N, ¹⁸O, ¹⁸F, ^{3S}S, ³²P or ¹²³I , at 1, 2 or more of the 1, 2, 3, 4, 5, 6, 7, 8, 9, 10, 11, 12, 13, 14, 15, 16, 17, 18, 19, 20, 21, 22 or other positions in any formula 1 steroid. Radiolabel or heavy isotope atoms may be directly bonded to, or for carbon atoms, replace a steroid nucleus atom, or they may be bonded through one, two or more intervening atoms, e.g., steroid-O-³²P(O)(OH)(OH). Suitably labeled compounds include any of the F1Cs disclosed herein. Such labeled compounds may comprise, e.g., a ¹³C at the 18 or 19 positions and 1, 2, 3 or 4 ³H may be bonded to the ¹³C atom(s) or to a ring carbon(s). Other formula 1 compounds may comprise one or two ²H or ³H atoms bonded to one or more of the 1, 2, 4, 5, 6, 11 or 12 positions and optionally a ¹³C at the 18 or 19 position(s). F1Cs and their metabolites are isolated or characterized using radiolabeled or mass labeled atoms. F1Cs are also optionally isolated by the use of antibodies capable of binding to epitopes in F1Cs or in metabolites.

Embodiments include a method (the "characterization method") to characterize or at least partially characterize a formula 1 compound that is at least partially uncharacterized for one or more given chemical or analytical properties, e.g., a known or potential metabolite of a parent formula 1 compound, comprising (a) providing a formula 1 compound having one, two or more known characteristics, e.g., a known or at least partially known or characterized chemical structure, XRD spectrum or melting point (a "CF1C"), and a formula 1 compound that is unknown or at least partially uncharacterized, i.e., is uncharacterized for at least one of the same analytical characteristics (a "UCF1C"), (b) obtaining one, two or more analytical characteristics of the UCF1C, and (c) comparing the 1, 2 or more analytical characteristics of the CF1C with the analytical characteristics of the UCF1C. The steps in this method may be conducted in any suitable order, e.g., analytical or chemical data for the CF1C will usually be obtained before or at about the same time as one obtains the analytical or chemical data for the UCF1C. Usually the CF1C will be more completely characterized than the UCF1C, particularly with regard to its chemical structure or its relative degree of purity or with regard to the analytical or chemical data that is being sought. This method allows further characterization of the UCF1C, e.g., by confirming the UCF1C's chemical structure or by determining the UCF1 C's stability under various storage or temperature conditions or in various formulations or by determining other analytical or chemical properties of interest. In this method, the CF1C itself may not be completely characterized, however, for the one, two or more analytical characteristics of interest, the CF1C will usually have a known or confirmed property or properties, while the UCF1C is unknown or at least unconfirmed for the same property or properties.

As is apparent from the present disclosure, the F1C may be prepared synthetically and typical embodiments will utilize a purified or at least partially purifiied F1C. Purified F1C can be free, essentially free or partially free, of other F1C or other compounds such as excipients. Thus, any given purified F1C can be present as a solid that contains, e.g., less than about 15% w/w or less than about 10% w/w or less than about 8% w/w or less than about 5% w/w or less than about 3% w/w or less than about 2% w/w or less than about 1% w/w of one, two or more other F1Cs, excipients, synthetic by-products, decomposition products or synthesis or purification reactants or reagents. Similarly, the F1 C can be present in a solution or suspension that contains at least about 90% w/w or at least about 95% w/w or at least about 97% w/w of the F1 C and one or more excipients and less than about 10% or 8% or 5% or 3% w/w or 1% w/w of one, two or more other F1 Cs, excipients, synthetic by-products, decomposition products or synthesis or purification reactants or reagents.

Dosages of F1C and dosing protocols or methods. In treating any of the conditions or symptoms disclosed herein, one can continuously or intermittently administer the F1 C(s) to a subject having or susceptible to developing the condition or symptom. In treating a condition such as an infection, a hyperproliferation condition, an inflammation condition or another condition disclosed herein with a F1C using an intermittent dosing can avoid or ameliorate some of the undesired aspects normally associated with discontinuous dosing. Such undesired aspects include development of resistance of a pathogen such as a pathogen disclosed herein, e.g., a virus or bacterium such as HIV or *Staphylococcus aureus* or a parasite such as a *Plasmodium* parasite, to the therapeutic agent, failure of the patient or subject to adhere to a daily dosing regimen or reduction of the dosages of other therapeutic agents and/or their associated unwanted side effects or toxicities, e.g., reduction or a toxic effect of a chemotherapy or radiation exposure. In any of the continuous or intermittent dosing protocols described herein, other appropriate treatments can be applied as the subject's clinical situation dictates. Suitable other appropriate treatments or therapeutic agents are described elsewhere herein and in the cited references.

In any of continuous daily dosing protocol, e.g., as described herein, or in any step(s) in the intermittent dosing protocols described herein, or in treating any of the diseases, conditions or symptoms described herein, the F1C(s) can be administered by one or more suitable routes, e.g., oral, buccal, sublingual, intramuscular (i.m.), subcutaneous (s.c.), intravenous (i.v.), intradermal, another parenteral route or by an aerosol. The effective daily dose in such methods will typically comprise about 0.05 mg/kg/day to about 200 mg/kg/day, about 0.1 to about 100 mg/kg/day, about 0.4 to about 80 mg/kg/day, about 1-45 mg/kg/day or about 1-6 mg/kg/day, including about 0.2 mg/kg/day, 0.5 mg/kg/day, about 1 mg/kg/day, about 2 mg/kg/day, about 4 mg/kg/day, about 6 mg/kg/day, about 10 mg/kg/day, about 20 mg/kg/day, about 40 mg/kg/day or about 100 mg/kg/day. Higher dosages, e.g., about 250 mg/kg/day, about 300 mg/kg/day or about 350 mg/kg/day can also be utilized, e.g., in veterinary applications. One can administer the F1 C(s) orally using about 4 to about 60 mg/kg/day, usually about 6-30 mg/kg/day. In some embodiments, the intermittent dosing methods exclude dosing protocols that are commonly used to deliver contraceptive steroids to, e.g., human females, such as daily dosing for 21 days, followed by no dosing for 7 days. For humans, dosing is generally about 0.05 mg/kg/day to about 30 mg/kg/day, typically about 0.5-10 mg/kg/day. Low dosages for humans such as about 0.005 mg/kg/day to about 0.2 mg/kg/day or about 0.25-10 mg/day, can be used with, e.g., local, topical, transmucosal or intravenous administration and higher dosages such as about 0.1 mg/kg/day to about 20 mg/kg/day or about 5-200 mg/day, can be used, e.g., for oral, subcutaneous or other systemic or local administration route. For non-human subjects, e.g., mammals such as rodents or primates, the effective daily dosage may comprise about 0.05 mg/kg/day to about 350 mg/kg/day.

Formulations and compositions for preparing formulations. Invention embodiments include formulations described here and elsewhere in this disclosure. While it is possible for the F1 C(s) to be administered to a subject or incubated with a subject's cells *in vitro* as the compound alone, it is usual to use F1C in a formulation or at least in a composition that contains 1, 2, 3, 4, 5, 6 or more excipients. The formulations, which are useful for veterinary or human pharmaceutical use, comprise at least one F1C, together with 1, 2, 3, 4, 5, 6 or more excipients and optionally one or more additional therapeutic ingredients. The formulations can contain one or more classes of excipients such as solubilizers, surfactants, co-solvents, complexation agents, lubricants, binding agents or binders, bulking agents, preservatives, buffers, disintegrants, colorants, sweeteners, souring agents, glidants, flavorants, flavor enhancers, oils such as hydrogenated oils, polymers such as starches, effervescent couples, amino acids, monosaccharides, disaccharides, oligosaccharides, dyes or colorants.

Exemplary effervescent couples include sodium bicarbonate and citric acid. Exemplary monosaccharides, disaccharides and oligosaccharides include sorbitol, glucose, dextrose, fructose, maltose, xylitol, sucrose, lactose, glucose, galactose, mannitol, dextrates and maltodextrins. Glidants include silicone dioxide. Lubricants include magnesium stearate. Flavorants include strawberry aroma, raspberry aroma, cherry flavor, magnasweet 135, key lime flavor, grape flavor, trusil art 5-11815, fruit extracts and prosweet. Flavor enhancers and sweeteners include aspartame, sodium saccharine, sorbitol, glucose and sucrose. Souring agents include citric acid.

The invention includes compositions comprising one or more pharmaceutically acceptable excipients or carriers. The compositions are used to prepare formulations suitable for human or animal use. Formulations may be designed or intended for oral, rectal, nasal, topical or transmucosal (including buccal, sublingual, ocular, vaginal and rectal) and parenteral (including subcutaneous, intramuscular, intravenous, intradermal, intrathecal, intraocular and epidural) administration. In general, aqueous and non-aqueous liquid or cream formulations are delivered by a parenteral, oral or topical route. In other embodiments, the F1C(s) may be present as an aqueous or a non-aqueous liquid formulation or a solid formulation suitable for administration by any route, e.g., oral, topical, buccal, sublingual, parenteral, aerosol, a depot such as a subcutaneous depot or an intraperitoneal or intramuscular depot or a rectal or vaginal suppository. The preferred route may vary with, for example, the subject's pathological condition or weight or the subject's response to therapy with a F1C or other therapy that is used or that is appropriate to the circumstances. The F1C formulations can also be administered by two or more routes, e.g., subcutaneous injection and buccal or sublingual, where these delivery methods are essentially simultaneous or they may be essentially sequential with little or no temporal overlap in the times at which the compound is administered to the subject.

The formulations include those suitable for the foregoing administration routes. The formulations may conveniently be presented in unit dosage form and may be prepared by any of the methods known in the art of pharmacy. Techniques, dosage forms and excipients such as binders, diluents, disintegrants, viscosity enhancers, stabilizing agents, water absorbing agents, suspending agents and lubricants, are found in, e.g., A.R. Gennaro et al., eds., Remington: The Science and Practice of Pharmacy, Lippincott Williams & Wilkins., Baltimore, MD 2000, 20th edition; Nema et al., PDA J. Pharm. Sci. Tech. 1997 51:166-171; Pharmaceutical Coating Technology, 1995, G. Cole, et al., editors, Taylor & Francis, ISBN 0 136628915; Pharmaceutical Dosage Forms, 1992 2nd revised edition, volumes 1 and 2, H.A. Lieberman, et al., editors, Marcel Dekker, ISBN 0824793870; Pharmaceutical Preformulation, 1998, pages 1-306, J.T. Carstensen, Technomic Publishing Co. ISBN 1566766907; Encyclopedia of Pharmaceutical Technology, volumes 1, 2 and 3, 2nd edition, 2002, J. Swarbrick and J.C Boylan, editors, Marcel Dekker, Inc., New York, N.Y.; and A.H. Kibbe, ed. Handbook of Pharmaceutical Excipients, 3rd ed., 2000, American Pharmaceutical Association, Washington, D.C.

Methods to make invention formulations include the step of bringing into association or contacting a F1C(s) with one or more excipient, such as one described herein or in the cited references. In general the formulations are prepared by uniformly and intimately bringing into association the F1 C(s) with liquid excipients or finely divided solid excipients or both, and then, if appropriate, shaping the product.

Formulations suitable for oral administration are prepared as discrete units or unit doses such as capsules, soft gelatin capsules (softgels), cachets, tablets or caplets each containing a predetermined amount of the F1C(s). F1C formulations can also be present as a powder or granules or as a solution or a suspension, colloid or gel in an aqueous liquid or base or in a non-aqueous liquid or base; or as an oil-in-water liquid emulsion or a water-in-oil liquid emulsion. The F1C formulations may also be a bolus, electuary or paste. Suspension formulations will typically contain about 0.5% w/w or about 1 % w/w to about 5%, 10%, 15% or 20% w/w of the F1 C, which can be for parenteral use or for other routes of administration, e.g., oral softgels.

A tablet is made by compression or molding, optionally with one or more accessory ingredients. Compressed tablets may be prepared by compressing in a suitable machine the F1C(s) in a free-flowing form such as a powder or granules, optionally mixed with a binder, lubricant, inert diluent, preservative, surface active or dispersing agent. Molded tablets may be made by molding in a suitable machine a mixture of the powdered or granulated F1 C and one or more excipients, which are optionally moistened, with an inert liquid diluent or excipient. The tablets may optionally be coated or scored and optionally are formulated so as to provide slow or controlled release of the F1C(s) therefrom. An exemplary tablet or caplet formulation suitable for buccal or sublingual delivery of a F1 C to a subject's tissues comprises about 25 or 50 mg of a F1C comprising per 25 mg of the F1C about 6.2 mg povidone, about 0.62 mg magnesium stearate, about 45 mg mannitol and about 48 mg of compressible sucrose.

For formulations adapted for administration to the eye or other external tissues e.g., the mouth, oral mucosa or skin, the formulations can be applied as a topical ointment or cream or sterile eye drops containing the F1 C(s) in an amount of, for example, about 0.075 to about 20% w/w (including F1C(s) in a range between about 0.1 % and 20% in increments of 0.1 % w/w such as about 0.6% w/w, about 0.7% w/w, about 1 % w/w, about 1.5% w/w, about 2% w/w, about 2.5 w/w, about 3% w/w, about 5% w/w, about 7% w/w, about 10% w/w etc.), including about 0.2 to 15% w/w and about 0.5 to 10% w/w. When formulated in an ointment, the F1 C(s) may be employed with either a paraffinic or a water-miscible ointment base. Alternatively, they may be formulated in a cream with an oil-in-water cream base. Ocular administration formulations are usually sterile and include a F1C(s) dissolved or suspended in a suitable excipient(s), including an aqueous solvent for a F1C(s) that comprise at least about 0.5, one, two or more charges at pH values near neutrality, e.g., about pH 6-8. The F1C(s) is typically present in such formulations in a concentration of about 0.5-20% w/w, about 1-10% w/w or about 2-5% w/w.

If desired, the aqueous phase of a cream base may include, for example, at least 30% w/w of a polyhydric alcohol, i.e. an alcohol having two or more hydroxyl groups such as propylene glycol, butane 1,3-diol, butane 1,4-diol, mannitol, sorbitol, glycerol and a polyethylene glycol (including, e.g., PEG 300 and PEG 400) and mixtures thereof. The topical formulations may include a compound that enhances absorption or penetration of the F1 C(s) through the skin or other affected areas. Examples of such dermal penetration enhancers include dimethyl sulphoxide and related analogs.

The oily phase of the emulsion formulations may be constituted from known excipients in a known manner. While the phase may comprise an emulsifier or emulgent, it typically comprises a mixture of at least one emulsifier with a fat or an oil or with both a fat and an oil. A hydrophilic emulsifier may be included together with a lipophilic emulsifier, which acts as a stabilizer. Some embodiments include both an oil and a fat. Together, the emulsifier(s) with or without stabilizer(s) make up the so-called emulsifying wax, and the wax together with the oil and fat make up the so-called emulsifying ointment base, which forms the oily dispersed phase of the cream formulations.

Emulgents and emulsion stabilizers suitable for use in the formulations include Tween60^{™}, Span80^{™}, cetostearyl alcohol, benzyl alcohol, myristyl alcohol, glyceryl mono-stearate and sodium lauryl sulfate. Other excipients include emulsifying wax, propyl gallate, citric acid, lactic acid, polysorbate 80, sodium chloride, isopropyl palmitate, glycerin and white petrolatum.

The choice of suitable oils or fats for the formulation is based on achieving the desired cosmetic properties. Creams are generally a non-greasy, non-staining and washable products with suitable consistency to avoid leakage from tubes or other containers. Straight or branched chain, mono- or dibasic alkyl esters such as di-isoadipate, isocetyl stearate, propylene glycol diester of coconut fatty acids, isopropyl myristate, decyl oleate, isopropyl palmitate, butyl stearate, 2-ethylhexyl palmitate or a blend of branched chain esters known as Crodamol CAP may be used. These may be used alone or in combination depending on the properties required. Alternatively, high melting point lipids such as white soft paraffin and/or liquid paraffin or other mineral oils are used.

Formulations suitable for topical administration to oral mucosa include lozenges or tablets comprising the F1 C(s) in a flavored basis or a monosaccharide or disaccharide such as sucrose, lactose or glucose and acacia or tragacanth; pastilles comprising the F1 C(s) in an inert basis such as gelatin and glycerin, or sucrose and acacia; and mouthwashes comprising the F1C(s) in a suitable liquid excipient(s). In some embodiments, the lozenges or tablets optionally comprise the property of rapid dissolution or disintegration, e.g., disintegration within about 15 seconds to about 2 minutes, while in others, the lozenges or tablets comprise the property of slower dissolution or disintegration, e.g., disintegration within about 2 minutes to about 10 minutes or more.

Formulations for rectal administration may be presented as a suppository with a suitable base comprising for example cocoa butter or a salicylate.

Formulations suitable for vaginal administration may be presented as pessaries, tampons, creams, gels, pastes, foams or spray formulations containing in addition to the F1 C(s) such excipients as are known in the art to be appropriate.

Formulations suitable for parenteral administration include aqueous and non-aqueous sterile injection solutions which may contain anti-oxidants, buffers, bacteriostats, salts (e.g., NaCl, potassium or sodium carbonate or bicarbonate or potassium or sodium phosphates) and solutes which render the formulation isotonic with the blood of the intended subject; and aqueous and non-aqueous sterile suspensions which may include suspending agents or thickening agents. In general, the F1C that is present in liquid compositions or formulations is completely dissolved in aqueous or non-aqueous excipients. However, in some embodiments, e.g., transient compositions or some formulations, the F1C is partially dissolved while the remaining portion is present as a solid, which can be a suspension or a colloid.

Formulations suitable for parenteral delivery of F1Cs to subjects such as humans or animals typically comprise 1, 2, 3, 4, 5, 6 or more excipients. Exemplary embodiments include (1) any two, three or four of propylene glycol, PEG200, PEG300, ethanol, benzyl alcohol and benzyl benzoate and (2) any two, three or four of propylene glycol, PEG100, PEG200, PEG300, PEG400, benzyl alcohol and benzyl benzoate. Typically such formulations will contain both propylene glycol and one or more PEGs, e.g., PEG100, PEG200, PEG300 or PEG400, which enhance the solubility of the F1C by a cosolvent effect.

Formulations, or compositions disclosed herein for use to make formulations suitable for administration by the routes disclosed herein optionally comprise an average particle size in the range of about 0.01 to about 500 microns, about 0.1 to about 100 microns or about 0.5 to about 75 microns. Average particle sizes include a range between 0.01 and 500 microns in 0.05 micron or in 0.1 micron or other increments, e.g., an average particle size of about 0.05, 0.1, 0.2, 0.3, 0.4, 0.5, 0.7, 1, 1.5, 2.0, 2.5, 3.0, 3.5, 4.0, 4.5, 5.0, 5.5, 6, 7, 8, 9, 10, 15, 20, 25, 30, 35, 40, 50, 60, 75, 85, 100, 120, 150, etc. microns). The F1C itself that is used to make a formulation can have one, two or more of these average particle sizes. When F1Cs or compositions that comprise a F1C are used as intermediates to make a formulation, they may comprise one, two, three or more of these average particle sizes, or size ranges. In preparing any of the compositions or formulations that are disclosed herein and that comprise a F1 C (and optionally one or more excipients), one may optionally mill, sieve or otherwise granulate the compound or composition to obtain a desired particle size, e.g., as described above.

Milling or micronization by any other method may occur before or after the F1 C is contacted with one or more excipients. For example, one may mill a F1 C to obtain an average particle size (or diameter) of about 0.05-50 µM or about 0.5-10 µM (e.g., about 0.02, 0.04, 0.05, 0.07, 0.1, 0.5, 1, 1.5, 2, 2.5, 5, 10, 15, 20, 30, 40, 50, 60, 80, 100 or 120 µM average particle size or diameter) before contacting the milled F1C with a liquid or solid excipient. In some cases the F1C is milled or sieved to obtain an average particle size of about 5 µm or about 10 µm before it is contacted with a solid or liquid excipient(s) to obtain a solution or suspension or a powder suitable for making a tablet, capsule or other dosage form as described herein or in the cited references. Micronized compound may be prepared using any suitable process for obtaining small particles, e.g., controlled precipitation from a solution, micronizing or milling, a number of which are known in the art. The micronized particles may include a percentage of particles that are less than or equal to about 0.1-20 µm in diameter. Ranges of average particle sizes include F1Cs of about 0.04-0.6 µm, about 0.04-1.0 µm, about 0.05-0.6 µm, about 0.05-1.0 µm, about 0.1-0.4 µm, about 0.5-1 µm, about 1-20 µm or about 2-50 µm.

As used herein, reference to an average particle size or an average particle diameter means that the material, e.g., a F1C(s), an excipient(s) or a composition that comprises both, is ground, milled, sieved or otherwise treated so as to comprise the specified average size. It is to be understood that some particles may be larger or smaller, but the composition or the F1 C(s) will comprise a significant proportion of the material with the specified size or within an acceptable range of the specified size, e.g., at least about 70% or about 80% of the particles within about 30% to about 50% of the average size or diameter. Micronization methods include milling by ball mills, pin mills, jet mills (e.g., fluid energy jet mills) and grinding, sieving and precipitation of a compound(s) from a solution, see, e.g., U.S. patents 4919341, 5202129, 5271944, 5424077 and 5455049. Average particle size is determined by known methods, e.g., transmission electron microscopy, scanning electron microscopy, light microscopy, X-ray diffractometry, light scattering methods or Coulter counter analysis.

Thus, the F1 Cs may comprise a powder that consists of one, two or more of these average particle sizes and the powder may be contacted with a solid excipient(s), suitably mixed and optionally compressed or formed into a desired shape. Alternatively, such a F1C(s) is contacted with a liquid excipient(s) to prepare a liquid formulation or a liquid composition that is incorporated into a solid formulation. Suitable micronized formulations thus include aqueous or oily solutions or suspensions of the F1C(s).

Formulations suitable for aerosol administration typically will comprise a fine powder, e.g., having an average particle size of about 0.1 to about 20 microns or any one, two or more of the average particle sizes within this range that are described above. The powder is typically delivered by rapid inhalation through the nasal passage or by inhalation through the mouth so as to reach the bronchioles or alveolar sacs of the lungs.

Formulations suitable for aerosol, dry powder or tablet administration may be prepared according to conventional methods and may be delivered with other therapeutic agents such as compounds heretofore used in the treatment or prophylaxis of viral or other infections as described herein. Such formulations may be administered, e.g., orally, parenterally (e.g., intravenous, intramuscular, subcutaneous, intradermal, intrathecal), topically, sublingually or by a buccal or sublingual route.

Micronized F1C is useful, e.g., to facilitate mixing, dissolution or uniform suspension of the F1C in one or more liquid or solid excipients, e.g., a PEG such as PEG 300 or PEG 400, propylene glycol, benzyl benzoate, a complexing agent, such as a cyclodextrin (e.g., an α-, β- or γ-cyclodextrin such as hydroxypropyl-β-cyclodextrin). Micronized F1C is also useful to facilitate uniformly distributing drug substance when the micronized compound is contacted with one or more solid excipients (e.g., a filler, a binder, a disintegrant, complexing agent (e.g., a cyclodextrin such as hydroxypropyl-β-cyclodextrin), a preservative, a buffer or a lubricant).

In related embodiments, suitable compositions or formulations comprise a F1C that is present in two or more physical forms. For example, a liquid composition or formulation may comprise a F1C that is present in solution and as undissolved particles, which may be milled as described herein. Alternatively, a solid composition or formulation may comprise a F1 C that is present as an amorphous form and as a crystal or in an encapsulated granule. Such encapsulated granules may comprise a slow release type formulation and the F1C that is present may be in one or more physical forms, e.g., liquids or solids as described herein, but usually as a solid in tablets or other solid formulations.

The formulations are presented in unit-dose or multi-dose containers, for example sealed ampules and vials, and may be stored in a freeze-dried (lyophilized) condition requiring only the addition of the sterile liquid excipient, for example water for injection, immediately prior to use. In general, solid, liquid or other formulations or compositions that comprise a F1C, e.g., unit dosages for solid or liquid formulations, are stored in a sealed container, which may optionally be opaque or nearly opaque (e.g., amber or blue glass or brown plastic) to reduce the amount of light that reaches the formulation or composition. Such containers are also optionally sealed, e.g., hermetically sealed, to prevent or limit exchange of air, water or other gases between the container's contents and air. Extemporaneous injection solutions and suspensions are prepared from sterile powders, granules and tablets as described above. Unit dosage formulations are those containing a daily dose or unit daily sub-dose, as recited herein, or an appropriate fraction thereof, of the F1C(s).

It should be understood that in addition to the ingredients particularly mentioned above the formulations of this invention may include other agents or excipients conventional in the art having regard to the type of formulation in question, for example those suitable for oral administration may include flavoring agents. Excipients include liquids, such as benzyl benzoate, cottonseed oil, N,N-dimethylacetamide, a C₂₋₁₂ alcohol (e.g., ethanol), glycerol, peanut oil, vitamin E, poppy seed oil, safflower oil, sesame oil, soybean oil and vegetable oil. Excipients may optionally exclude one or more excipient, e.g., chloroform, dioxane, vegetable oil, DMSO, other excipients or any combination of these. Other excipients are components typically used in the pharmaceutical formulation arts, e.g., one, two or more of fillers, binders, disintegrants, dispersants, preservatives, glidants and lubricants, e.g., povidone, crospovidone, corn starch, carboxymethyl cellulose, hydroxypropyl methylcellulose, microcrystalline cellulose, gum arabic, polysorbate 80, butylparaben, propylparaben, methylparaben, BHA, EDTA, sodium lauryl sulfate, sodium chloride, potassium chloride, titanium dioxide, magnesium stearate, castor oil, olive oil, vegetable oil, buffering agents such as sodium hydroxide, monobasic sodium phosphate, dibasic sodium phosphate, potassium hydroxide, monobasic potassium phosphate, dibasic potassium phosphate, tribasic potassium phosphate, potassium carbonate, potassium bicarbonate, ammonium hydroxide, ammonium chloride, saccharides such as mannitol, glucose, fructose, sucrose or lactose any of which may be compressible or any of which may be spray dried, milled, micronized or otherwise treated to obtained desired characteristics.

Formulations made from or comprising a F1C are optionally stored under conditions that limit the amount of light or water that reaches the formulation, e.g., in a sealed container that holds a formulation or unit dosage form and optionally contains silica gel or activated carbon. Water permeation characteristics of containers have been described, e.g., Containers - Permeation, Chapter, USP 23, 1995, U.S. Pharmacopeial Convention, Inc., Rockville, MD, p.1787.

The invention further provides veterinary compositions comprising at least one F1 C together with a veterinary excipient(s) therefor. Veterinary excipients are materials useful for the purpose of administering the composition and may be solid, liquid or gaseous materials that are otherwise inert or acceptable in the veterinary art and are compatible with the F1C(s). These veterinary compositions may be administered orally, parenterally or by any other desired route.

Invention formulations include controlled release or slow release formulations containing a F1 C(s) in which the release of the F1 C(s) is controlled or regulated to allow less frequency dosing or to improve the pharmacokinetic or toxicity profile of a given F1C(s). Polymers and other materials that are suitable to prepare controlled release formulations that comprise a F1C have been described, e.g., U.S. patents 4652443, 4800085, 4808416, 5013727, 5188840.

Formulations may comprise a liposome or lipid complex that comprises or contains a F1C(s). Such formulations are prepared according to known methods, e.g., U.S. patents 4427649, 5043165, 5714163, 5744158, 5783211, 5795589, 5795987, 5798348, 5811118, 5820848, 5834016 and 5882678. The liposomes optionally contain an additional therapeutic agent(s), e.g., amphotericin B, cis-platin, adriamycin, a protease inhibitor, a nucleoside or a nucleotide analog, such as one of those mentioned herein. Formulations that comprise liposomes can be delivered to a subject by any standard route, e.g., oral, aerosol or parenteral (e.g., s.c., i.v. or i.m.).

Invention embodiments include the product made by a process of combining, mixing or otherwise contacting a F1 C and one, two or more excipients. Such products are produced by routine methods of contacting the ingredients. Such products optionally contain a diluent, a disintegrant, a lubricant, a binder, or other excipients described herein.

Other embodiments include compositions that transiently occur when a method step or operation is performed. For example, when a F1C, containing less than about 3% w/w water is contacted with an excipient, e.g., a PEG, an alcohol, propylene glycol benzyl alcohol or benzyl benzoate, the composition before addition of one ingredient with another is a non-homogenous mixture. As the ingredients are contacted, the mixture's homogeneity increases and the proportion of ingredients relative to each other approaches a desired value. Thus, invention compositions, which contain less than about 3% w/w or less than about 2% w/w or less than about 1% w/w or less than about 0.5% w/w water can comprise about 0.0001-99% w/w of a F1 C and 1, 2, 3 or more excipients. These transient compositions are intermediates that necessarily arise when one makes an invention composition or formulation and they are included in invention embodiments.

When a F1C and an excipient(s) is contacted or mixed, the final composition may comprise a homogenous mixture or it may comprise a mixture that is not homogenous for one or more of the compounds that are present in the composition. Compositions and formulations that are either homogenous or non-homogenous are included in the scope of the invention. Non-homogenous compositions can be used, e.g., to make controlled release formulations.

Invention embodiments include compositions and formulations that comprise less than about 3% water, a F1 C and a compound that is not generally considered suitable for human use but is useful to make an invention formulation for veterinary use. Veterinary formulations are compositions useful for the purpose of administering invention compositions to primates, cats, dogs, horses, cows, rabbits and other subjects and may contain excipients acceptable in the veterinary art and are compatible with F1Cs. These veterinary compositions may not always be suitable for human use because they contain an excipient that is not suitable for human use, e.g., an alcohol other than ethanol such as methanol, propanol or butanol. Typically such excipients will be present at relatively low levels, e.g., about 1-30%, usually about 1-5%.

Invention embodiments include non-aqueous compositions and formulations, e.g., unit dosage forms and sterile solutions or suspensions, that comprise about 1-25% w/w of a F1C, about 20-60% w/w propylene glycol, about 15-55% w/w of more or more PEGs, e.g., PEG100 or PEG200, about 0-5% w/w benzyl benzoate, about 0-5% w/w benzyl alcohol and optionally one or more additional excipients. These non-aqueous formulations will usually contain less than about 3%, 2%, 1%, 0.8%, 0.5%, 0.4%, 0.3%, 0.2% or 0.1 % w/w of water. In formulations that contain non-aqueous excipients, the F1 C will usually be relatively hydrophobic and will usually not contain any easily charged or ionizable moieties such as free carboxyl groups.

F1Cs may be administered to subjects by transmucosal dosing, e.g., by buccal or sublingual administration. The buccal area generally refers to the subject's mouth and pharynx, and the buccal mucosa includes the mucosa of the mouth and pharynx. The sublingual area refers generally to the mucosa below and adjacent to the tongue. Formulations suitable for buccal or sublingual administration typically comprise about 1-100 mg of F1C per unit dose, often about 2-60 mg. Transmucosal dosages may comprise a slow release or a rapid release formulation or tablet that contains about 1, 5, 10, 15, 20, 25, 30, 35, 40, 50 or 60 mg of a F1C. Slow release formulations will generally degrade or release the F1 C from the dosage over a period of about 2 minutes to about 60 minutes or more. Rapid release formulations will generally release the F1C over a period of about 4 seconds to about 2 minutes, typically over about 0.1 to about 1 minute. Solid and liquid buccal or sublingual formulations optionally include one, two, three or more excipients such as fillers, binders, lubricants, antioxidants, preservatives, flavoring agents or disintegrants, e.g., lactose, sucrose, mannitol, Tween-80, magnesium stearate, butylated hydroxyanisole, butylated hydroxytoluene, cyclodextrins (e.g., α-cyclodextrins, β-cyclodextrins, γ-cyclodextrins, hydroxypropyl-β-cyclodextrin, β-cyclodextrin ether comprising one or more hydroxybutyl sulfonate moieties, cyclodextrins as described in US 6610671 or US 6566347), carbomers, hydrolyzed polyvinylalcohol, polyethylene oxide, polyacrylates, hydroxypropylmethylcellulose, hydroxypropylcellulose, and combinations thereof. Such formulations may be a unit solid such as a tablet or powder or a liquid. Buccal tablets may comprise a concave surface for contacting the buccal mucosa and adhering to it. A buccal or sublingual dosage may comprise a compressed tablet of a substantially uniform mixture of a bioerodible polymeric carrier, which on sustained contact with the oral mucosa, substantially or completely erodes within a predetermined period in the range of about 10 minutes to about 24 hours. In some embodiments, the F1C is administered by a method for administering the compound to the subject, e.g., to a mammal or a human, comprising affixing a unit dosage or tablet to the subject's buccal mucosa in a region at or near the upper gum between the first bicuspid on the left and the first bicuspid on the right (or an alternative location for the dosage unit is the inner lip area opposing the this upper gum area) and optionally allowing the tablet to remain in place until erosion thereof is complete or nearly complete. Exemplary excipients may comprise a combination of polyethylene oxide and a carbomer, e.g., wherein the polyethylene oxide and the carbomer are in an approximately 1:5 to 5:1 ratio by weight.

Tablets or unit dosages for buccal or sublingual delivery may be about 5 mm in diameter and 2 mm in height, so that the unit dosage occupies about 40 mm³. Such dosages will typically weigh less than about 100 mg (e.g., about 5 to 60 mg), with a contact surface area of about 10-30 mm², e.g., about 15-20 mm². Such dosages will generally be about 4-10 mm in diameter and about 1-3 mm in height. When a polymer excipient is used, it optionally comprises a polymer having sufficient tack to ensure that the dosage unit adheres to the buccal mucosa for a sufficient time period, e.g., the time period during which drug is to be delivered to the buccal mucosa. The polymeric excipient is gradually "bioerodible," and it hydrolyzes, dissolves, erodes or disintegrates (collectively "erodes") at a predetermined rate upon contact with water or saliva. The polymeric carrier is generally sticky when moist, but not when dry, for convenience in handling. The average molecular weight of the polymer may be about 400 to 1,000,000, or about 1,000 to 100,000. Higher the molecular weight polymers generally erode more slowly.

For these buccal and sublingual dosages, a pharmaceutically acceptable polymer(s) can be used. Such polymers will provide a suitable degree of adhesion and the desired drug release profile, and are generally compatible with the drug to be administered and any other components that may be present in the buccal dosage unit. The polymeric carriers optionally comprise hydrophilic (water-soluble and water-swellable) polymers that adhere to the wet surface of the buccal mucosa. Examples of polymeric carriers that are useful herein include acrylic acid polymers, e.g., those known as "carbomers" (Carbopol^{™}, which may be obtained from B.F. Goodrich, is one such polymer). Other suitable polymers include hydrolyzed polyvinylalcohol; polyethylene oxides (e.g., Sentry Polyox^{™} water soluble resins, available from Union Carbide); polyacrylates (e.g., Gantrez^{™}, which may be obtained from GAF); vinyl polymers and copolymers; polyvinylpyrrolidone; dextran; guar gum; pectins; starches; and cellulosic polymers such as hydroxypropyl methylcellulose, (e.g., Methocel ^{™}, which may be obtained from the Dow Chemical Company), hydroxypropyl cellulose (e.g., Klucel^{™}, which may be obtained from Dow), hydroxypropyl cellulose ethers (see, e.g., U.S. Pat. No. 4,704,285 to Alderman), hydroxyethyl cellulose, carboxymethyl cellulose, sodium carboxymethyl cellulose, methylcellulose, ethyl cellulose, cellulose acetate phthalate, cellulose acetate butyrate, and the like. The carrier may also comprise two or more suitable polymers in combination, for example, a carbomer combined in an approximately 1:5 to 5:1 ratio, by weight, with a polyethylene oxide.

Buccal dosages may contain only the F1C and the polymer(s). However, it may be desirable in some cases to include one or more additional excipients. For example, a lubricant may be included to facilitate the process of manufacturing the dosage units; lubricants may also optimize erosion rate and drug flux. If a lubricant is present, it may optionally represent about 0.01 wt. % to about 2 wt. %, or about 0.01 wt. % to 0.5 wt. %, of the dosage unit. Suitable lubricants include, but are not limited to, magnesium stearate, calcium stearate, stearic acid, sodium stearylfumarate, talc, hydrogenated vegetable oils and polyethylene glycol. However, modulating the particle size of the components in the dosage unit and/or the density of the unit can provide a similar effect, e.g., improved manufacturability, and optimization of erosion rate and drug flux without addition of a lubricant.

Other excipients are also optionally incorporated into buccal unit dosages. Such additional optional excipients include, one or more disintegrants, diluents, binders, enhancers, or the like. Examples of disintegrants that may be used include, but are not limited to, cross-linked polyvinylpyrrolidones, such as crospovidone (e.g., Polyplasdone^{™} XL, which may be obtained from GAF), cross-linked carboxylic methylcelluloses, such as croscarmelose (e.g., Ac-di-sol^{™}, which may be obtained from FMC), alginic acid, and sodium carboxymethyl starches (e.g., Explotab^{™}, which may be obtained from Edward Medell Co., Inc.), methylcellulose, agar bentonite and alginic acid. Suitable diluents are those which are generally useful in pharmaceutical formulations prepared using compression techniques, e.g., dicalcium phosphate dihydrate (e.g., Di-Tab^{™}, which may be obtained from Stauffer), sugars that have been processed by cocrystallization with dextrin (e.g., co-crystallized sucrose and dextrin such as Di-Pak^{™}, which may be obtained from Amstar), lactone, calcium phosphate, cellulose, kaolin, mannitol, sodium chloride, dry starch, powdered sugar and the like. Binders, if used, are those that enhance adhesion. Examples of such binders include, but are not limited to, starch, gelatin and sugars such as sucrose, dextrose, molasses, and lactose. Permeation enhancers may also be present in the novel dosage units in order to increase the rate at which the active agent passes through the buccal mucosa. Examples of permeation enhancers include, but are not limited to, polyethylene glycol monolaurate ("PEGML"), glycerol monolaurate, lecithin, the 1-substituted azacycloheptan-2-ones, particularly 1-n-dodecylcyclaza-cycloheptan-2-one (available under the trademark Azone^{™} from Nelson Research & Development Co., Irvine, Calif.), lower alkanols (e.g., ethanol), SEPA^{™} (available from Macrochem Co., Lexington, Mass.), cholic acid, taurocholic acid, bile salt type enhancers, and surfactants such as Tergitol^{™}, Nonoxynol-9^{™} and TWEEN-80^{™}.

Flavorings are optionally included in buccal or sublingual formulations. Any suitable flavoring may be used, e.g., one or more of mannitol, sucrose, glucose, lactose, lemon, lemon lime, orange, menthol or artificial sweeteners such as aspartame, saccharin sodium, dipotassium glycyrrhizinate, stevia and thaumatin. Some sweeteners such as sucrose may also aid in dissolution or erosion of solid formulations. Coloring agents may also be added, e.g., any of the water soluble FD&C dyes or mixtures thereof, e.g., one or more of FD&C Yellow No. 5, FD&C RED No.2, FD&C Blue No.2, etc., food lakes or red iron oxide. In addition such formulations dosages may be formulated with one or more preservatives or bacteriostatic agents, e.g., methyl hydroxybenzoate, propyl hydroxybenzoate, chlorocresol, benzalkonium chloride, or the like.

Other embodiments include solid buccal or sublingual formulations comprising (i) a F1C and (ii) erythritol, (iii) crystalline cellulose and (iv) a disintegrant, e.g., crospovidone. These formulations are capable of buccal disintegration or dissolution and may further comprise mannitol. These formulations may dissolve completely in solely saliva within about 1-10 minutes of administration to a subject. The erythritol is optionally contained in a proportion of about 5-90 parts by weight, based on 100 parts by weight of the solid buccal formulation. The crystalline cellulose is optionally contained in a proportion of about 3-50 parts by weight, based on 100 parts by weight of the formulation. The disintegrant is optionally contained in a proportion of 1-10 parts by weight. In any of the solid buccal or sublingual formulations the ingredients are generally uniformly mixed, although non-uniform mixtures may be used. An exemplary formulation comprises a solid capable of buccal disintegration or dissolution, which comprises (i) about 0.3-50 parts by weight of a F1 C, (ii) about 50-80 parts by weight of erythritol, (iii) about 5-20 parts by weight of crystalline cellulose and (iv) about 3-7 parts by weight of a disintegrant, which optionally is one or more of crospovidone, croscarmellose, croscarmellose sodium, carmellose calcium, carboxymethylstarch sodium, low substituted hydroxypropyl cellulose or corn starch. Examples of the crystalline cellulose include products of various grade such as CEOLUS KG801, avicel PH101, avicel PH102, avicel PH301, avicel PH302, avicel RC-591 (crystalline cellulose carmellose sodium) and so on. One crystalline cellulose may be used or two or more species may be used in combination. The disintegrant, e.g., crospovidone, may be used singly or in combination with other disintegrants. Crospovidone includes any cross-linked 1-ethenyl-2-pyrrolidinone homopolymer, and may comprise a polymer of molecular weight of 1,000,000 or more. Examples of commercially available crospovidone include Cross-linked povidone, Kollidon CL, Polyplasdone XL, Polyplasdone XL-10, INF-10 (manufactured by ISP, Inc.), polyvinylpolypyrrolidone, PVPP and 1-vinyl-2-pyrrolidinone homopolymer. The disintegrants are optionally incorporated in a proportion of about 1-15 parts by weight, or about 1-10 parts by weight, or about 3-7 parts by weight, based on 100 parts by weight of the solid formulation.

Some embodiments include a solid buccal or sublingual formulation containing a F1 C where unit doses of the formulation substantially or completely disintegrates or erodes within about 20-120 seconds in water at 37°C or on insertion of the unit dose into the buccal area or upon placement under the tongue. Such formulations may comprise a swellable hydrophilic excipient, a water-soluble or a water-dispersible excipient, e.g., one or more of partially hydrolyzed gelatin, hydrolyzed dextran, dextrin, mannitol, alginates, polyvinyl alcohol, polyvinyl pyrrolidine, water soluble cellulose derivatives, methylcellulose, ethyl cellulose, carboxymethyl cellulose, hydroxymethylcellulose, hydroxypropyl methylcellulose, microcrystalline cellulose, alginates, gelatin, guar gum, gum tragacanth, gum acacia, polyacrylic acid, polymethacrylic acid, polysilicic acid, polylactic acid, polymaleic acid, polyvinyl alcohol, polyethylene glycol, polyvinyl pyrrolidone, nonionic blocked polymers, carbomers, polycarbophils, a water soluble starch, dicalcium phosphate, calcium carbonate, silica or polyethyleneglycol, e.g., PEG1000, PEG2000 or a polyethylene oxide ("PEO"), PEO1000, PEO100000 or PEO5000000.

Other embodiments include the product obtained by storing invention compositions or formulations, e.g., unit dosage forms or compositions used to make formulations, at about 4-40°C for at least about 30 days, e.g., storage at ambient temperature for about 1-24 months. Invention formulations will typically be stored in hermetically or induction sealed containers for these time periods. Compositions and formulations that comprise a F1C will typically be held in closed or sealed containers, particularly when the composition is a formulation for pharmaceutical or veterinary use.

Typical containers for storage of compositions and formulations that comprise a F1 C will limit the amount of water that reaches the materials contained therein. Typically, formulations are packaged in hermetically or induction sealed containers. The containers are usually induction sealed. Water permeation characteristics of containers have been described, e.g., Containers - Permeation, chapter, USP 23 <671 >, United States Pharmacopeial Convention, Inc., 12601 Twinbrook Parkway, Rockville, MD 20852, pp.: 1787 et seq. (1995).

Immune modulation. The F1Cs, or the biologically active substances produced from these compounds by hydrolysis or metabolism *in vivo*, have a number of clinical and non-clinical applications. The compounds are generally useful to correct immune dysregulation, e.g., imbalanced immune responses to disease conditions, pathogens or the like, suppression of an innate or acquired immune response(s) and inflammation conditions in vertebrate or mammalian subjects, e.g., as disclosed herein. Thus, while the compounds will generally enhance a deficient immune response in a given clinical condition, they will generally reduce the same immune response when it is too active in a different clinical condition. For example, they can enhance insufficient or suboptimal Th1 immune responses, reduce excess or undesirable Th2 immune responses, reduce excess or undesirable Th1 immune responses or enhance insufficient or suboptimal Th2 immune responses or they can reduce excess or undesirable inflammation or one or more of its symptoms. The compounds will generally also modulate dysregulated Tc1 and Tc2 immune responses (associated with CD8⁺ T cells) in a similar manner, e.g., excessive Tc1 or Tc2 responses will be detectably decreased and deficient or suboptimal Tc1 or Tc2 responses will generally be detectably enhanced.

Clinical indications that have an association with or have a symptom(s) that is consistent or associated with an excessive or unwanted Th2 immune response include, e.g., fatigue, pain, fever or an increased incidence of infection, schizophrenia, acute myelitis, tumor progression, progressive systemic sclerosis, Omenn's syndrome, atopic disease, atopy, allergen hypersensitivity, atopic asthma, atopic dermatitis, burns, trauma (e.g., bone fracture, hemorrhage, surgery), immune responses to xenotransplantation, chronic periodontitis, SLE (systemic lupus erythematosus), discoid lupus erythematosus, osteoporosis, myasthenia gravis, Graves disease, mite-associated ulcerative dermatitis, rheumatoid arthritis and osteoarthritis. Excessive Th2 immune responses are also associated with an unwanted symptom or pathology, e.g., fatigue, pain, fever or an increased incidence of infection, that is associated with aging, allergy and inflammation conditions such as allergic bronchopulmonary aspergillosis in cystic fibrosis patients, allergic respiratory disease, allergic rhinitis, atopic dermatitis, subepithelial fibrosis in airway hyperresponsiveness, chronic sinusitis, perennial allergic rhinitis, fibrosing alveolitis (lung fibrosis). This common underlying immune component is at least part of the pathology or symptoms of all of these conditions. This allows a F1 C to be effectively used to prevent or treat the condition or to treat or ameliorate one or more symptoms that are associated with these conditions. Thus, in some embodiments, an unwanted or excessive Th2 response is present and amelioration of one or more symptoms associated with this condition is accomplished by administering an effective amount of a F1C according to the methods described herein, e.g., F1C is administered using a formulation and a route of administration essentially as described herein on an intermittent or a daily basis.

Exemplary conditions where an immune imbalance or an excessive Th1 immune response is involved include autoimmune diseases such as multiple sclerosis, Crohn's disease (regional enteritis), ulcerative colitis, inflammatory bowel disease, rheumatoid arthritis, reactive arthritis, acute allograft rejection, sarcoidosis, type 1 diabetes mellitus, *Helicobacter pylori* associated peptic ulcer, graft versus host disease and Hashimotos' thyroiditis. Because these conditions are associated with a similar type immune dysfunction, a F1C can be effectively used to prevent or treat these conditions or to treat or ameliorate one or more symptoms associated therewith. Thus, in some embodiments, an unwanted or excessive Th1 response is present and amelioration of one or more symptoms associated with this condition is accomplished by administering an effective amount of a F1C according to the methods described herein, e.g., F1C is administered using a formulation and a route of administration essentially as described herein on an intermittent or a daily basis. In other embodiments, an deficient Th1 response is enhanced, which is optionally observed as a detectable increase in one or more of IFNγ, IL-2, IL-12 or IL-18 in Th1 cells or in accessory cells such as a dendritic cell or macrophage. In all of the conditions where an insufficient or excess Th1, Th2, Tc1 or Tc2 response is present, amelioration of one or more symptoms associated with the condition is accomplished by administering an effective amount of a F1C according to the methods described herein.

Thus, an aspect of the invention is a method to enhance the migration of one or more immune cell types in a subject from one location (e.g., bone marrow, circulating blood or a tissue such as the skin, liver, central nervous system or lung) to another (e.g., to the blood or to a lymphoid tissue such as a lymph node, spleen or a mucosal tissue such as GALT) by administration to a subject as described herein of an effective amount of a F1C essentially as described by any of the methods disclosed herein. A related aspect is the monitoring, e.g., by suitable blood counts or tissue biopsy, of the subject's response to determine the timing and extent of such immune cell migration.

Treatment of a subject with a F1 C can result in a change of at least about 20-80% or about 25-50% above or below (e.g., at least 30% or at least 40% above or below) the control or basal level of affected immune cell subsets. For example, increases of more than about 30% in the total numbers of activated CD8⁺ T cells, e.g., CD8⁺, CD69⁺, CD25⁺ T cells, CD8⁺, CD69⁺, CD25- T cells or CD8⁺, CD69⁻, CD25⁺ T cells, can occur by 7 days after a single dose of a F1 C to a subject. Such increases may be greater than 50%, 60% or 100% in the total numbers of activated CD8⁺ T cells or subsets of activated CD8⁺ T cells in individual subjects. Typically such increases are about in the total numbers of activated CD8⁺ T cells or subsets of activated CD8⁺ T cells averages about 30-40%, with individual subjects experiencing increases over 100% in the numbers of activated CD8⁺ T cells per unit blood volume compared to the basal level.

Administration of the F1Cs can affect other immune cell subsets. For example, the concentration of circulating CD4⁺, CD69⁺, CD25- (Th1 helper cells) and CD8⁺, CD16⁺, CD38⁺ LAK cells or CD8⁻, CD16⁺, CD38⁺ LAK cells typically increases during or after the course of dosing a subject with a F1C. Also, CD8⁻, CD16⁺, CD38⁺ and CD8⁺, CD16⁺, CD38⁺ (ADCC effector cells) and low side scatter Lin⁻, DR⁺, CD123⁺ (dendritic precursors) or low side scatter Lin⁻, DR⁺, CD11c⁺ (dendritic cells or precursors) may show modest to significant increases.

Infection treatments. In some embodiments, the F1C(s) is administered to a subject who has a pathogen infection, such as a viral, bacterial, fungal, yeast, intracellular parasite or extracellular parasite infection. The F1Cs can be considered for use in a broad scope of infections (see, e.g., J.B. Peter, editor, Use and Interpretation of Laboratory Tests in Infectious Disease, 5th edition, Specialty Laboratories, Santa Monica, CA 90404, 1998, pages 1-271), since the compounds generally enhance Th1 immune responses and/or reduce Th2 immune responses and/or reduce inflammation or its symptoms. Difficulty in treating many infections, e.g., progressive toxoplasmic encephalitis, malaria, tuberculosis, Leishmaniasis and schistosomiasis, often appear to be associated with one or more of an unwanted Th2 immune responses, a suboptimal Th1 response or the development of resistance of the infectious agent to antimicrobial agents. For example, in disseminated or diffuse tuberculosis, a reduced Th2 response would be desirable to allow a patient to slow progression of the disease or to clear infected cells more efficiently. In treating chloroquine resistant or sensitive malaria, the F1Cs have essentially the same activity.

Exemplary viral infections that the F1 Cs can be used to treat, prevent or ameliorate include infections by one or more DNA or RNA viruses, or a symptom(s) associated with such infection(s), such as a genogroup, clade, serotype, serotype subtypes, isolate, strain, subtype or so forth of influenza viruses (e.g., a human influenza A virus, a human influenza B virus, an avian (e.g., chicken, duck, goose) influenza virus, a swine influenza virus or a recombinant avian-swine influenza virus), respiratory syncytial viruses, Rotaviruses, Hantaviruses, animal or human Papillomaviruses (e.g., HPV-1, HPV-2, HPV-6, HPV-7, HPV-10, HPV-11, HPV-13, HPV-16, HPV-18, HPV-32, HPV-33, HPV-35, HPV-39, HPV-42, HPV-43, HPV-44, HPV-45, HPV-61, HPV-72 or HPV-83), Poxviruses, Poliovirus, rabies viruses, human and animal Retroviruses (e.g., HIV-1, HIV-2, LAV, human T-cell leukemia virus I ("HTLV I"), HTLV II, HTLV III, SIV, SHIV, FIV or FeLV), Togaviruses and Flaviviruses (e.g., West Nile Virus, Yellow Fever Virus, Dengue viruses), Herpesviruses (e.g., CMV, EBV, Varicella Zoster Virus (human Herpesvirus 3), Herpes simplex virus 1 ("HSV-1"), Herpes simplex virus 2 ("HSV-2"), human Herpesvirus 6 ("HHV-6"), human Herpesvirus 7, human Herpesvirus 8 ("HHV-8")), measles viruses, mumps viruses, rubella virus, Hepadnaviruses or hepatitis viruses, Adenoviruses, Retroviruses, Togaviruses, Alphaviruses, Arboviruses, Coronaviruses (e.g., human severe acute respiratory syndrome virus, Urbani SARS-associated coronavirus, human respiratory coronaviruses such as HCV-229E or HCV-OC43, including serogroups, genotypes, strains or variants of any of these viruses), Flaviviruses, Filoviruses, Rhinoviruses, Picornaviruses, Papovaviruses, Bunyaviruses, Picornaviruses, Poxviruses, Parvoviruses (e.g., human B19 parvovirus) and/or Pestiviruses.

Cancer and hyperproliferation conditions. Many cancers, precancers, malignancies or hyperproliferation conditions are associated with an unwanted Th2 immune response, a deficient Th1 response or unwanted inflammation. An insufficient Th1 immune response may play a role in the capacity of malignant or premalignant cells to escape immune surveillance. Any of the F1Cs disclosed herein, may thus be used to treat, prevent or slow the progression of one or more cancers, precancers or cell hyperproliferation conditions or they may be used to ameliorate one or more symptoms thereof. In these conditions, the F1Cs are useful to enhance the subject's Th1 responses or to reestablish a more normal Th1-Th2 balance in the subject's immune responses. The F1 Cs may function at least in part by decreasing inflammation or inflammation associated markers such as IL-6 and/or by enhancing hematopoiesis in many of these conditions.

These conditions include cancers or precancers comprising carcinomas, sarcomas, adenomas, blastoma, disseminated tumors and solid tumors such as one associated with or arising from prostate, lung, breast, ovary, skin, stomach, intestine, pancreas, neck, larynx, esophagus, throat, tongue, lip, oral cavity, oral mucosa, salivary gland, testes, liver, parotid, biliary tract, colon, rectum, cervix, uterus, vagina, pelvis, endometrium, kidney, bladder, central nervous system, glial cell, astrocyte, squamous cell, blood, bone marrow, muscle or thyroid cells or tissue. The F1 Cs are thus useful to treat, prevent, slow the progression of, or ameliorate one or more symptoms of a precancer, cancer or related hyperproliferation condition such as myelodysplastic syndrome, actinic keratoses, endometriosis, Barrett's esophagus, leiomyoma, fibromyoma, benign or precancerous intestinal or bowel polyps or benign prostatic hyperplasia. The compounds can also be used to treat, prevent, slow the progression of, slow the replication or growth of, or to ameliorate one or more symptoms of a primary tumor, a metastasis, an advanced malignancy, a blood born malignancy, a leukemia or a lymphoma. Any of these conditions may be in an early or mild form or can be moderate or advanced in the existent or progression of the disease or a symptom.

Cardiovascular applications. Any of the F1Cs disclosed herein, may be used to treat, prevent or slow the progression of one or more of congenital heart defects, cardiovascular diseases, disorders, abnormalities and/or conditions, or to ameliorate one or more symptoms thereof in a subject. These include peripheral artery disease, arterio-arterial fistula, arteriovenous fistula, cerebral arteriovenous malformations, aortic coarctation, cor triatum, coronary vessel anomalies, patent ductus arteriosus, Ebstein's anomaly, hypoplastic left heart syndrome, levocardia, transposition of great vessels, double outlet right ventricle, tricuspid atresia, persistent truncus arteriosus, and heart septal defects, such as aortopulmonary septal defect, endocardial cushion defects, Lutembacher's Syndrome, ventricular heart septal defects, cardiac tamponade, endocarditis (including bacterial), heart aneurysm, cardiac arrest, congestive heart failure, congestive cardiomyopathy, paroxysmal dyspnea, cardiac edema, post-infarction heart rupture, ventricular septal rupture, heart valve diseases, myocardial diseases, pericardial effusion, pericarditis (including constrictive and tuberculous), pneumopericardium, postpericardiotomy syndrome, pulmonary heart disease, rheumatic heart disease, ventricular dysfunction, hyperemia, cardiovascular pregnancy complications, cardiovascular syphilis, cardiovascular tuberculosis, arrhythmias such as sinus arrhythmia, atrial fibrillation, atrial flutter, bradycardia, extrasystole, Adams-Stokes Syndrome, bundle-branch block, sinoatrial block, long QT syndrome, parasystole, sick sinus syndrome, ventricular fibrillations, tachycardias such as paroxysmal tachycardia, supraventricular tachycardia, accelerated idioventricular rhythm, atrioventricular nodal reentry tachycardia, ectopic atrial tachycardia, ectopic junctional tachycardia, sinoatrial nodal reentry tachycardia, sinus tachycardia, Torsades de Pointes, and ventricular tachycardia and heart valve diseases such as aortic valve insufficiency, aortic valve stenosis, hear murmurs, aortic valve prolapse, mitral valve prolapse, tricuspid valve prolapse, mitral valve insufficiency, mitral valve stenosis, pulmonary atresia, pulmonary valve insufficiency, pulmonary valve stenosis, tricuspid atresia, tricuspid valve insufficiency, and tricuspid valve stenosis.

The F1 Cs can be used to treat, prevent or ameliorate one or more symptoms of myocardial diseases or pathological myocardial or vascular conditions such as alcoholic cardiomyopathy, congestive cardiomyopathy, hypertrophic cardiomyopathy, aortic subvalvular stenosis, pulmonary subvalvular stenosis, restrictive cardiomyopathy, Chagas cardiomyopathy, endocardial fibroelastosis, myocardial fibrosis, endomyocardial fibrosis, Kearns Syndrome, myocardial reperfusion injury, myocarditis, cardiovascular or vascular diseases such as dissecting aneurysms, false aneurysms, infected aneurysms, ruptured aneurysms, aortic aneurysms, cerebral aneurysms, coronary aneurysms, heart aneurysms, and iliac aneurysms, angiodysplasia, angiomatosis, bacillary angiomatosis, Sturge-Weber Syndrome, angioneurotic edema, aortic diseases, Takayasu's Arteritis, aortitis, Leriche's Syndrome, arterial occlusive diseases, arteritis, enarteritis, polyarteritis nodosa, cerebrovascular diseases, disorders, and/or conditions, diabetic angiopathies, diabetic retinopathy, thrombosis, erythromelalgia, hemorrhoids, hepatic veno-occlusive disease, hypertension, hypotension, idiopathic pulmonary fibrosis, peripheral vascular diseases, phlebitis, pulmonary veno-occlusive disease, Raynaud's disease, CREST syndrome, retinal vein occlusion, Scimitar syndrome, superior vena cava syndrome, telangiectasia, atacia telangiectasia, hereditary hemorrhagic telangiectasia, varicocele, varicose veins, varicose ulcer, vasculitis, venous insufficiency and arterial occlusive diseases such as arteriosclerosis, intermittent claudication, carotid stenosis, fibromuscular dysplasias, mesenteric vascular occlusion, Moyamoya disease retinal artery occlusion, thromboangiitis obliterans or atherosclerosis, any of which may be at an early stage or at a more advanced or late stage.

The F1 Cs can also be used to treat, prevent or ameliorate one or more symptoms of cerebrovascular diseases, thrombosis, and/or conditions such as carotid artery diseases, cerebral amyloid angiopathy, cerebral aneurysm, cerebral anoxia, cerebral arteriosclerosis, cerebral arteriovenous malformation, cerebral artery diseases, cerebral embolism and thrombosis, carotid artery thrombosis, sinus thrombosis, Wallenberg's syndrome, cerebral hemorrhage, epidural hematoma, subdural hematoma, subarachnoid hemorrhage, cerebral infarction, cerebral ischemia (including transient), subclavian steal syndrome, periventricular leukomalacia, vascular headache, cluster headache, migraine, vertebrobasilar insufficiency, air embolisms, embolisms such as cholesterol embolisms, fat embolisms, pulmonary embolisms or amniotic fluid embolism, thromoboembolisms, thrombosis such as coronary thrombosis, hepatic vein thrombosis, retinal vein occlusion, carotid artery thrombosis, sinus thrombosis, Wallenberg's syndrome, and thrombophlebitis.

The F1 Cs also can limit inflammation or cell injury that is associated with ischemia or oxygen reperfusion after ischemia. Ischemia, which is a detrimental decrease in oxygenated blood delivery to affected cells or tissues, may arise from a cardiovascular condition or event such as an infarction, or from thermal injury or burns. Ischemia may also arise from accidental or surgical trauma. Reperfusion after cells have become hypoxic for a sufficient period of time can lead to tissue or cell injury that varies from slight to lethal. The compounds can reduce cell or tissue injury or death associated with ischemia and reperfusion, by, e.g., reducing inflammation or the level of a molecule associated with inflammation. Thus, levels of a proinflammatory cytokine or molecule such as leukotriene B4, platelet activating factor or levels of extracellular P-selectin may result from administration of a F1 C to a subject who may experience reperfusion injury. Thus, the compounds can reduce injury or death of, e.g., neuron, cardiac, vascular endothelium, myocardial, pulmonary, hepatic or renal cells or tissues. Without wishing to be bound by any theory, the compounds may act in part by reducing one or more of neutrophil activation, platelet activation, platelet aggregation, endothelial cell activation and neutrophil adherence or adhesion to endothelial cells in these conditions.

The compounds can thus be used in diabetes, obesity, hyperlipidemia or hypercholesterolemia conditions to reduce body fat mass, increase muscle mass or to lower one or more of serum or blood low density lipoprotein, triglyceride, cholesterol, apolipoprotein B, free fatty acid or very low density lipoprotein compared to a subject that would otherwise be considered normal for one or more of these characteristics. These beneficial effects are typically obtained with little or no effect on serum or blood high density lipoprotein levels. The F1Cs are useful to reduce or slow the rate of myocardial tissue or myocyte damage, e.g., fibrosis, or to enhance cardiac fatty acid metabolism in conditions, such as inflammation, where fatty acid metabolism is depressed or decreased. Elevated cholesterol levels are often associated with a number of other disease states, including coronary artery disease, angina pectoris, carotid artery disease, strokes, cerebral arteriosclerosis, and xanthoma, which the F1Cs can ameliorate or slow the progression or severity of. Abnormal lipid and cholesterol conditions that can be treated include exogenous hypertriglyceridemia, familial hypercholesterolemia, polygenic hypercholesterolemia, biliary cirrhosis, familial combined hyperlipidemia, dysbetalipoproteinemia, endogenous hypertriglyceridemia, mixed hypertriglyceridemia and hyperlipidemia or hypertriglycidemia secondary to alcohol consumption, diabetic lipemia, nephrosis or drug treatments, e.g., corticosteroid, estrogen, colestipol, cholestyramine or retinoid treatments. Dosages, routes of administration and dosing protocols for the F1 Cs are essentially as described herein. Where the condition is chronic, the F1 Cs will generally be administered to a subject such as a human for a relatively long time period, e.g., for about 3 months to about 10 years or more. Dosages, routes of administration and dosing protocols for the F1 Cs are essentially as described herein. Dosing of the compound can be daily or intermittent using a dosing protocol using dosages as described herein, e.g., about 0.01 to about 20 mg/kg of a F1 C administered to a subject once or twice per day daily or intermittently. The use of the F1 Cs can be combined with one, two or more other suitable treatments, e.g., treatment for cessation of smoking, diet control, e.g., caloric restriction, reduced fat intake or reduced carbohydrate intake, or treatment with fibrates, non-steroidal anti-inflammatory drugs, angiotensin-converting enzyme inhibitors or HMG-CoA reductase inhibitors such as aspirin, clofibrate, fenofibrate, ciprofibrate, gemfibrozil, Simvastatin^{™}, Pravastatin^{™}, Mevastatin^{™} or Lovastatin^{™}.

The use of any F1C or species in any genus of F1Cs disclosed herein to treat, prevent or ameliorate any of these cardiovascular or metabolic disorders or symptoms will generally use one or more of the routes of administration, dosages and dosing protocols as disclosed herein. Thus, in exemplary embodiments, about 0.5 to about 100 mg/kg or about 1 to about 25 mg/kg, of the F1C will be administered per day by an oral, buccal, sublingual or parenteral route. Such administration can be, e.g., daily for about 5 to about 60 days in acute conditions or it can be intermittent for about 3 months to about 2 years or more for chronic conditions. Alternatively, intermittent dosing can be used essentially as described herein for acute cardiovascular conditions. In some embodiments, for conditions such as ischemia or trauma, administration of the F1 C is provided before or as soon after the ischemic or traumatic event as possible, e.g., within about 6 hours of an ischemic or traumatic event or about 12-24 hours before an anticiapted ischemic or traumatic event. In other embodiments, administration of the F1C can be delayed for, e.g., about 6, 7, 8, 9, 10, 11, 12, 13, 14, 15, 16, 18, 19, 20, 21, 24, 28, 32, 36, 40, 48 or more hours after an ischemic or traumatic event has occurred and a course of daily or intermittent dosing is initiated af one of these times, or in a range between any of these times after the event. Thus, administration of the F1C can begin at about 10-14 hours, at about 11-13 hours or at about 8-16 hours after the ischemic or traumatic event.

In another aspect of the invention, the F1Cs can be used to prevent, treat or to reduce the severity of vascular or microvascular occlusions in human sickle cell diseases (SCD). SCD is heterogenous and includes subgroups with high transcranial velocities, which is a group with an increased risk of infarctive stroke or cereberal thrombosis. SCD types also include sickle cell-β⁺ thalassemia, sickle cell-β^{o} thalassemia, sickle cell-δβ^{o} thalassemia and sickle cell-HPFH (hereditary of persistent fetal hemoglobin). Another subgroup of SCD patients is characterized by the presence of a *Plasmodium* parasite infection. SCD is usually accompanied by acute vaso-occlusive episodes such as microvascular occlusions, ischemia and infarctions that arise from adhesion of sickle cells and other blood cell types, e.g., platelets or leukocytes, to vascular endothelial cells. Reduced sickle cell adhesion in response to treatment with a F1C and related responses is facilitated at least in part by decreased production or activity of one or more biological response mediators such as one, two, three or more of thrombospondin, von Willebrand factor, epinephrine, C reactive protein, cAMP, basal cell adhesion molecule/Lutheran (BCAM/Lu), P-selectin, L-selectin, E-selectin, VCAM-1, ICAM-1, fibronectin, annexin V, placenta growth factor, superoxide, CD11a, CD11b, CD11c, CD15, CD18, CD31, CD36, TNFα, NF-κB, IL-1β or IL-6 by endothelial cells or one or more immune cell types as described herein. In treating sickle cell disease, the F1Cs will also increase the activity or levels of one, two or more desired response mediators including fetal hemoglobin, erythropoietin, heme oxygenase, nitric oxide, PPARα, PPARγ or GM-CSF. The F1Cs will thus ameliorate one or more symptoms of sickle cell disease such as anemia, stroke, pain, e.g., chest or abdominal pain, skin ulcers, dyspnea, organ damage, retinopathy or the level of infected red cells in *Plasmodium*-infected subjects. Treatment of acute SCD episodes or of chronic SCD with F1Cs can be combined with other suitable therapies, e.g., inhaled nitric oxide, hydroxyurea treatment, anti-adhesion molecule antibody treatment or analgesic use such as morphine, oxycodone, or codeine. The F1Cs can also be used to reduce cellular damage from reactive oxygen species associated with hydroxyurea treatment, since the F1Cs will enhance cellular antioxidant capacity.

Respiratory and pulmonary conditions. F1Cs can be used to treat, ameliorate, prevent or slow the progression of a number of pulmonary conditions or their symptoms such as 1, 2, 3 or more of cystic fibrosis, bronchiectasis, cor pulmonale, pneumonia, lung abcess, acute bronchitis, chronic bronchitis, a chronic obstructive pulmonary disease (COPD) condition, bronchopulmonary dysplasia, emphysema, pneumonitis, e.g., hypersensitivity pneumonitis or pneumonitis associated with radiation exposure, alveolar lung diseases and interstitial lung diseases, e.g., associated with asbestos, fumes or gas exposure, aspiration pneumonia, pulmonary hemorrhage syndromes, amyloidosis, connective tissue diseases, systemic sclerosis, ankylosing spondylitis, allergic granulomatosis, granulomatous vasculitides, asthma, e.g., mild intermittent asthma, mild persistent asthma, moderate persistent asthma, severe persistent asthma, acute asthma, chronic asthma, atopic asthma, allergic asthma or idiosyncratic asthma, cystic fibrosis and associated conditions, e.g., allergic bronchopulmonary aspergillosis, chronic sinusitis, pancreatic insufficiency, lung or vascular inflammation, bacterial or viral infection, e.g., *Haemophilus influenzae*, *S. aureus, Pseudomonas aeruginosa* or RSV infection or an acute or chronic adult or pediatric respiratory distress syndrome (RDS) suh as grade I, II, III or IV RDS or an RDS associated with, e.g., sepsis, pneumonia, reperfusion, atelectasis or chest trauma. Chronic obstructive pulmonary diseases include conditions where airflow obstruction is located at upper airways, intermediate-sized airways, bronchioles or parenchyma, which can be manifested as, or associated with, tracheal stenosis, tracheal right ventricular hypertrophy, pulmonary hypertension, polychondritis, bronchiectasis, bronchiolitis, e.g., idiopathic bronchiolitis, ciliary dyskinesia, asthma, emphysema, connective tissue disease, bronchiolitis of chronic bronchitis or lung transplantation. The F1C can be used to treat or ameliorate acute or chronic asthma or their symptoms or complications, including airway smooth muscle spasm or hyperresponsiveness, airway mucosa edema, increased mucus secretion, excessive T cell activation, airway epithelium injury or desquamation, atelectasis, cor pulmonale, pneumothorax, subcutaneous emphysema, dyspnea, coughing, wheezing, shortness of breath, tachypnea, fatigue, decreased forced expiratory volume in the 1^{st} second (FEV₁), arterial hypoxemia, respiratory acidosis, inflammation including unwanted elevated levels of mediators such as IL-4, IL-5, IgE, histamine, substance P, neurokinin A, calcitonin gene-related peptide or arachidonic acid metabolites such as thromboxane or leukotrienes (LTD₄ or LTC₄), and cellular airway wall cellular infiltration, e.g., by eosinophils, lymphocytes, macrophages or granulocytes. Any of these and other pulmonary conditions or symptoms that can be treated with F1C are described elsewhere, e.g., The Merck Manual, 17th edition, M.H. Beers and R. Berkow editors, 1999, Merck Research Laboratories, Whitehouse Station, NJ, ISBN 0911910-10-7, or in other references cited herein. In some of these conditions where inflammation plays a role in the pathology of the condition, the F1Cs can ameliorate or slow the progression of the condition by reducing damage from inflammation. In other cases, the F1Cs act to limit pathogen replication or pathogen-associated lung tissue damage. Other standard treatments can be combined with the use of the F1Cs to treat these conditions or symptoms, e.g., asthma, RDS or COPD, including the use of anticholinergic agents, β2-adrenoreceptor agonists such as formoterol or salmeterol, corticosteroids, antibiotics or antihypertension agents.

For these conditions, the severity of the disease or the type or severity of associated symptoms can vary. For example, in humans having pediatric, e.g., infants or children of about 1 month or about 4 months of age to about 16 or 17 years of age, or adult cystic fibrosis ("CF"), the disease may be associated with the presence of one or more symptoms, syndromes, genetic mutations or the like. Symptoms or syndromes that can be observed in human CF patients include 1, 2, 3, 4 or more of *Staphylococcus* (e.g., *S. aureus*), *Haemophilus influenzae, Pseudomonas* or *Burkholderia* respiratory tract or lung infection or propensity to develop detectable infection or colonization, coughing, wheezing, cyanosis, bronchiolitis, bronchospasm, pneumothorax, hemoptysis, pancreatic exocrine insufficiency, bronchiectatic lung disease, atelectasis-consolidation, pulmonary edema, increased lung vascular hydrostatic pressure, increased lung vascular permeability, sinusitis, respiratory insufficiency, bronchial wall or interlobular septa thickening, reduction of forced expiratory volume in 1 second, dyspnea, impaired male fertility, elevated sweat chloride (e.g., > 60 mmol/L), mucous plugging, tree-in-bud sign, mosaic perfusion pattern, glucose intolerance or abnormal elevation of one or more of IL-4, IL-8, RANTES, neutrophil elastase, eosinophils, macrophages, neutrophils, eosinophil cationic protein or cysteinyl leukotrienes. Any of these symptoms or syndromes can be acute, intermittent or chronic and/or mild, moderate or severe. Relevant mutations include, e.g., a homozygous or heterozygous, dominant or recessive deletion, insertion and/or point mutation in (1) the cationic trypsinogen gene or (2) the cystic fibrosis transmembrane conductance regulator (CFTR) gene, such as one, two or more of , a CFTR F508del deletion mutation or CFTR lacking phe508, 3272-26A>G/F508del, 3659delC, 394delTT, S1455X or Δ26, I1234V, 2183AA>G, 2043delG, 548A>T, I148T, R334W, S1196X, 4041C>G, 1161delC, 1756G>T or 3120+1 G>A mutation.

The use of a F1 C to treat, ameliorate or slow the progression of conditions such as CF can be optionally combined with other suitable treatments. For CF, this includes, e.g., one, two or more of oral or aerosol corticosteroid treatment, ibuprofen treatment, DNAse or IL-10 treatment, diet control, e.g., vitamin E supplementation, vaccination against pathogens, e.g., *Haemophilus influenzae*, or chest physical therapy, e.g., chest drainage or percussion.

Applications in autoimmunity, allergy, inflammation and related conditions. As mentioned above, the F1Cs may be used to treat, prevent or slow the progression of one or more autoimmune allergic or inflammatory diseases, disorders, or conditions, or to ameliorate one or more symptoms thereof in a subject. These diseases and conditions include Addison's Disease, autoimmune hemolytic anemia, autoimmune sensorineural hearing loss, antiphospholipid syndrome, acute or chronic rheumatoid arthritis and other synovial disorders, an osteoarthritis including post-traumatic osteoarthritis and hypertrophic pulmonary osteoarthropathy, psoriatic arthritis, polyarthritis, epichondylitis, type I diabetes, type II diabetes, rheumatic carditis, bursitis, ankylosing spondylitis, multiple sclerosis, a dermatitis such as contact dermatitis, atopic dermatitis, exfoliative dermatitis or seborrheic dermatitis, mycosis fungoides, allergic encephalomyelitis, autoimmune glomerulonephritis, Goodpasture's Syndrome, Graves' Disease, Hashimoto's Thyroiditis, multiple sclerosis, myasthenia gravis, neuritis, bullous pemphigoid, pemphigus, polyendocrinopathies, purpura, Reiter's Disease, autoimmune thyroiditis, systemic lupus erythematosus, systemic lupus erythematosus, lupus erythematosus-related arthritis, discoid lupus erythematosus, subacute cutaneous lupus erythematosus, scleroderma, fibromyalgia, chronic fatigue syndrome, autoimmune pulmonary inflammation, Guillain-Barre Syndrome, type 1 or insulin dependent diabetes mellitus, autoimmune inflammatory eye disease, hepatitis C virus associated autoimmunity, postinfectious autoimmunity associated with, e.g., virus or bacterial infection such as a parvovirus such as human parvovirus B19 or with rubella virus, autoimmune skin and muscle conditions such as pemphigus vulgaris, pemphigus foliaceus, systemic dermatomyositis or polymyositis or another inflammatory myopathy, myocarditis, asthma such as allergic asthma, allergic encephalomyelitis, allergic rhinitis, a vasculitis condition such as polyarteritis nodosa, giant cell arteritis or systemic necrotizing vasculitis, chronic and an acute or chronic inflammation condition such as chronic prostatitis, granulomatous prostatitis and malacoplakia, ischemia-reperfusion injury, endotoxin exposure, complement- mediated hyperacute rejection, nephritis, cytokine or chemokine induced lung injury, cachexia, sarcoidosis, inflammatory bowel disease, regional enteritis, ulcerative colitis, Crohn's disease, inflammatory bowel disease or inflammation associated with an infection, e.g., septic shock, sepsis, or systemic inflammatory response syndrome. Any of these diseases or conditions or their symptoms may be acute, chronic, mild, moderate, severe, stable or progressing before, during or after the time administration of the F1 C to a subject such as a human, is initiated. In general, a detectable improvement is observed in the subject within a period of about 3 days to about 12 months after initiation of a dosing protocol, e.g., the severity of the disease or condition will detectably decrease, the rate of progression will detectably slow or the severity of a symptom(s) will detectably decrease.

As used herein, acute inflammation conditions are characterized as an inflammation that typically has a fairly rapid onset, quickly becomes moderate or severe and usually lasts for only a few days or for a few weeks. Chronic inflammation conditions as used herein are characterized as an inflammation that may begin with a relatively rapid onset or in a slow, or even unnoticed manner, tends to persist for at least several weeks, e.g., about 3-6 weeks, months, or years and may have a vague or indefinite termination. Chronic inflammation may result when the injuring agent (or products resulting from its presence) persists in the lesion, and the subject's tissues respond in a manner (or to a degree) that is not sufficient to overcome completely the continuing effects of the injuring agent. Other exemplary conditions are described in, e.g., Textbook of Autoimmune Diseases, R.G. Lahita, editor, Lippincott Williams & Wikins, Philadelphia, PA, 2000, ISBN 0-7817-1505-9, pages 175-851 and Rheumatology, 2nd edition, J.H. Klippel et al., editors, 1998, ISBN 0-7234-2405-5, volume 1, sections 1-5 and volume 2, sections 6-8, Mosby International, London, UK.

A F1 C can be used to inhibit or ameliorate one or more inappropriate immune responses or their symptoms in autoimmunity, inflammation, allergy or related conditions. The effects of the F1Cs include detectably ameliorating one or more of (1) the proliferation, differentiation or chemotaxis of T cells, (2) reducing unwanted cytotoxic T cell responses, (3) reducing unwanted autoantibody or other antibody synthesis, e.g., an unwanted IgA, IgE, IgG or IgM, in allergy, asthma or another autoimmune or inflammation condition, (4) inhibiting the development, proliferation or unwanted activity of autoreactive T or B cells, (5) altering the expression of one or more cytokines, interleukins or cell surface antigens, e.g., a cytokine, interleukin or cell surface antigen described herein (decreasing IL-8 in an autoimmune condition, decreasing the level of acute phase proteins such as C reactive protein or fibrinogen in inflammation conditions, (6) decreasing eosinophilia in allergy conditions, (7) detectably decreasing the level or activity of one or more of ICAM-1, IL-1α, IL-1β, TNFα, IL-6 or IL-8 in, e.g., inflammation conditions or in autoimmune conditions such as an arthritis or a myocarditis condition such as osteoarthritis, rheumatoid arthritis, toxic myocarditis, indurative myocarditis or idiopathic myocarditis, (8) decreasing the level or biological activity of one or more of anti-islet antibody, TNF, IFN-γ, IL-1, an arthritis symptom(s), nephritis, skin rash, photosensitivity, headache frequency or pain, migraine frequency or pain, abdominal pain, nausea or anti-DNA antibodies in , e.g., insulin dependent diabetes mellitus or an autoimmune or inflammation condition such as systemic lupus erythematosus, rheumatoid arthritis or Crohn's disease, (9) reducing induction of arachidonic acid metabolism or reducing eicosanoid metabolites such as thromboxanes or prostaglandins in, e.g., inflammation, asthma or allergy, (10) reducing IL-4, IL-8 or IL-10 synthesis, levels or activity in, e.g., allergy or inflammation such as idiopathic pulmonary fibrosis or allergic asthma or (11) reducing or interfering with neutrophil chemotaxis by, e.g., reducing thioredoxin release from affected cells in conditions such as cancer, infections, inflammation or autoimmunity.

Exemplary symptoms that the use of the F1Cs can ameliorate in these autoimmune, inflammatory and allergy conditions include one or more of pain such as shoulder, hip, joint, abdominal or spine pain, joint stiffness or gelling, bursitis, tendonitis, edema or swelling, fatigue or malaise, headache, dyspnea, skin rash, fever, night sweats, anorexia, weight loss, skin or intestine ulceration, muscle weakness, pericarditis, coronary occlusion, neuropathy and diarrhea. In treating one of these conditions in a subject or in improving one or more symptoms thereof, the F1Cs may accomplish one or more of decreasing levels of one or more of IL-1, IL-4, IL-6 or TNFα, decreasing levels of C reactive protein, fibrinogen or creatinine kinase. Other biological effects associated with treatment using a F1C may also be monitored or observed, e.g., an increase or decrease of a cell surface antigen, a cytokine or an interleukin as disclosed herein.

In another aspect of the invention, the F1 Cs can be used to treat or to reduce the severity of chronic allergies or atopic diseases such as allergic rhinitis, psoriasis, eczema, gastrointestinal allergies, atopic dermatitis conditions, allergic asthma, food allergies and hay fever. These conditions are typically characterized by the presence of elevated levels of allergen specific antibodies of the IgE isotype. In treating or ameliorating these conditions, the F1Cs reduce the generation of IgE by "isotype switching", which is increasing allergen-specific IgA production and/or decreasing IgE production from preexisting allergen-primed cells. Allergen specific IgG may also be increased from new cells that might otherwise have responded to allergen exposure by generating unwanted IgE.

Regeneration and wound healing. The F1Cs can be used to facilitate cell differentiation, proliferation or repair where regeneration of tissues is desired. The regeneration of tissues could be used to repair, replace, protect or limit the effects of tissue damaged by congenital defects, trauma (wounds, burns, incisions, or ulcers), age, disease (e.g. osteoporosis, osteoarthritis, periodontal disease, liver failure), surgery, including cosmetic plastic surgery, fibrosis, reperfusion injury, toxin exposure or systemic cytokine damage. Ulcers or skin lesions can arise from ionizing radiation exposure, cytotoxic chemotherapy or pressure, e.g., a pressure or decubitis ulcer or vascular insufficiency, e.g., associated with diabetes or vascular occlusion. Tissues for which regeneration may be enhanced include organs (e.g., pancreas, liver, lung, intestine, kidney, skin, endothelium, oral mucosa, gut or intestinal mucosa), muscle (e.g., smooth, skeletal or cardiac), vasculature (including vascular and lymphatics), central or peripheral nervous tissue, hematopoietic tissue, and skeletal tissue (e.g., bone, cartilage, tendon, and ligament). Decreased scarring or an increased rate or quality of healing may accompany these effects.

The F1 Cs are thus useful to enhance healing or tissue repair in a subject having a bone fracture(s), e.g., a simple or compound skull, spine, hip, arm or leg bone fracture. Similarly, nerve or brain tissue treatment using a F1C allows treating, slowing the progression of, ameliorating or preventing diseases such as central and peripheral nervous system diseases, neuropathies, or mechanical and traumatic diseases, disorders, and/or conditions (e.g., spinal cord disorders, head trauma, cerebrovascular disease, and stoke). The compounds are useful to treat diseases associated with peripheral nerve injuries, peripheral neuropathy (e.g., resulting from chemotherapy, radiation exposure or therapy or other medical therapies), localized neuropathies, and central nervous system diseases such as Alzheimer's disease, Parkinson's disease, Huntington's disease and amyotrophic lateral sclerosis. The subjects undergoing treatment in these conditions may be elderly, e.g., a human at least about 55, 60, 65 or 70 years of age. Where the condition is acute, e.g., a bone fracture or a burn, the treatment may comprise administration of a F1C to the subject on a daily or intermittent basis for about 3 days to about 12 months, e.g., administration for about 2-12 weeks beginning after the subject sustains an injury.

An aspect of the F1 Cs is their capacity to facilitate wound or trauma healing or to slow or at least partially reverse tissue organ impairment or damage associated with surgery, aging, chemotherapy or radiation exposure by increasing the proliferation or self-renewal of stem cells and pluripotent derivatives of stem cells and/or by increasing the rate of differentiation of stem cells or their pluripotent derivatives to more mature cell types. Thus, the F1Cs can increase the numbers, rate of differentiation, growth or activity of stem cells in, e.g., skin, central or nervous system tissue, blood vessels, heart tissue, lung, liver, pancreas, kidney, thymus, spleen, oral mucosa, intestine, bone marrow, or connective tissue some of which is discussed elsewhere herein. Cell types that can be affected include cells that give rise to neurons, glial cells, astrocytes, hepatocytes, thymus cells, spleen cells, lung tissue, skin tissue, fibroblasts, myocytes such as smooth muscle and striated muscle, including cardiac muscle cells, chondrocytes, osteoblasts and other stem cell types in other organs or tissue. Increased numbers of mature cell types typically is observed beginning at about 2-28 days after treatment with a F1C is started, usually after about 2-21 days. Thus, the F1Cs can enhance the numbers, activities or differentiation of, e.g., crypt cells in intestinal mucosa, skin cells, e.g., stem cells, in the oral mucosa or cardiac precursor cells after damage to those cells or tissues. Such damage can arise, e.g., from trauma, infection, ionizing radiation exposure, toxin exposure and/or cytotoxic chemotherapy. The F1Cs can thus be used to facilitate or enhance healing, reepithelialization or reendothelialization of skin, intestine, mucosal or endothelial cells after the occurrence of a wound, infarction, burn or other event that damages or impairs the barrier function or hemostasis capacity of such tissues. The barrier function or hemostasis capacity of such tissues includes their capacity to act as a physical barrier against infection or to maintain normal blood flow or composition. Optimal modulation of stem cell survival, self-renewal and differentiation in these embodiments is usually obtained by dosing the F1 C at a time period near the time that the subject is exposed to an agent, event or treatment that can cause significant tissue damage. Typically this time period is about 1, 2, 3, 4 or 5 days before, on the same day as or within 1, 2, 3, 4 or 5 days after the damaging event or exposure occurs. For chronic toxin exposure, e.g., alcohol, chronic continuous or intermittent administration of the F1C can be used. Dosages of the F1Cs, routes of administration and dosing protocols for these embodiments are as described herein.

As noted above, the F1 Cs are useful to enhance healing in a subject who has experienced or who is expected to experience one or more traumas or acute injuries such as a wound, burn, bone fracture, nervous system tissue trauma, gastrointestinal damage or intestinal cell damage or other traumatic events. In some embodiments, such subjects have experienced a trauma and who are immune suppressed or are anticipated to become immune suppressed. The immune suppression may arise from, e.g., a myelosuppressive cancer therapy, a glucocorticoid therapy or from radiation exposure. Thus, in some cases a subject such as a human or a primate who has experienced a trauma, e.g., a bone fracture, a chemical or thermal burn, a cut or a laceration, is also exposed to, e.g., an ionizing radiation as described herein such as γ-radiation, β-radiation, X-radiation or neutron radiation in an immune suppressive amount or dose, e.g., about 0.3 Gy ("gray") to about 30 Gy, typically about 0.5 Gy to about 12 Gy or about 0.7 Gy to about 8 Gy. The subject's radiation exposure can be localized or whole body and can occur rapidly, e.g., over a period of up to about 20 minutes, or more slowly, e.g., over a period of about 5-25 minutes to about 5-72 hours or more. A Gy of radiation is 1 joule per kg of absorbed ionizing radiation. The trauma event and the radiation exposure event may occur at about the same time, e.g., on the same day, or within a time period of about 1, 2, 3, 4, 5 or 6 days to about 1, 2, 3 or 4 weeks, when detectable clinical effects of both events are present. Treatment with the F1 C will use the dosing protocols, dosages and routes of F1C administration as described herein, e.g., dosing daily or every other day for about 1-12 days using dosages of about 0.1 mg/kg to about 30 mg/kg, depending on the route of administration and the subject's condition. Dosing of the F1C will usually commence within a few days of the radiation exposure event, e.g., within 0, 1, 2, 3 or 4 days. Similarly, such healing or repair of traumas in subjects who are or are expected to become immune suppressed, e.g., from an immunosuppressive chemotherapy, cancer, stress, infection or from aging, can be treated in the same manner.

The F1 Cs are also useful in the prophylaxis or treatment of nosocomial infections, such as those associated with elderly or special population patients that are hospitalized for trauma or other treatments. Such treatments include treatment of cancer, stroke or bone fracture patients with a F1C to prevent or to reduce the severity of nosocomial or other infections. Typical bone fractures include hip, arm or leg fractures. F1Cs in these treatments include those described herein such as 3β,17β-dihydroxyandrostane, 3α,17β-dihydroxyandrostane, 3-oxo-17β-hydroxyandrostane, 3-oxo-17α-hydroxyandrostane, 3β,17β-dihydroxyandrost-5-ene, 3α,17β-dihydroxyandrost-5-ene, 3β,17β-dihydroxyandrost-5(10)-ene, 3β-hydroxy-17β-mercaptoandrost-5-ene, 3α-hydroxy-17β-mercaptoandrost-5-ene, 3β-hydroxy-17β-mercaptoandrost-5(10)-ene and analogs of these compounds that contain (1) an oxygen-, nitrogen- or sulfur-linked moiety as described herein in the α- or β-configuration such as -OH, =O, -SH, =S, -NH₂, -NHCH₃, -N(CH₃)₂, =NOH, ether, ester at one, two or more of, e.g., the 2-, 6-, 7-, 12- or 16-position, (2) -F, -Cl, -Br or -I at the 9-position in the α- or β-configuration and/or (3) one or more double bonds at the 1-, 2-, 7- 11-, 14- or 15-positions. In these embodiments, the F1 C can be given as a depot injection at the time of hospital admission and periodically thereafter, e.g., every second or third day, to the hospital or as daily doses as needed. In other embodiments, the F1C is administered to hospitalized patients who are given antibiotics or antimicrobials prophylactically or to treat an existing infection.

Neurological conditions. Nervous system diseases, disorders, conditions, or their symptoms (collectively 'neurological conditions') that can be ameliorated, treated or prevented with any of the F1Cs disclosed herein include, but are not limited to, nervous system trauma or injury, and neurological conditions that result in an unwanted pathology or symptom, e.g., demyelination, pain, impairment of cognitive function, discernable memory loss, depression, anxiety, a disconnection of axons, a diminution of neuron, astrocyte or glia function or degeneration or death of nervous system cells or tissues such as one or more of those described herein.

Neurological conditions, including nervous system lesions that may be treated, prevented, or ameliorated in a subject include but are not limited to, the following lesions of either the central (including spinal cord, brain) or peripheral nervous systems. Exemplary neurological conditions include (1) ischemic lesions, in which a lack of oxygen in a portion of the nervous system results in neuronal injury or death, including cerebral infarction, ischemia or stroke, or spinal cord infarction or ischemia, (2) traumatic lesions, including lesions caused by physical injury or associated with surgery, for example, lesions which sever a portion of the nervous system, or compression injuries, (3) malignant lesions, in which a portion of the nervous system is destroyed or injured by malignant tissue which is either a nervous system associated malignancy or a malignancy derived from non-nervous system tissue, (4) infectious lesions, in which a portion of the nervous system is destroyed or injured as a result of infection, for example, by an abscess or associated with infection by human immunodeficiency virus, herpes zoster, or herpes simplex virus or with Lyme disease, tuberculosis or syphilis, (5) degenerative lesions or conditions, in which a portion of the nervous system is destroyed or injured as a result of a degenerative process including but not limited to degeneration associated with Parkinson's disease, Alzheimer's disease, Huntington's chorea, AIDS associated dementia, eplieptic dementia, presenile dementia, senile dementia, vascular dementia, post stroke dementia, post traumatic dementia or amyotrophic lateral sclerosis (ALS), (6) lesions associated with nutritional diseases, disorders, and/or conditions, in which a portion of the nervous system is destroyed or injured by a nutritional disorder or disorder of metabolism including but not limited to, vitamin B 12 deficiency, folic acid deficiency, Wernicke disease, tobacco-alcohol amblyopia, Marchiafava-Bignami disease (primary degeneration of the corpus callosum), and alcoholic cerebellar degeneration, (7) neurological lesions associated with systemic diseases including, but not limited to, diabetes (diabetic neuropathy, Bell's palsy), systemic lupus erythematosus, carcinoma, or sarcoidosis, (8) lesions caused by toxic substances including alcohol, lead, or neurotoxins, (9) demyelinated lesions in which a portion of the nervous system is destroyed or injured by a demyelinating disease including, but not limited to, multiple sclerosis, human immunodeficiency virus-associated myelopathy, progressive multifocal leukoencephalopathy, and central pontine myelinolysis or a myelopathy, e.g., diabetic meylopathy or a transverse myelopathy, (10) neurological conditions such as insomnia (e.g., transient or chronic), epilepsy, schizophrenia, psychosis, delusion, a unipolar mood disorder, a bipolar mood disorder, psychomotor dysfunction, depression, anxiety, addiction to or abuse of a drug substance such as tobacco, nicotine, caffeine, alcohol, a barbiturate, a tranquilizer, a narcotic such as hydromorphone HCl, propoxyphene napsylate, meperidine HCl, valium, codeine, cocaine, morphine, heroin or methadone, (11) cognitive dysfunction conditions or diseases such as one or more of impaired long-term or short-term memory, impaired concentration, impaired attention or impaired learning, where the cognitive dysfunction condition or disease is optionally associated with chemotherapy, radiation therapy or exposure, aging, trauma, e.g., CNS trauma, or neurodegeneration and (12) genetic disorders with a neurological pathology or component such as Down's syndrome or Tay Sach's disease.

The F1 Cs are useful to ameliorate, treat or prevent the onset, severity or length of other neurological diseases or conditions such as headache or a migraine condition or symptom such as classic migraine, cluster headache, abdominal migraine, common migraine, hemiplegic migraine, ocular migraine, fulminating migraine, complicated migraine or a symptom of any of these such as head pain, vertigo, nausea, vomiting or potophobia.

In some embodiments, the F1C is used to protect neural cells from the damaging effects of cerebral hypoxia, cerebral ischemia or neural cell injury associated with cerebral infarction, heart attack, stroke or elevated levels of glucocorticoids such as cortisol. The compounds that are also useful for treating or preventing a nervous system disorder may be selected, e.g., by assaying their biological activity in promoting the survival or differentiation of neurons. For example, and not by way of limitation, the F1Cs can be used to elicit any of the following useful effects: (1) increased survival time of neurons in culture, (2) increased sprouting of neurons in culture or *in vivo,* (3) increased production of a neuron-associated molecule in culture or *in vivo, e.g.,* dopamine or choline acetyltransferase or acetylcholinesterase with respect to motor neurons or (4) decreased symptoms of neuron dysfunction *in vivo.* Such effects may be measured by any method known in the art. Increased survival of neurons may be measured using known methods, such as, for example, the method set forth in Arakawa et al. (J. Neurosci. 10:3507-3515 1990); increased sprouting of neurons may be detected by methods known in the art, such as the methods set forth in Pestronk et al. (Exp. Neurol. 70:65-82 1980) or Brown et al. (Ann. Rev. Neurosci. 4:17-42 1981). Increased production of neuron-associated molecules may be measured by, e.g., bioassay, enzymatic assay, antibody binding or Northern blot assay, using techniques known in the art and depending on the molecule to be measured. Motor neuron dysfunction may be measured by assessing the physical manifestation of motor neuron disorder, e.g., weakness, motor neuron conduction velocity, or functional disability. Motor neuron conditions may arise from infarction, cancer, infection, exposure to toxin, trauma, surgical damage or a degenerative disease that affects motor neurons as well as other components of the nervous system.

Other neurological condtions that can be treated using F1Cs include conditions that selectively affect neurons or adjacent tissues such as amyotrophic lateral sclerosis, progressive spinal muscular atrophy, progressive bulbar palsy, primary lateral sclerosis, infantile and juvenile muscular atrophy, poliomyelitis and the post polio syndrome, hereditary motorsensory neuropathy, spinal cord compression and a myelitis such as necrotizing myelitis, transverse myelitis, ascending myelitis, bulbar myelitis, concussion myelitis, demyelinated myelitis, postinfectious myelitis, systemic myelitis or transverse myelitis.

In some neurological conditions such as mood changes, depression, anxiety, memory loss or motor function impairment, the F1 Cs can modulate one or more biological activities of a transcription factor or a nuclear hormone receptor such as ERα in tissue such as the hypothalamus or amygdala or ERβ in tissue such as the hippocampus, thalamus or entorhinal cortex.

In neurological conditions or other conditions where loss or damage to nervous system cells or tissue is typically present, e.g., multiple sclerosis, cerebral infarction, cerebral trauma, elevated glucocorticoid levels or Alzheimer's disease, use of the F1 Cs can lead to detectable repair of damaged cells or replacement of at least some killed cells. Elevated glucocorticoids can result from endogenous production of natural glucocorticoids, e.g., cortisol or hydrocortisone, or from administration of synthetic glucocorticoids, e.g., dexamethasone, triamcinolone, betamethasone or other synthetic agents disclosed herein or in the cited references. Repair or replacement can occur for cell types that are present in nervous system tissues, e.g., neurons, Schwann cells, glial cells, astrocytes, oligodendrocytes, macroglia cells, endothelial cells, or stem or progenitor cells of any of these cell types. The cells may reside in discrete regions of nervous organs, e.g., hippocampus, cerebrum or cerebellum, or they may reside in multiple regions. Any of the neurological conditions that cen be treated with the F1Cs may be acute, subacute or chronic and they may be subclinical (having few or no overt symptoms), mild, moderate or severe.

In treating neurological conditions, the F1Cs will generally enhance function, self renewal and/or differentiation of stem or progenitor cells and/or they will reduce the severity of cell damage or impairment compared to similar subjects that are not treated with the F1Cs. In cases where myelin damage or nerve death occurs, the F1 Cs can reduce the rate at which damage or death occurs or they can detectably reverse damage or enhance replacement of killed cells, particularly where the extent of such damage or killing is mild or moderate. Without wishing to be bound to any theory, the F1Cs may exert these properties (1) by directly acting as a hormone, growth factor or modulator of a biomolecule disclosed herein such as an enzyme, a glucocorticoid receptor, PPARα, a neural stem cell helix-loop-helix transcription factor such as HES1 or an estrogen receptor to enhance replication, synaptogenesis or other repair or maintenance functions, (2) by enhancing recruitment and/or differentiation of cells involved in cell or tissue repair, e.g., enhanced recruitment and differentiation of oligodendrocyte cells to a demyelinated lesion in multiple sclerosis and/or (3) indirectly by modulating the level or activity of autocrine, paracrine or endocrine factors such as one or more inflammatory cytokines or markers as disclosed herein that can modulate disease progression, e.g., cortisol, IL-1α, IL-1β, TNF-α, IL-6, a thromboxane, a prostaglandin or a neuregulin.

In treating chronic or progressive disorders such as multiple sclerosis or Alzheimer's disease, the F1Cs will typically slow the rate of progression of the disease. The F1Cs act at least in part by decreasing the activity or levels of chemokines and/or pro-inflammatory cytokines, e.g., one, two or more of MCP-1, MIP-1, ICAM, V-CAM, E-selectin, RANTES, IL-1α, IL-1β, IL-6, IL-8 and TNF-α. This reduction can be accompanied by a reduced rate of deposition of amyloid-β (AJ3) protein, which results in slowed disease progression and in reduced severity and/or frequency of one or more symptoms such as short term memory loss, impaired concentration, impaired judgement, episodes of disorientation or confusion and periods of mood or behavior changes such as irritability, anxiety or aggression. Treatment of chronic or progressive disorders such as Alzheimer's disease with a F1C is optionally accompanied by other suitable treatments, e.g., treatment with one or more non-steroidal anti-inflammatory drugs or other palliative measures.

Factors such as increased levels of cortisol or thromboxane, that are associated with increased cell or tissue damage or with inhibition of cell growth or differentiation are generally decreased or reregulated to express in a normal manner by the appropriate cells such as neurons, astrocytes, glial cells or their stem or precursor cells. Factors that facilitate normal differentiation or repair, e.g., basic fibroblast growth factor 2 or neuregulin, are generally increased or reregulated to express in a normal manner by the appropriate cells such as neurons, astrocytes, glial cells or their stem or precursor cells.

Because of these properties, the F1Cs can be used in various protocols or methods to enhance differentiation or proliferation of these cell types *in vivo* or *in vitro.* Typically, the concentration of the F1Cs will exert one or more of these beneficial effects at extracellular concentrations of about 1 x 10⁻¹² M to about 5 x 10⁻⁶ M, e.g., about 1 x 10⁻¹¹ M to about 5 x 10⁻⁷ M or about 1 x 10⁻¹⁰ M to about 1 x 10⁻⁷ M. Such concentrations can suitably be established transiently, e.g., for about 10 minutes to about 6 hours or about 12 hours once or twice per day on one, two or more days. Alternatively, such concentrations may be maintained more or less constantly, e.g., within these ranges for at least about 12 hours per day for one, two or more days, particularly for *in vitro* use to enhance cell or tissue growth, differentiation or viability in tissue culture. Methods to administer the F1 Cs for *in vivo* use are essentially as described herein.

For any of these neurological conditions or their associated symptoms, the presence of the condition or its pathological manifestation, e.g., cell or tissue damage, or symptom may be determined by suitable objective or subjective means, e.g., assays to detect tissue damage, levels of diagnostic markers or an etiological agent, performance of histopathological examination of cells or tissues, patient questionnaires or behavior performance tests, measurement of a diagnostic marker(s), e.g., an enzyme, hormone, cytokine or drug substance in blood or tissue, electroencephalography, imaging methods such as X-ray, MRI scan or CAT scan, observation and diagnosis of clinical features or symptoms or biopsy of affected tissue or cells, e.g., aspiration biopsy, needle biopsy, incision biopsy or punch biopsy of tissue or cells. Neurological conditions, diseases and symptoms, which the F1Cs can be used to treat or ameliorate and methods to diagnose and characterize such conditions or diseases have been described. See, e.g., Ph. Demaerel, A.L. Baert et al., eds. Recent Advances in Diagnostic Neuroradiology (Medical Radiology: Diagnostic Imaging) 2001 Springer Verlag, ISBN: 3504657231, W.G. Bradley et al., Neurology in Clinical Practice: Principles of Diagnosis and Management 1995, see, e.g., vol. 1 Ch. 1-55 and vol. 2. Ch. 1-66, Butterworth-Heinemann Medical, ISBN 0750694777, H.J.M. Barnett et al., eds. Stroke: Pathophysiology, Diagnosis and Management 3rd edition, 1998, see, e.g., pages 10-1450, Churchill Livingstone, ISBN 0443075514, P.J. Vinken et al., eds. Neurodystrophies and Neurolipidoses 2nd ed. 1996, see, e.g., pages 8-780, Elsevier Science, ISBN 0444812857, P.L. Peterson and J.W. Phillis eds. Novel Therapies for CNS Injuries: Rationales and Results 1995, see, e.g., pages 8-380, CRC Press, ISBN 0849376521, D. Schiffer, Brain Tumors: Pathology and Its Biological Correlates 2nd ed. 1997, see, e.g., pages 5-450, Springer Verlag, ISBN 3540616225 and E. Niedermeyer and F. Lopes Da Silva, eds. Electroencephalography: Basic Principles, Clinical Applications and Related Fields 4th ed. 1999 see, e.g., pages 13-1238, Lippincott, Williams & Wilkins, ISBN 0683302841.

The use of the F1Cs in these conditions is optionally combined with one or more of the therapeutic treatments that are described in these references. The F1 C may be administered before, during or after another treatment is employed to prevent, treat or ameliorate a given neurological condition or symptom thereof. Any of these neurological conditions or symptoms may be mild or at an early stage, moderate or severe or advanced.

Skin treatments. The affect of the F1Cs on immune function permits their use to improve the function of organs organ systems that rely on the optimal functioning of one or more immune responses. Thus, the F1Cs can be administered to a subject to prevent, treat, ameliorate, slow the progression of or enhance the healing of certain skin conditions such as skin inflammation, lesions, atrophy or rash. Conditions that can give rise to skin pathology or an unwanted skin condition include autoimmune diseases, inflammation, allergy, age, exposure to sunlight, cancer, infection or the like.

As used here, skin includes external skin and internal skin or surfaces such as oral, intestinal and rectal mucosa. These conditions include lesions, rashes or inflammation associated with, e.g., burns, infections and the thinning or general degradation of the dermis often characterized by a decrease in collagen or elastin as well as decreased number, size and doubling potential of fibroblast cells. Such skin conditions include keratoses such as actinic keratosis, psoriasis, eczema, warts such as papillomavirus-induced warts, ulcers or lesions such as herpesvirus-induced ulcers or lesions or diabetes associated ulcers or lesions, discoid lupus erythematosus, erythema nodosum, erythema multiform, cutaneous T cell lymphoma, atopic dermatitis, inflammatory vasculitis, relapsing polychondritis, exfoliative dermatitis, sarcoidosis, burns, melanoma, rash or irritation from poison oak, poison ivy or poison Sumac, blemished or hyperpigmented skin, hyperkeratotic skin, dry skin, dandruff, acne, inflammatory dermatoses, scarring such as from a chemical or thermal burn and age-related skin changes. In these embodiments, treatment with the F1Cs is optionally combined with other appropriate treatments or therapies essentially as described herein, e.g., one or more of a corticosteroid such as hydrocortisone or cortisol, prednisone, or prednisolone, an α-hydroxybenzoic acid or an α-hydroxycarboxylic acid(s) is coadministered with a F1 C to treat, prevent or ameliorate a skin condition such as atrophy or a lesion. α-Hydroxybenzoic acids and α-hydroxycarboxylic acids suitable for use in these embodiments are described in, e.g., U.S. patents 5262407, 5254343, 4246261, 4234599 and 3984566. The F1C can be used to minimize cutaneous atrophy caused by corticosteroids, a common side effect of their application to the skin.

As is apparent from the forgoing, F1Cs can be used in cosmetic preparations for treating one or more skin conditions such as unwanted pigment spots, dry skin, dry scalp, cutaneous signs of aging such as wrinkles, especially fine wrinkles, reduction in the thickness of the dermis, degradation or loss of collagen fibers, withered skin, skin with reduced thickness, dull skin and skin with no brightness, lack of elasticity and/or lack of skin tone. In these applications, the F1Cs may act at least in part by stimulating collagen synthesis, inhibiting collagenase activity and/or increasing keratinocyte proliferation. In cosmetic applications the F1Cs can be administered systemically or they can be included in topical costemtic preparations or formulations. Topical or systemic preparations or formulations for treating skin or other conditions described herein can also optionally contain one or more additional active ingredients such as a carotenoid, a flavanoid, an isoflavanoid, an isoflavone, a retinoid, a lipis synthesis stimulator, a keratinocyte proliferation stimulator, a desquamating agent, a moisturizer, a ultraviolet light absorbing agent or an antibacterial agent. Exemplary active ingredients include one or more of pterocarpan, isoflavan, isoflavan-3-ene, 3-arylcoumarin, 3-aryl-4-hydroxycoumarin, coumestan, coumaronochromone, α-methyldeoxybenzoin, 2-arylbenzofuran, daidzein, formononetin, cuneatin, genistein, isoprunetin and prunetin, cajanin, orobol, pratensein, santal, junipegenin A, glycitein, afrormosin, retusin, tectorigenin, irisolidone, jamaicin, a plant hormone, an auxin, a compound of plant origin, cinnamic acid, indoleacetic acid, 4-chloroindole-3-acetic acid, phenylacetic acid, indole-3-butyric acid, 2,4-dichlorophenoxyacetic acid, α-naphthaleneacetic acid, β-naphthoxyacetic acid, indoleethanol, idoleacetaldehyde, indoleacetonitrile, phloroglucinol, *p*-methylbenzylidenecamphor, *p-*methylbenzylidenecamphor, a 4,4-diarylbutadiene and a dibenzoylmethane compound such as 2-methyldibenzoylmethane, 4-methyldibenzoylmethane, 4-isopropyldibenzoylmethane, 4-tert-butyldibenzoylmethane, 2,4-dimethyldibenzoylmethane, 2,5-dimethyldibenzoylmethane, 4,4'-diisopropyldibenzoylmethane, 4,4'-dimethoxydibenzolymethane, 4-tert-butyl-4'-methoxydibenzoylmethane, 2-methyl-5-isopropyl-4'-methoxyd- ibenzoylmethane, 2-methyl-5-tert-butyl-4'-methoxydibenzoylmethane, 2,4-dimethyl-4'-methoxydibenzoylmethane or 2,6-dimethyl-4-tert-butyl-4'-met- hoxydibenzoyl-methane, and mixtures thereof.

Enhancement of hematopoiesis. The invention includes methods to modulate hematopoiesis by administering a F1C to a subject, which can be used to treat or prevent various blood cell deficiencies such as thrombocytopenia ("TP") or neutropenia ("NP"). Hematopoiesis or hemopoiesis is the formation and development of the various types of blood cells and their progenitor cells. Mature cells are found in circulation or tissues such as the lymph nodes, spleen or the thymus. Many of the stem cells that give rise to mature forms reside in the bone marrow, although some may circulate in the blood for some time. Clinical blood cell deficiencies such as thrombocytopenia, neutropenia or erythropenia can arise from causes such as impaired hematopoiesis or abnormal loss or destruction of mature or immature blood cells.

Without being bound to any theory, the treatment methods at least in part result in enhanced hematopoiesis, enhanced movement of blood cells into the circulation and/or in reduced loss of blood cells such as platelets or neutrophils. The F1 Cs can enhance self-renewal or numbers of hematopoietic stem cells, precursor cells, mature blood cells and/or they can enhance or accelerate differentiation of stem or any progenitor cell that can give rise to a mature blood cell. The stem or progenitor cells include early lineage cells showing little or no characteristics of fully differentiated blood cells and/or they can be partially differentiated. Increased platelet or neutrophil production, enhanced survival or reduced loss is typically observed as increased circulating blood cell counts. Increases in blood cells appear to arise from enhanced proliferation of precursor cells and/or from enhanced or accelerated differentiation of precursor cells. Increased cell numbers, e.g., platelets or neutrophils, can also arise from from reduced loss or death of such cells, increased demargination of cells such as neutrophils from the vasculature into circulating blood or other tissues and/or shorter transit time of mature or precursor cells from the bone marrow into blood.

Thus, invention embodiments comprise a method to treat or prevent a blood cell deficiency such as TP or NP in a subject in need thereof, comprising administering to the subject, or delivering to the subject's tissues, an effective amount of a F1C. Related embodiments include a method to increase self-renewal of hematopoietic stem cells or hematopoietic progenitor cells or to increase the commitment of such cells to transition to a more differentiated blood precursor cell or mature blood cell. In other embodiments, the invention provides a method for stimulating the proliferation or differentiation of neutrophil precursors or to increase demargination of neutrophils or to reduce transit time from bone marrow to blood in a subject having or susceptible to developing NP comprising administering an effective amount of a F1C to the subject in need thereof. The F1C treatment will stimulate the activity of, e.g., neutrophils, or enhance their production from progenitor cells, enhance their survival and/or limit their loss. Hematopoietic stem cells, e.g., GEMM cells, are pluripotent and can give rise to more than one type of mature blood cell, while hematopoietic progenitor cells are usually not pluripotent, but are bipotent or monopotent. Hematopoietic progenitor cells reside primarily in bone marrow, but can also be found in blood, spleen or lymph tissue or fluids.

Neutropenia ("NP"), is considered to exist clinically when neutrophils drop to below a level considered normal. NP can arise from impaired production of neutrophil precursors or mature neutrophils, movement of neutrophils from the circulation to tissue, abnormal circulating neutrophil loss or a combination of these causes. Impaired neutrophil production can be acquired from, e.g., treatment with a cytotoxic or cytostatic drug, chemotherapy, radiation therapy or an autoimmune response as described herein. The abnormal loss of circulating neutrophils in autoimmunity is typically associated with autoreactive antibodies that bind to the cells and reduce their life span. These underlying causes give rise to the various clinical forms of NP, such as postinfectious NP, drug-induced NP, autoimmune NP, or chronic idiopathic NP. The sources of NP and treatment options have been described. See, e.g., Hematology - Basic Principles and Practice, 3rd edition, R. Hoffman, E.J. Benz Jr. et al., editors, Churchill Livingstone, New York, 2000 (see, e.g., Chapters 19, 41, 51, 79, 134 and 137 at pages 297-331, 720-762, 939-979, 1443-1500, 2220-2248 and 2257-2263).

In some embodiments, the F1Cs that are used to enhance hematopoiesis or to treat associated conditions such as a TP or a NP disease or condition as disclosed herein, are characterized by having a lack of appreciable androgenicity. In these embodiments, the F1 Cs are characterized by having about 15% or less, about 10% or less, about 5% or less, about 2% or less, about 1% or less or about 0.5% or less of the androgenicity of a reference androgen such as testosterone, testosterone proprionate, dihydrotestosterone or dihydrotestosterone proprionate as measured in a suitable assay using suitable positive and/or negative controls. F1Cs having, e.g., a substitution at the 6- or 7-position or having no double bond at the 4-5 or 5-6 positions, will generally have relatively low levels of androgen activity. Suitable assays for androgenicity of various compounds have been described, e.g., J.R. Brooks, et al., Prostate 1991, 18:215-227, M. Gerrity et al., Int. J. Androl. 1981 4:494-504, S.S. Rao et al., Indian J. Exp. Biol. 1969 7:20-22, O. Sunami et al., J. Toxicol. Sci. 2000 25:403-415, G.H. Deckers et al., J. Steroid Biochem. Mol. Biol. 2000 74:83-92. The androgenicity of the F1Cs are optionally determined as described or essentially as described in one or more of these assays or any other assay. Thus, one such embodiment comprises a method to enhance hematopoiesis or to treat TP or NP comprising administering to a subject in need thereof an effective amount of a F1C, or delivering to the subject's tissues an effective amount of a F1C, wherein the F1C has about 30% or less, about 20% or less, about 10% or less or about 5% or less of the androgenicity of an androgen such as testosterone, testosterone proprionate, dihydrotestosterone or dihydrotestosterone proprionate as measured in a suitable assay, e.g., as described in the citations above. In conducting such methods, the subjects, e.g., rodents, humans or primates, are optionally monitored for e.g., amelioration, prevention or a reduced severity of a disease, condition or symptom. Such monitoring can optionally include measuring one or more of cytokines (e.g., TNFα, IL-1β), WBCs, platelets, granulocytes, neutrophils, RBCs, NK cells, macrophages or other immune cell types, e.g., as described herein or in the cited references, in circulation at suitable times, e.g., at baseline before treatment is started and at various times during or after treatment with a F1 C, e.g., at about 2-45 days after treatment with a F1 C has ended.

Delayed radiation effects. Invention embodiments include a method to prevent, treat or ameliorate a symptom or condition associated with one or more delayed adverse effect, symptom or condition from ionizing radiation exposure in a subject in need thereof comprising administering to the subject, or delivering to the subject's tissues, an effective amount of a F1C. In these embodiments, administration of the F1C commences at least 2 weeks after the subject has been exposed to a dose or subdose of radiation that could give rise to a delayed radiation effect. Dosing with the F1C can thus begin at 14 days to about 2 years or more after ionizing radiation exposure. Typically dosing will begin at about 2 weeks, 3 weeks, or 1, 2, 3 or 4 months after exposure of the subject to sufficient ionizing radiation to potentially cause delayed effects. Radiation exposure may arise from a radiation therapy where exposure is intentional, or it may arise from an accidental exposure.

Radiation therapy ("RT") can generate a number of late delayed-onset conditions or symptoms. Delayed radiation effects are conditions or symptoms that generally arise or become detectable to the subject or to a health care provider at least about 1 month after exposure to radiation. Thus the conditions or symptoms may be detectable at about 2 months, about 3 months, about 4 months, about 5 months, about 1 year, about 20 years or more after radiation exposure. For example, transient nervous system symptoms may develop early after RT, but progressive, permanent, often disabling nervous system damage may appear months or years later. The total radiation dose, size of the fractions, duration of RT, and volume of tissue irradiated influence the probability of the injury and its severity. Individual patient and tissue susceptibility to delayed injuries is variable, which factors into the selection of safe and effective radiation doses for RT. Total radiation doses that a subject may receive may comprise single doses or 2, 3, 4, or more doses within a range of about 1 to about 400 Gy, e.g., about 1, 1.4, 1.6, 1.8, 2, 2.5, 3, 5, 10, 20, 40, 50, 80, 100, 130, 150, 180, 200, 250, 300, 400 Gy. Typical doses are about 1-12 Gy or about 1-8 Gy. Such doses in a given course of treatment may be the same or different and can occur over a period of time, e.g., over 1 day to about 1 or 2 years.

For example, in lung pneumonitis, administration of a F1C can lead to detectably increased oxygen saturation in the subject's blood by about 5% or by about 10% or more, e.g., oxygen saturation can rise from about 83% to about 88%, which would typically be detectable by the subject and the health care provider. Such decreased severity of a condition or symptom may be objectively measured in some instances, e.g., by determining the number or activity of circulating platelets or neutrophils or by evaluation of fever, severity or frequency of diarrhea or blood oxygen saturation levels. For other symptoms or conditions, prevention may be subjectively observed by a significant or detectable improvement in a relevant score, e.g., decreased fever or pain or a decreased need for treatment of fever, pain or inflammation.

Characterization of F1C biological activity and measurement of the status profile. Some aspects of the invention and related subject matter center on (i) methods to determine the status profile for a subject or groups of subjects that have been exposed to a biological insult that is potentially life-threatening and that are treated with a F1C and (ii) identification of biological parameters, typically biological results or symptoms of the biological insult, that can be used to obtain a status profile. The status profile is a predictor for survival or for non-survival after exposure of a subject to a potentially lethal biological insult. Once a status profile is obtained for a given subject species, the effect of treatment with a F1 C on the status profile for that same species or for a closely related species can be determined. A survival status profile, or Pₛᵤᵣᵥᵢᵥₐₗ, is the probability that the subject will survive the biological insult, absent treatment other than palliative treatments such as management of symptoms, pain, fever, suffering, nutrition, body or peripheral temperature, water or electrolyte management or other typical palliative treatments. A lethality status profile, or P_{lethality}, is the probability that the subject will not survive the biological insult, absent treatment other than palliative treatments such as management of symptoms, pain, fever, suffering, nutrition, body or peripheral temperature, water or electrolyte management or other typical palliative treatments. As the foregoing indicates, methods to obtain Pₛᵤᵣᵥᵢᵥₐₗ or P_{lethality} that have high probabilities of predicting survival or non-survival after a biological insult are useful for many purposes, e.g., to assess or diagnose a subject's clinical condition or prognosis or to tailor palliative or other therapies to fit the subject's clinical condition. This information is particularly useful where the status profile is obtained soon, e.g., within about 12-48 hours, after a biological insult that can cause death at a much later time, e.g., at about 1, 2 or 3 weeks later.

When the subject species is a human, the exposed patients will usually be treated with at least the minimal acceptable treatment consistent with the subject's clinical condition and/or the biological insult and/or the subject's local clinical standards of care and/or any standard of care that is practical under the circumstances. In cases where medical care is limited or at least temporarily unavailable, measurements, e.g., non-invasive measurements of temperature, to obtain status profile information can be obtained. Such information can be used to assess or triage the patient's clinical condition.

In general Pₛᵤᵣᵥᵢᵥₐₗ or P_{lethality} values that are near to or at least at statistical significance are of the greatest interest or, for human clinical practice, utility. As used herein, any Pₛᵤᵣᵥᵢᵥₐₗ means that the value predicts survival of the exposed subject with at least about a 80%, 85%, 90%, 91%, 92%, 93%, 94% degree of confidence, or preferably at least about a 95% degree of confidence, which is typically considered at statistical significance. Similarly, a P_{lethality} means that the value predicts non-survival of the exposed subject with at least about 80%, 85%, 90%, 91%, 92%, 93%, 94% degree of confidence, or preferably at least about a 95% degree of confidence. Typically, Pₛᵤᵣᵥᵢᵥₐₗ values of at least about 0.8, at least about 0.85, at least about 0.9, at least about 0.92, at least about 0.93, at least about 0.94, at least about 0.95, at least about 0.96, at least about 0.97, at least about 0.98, at least about 0.99, at least about 0.995, at least about 0.999 or better are generally useful in the invention. Typically, P_{lethality} values of about 0.2, about 0.15, about 0.1, about 0.08, about 0.07, about 0.06, about 0.05, about 0.04, about 0.03, about 0.02, about 0.01, about 0.005, about 0.001 or better are generally useful in the invention.

In some of these embodiments, the invention provides methods to determine a subject's status profile, where the methods comprise, (1) exposing the subject to a sufficient amount of a biological insult (or exposing a group of subjects, where the group has been exposed to the same, essentially the same or a similar, but comparable biological insult) to potentially (e.g., the probability is at least 10%, about 30%, about 50% or more to at least about 60% or about 70%, about 80% or more) cause or elicit one, two or more biological responses that are potentially life-threatening to obtain an exposed subject (or group of subjects); (2) measuring on 1, 2, 3, 4 or more occasions in or from the exposed subject (or group of subjects) 2, 3, 4 or more parameters selected from temperature, red blood cell counts, hematocrit, red blood cell precursors optionally selected from CFU-GEMM, BFU-E, CFU-E, proerythroblasts, pronormoblasts, basophilic normoblasts, polychromatic normoblasts, orthochromatic normoblasts and reticulocytes, platelets, platelet precursors optionally selected from megakaryocytes, megakaryocyte progenitor cells, megakaryocyte precursor cells, promegakaryoblasts, immature megakaryocyte colony forming units, mature megakaryocyte colony forming units and megakaryocyte lineage markers optionally selected from GP-IIb, GP-IX, PF4 and GP-Ibα, macrophages, monocytes or monocyte precursors optionally selected from CD34⁻CD90⁺CD123⁺CD117⁺CD135⁺ stem cells, CD34⁺CD33⁻CD38⁻CD45R0⁺CD45RA- progenitor cells, CFU-GEMM (e.g., CD34⁺CD33⁺CD38⁻), CFU-GM (e.g., CD64⁺), CFU-M (e.g., CD34⁺CD33⁺CDC13⁺), monoblasts (e.g., CD33⁺CD38⁺CD14⁺), promonocytes (e.g., CD64⁺CD11c⁺CD14⁺), C reactive protein, fibrinogen, sepsis, respiration rate, pulse rate, blood or arterial pH, blood pressure, pH or composition of sweat, pH or composition of saliva, respired breath composition, urine pH or composition, blood SaO₂ or oxygen saturation of arterial oxyhemoglobin (e.g., as measured by a pulse oximeter), a circadian, diurnal or nocturnal rhythm parameter, optionally selected from one, two or more of rapid eye movement sleep, sleeping brain theta waves, leptin, glucose, insulin, melatonin, heart rate, temperature, locomotor activity, autonomic nervous function, hormone, glucocorticoid such as cortisol, blood enzyme levels, B-cells, T-cells, natural killer cells, dendritic cells, neutrophils, eosinophils, basophils, CFU-Eos, CFU-Baso or a progenitor or precursor of any of these such as a neutrophil or other precursor optionally selected from CD34-CD90⁺CD123⁺CD117⁺CD135⁺ stem cells, CD34⁺CD33⁻CD38⁻CD45RO⁺CD45RA⁻ progenitor cells, CFU-GEMM (e.g., CD34⁺CD33⁺CD38⁻), CFU-GM (e.g., CD64⁺), CFU-G (e.g., CD45RA⁺MPO⁺), myeloblasts (e.g., CD33⁺CD38⁺), complement protein C3a, sepsis, e.g., as determined by detection of bacteria in blood, liver, lung or other tissue on 1, 2, 3 or more occasions, septic shock, myelocytes, neurological damage (e.g., motor function impairment, cognitive impairment or autonomic function impairment), wherein the measurements are obtained at times before, during or overlapping with, and/or after the biological insult to obtain a status profile for the exposed subject or the treated exposed subject (or the exposed group of subjects, and/or the exposed treated group of subjects); (3) optionally administering one or more palliative or ameliorative therapies to treat one or more side effects of the biological insult to obtain an exposed treated subject(s); (4) measuring the survival rate of the exposed subject(s) and/or the exposed treated subject(s); and (5) identifying one or more status profiles that corresponds to a defined probability of surviving the biological insult (Pₛᵤᵣᵥᵢᵥₐₗ) or of not surviving the biological insult (P_{lethality}). Types of mature blood cells, their progenitors and methods to measure or identify them have been described, e.g., Hematology - Basic Principles and Practice, 3rd edition, R. Hoffman, E.J. Benz Jr. et al., editors, Churchill Livingstone, New York, 2000, see, e.g., chapter 12 at pages 126-138 and chapter 13 at pages 139-154, chapter 15 at pages 202-219, chapter 16 at pages 220-222 and chapter 17 at pages 245-260. These methods and descriptions can be used in the invention methods.

In these embodiments, the biological insult typically comprises exposure of one or more subjects to one or more of radiation, toxin, trauma and/or chemotherapy. Biological responses to a biological insult that is potentially life-threatening can be associated with a variety of conditions, e.g., a toxicity or tissue damage from an infectious agent, side-effects of trauma such as blood loss, and/or impairment, failure or death of one or more organs or tissues, e.g., kidney, liver, heart, intestine, stomach or skin or bone marrow failure or impairment after exposure to radiation or a toxic chemotherapy. To obtain measurements for assembling a status profile, cells or tissue can be obtained from marrow, spleen, thymus, lymph node, lymph fluid, liver or lung blood, serum or tissue from the exposed subject(s) and/or the exposed treated subject(s). Types of mature blood cell, their progenitors and methods to measure or identify them have been described, e.g., Hematology - Basic Principles and Practice, 3rd edition, R. Hoffman, E.J. Benz Jr. et al., editors, Churchill Livingstone, New York, 2000, see, e.g., chapter 12 at pages 126-138 and chapter 13 at pages 139-154, chapter 15 at pages 202-219, chapter 16 at pages 220-222 and chapter 17 at pages 245-260. For small subjects such as mice or rats, measurement of some parameters, e.g., measuring a particular cell type in bone marrow tissue, on more than one occasion may not be easily accomplished. In these situations, obtaining more than one measurement of a parameter will thus typically be accomplished using measurements from one or more exposed subjects or exposed treated subjects once and other one or more exposed subjects or exposed treated subjects (s) at one or two other occasions to get the needed time points.

Specific exemplary status profiles include status profiles that are based on measuring the following combinations of biological parameters, which are measured on one or more occasions:

(i) a temperature increase (or a measure of central tendency) of at least about 0.5°C, at least about 0.6°C, at least about 0.7°C, at least about 0.8°C, at least about 0.9°C, at least about 1.0°C, at least about 1.1°C, at least about 1.2°C, at least about 1.3°C, at least about 1.4°C, at least about 1.5°C, at least about 1.6°C, at least about 1.7°C, at least about 1.8°C, at least about 1.9°C, at least about 2.0°C, at least about 2.1°C or at least about 2.3°C above the baseline of the normal temperature for the subject species, e.g., about 37.2°C for Rhesus monkeys or about 98.6°F for humans, optionally where the temperature increase optionally is (a) completely or mostly (at least about 80% or at least about 90% or at least about 95% or at least about 98% of the time) maintained at that level for a period of at least about 0.5 minute, at least about 1 minute, at least about 5 minutes, at least about 10 minutes, at least about 0.25 hour, at least about 0.5 hour at least about 0.75 hour, at least about 1 hour or at least about 2 hours, at least about 3 hours, at least about 4 hours or at least about 6 hours, or at least about 8 hours, optionally where the temperature increase occurs within about 4 hours, about 8 hours, about 12 hours, about 24 hours or about 48 hours of the biological insult, and/or (b) when the subject is a human or a non-human primate, core or peripheral temperature is measured within a period of about 1, 2, 3, 4, 5, 6, 7, 8, 9, 10, 11, 12, 13, 14, 15, 16, 17, 18, 19, 20 or 21 days after the biological insult and/or (c) the core body temperature is measured, e.g., using rectal temperature, an implanted monitoring device and/or a thermister in an indwelling catheter or line, and/or (d) the status profile correlates with lethality of the biological insult for the exposed subject, or status profile correlates with survival after the biological insult for the exposed subject when the temperature increase is not observed;

(ii) disruption of the circadian rhythm as described or defined as, e.g., a significant change in the normal rhythm or signature in any of the elements of the composite of a circadian rhythm such as temperature, in the subject species, optionally where the disruption is (a) completely or mostly (at least about 80% or at least about 90% or at least about 95% or at least about 98% of the time) maintained for a period of at least about 1 hour or at least about 2 hours, at least about 3 hours, at least about 4 hours or at least about 6 hours, at least about 8 hours, at least about 12 hours, at least about 24 hours or at least about 48 hours, and/or (b) when the subject is a human or a non-human primate, core or peripheral temperature is measured within a period of about 1, 2, 3, 4, 5, 6, 7, 8, 9, 10, 11, 12, 13, 14, 15, 16, 17, 18, 19, 20, 21, 22, 23 or 24 days after the biological insult and/or (c) the status profile correlates with lethality of the biological insult for the exposed subject when the circadian rhythm is completely or mostly disrupted or the status profile correlates with survival after the biological insult for the exposed subject when the circadian rhythm is not completely or mostly disrupted;

(iii) a mean or absolute decrease (or a measure of central tendency) of at least about 20%, at least about 21%, at least about 22%, at least about 23%, at least about 24%, at least about 25%, at least about 26%, at least about 27% or at least about 28%, at least about 29% or at least about 30%, in red blood cell or erythrocyte counts, hematocrit, hemoglobin and/or reticulocytes, optionally where (a) the mean decrease in red blood cell or erythrocyte counts, hematocrit, hemoglobin and/or reticulocytes is obtained from the nadir or lowest measurement, optionally, and/or (b) when the subject is a human or a non-human primate, the red blood cell or erythrocyte count, hematocrit, hemoglobin and/or reticulocyte count is measured within a period of about 7, 8, 9, 10, 11, 12, 13, 14, 15, 16, 17, 18, 19, 20, 21, 22, 23 or 24 days after the biological insult and/or (c) the status profile correlates with lethality of the biological insult for the exposed subject, and/or (d) temperature variation or increase or circadian rhythm disruption is also measured, e.g., as described in (i), (ii) or elsewhere herein;

(vi) an absolute decrease of at least about 78%, about 79% or about 80% or about 85% in red blood cell or erythrocyte counts, hematocrit, hemoglobin and/or reticulocytes for individual exposed subjects or for groups of exposed subjects, optionally where (a) the mean decrease in red blood cell or erythrocyte counts, hematocrit, hemoglobin and/or reticulocytes is obtained from the nadir or lowest measurement, optionally, and/or (b) when the subject is a human or a non-human primate, the red blood cell or erythrocyte count, hematocrit, hemoglobin and/or reticulocyte count is measured within a period of about 7, 8, 9, 10, 11, 12, 13, 14, 15, 16, 17, 18, 19, 20, 21, 22, 23 or 24 days after the biological insult and/or (c) the status profile correlates with survival of the exposed subject after the biological insult, and/or (d) temperature variation or increase or circadian rhythm disruption is also measured, e.g., as described in (i), (ii) or elsewhere herein;

(v) an absolute decrease of at least about 80%, at least about 85%, at least about 90%, at least about 95%, at least about 97%, in platelets, megakaryocytes or a megakaryocyte precursor described herein, or, for a human or a non-human primate, a mean count of about 6500 per µL or less, about 6600 per µL or less, about 6700 per µL or less, about 6800 per µL or less, about 6900 per µL or less or about 7000 per µL or less for non-human primates or humans or about 10,000 per µL or less, about 9,500 per µL or less, about 9,000 per µL or less, about 8,500 per µL or less or about 8,000 per µL or less, optionally where (a) the mean decrease in platelets, megakaryocytes or megakaryocyte precursors is obtained from the nadir or lowest measurement, and/or (b) when the subject is a human or a non-human primate, the platelet, megakaryocyte or megakaryocyte precursor count is measured within a period of about 6, 7, 8, 9, 10, 11, 12, 13, 14, 15, 16, 17, 18, 19, 20, 21, 22, 23 or 24 days after the biological insult and/or (c) the status profile correlates with lethality of the biological insult for the exposed subject, and/or (d) temperature variation or increase or circadian rhythm disruption is also measured, e.g., as described in (i), (ii) or elsewhere herein;

(vi) an absolute decrease of less than about 78%, less than about 75%, less than about 70% or less than about 65%, in platelets, megakaryocytes or megakaryocyte precursors, optionally where (a) the mean decrease in platelets, megakaryocytes or a megakaryocyte precursor described herein is obtained from the nadir or lowest measurement, and/or (b) when the subject is a human or a non-human primate, the platelet, megakaryocyte or megakaryocyte precursor count is measured within a period of about 6, 7, 8, 9, 10, 11, 12, 13, 14, 15, 16, 17, 18, 19, 20, 21, 22, 23 or 24 days after the biological insult and/or (c) the status profile correlates with survival of the exposed subject after the biological insult, and/or (d) temperature variation or increase or circadian rhythm disruption is also measured, e.g., as described in (i), (ii) or elsewhere herein;

(vii) an absolute decrease of at least about 80%, at least about 85%, at least about 90%, at least about 95%, at least about 97%, in neutrophils or a neutrophil precursor described herein, and/or, for a human or a non-human primate, an absolute count of about 30 per mm³ or less, about 40 per mm³ or less, about 45 per mm³ or less, about 50 per mm³ or less or about 55 per mm³ or less, optionally where (a) the mean decrease in neutrophil or neutrophil precursor is obtained from the nadir or lowest measurement, and/or (b) when the subject is a human or a non-human primate, the neutrophil or neutrophil precursor count is measured within a period of about 7, 8, 9, 10, 11, 12, 13, 14, 15, 16, 17, 18, 19, 20, 21, 22, 23 or 24 days after the biological insult and/or (c) the status profile correlates with lethality of the biological insult for the exposed subject, and/or (d) temperature variation or increase or circadian rhythm disruption is also measured, e.g., as described in (i), (ii) or elsewhere herein, and/or (e) a decrease in one or more of platelets, megakaryocytes or another thrombopoiesis marker as described in (v) or (vi) or elsewhere herein and/or (f) a decrease in one or more of red cell counts or hematocrit or other erythropoiesis marker as described in (iii) or (iv) or elsewhere herein;

(viii) an absolute decrease of less than about 78%, less than about 75%, less than about 70% or less than about 65%, in neutrophils or in a neutrophil precursor described herein, or, for a human or a non-human primate, a mean count of at least about 50 per mm³, at least about 55 per mm³, at least about 60 per mm³, at least about 65 per mm³, at least about 70 per mm³, at least about 80 per mm³, at least about 90 per mm³, at least about 100 per mm³, at least about 150 per mm³, at least about 200 per mm³, at least about 300 per mm³ or at least about 400 per mm³, optionally where (a) the mean decrease in neutrophils or neutrophil precursor is obtained from the nadir or lowest measurement, and/or (b) when the subject is a human or a non-human primate, the neutrophil or a neutrophil precursor count is measured within a period of about 7, 8, 9, 10, 11, 12, 13, 14, 15, 16, 17, 18, 19, 20, 21, 22, 23 or 24 days after the biological insult and/or (c) the status profile correlates with survival of the exposed subject after the biological insult, and/or (d) temperature variation or increase or circadian rhythm disruption is also measured, e.g., as described in (i), (ii) or elsewhere herein, and/or (e) a decrease in one or more of platelets, megakaryocytes or another thrombopoiesis marker as described in (v) or (vi) or elsewhere herein and/or (f) a decrease in one or more of red cell counts or hematocrit or other erythropoiesis marker as described in (iii) or (iv) or elsewhere herein;

(ix) the first time after the biological insult that the exposed subject, usually a human or a non-human primate, has a Grade III or IV thrombocytopenia or an equivalent condition, e.g., a platelet count of less than 50,000 per mm³, optionally combined with one or more of the biological parameters described in (i), (ii), (iii), (iv), (v), (vi), (vii) or (viii) above or one, two or more biological parameters described elsewhere herein;

(x) the first time after the biological insult that the exposed subject, usually a human or a non-human primate, has a Grade III or IV anemia or an equivalent condition, e.g., hemoglobin measurement of less than 8.0 g per dL, optionally combined with one or more of the biological parameters described in (i), (ii), (iii), (iv), (v), (vi), (vii), (viii) or (ix) above or one, two or more biological parameters described elsewhere herein; and/or

(xi) the status profile of any of (i), (ii), (iii), (iv), (v), (vi), (vii), (viii), (ix) or (x) wherein (a) the subject is a treated exposed subject and the treatment optionally is one, two or more of administration of an effective amount of a hematopoiesis stimulator, an immune system stimulator, an apoptosis inhibitor, an antibiotic, an antifever treatment or agent, an analgesic, whole blood, platelets, red cells, neutrophils, electrolytes, anti-fever agents, analgesics, G-CSF, GM-CSF, IL-6, IL-11, IFNγ, intravenous fluids, intravenous immunoglobulin, intravenous nutrients or sugars, anti-TNF-α antibody or monoclonal antibody or antibody fragment, thrombopoietin, erythropoietin, stem cell factor, pegfilgrastim, α-1 thymosin, thymopoietin, serum thymic factor, an antioxidant, a CpG oligonucleotide, allopurinol, vitamin E or related compounds, superoxide dismutase mimetics, a benzyl styryl sulfone, dipeptide peptidase inhibitors, phenylacetic acid, phenylbutyric acid, an apoptosis inhibitor or hematopoiesis stimulator optionally selected from a steroid of formula 1, a bacterial flagellin and an antiapoptotic fragment thereof, a biologically active fragment of any of these proteins, a polymer conjugate of any of these proteins or any biologically active fragment of any of these proteins, a statin, e.g., as described herein or in the cited references, a F1 C, and/or (b) the subject is a human or a non-human primate, optionally selected from a Rhesus monkey and a Cynomolgus monkey, and/or (c) the status profile is obtained from exposed subjects, exposed treated subjects and/or both exposed subjects and exposed treated subjects, and/or (d) the biological insult is radiation exposure, optionally at a dose of about an LD₃₀ or LD₄₀ or LD₄₅ to about an LD₅₅, LD₆₀ or LD₇₀ or at a dose of about an LD₅₀, or at another dose or dose range described herein, where survival is determined at 30 days post exposure or at 60 days post exposure, and optionally where the radiation is γ-radiation such as ⁶⁰Co or ¹²⁷Cs, particle radiation, e.g., silicon or boron, fast neutrons or slow neutrons, and optionally wherein the radiation is whole body radiation that the subject(s) is exposed to over a period of about 30 minutes or less or about 20 minutes or less or where the subject(s) is exposed to the radiation for a period of about 10 +/- 3 minutes, and optionally where a treatment agent selected from administration of an effective amount of a steroid of formula 1, IL-6, IFNγ,G-CSF, GM-CSF or another treatment described herein is administered to the subject, optionally where the administration results in the treatment agent being systemically present in the subject at 1, 2 or more times within about 0.5 hours, about 1 hour, about 1.5 hours, about 2 hours, about 2.5 hours about 3 hours about 3.5 hours, about 4 hours, about 4.5 hours, about 5 hours, about 5.5 hours, about 6 hours, about 8 hours, about 12 hours or about 24 hours after the subject was exposed to the radiation; and/or (e) the biological insult is radiation exposure, optionally at a dose of about 450 cGy, about 500 cGy, about 550 cGy, about 560 cGy, about 570 cGy, about 580 cGy, about 590 cGy, about 600 cGy, about 610 cGy, about 620 cGy, about 630 cGy, about 640 cGy, about 650 cGy, about 700 cGy, about 750 cGy, about 800 cGy, about 850 cGy, about 9 Gy, about 9.5 Gy, about 10 Gy, about 10.5 Gy, about 11 Gy, about 12 Gy, about 15 Gy, about 20 Gy or another radiation dose or dose range described herein, optionally wherein the radiation is whole body radiation that the subject(s) is exposed to over a period of about 30 minutes or less or about 20 minutes or less or where the subject(s) is exposed to the radiation for a period of about 10 +/- 3 minutes and optionally where the radiation is a radiation disclosed herein, e.g., γ-radiation such as ⁶⁰Co or ¹²⁷Cs or fast neutrons, and optionally where a treatment agent selected from administration of an effective amount of a steroid of formula 1, IL-6, IFNγ,G-CSF, GM-CSF, thrombopoietin, erythropoietin or another treatment described herein is administered to the subject, optionally where the administration results in the treatment agent being systemically present in the subject at 1, 2 or more times within about 0.5 hours, about 1 hour, about 1.5 hours, about 2 hours, about 2.5 hours about 3 hours about 3.5 hours, about 4 hours, about 4.5 hours, about 5 hours, about 5.5 hours, about 6 hours, about 8 hours, about 12 hours or about 24 hours after the subject was exposed to the radiation; and/or (f) the biological insult is 1, 2, 3, 4, 5, 6 or more rounds of 1, 2, 3, 4 or more cancer chemotherapies or cancer chemotherapy agents or a bone marrow transplantation protocol, or a surgery, any of which are optionally combined with radiation exposure, optionally wherein the biological insult occurs over a time period of about 1, 2, 3, 4, 5, 6, 7, 8, 9, 10, 11, 12, 13, 14, 15, 16, 17, 18, 19, 20 or more days or over a time period of about 3, 4, 5, 6, 7, 8, 9, 10, 11, 12, 13, 14, 15, 16, 17, 18, 19 or more weeks or aver a time period of about 5, 6, 7, 8, 8, 10, 11, 12 or more months, optionally wherein one, two or more biological parameter measurements to obtain the status profile are begun at about at time when the subject(s) would be expected to have a significant chance (P > about 0.1, about 0.2, about 0.3, about 0.4, about 0.5, about 0.6 or more) of not surviving the biological insult, where the assessment of the significant chance of not surviving the biological insult is a subjective or objective assessment based on clinical observations and/or comparison of the subject(s)' condition with similarly situated subjects of the same or a closely related species, optionally wherein, for a subject(s) that has a cancer, the cancer is optionally selected from lung cancer, prostate cancer, breast cancer, colon cancer, skin cancer, a cancer of the central or peripheral nervous system, ovarian cancer, cervical cancer and endometrial cancer.

In general, a significant change in the normal rhythm or signature in any of the elements of the composite of a circadian rhythm can occur as a biological response to the biological insult. A significant change is generally a change that is statistically significantly not zero, e.g., P < 0.05, or a change that is nearly statistically significantly not zero, e.g., P < 0.15, P < 0.12 or P < 0.10. A change(s) can be observed in one, two, three or more of the elements of the composite of a circadian rhythm, e.g., circadian sex steroid levels, cortisol, IL-6, melatonin or other molecules described herein. Change in circadian temperature can be observed as a relatively flat daily temperature profile, intermittent changes, e.g., hectic fever, as significant shifts in cycle timing, exaggerated temperature peaks and/or valleys or as combinations of these situations.

Specific embodiments. Aspects of the invention and related subject matter include the following specific embodiments. In the following embodiments and elsewhere herein, when reference is made to a variable group such as R¹, R⁴, R⁶ or R¹⁰ in the α-configuration or the β-configuration, a double bond will usually not be present at the carbon or at the position to which the variable group is bonded. Thus, R¹ in the α-configuration or the β-configuration usually means that no double bond is present at the 3-position or the position to which R¹ is bonded, or if a double bond is present, the variable goup is bonded to the ring without a defined configuration.

1. A method to treat, prevent, ameliorate, delay the onset of or slow the progression of one or more of a condition or symptom described herein such as an unwanted inflammation, allergy, immune suppression condition (e.g., an innate immune suppression condition, an adaptive immune suppression condition or an adaptive immune suppression condition), immunosenescence, autoimmune disorder, infection, cancer or precancer, hereditary condition, blood cell deficiency (such as grade III or IV neutropenia, anemia or thrombocytopenia), a neurological disorder, a cardiovascular disorder, trauma, hemorrhage, bone fracture, unwanted or excess bone loss, androgen deficiency, estrogen deficiency, a congenital or hereditary disorder or a symptom of any of these conditions in a subject who has the condition or who is subject to developing the condition, comprising administering to a subject, or delivering to the subject's tissues, an effective amount of a formula 1 compound

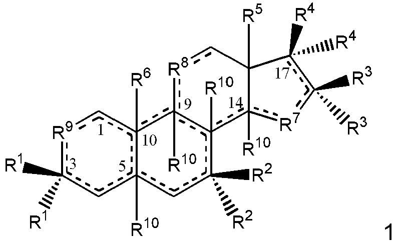

or a metabolic precursor, a metabolite, salt or tautomer thereof, wherein the dotted lines are optional double bonds; each R¹, R², R³, R⁴, R⁵, R⁶ and R¹⁰ independently or together are -H, -OH, -OR^{PR}, -SR^{PR}, -SH, -N(RP^{R})2, -NH₂, -O-Si-(R¹³)₃, - CHO, -CHS, -CN, -SCN, -NO₂, -COOH, -OSO₃H, -OPO₃H₂, =O, =S, =N-OH, =N-OCH₃, =CH₂, =CH-CH₃, =CH-optionally substituted alkyl, =N-O-optionally substituted alkyl, =N-optionally substituted alkyl, an ester, a thioester, a thionoester, a phosphoester, a phosphothioester, a phosphonate, a phosphonate ester, a thiophosphonate, a thiophosphonate ester, a phosphiniester, a sulfite ester, a sulfate ester, a sulfamate, a sulfonate, a sulfonamide, an amide, an amino acid, a peptide, an ether, a thioether, an acyl group, a thioacyl group, a carbonate, a carbamate, a halogen, optionally substituted alkyl, optionally substituted alkenyl, optionally substituted alkynyl, an optionally substituted aryl moiety, an optionally substituted heteroaryl moiety, an optionally substituted heterocycle, an optionally substituted monosaccharide, an optionally substituted oligosaccharide, a polymer, a spiro ring, an epoxide, an acetal, a thioacetal, a ketal or a thioketal; R⁷ is -O-, -S-, -NR^{PR}-, -C(R¹⁰)₂-, -C(R¹⁰)₂-C(R¹⁰)₂-, -C(R¹⁰)₂-C(R¹⁰)₂-C(R¹⁰)₂-, -C(R¹⁰)₂-O-C(R¹⁰)₂-, -C(R¹⁰)₂-S-C(R¹⁰)₂-, -C(R¹⁰)₂-NR^{PR}-C(R¹⁰)₂-, O-C(R¹⁰)₂-, - S-C(R¹⁰)₂- or -NR^{PR}-C(R¹⁰)₂-; R⁸ and R⁹ independently are -C(R¹⁰)₂-, -C(R¹⁰)₂-C(R¹⁰)₂-, - O-, -O-C(R¹⁰)₂-, -S-, -S-C(R¹⁰)₂-, -NR^{PR}- or -NR^{PR}-C(R¹⁰)₂-, or one or both of R⁸ or R⁹ independently are absent, leaving a 5-membered ring; R¹³ independently is C₁₋₆ alkyl; R^{PR} independently are -H or a protecting group; and optionally wherein one, two or three of the 1-, 4-, 6- and/or 12-positions are optionally substituted with (i) an independently selected R¹⁰ moiety when a double bond is present at the corresponding 1-, 4-, 6- or 12-position, or (ii) one or two independently selected R¹⁰ moieties when no double bond is present at the corresponding 1-, 4-, 6- and/or 12-position.

2. The method of embodiment 1 wherein one each of R¹, R², R³ and R⁴ are -H, no double bond is present at the 3-, 7-, 16-, or 17-position, the second R¹, R², R³ and R⁴ are bonded by a single bond and respectively are in the α,α,α,α, α,α,α,β, α,α,β,α, α,β,α,α, β,α,α,α, α,α,β,β, α,β,α,β, β,α,α,β, β,α,β,α, β,β,α,α, α,β,β,α, α,β,β,β, β,α,β,β, β,β,α,β, β,β,β,α o r β,β,β,β configurations and the second R¹, R², R³ and R⁴ are optionally independently selected from -H, -F, -Cl, -Br, -I, -OH, -SH, -NH₂, -COOH, -CH₃, -C₂H₅, -C(CH₃)₃, -OCH₃, -OC₂H₅, -CF₃, -CH₂OH, -C(O)CH₃, -C(O)CH₂OH, -C(O)CH₂F, - C(O)CH₂Cl, -C(O)CH₂Br, -C(O)CH₂I, -C(O)CF₃, -C₂F₅, =O, =CH₂, =CHCH₃, amino acid, carbamate, carbonate, optionally substituted C1-C20 alkyl, optionally substituted C1-C20 ether, optionally substituted C1-C20 ester, optionally substituted C1-C20 thioether, optionally substituted C1-C20 thioester, optionally substituted monosaccharide, optionally substituted disaccharide, optionally substituted oligosaccharide and polymer.

3. The method of embodiment 1 or 2 wherein the F1C is (1) a compound or genus of compounds described in a compound group described herein, or (2) an androstane, a 5β-androstane, 1-ene, 2-ene, 3-ene, 5(6)-ene (or a "5-ene"), 5(10)-ene, 6-ene, 7-ene, 8(9)-ene, 8(14)-ene, 9(10)-ene, 9(11)-ene, 11-ene, 12-ene, 13(17)-ene, 14-ene, 15-ene, 16-ene, 1,3-diene, 1,5-diene, 1,5(10)-diene, 1,6-diene, 1,7-diene, 1,8(9)-diene, 1,8(14)-diene, 1,9(11)-diene, 1,11-diene, 1,12-diene, 1,13(17)-diene, 1,15-diene, 1,16-diene, 2,4-diene, 2,5-diene, 2,5(10)-diene, 2,6-diene, 2,7-diene, 2,8(9)-diene, 2,8(14)-diene, 2,11-diene, 2,12-diene, 2,13(17)-diene, 2,14-diene, 2,15-diene, 2,16-diene, 3,5-diene, 3,6-diene, 3,7-diene, 3,8(9)-diene, 3,8(14)-diene, 3,9(10)-diene, 3,9(11)-diene, 3,11-diene, 3,12-diene, 3,13(17)-diene, 3,14-diene, 3,15-diene, 3,16-diene, 4,6-diene, 4,7-diene, 4,8(9)-diene, 4,8(14)-diene, 4,9(10)-diene, 4,9(11)-diene, 4,11-diene, 4,12-diene, 4,13(17)-diene, 4,14-diene, 4,15-diene, 4,16-diene, 5(6),15-diene (or a "5,15-diene"), 5,7-diene, 5,8(9)-diene, 5,8(14)-diene, 5,9(11)-diene, 5,11-diene, 5,12-diene, 5,13(17)-diene, 5,14-diene, 5,15-diene, 5,16-diene, 5(10),7-diene, 5(10),8(9)-diene, 5(10),8(14)-diene, 5,9(11)-diene, 5(10),11-diene, 5(10),12-diene, 5(10),13(17)-diene, 5(10),14-diene, 5(10),15-diene, 5(10),16-diene, 6,9(11)-diene, 6,9(14)-diene, 6,10-diene, 6,11-diene, 6,13(17)-diene, 6,14-diene, 6,15-diene, 6,16-diene, 7,9(10)-diene, 7,9(11)-diene, 7,12-diene, 7,13(17)-diene, 7,14-diene, 7,15-diene, 7,16-diene, 8(9),11-diene, 8(9),12-diene, 8(9),13(17)-diene, 8(9),14-diene, 8(9),15-diene, 8(9),16-diene, 8(14),9-diene, 8(14),11-diene, 8(14),12-diene, 8(14),13(17)-diene, 8(14),15-diene, 8(14),16-diene, 9(10),11-diene, 9(10),12-diene, 9(10),13(17)-diene, 9(10),14-diene, 9(10),15-diene, 9(10),16-diene, 9(11),13-diene, 9(11),13(17)-diene, 9(11),14-diene, 9(11),15-diene, 9(11),16-diene, 11,13(17)-diene, 11,14-diene, 11,15-diene, 11,16-diene, 12,14-diene, 12,15-diene, 12,16-diene, 13(17),14-diene, 13(17),15-diene, 14,16-diene, 1,3,5-triene, 1,3,5(10)-triene, 1,3,6-triene, 1,3,7-triene, 1,3,8-triene, 1,3,8(14)-triene, 1,3,9-triene, 1,3,9(11)-triene, 1,3,12-triene, 1,3,13(17)-triene, 1,3,14-triene, 1,3,15-triene, 1,3,16-triene, 1,4,6-triene, 1,4,7-triene, 1,4,8-triene, 1,4,8(14)-triene, 1,4,9-triene, 1,4,9(11)-triene, 1,4,12-triene, 1,4,13(17)-triene, 1,4,14-triene, 1,4,15-triene, 1,4,16-triene, 1,5,7-triene, 1,5,8-triene, 1,5,8(14)-triene, 1,5,9-triene, 1,5,9(11)-triene, 1,5,12-triene, 1,5,13(17)-triene, 1,5,14-triene, 1,5,15-triene, 1,5,16-triene, 1,5(10),6-triene, 1,5(10),7-triene, 1,5(10),8-triene, 1,5(10),8(14)-triene, 1,5(10),9(11)-triene, 1,5(10),12-triene, 1,5(10),13(17)-triene, 1,5(10),14-triene, 1,5(10),15-triene, 1,5(10),16-triene, 1,6,8-triene, 1,6,8(14)-triene, 1,6,9-triene, 1,6,9(11)-triene, 1,6,12-triene, 1,6,13(17)-triene, 1,6,14-triene, 1,6,15-triene, 1,6,16-triene, 1,7,9-triene, 1,7,9(11)-triene, 1,7,12-triene, 1,7,13(17)-triene, 1,7,14-triene, 1,7,15-triene, 1,7,16-triene,2,4,6-triene, 2,5,6-triene, 2,5(10),6-triene, 2,4,7-triene, 2,5,7-triene, 2,5(10),7-triene, 2,4,8-triene, 2,5,8-triene, 2,5(10),8-triene, 2,4,8(14)-triene, 2,5,8(14)-triene, 2,5(10),8(14)-triene, 2,4,9(11)-triene, 2,5,9(11)-triene, 2,5(10),9(11)-triene, 2,4,11-triene, 2,5,11-triene, 2,5(10),11-triene, 2,4,12-triene, 2,5,12-triene, 2,5(10),12-triene, 2,4,14-triene, 2,5,14-triene, 2,5(10),14-triene, 2,4,15-triene, 2,5,15-triene, 2,5(10),15-triene, 2,4,16-triene, 2,5,16-triene, 2,5(10),16-triene, 2,6,8-triene, 2,6,8(14)-triene, 2,6,9-triene, 2,6,9(11)-triene, 2,6,12-triene, 2,6,13(17)-triene, 2,6,14-triene, 2,6,15-triene, 2,6,16-triene, 2,7,9-triene, 2,7,9(11)-triene, 2,7,12-triene, 2,7,13(17)-triene, 2,7,14-triene, 2,7,15-triene, 2,7,16-triene, 3,5,9-triene, 3,5,11-triene, 3,5,12-triene, 3,5,13-triene, 3,5,14-triene, 3,5,15-triene, 3,5,16-triene, 3,6,8-triene, 3,6,8(14)-triene, 3,6,9-triene, 3,6,9(11)-triene, 3,6,11-triene, 3,6,12-triene, 3,6,13(17)-triene, 3,6,14-triene, 3,6,15-triene, 3,6,16-triene, 3,7,9-triene, 3,7,11-triene, 3,7,12-triene, 3,7,13(17)-triene, 3,7,14-triene, 3,7,15-triene, 3,7,16-triene,3,8,11-triene, 3,8,12-triene, 3,8,13(17)-triene, 3,8,14-triene, 3,8,15-triene, 3,8,16-triene, 3,8(14),11-triene, 3,8(14),12-triene, 3,8(14),13(17)-triene, 3,8(14),15-triene, 3,8(14),16-triene, 3,9,11-triene, 3,9,12-triene, 3,9,13(17)-triene, 3,9,14-triene, 3,9,15-triene, 3,9,16-triene, 3,9(11),12-triene, 3,9(11),13(17)-triene, 3,9(11),14-triene, 3,9(11),15-triene, 3,9(11),16-triene, 3,11,13(17)-triene, 3,11,14-triene, 3,11,15-triene, 3,11,16-triene, 3,12,14-triene, 3,12,15-triene, 3,12,16-triene, 3,13(17),14-triene, 3,13(17),15-triene, 3,14,16-triene, 4,6,8-triene, 4,6,8(14)-triene, 4,6,9-triene, 4,6,9(11)-triene, 4,6,11-triene, 4,6,12-triene, 4,6,13(17)-triene, 4,6,14-triene, 4,6,15-triene, 4,6,16-triene, 4,7,9-triene, 4,7,11-triene, 4,7,12-triene, 4,7,13(17)-triene, 4,7,14-triene, 4,7,15-triene, 4,7,16-triene, 4,8,9-triene, 4,8,9(11)-triene, 4,8,11-triene, 4,8,12-triene, 4,8,13(17)-triene, 4,8,14-triene, 4,8,15-triene, 4,8,16-triene, 4,8(14),9-triene, 4,8(14),9(11)-triene, 4,8(14),11-triene, 4,8(14),12-triene, 4,8(14),13(17)-triene, 4,8(14),15-triene, 4,8(14),16-triene, 4,9,11-triene, 4,9,12-triene, 4,9,13(17)-triene, 4,9,14-triene, 4,9,15-triene, 4,9,16-triene, 4,9(11),12-triene, 4,9(11),13(17)-triene, 4,9(11),14-triene, 4,9(11),15-triene, 4,9(11),16-triene, 4,11,13(17)-triene, 4,11,14-triene, 4,11,15-triene, 4,11,16-triene, 4,12,14-triene, 4,12,15-triene, 4,12,16-triene, 4,13(17),14-triene, 4,13(17),15-triene, 4,14,16-triene, 5,7,9-triene, 5,7,9(11)-triene, 5,7,12-triene, 5,7,13(17)-triene, 5,7,14-triene, 5,7,15-triene, 5,7,16-triene, 5,8,11-triene, 5,8,12-triene, 5,8,13(17)-triene, 5,8,14-triene, 5,8,15-triene, 5,8,16-triene, 5,8(14),9-triene, 5,8(14),9(11)-triene, 5,8(14),12-triene, 5,8(14),13(17)-triene, 5,8(14),15-triene, 5,8(14),16-triene, 5,9,11-triene, 5,9,12-triene, 5,9,13(17)-triene, 5,9,14-triene, 5,9,15-triene, 5,9,16-triene, 5,9(11),12-triene, 5,9(11),13(17)-triene, 5,9(11),14-triene, 5,9(11),15-triene, 5,9(11),16-triene, 5,11,13(17)-triene, 5,11,14-triene, 5,11,15-triene, 5,11,16-triene, 5,12,14-triene, 5,12,15-triene, 5,12,16-triene, 5,13(17),14-triene, 5,13(17),15-triene, 5,14,16-triene, 6,8,11-triene, 6,8,12-triene, 6,8,13(17)-triene, 6,8,14-triene, 6,8,15-triene, 6,8,16-triene, 6,8(14),9-triene, 6,8(14),9(11)-triene, 6,8(14),11-triene, 6,8(14),12-triene, 6,8(14),13(17)-triene, 6,8(14),15-triene, 6,8(14),16-triene, 6,9,11-triene, 6,9,12-triene, 6,9,13(17)-triene, 6,9,14-triene, 6,9,15-triene, 6,9,16-triene, 6,9(11),12-triene, 6,9(11),13(17)-triene, 6,9(11),14-triene, 6,9(11),15-triene, 6,9(11),16-triene, 6,11,13(17)-triene, 6,11,14-triene, 6,11,15-triene, 6,11,16-triene, 6,12,14-triene, 6,12,15-triene, 6,12,16-triene, 6,13(17),14-triene, 6,13(17),15-triene, 6,14,16-triene, 7,9,11-triene, 7,9,12-triene, 7,9,13(17)-triene, 7,9,14-triene, 7,9,15-triene, 7,9,16-triene, 7,9(11),12-triene, 7,9(11),13(17)-triene, 7,9(11),14-triene, 7,9(11),15-triene, 7,9(11),16-triene, 7,12,14-triene, 7,12,15-triene, 7,12,16-triene, 7,13(17),14-triene, 7,13(17),15-triene, 7,14,16-triene, 8,11,13(17)-triene, 8,11,14-triene, 8,11,15-triene, 8,11,16-triene, 8,12,14-triene, 8,12,15-triene, 8,12,16-triene, 8,13(17),14-triene, 8,13(17),15-triene, 8,14,16-triene, 8(14),9,11-triene, 8(14),9,12-triene, 8(14),9,13(17)-triene, 8(14),9,15-triene, 8(14),9,16-triene, 8(14),9(11),12-triene, 8(14),9(11),13(17)-triene, 8(14),9(11),15-triene, 8(14),9(11),16-triene, 9,11,13(17)-triene, 9,11,14-triene, 9,11,15-triene, 9,11,16-triene, 9(11),13(17),14-triene, 9(11),13(17),15-triene, 11,13(17),14-triene, 11,13(17),15-triene, 12,14,16-triene, 1,3,5(10),6-tetraene, 1,3,5(10),7-tetraene, 1,3,5(10),8(9)-tetraene, 1,3,5(10),8(14)-tetraene, 1,3,5(10),9(11)-tetraene, 1,3,5(10),11-tetraene, 1,3,5(10),12-tetraene, 1,3,5(10),13(17)-tetraene, 1,3,5(10),14-tetraene, 1,3,5(10),15-tetraene, 1,3,5(10),16-tetraene, 1,3,5,7-tetraene, 1,3,5,8-tetraene, 1,3,5,8(14)-tetraene, 1,3,5,9-tetraene, 1,3,5,9(11)-tetraene, 1,3,5,12-tetraene, 1,3,5,13(17)-tetraene, 1,3,5,14-tetraene, 1,3,5,15-tetraene, 1,3,5,16-tetraene, 1,3,6,8-tetraene, 1,3,6,8(14)-tetraene, 1,3,6,9-tetraene, 1,3,6,9(11)-tetraene, 1,3,6,12-tetraene, 1,3,6,13(17)-tetraene, 1,3,6,14-tetraene, 1,3,6,15-tetraene, 1,3,6,16-tetraene, 1,3,7,9-tetraene, 1,3,7,9(11)-tetraene, 1,3,7,11-tetraene, 1,3,7,12-tetraene, 1,3,7,13(17)-tetraene, 1,3,7,14-tetraene, 1,3,7,15-tetraene, 1,3,7,16-tetraene,1,3,8,9-tetraene, 1,3,8,9(11)-tetraene, 1,3,8,12-tetraene, 1,3,8,13(17)-tetraene, 1,3,8,14-tetraene, 1,3,8,15-tetraene, 1,3,8,16-tetraene, 1,3,8(14)9-tetraene, 1,3,8(14)9(11)-tetraene, 1,3,8(14)12-tetraene, 1,3,8(14)13(17)-tetraene, 1,3,8(14)15-tetraene, 1,3,8(14)16-tetraene, 1,3,9,11-tetraene, 1,3,9,12-tetraene, 1,3,9,13(17)-tetraene, 1,3,9,14-tetraene, 1,3,9,15-tetraene, 1,3,9,16-tetraene, 1,3,9(11),12-tetraene, 1,3,9(11),113(17)-tetraene, 1,3,9(11),14-tetraene, 1,3,9(11),15-tetraene, 1,3,9(11),16-tetraene, 1,3,12,14-tetraene, 1,3,12,15-tetraene, 1,3,12,16-tetraene, 1,3,13(17),14-tetraene, 1,3,13(17),15-tetraene, 1,3,13(17),16-tetraene, 1,3,14,16-tetraene, 1,4,6,8-tetraene, 1,4,6,8(14)-tetraene, 1,4,6,9-tetraene, 1,4,6,9(11)-tetraene, 1,4,6,11-tetraene, 1,4,6,12-tetraene, 1,4,6,13(17)-tetraene, 1,4,6,14-tetraene, 1,4,6,15-tetraene, 1,4,6,16-tetraene, 1,5,7,9-tetraene, 1,5,7,9(11)-tetraene, 1,5,7,11-tetraene, 1,5,7,12-tetraene, 1,5,7,13(17)-tetraene, 1,5,7,14-tetraene, 1,5,7,15-tetraene, 1,5,7,16-tetraene, 1,5,8,11-tetraene, 1,5,8,12-tetraene, 1,5,8,13(17)-tetraene, 1,5,8,14-tetraene, 1,5,8,15-tetraene, 1,5,8,16-tetraene, 1,5,8(14),9-tetraene, 1,5,8(14),9(11)-tetraene, 1,5,8(14),11-tetraene, 1,5,8(14),12-tetraene, 1,5,8(14),13(17)-tetraene, 1,5,8(14),15-tetraene, 1,5,8(14),16-tetraene, 1,5,9,11-tetraene, 1,5,9,12-tetraene, 1,5,9,13(17)-tetraene, 1,5,9,14-tetraene, 1,5,9,15-tetraene, 1,5,9,16-tetraene, 1,5,9(11), 12-tetraene, 1,5,9(11), 13(17)-tetraene, 1,5,9(11),14-tetraene, 1,5,9(11),15-tetraene, 1,5,9(11),16-tetraene, 1,5,11,13(17)-tetraene, 1,5,11,14-tetraene, 1,5,11,15-tetraene, 1,5,11,16-tetraene, 1,5,12,14-tetraene, 1,5,12,15-tetraene, 1,5,12,16-tetraene, 1,5,13(17),14-tetraene, 1,5,13(17),15-tetraene, 1,5,14,16-tetraene, 1,4,7,15-tetraene, 1,5,7,15-tetraene, 1,3,7,16-tetraene, 1,4,6,8-tetraene, 1,4,6,9-tetraene, 1,4,6,9(11)-tetraene, 1,4,6,11-tetraene, 1,4,6,12-tetraene, 1,4,6,13(17)-tetraene, 1,4,6,14-tetraene, 1,4,6,15-tetraene, 1,4,6,16-tetraene, 1,4,7,9-tetraene, 1,4,7,9(11)-tetraene, 1,4,7,11-tetraene, 1,4,7,12-tetraene, 1,4,7,13(17)-tetraene, 1,4,7,14-tetraene, 1,4,7,15-tetraene, 1,4,7,16-tetraene, 1,6,8,11-tetraene, 1,6,8,12-tetraene, 1,6,8,13(17)-tetraene, 1,6,8,14-tetraene, 1,6,8,15-tetraene, 1,6,8,16-tetraene, 1,6,8(14),9-tetraene, 1,6,8(14),9(11)-tetraene, 1,6,8(14),11-tetraene, 1,6,8(14),12-tetraene, 1,6,8(14),13(17)-tetraene, 1,6,8(14),15-tetraene, 1,6,8(14),16-tetraene, 1,6,9,11-tetraene, 1,6,9,12-tetraene, 1,6,9,13(17)-tetraene, 1,6,9,14-tetraene, 1,6,9,15-tetraene, 1,6,9,16-tetraene, 1,6,9(11),12-tetraene, 1,6,9(11),13(17)-tetraene, 1,6,9(11),14-tetraene, 1,6,9(11),15-tetraene, 1,6,9(11), 16-tetraene, 1,6,11,13(17)-tetraene, 1,6,11,14-tetraene, 1,6,11,15-tetraene, 1,6,12,14-tetrane, 1,6,12,15-tetrane, 1,6,12,16-tetrane, 1,6,13(17),14-tetraene, 1,6,13(17),15-tetraene, 1,6,14,16-tetraene, 1,7,9,11-tetraene, 1,7,9,12-tetraene, 1,7,9,13(17)-tetraene, 1,7,9,14-tetraene, 1,7,9,15-tetraene, 1,7,9,16-tetraene, 1,8,11,13(17)-tetraene, 1,8,11,14-tetraene, 1,8,11,15-tetraene, 1,8,11,16-tetraene, 1,8(14),9,11-tetraene, 1,8(14),9,12-tetraene, 1,8(14),9,13(17)-tetraene, 1,8(14),9,15-tetraene, 1,8(14),9,16-tetraene, 1,9,11,13(17)-tetraene, 1,9,11,14-tetraene, 1,9,11,15-tetraene, 1,9,11,16-tetraene, 1,9(11),12,14-tetraene, 1,9(11),12,15-tetraene, 1,9(11),12,16-tetraene, 1,11,13(17),14-tetraene, 1,11,13(17),15-tetraene, 1,11,13(17),16-tetraene, 1,12,14,16-tetraene, 1,8,11,13(17)-tetraene, 1,8,11,14-tetraene, 1,8,11,15-tetraene, 1,9,11,13(17)-tetraene, 1,9,11,14-tetraene, 1,9,11,15-tetraene, 1,9,11,16-tetraene, 1,9(11),12,14-tetraene, 1,9(11),12,15-tetraene, 1,9(11),12,16-tetraene, 1,11,13(17),14-tetraene, 1,11,13(17),15-tetraene, 1,11,13(17),16-tetraene, 1,12,14,16-tetraene, 2,4,6,8-tetraene, 2,4,6,8(14)-tetraene, 2,4,6,9-tetraene, 2,4,6,9(11)-tetraene, 2,4,6,11-tetraene, 2,4,6,12-tetraene, 2,4,6,13(17)-tetraene, 2,4,6,14-tetraene, 2,4,6,15-tetraene, 2,4,6,16-tetraene, 2,5,7,9-tetraene, 2,5,7,9(11)-tetraene, 2,5,7,11-tetraene, 2,5,7,12-tetraene, 2,5,7,13(17)-tetraene, 2,5,7,14-tetraene, 2,5,7,15-tetraene, 2,5,7,16-tetraene, 2,5,8,11-tetraene, 2,5,8,12-tetraene, 2,5,8,13(17)-tetraene, 2,5,8,14-tetraene, 2,5,8,15-tetraene, 2,5,8,16-tetraene, 2,5,8(14),9-tetraene, 2,5,8(14),9(11)-tetraene, 2,5,8(14),11-tetraene, 2,5,8(14),12-tetraene, 2,5,8(14),13(17)-tetraene, 2,5,8(14),15-tetraene, 2,5,8(14),16-tetraene, 2,5,9,11-tetraene, 2,5,9,12-tetraene, 2,5,9,13(17)-tetraene, 2,5,9,14-tetraene, 2,5,9,15-tetraene, 2,5,9,16-tetraene, 2,5,9(11),12-tetraene, 2,5,9(11),13(17)-tetraene, 2,5,9(11),14-tetraene, 2,5,9(11),15-tetraene, 2,5,9(11),16-tetraene, 2,5,11,13(17)-tetraene, 2,5,11,14-tetraene, 2,5,11,15-tetraene, 2,5,11,16-tetraene, 2,5,12,14-tetraene, 2,5,12,15-tetraene, 2,5,12,16-tetraene, 2,5,13(17),14-tetraene, 2,5,13(17),15-tetraene, 2,5,14,16-tetraene, 2,4,7,15-tetraene, 2,5,7,15-tetraene, 2,4,6,8-tetraene, 2,4,6,9-tetraene, 2,4,6,9(11)-tetraene, 2,4,6,11-tetraene, 2,4,6,12-tetraene, 2,4,6,13(17)-tetraene, 2,4,6,14-tetraene, 2,4,6,15-tetraene, 2,4,6,16-tetraene, 2,4,7,9-tetraene, 2,4,7,9(11)-tetraene, 2,4,7,11-tetraene, 2,4,7,12-tetraene, 2,4,7,13(17)-tetraene, 2,4,7,14-tetraene, 2,4,7,15-tetraene, 2,4,7,16-tetraene, 2,6,8,11-tetraene, 2,6,8,12-tetraene, 2,6,8,13(17)-tetraene, 2,6,8,14-tetraene, 2,6,8,15-tetraene, 2,6,8,16-tetraene, 2,6,8(14),9-tetraene, 2,6,8(14),9(11)-tetraene, 2,6,8(14),11-tetraene, 2,6,8(14),12-tetraene, 2,6,8(14),13(17)-tetraene, 2,6,8(14),15-tetraene, 2,6,8(14),16-tetraene, 2,6,9,11-tetraene, 2,6,9,12-tetraene, 2,6,9,13(17)-tetraene, 2,6,9,14-tetraene, 2,6,9,15-tetraene, 2,6,9,16-tetraene, 2,6,9(11),12-tetraene, 2,6,9(11),13(17)-tetraene, 2,6,9(11),14-tetraene, 2,6,9(11),15-tetraene, 2,6,9(11),16-tetraene, 2,6,11,13(17)-tetraene, 2,6,11,14-tetraene, 2,6,11,15-tetraene, 2,6,12,14-tetrane, 2,6,12,15-tetrane, 2,6,12,16-tetrane, 2,6,13(17),14-tetraene, 2,6,13(17),15-tetraene, 2,6,14,16-tetraene, 2,7,9,11-tetraene, 2,7,9,12-tetraene, 2,7,9,13(17)-tetraene, 2,7,9,14-tetraene, 2,7,9,15-tetraene, 2,7,9,16-tetraene, 2,8,11,13(17)-tetraene, 2,8,11,14-tetraene, 2,8,11,15-tetraene, 2,8,11,16-tetraene, 2,8(14),9,11-tetraene, 2,8(14),9,12-tetraene, 2,8(14),9,13(17)-tetraene, 2,8(14),9,15-tetraene, 2,8(14),9,16-tetraene, 2,9,11,13(17)-tetraene, 2,9,11,14-tetraene, 2,9,11,15-tetraene, 2,9,11,16-tetraene, 2,9(11),12,14-tetraene, 2,9(11),12, 15-tetraene, 2,9(11),12, 16-tetraene, 2,11,13(17),14-tetraene, 2,11,13(17),15-tetraene, 2,11,13(17),16-tetraene, 2,12,14,16-tetraene, 2,8,11,13(17)-tetraene, 2,8,11,14-tetraene, 2,8,11,15-tetraene, 2,9,11,13(17)-tetraene, 2,9,11,14-tetraene, 2,9,11,15-tetraene, 2,9,11,16-tetraene, 2,9(11),12,14-tetraene, 2,9(11),12,15-tetraene, 2,9(11),12,16-tetraene, 2,11,13(17),14-tetraene, 2,11,13(17),15-tetraene, 2,11,13(17),16-tetraene, 2,12,14,16-tetraene, 3,5,7,9-tetraene, 3,5,7,9(11)-tetraene, 3,5,7,11-tetraene, 3,5,7,12-tetraene, 3,5,7,13(17)-tetraene, 3,5,7,14-tetraene, 3,5,7,15-tetraene, 3,5,7,16-tetraene, 3,5,8,11-tetraene, 3,5,8,12-tetraene, 3,5,8,13(17)-tetraene, 3,5,8,14-tetraene, 3,5,8,15-tetraene, 3,5,8,16-tetraene, 3,5,8(14),9-tetraene, 3,5,8(14),9(11)-tetraene, 3,5,8(14),11-tetraene, 3,5,8(14),12-tetraene, 3,5,8(14),13(17)-tetraene, 3,5,8(14),15-tetraene, 3,5,8(14),16-tetraene, 3,5,9,11-tetraene, 3,5,9,12-tetraene, 3,5,9,13(17)-tetraene, 3,5,9,14-tetraene, 3,5,9,15-tetraene, 3,5,9,16-tetraene, 3,5,9(11),12-tetraene, 3,5,9(11),13(17)-tetraene, 3,5,9(11),14-tetraene, 3,5,9(11),15-tetraene, 3,5,9(11),16-tetraene, 3,5,11,13(17)-tetraene, 3,5,11,14-tetraene, 3,5,11,15-tetraene, 3,5,11,16-tetraene, 3,5,12,14-tetraene, 3,5,12,15-tetraene, 3,5,12,16-tetraene, 3,5,13(17),14-tetraene, 3,5,13(17),15-tetraene, 3,5,14,16-tetraene, 3,4,7,15-tetraene, 3,5,7,15-tetraene, 3,5,7,16-tetraene, 3,4,6,8-tetraene, 3,4,6,9-tetraene, 3,4,6,9(11)-tetraene, 3,4,6,11-tetraene, 3,4,6,12-tetraene, 3,4,6,13(17)-tetraene, 3,4,6,14-tetraene, 3,4,6,15-tetraene, 3,4,6,16-tetraene, 3,4,7,9-tetraene, 3,4,7,9(11)-tetraene, 3,4,7,11-tetraene, 3,4,7,12-tetraene, 3,4,7,13(17)-tetraene, 3,4,7,14-tetraene, 3,4,7,15-tetraene, 3,4,7,16-tetraene, 3,6,8,11-tetraene, 3,6,8,12-tetraene, 3,6,8,13(17)-tetraene, 3,6,8,14-tetraene, 3,6,8,15-tetraene, 3,6,8,16-tetraene, 3,6,8(14),9-tetraene, 3,6,8(14),9(11)-tetraene, 3,6,8(14),11-tetraene, 3,6,8(14),12-tetraene, 3,6,8(14),13(17)-tetraene, 3,6,8(14),15-tetraene, 3,6,8(14),16-tetraene, 3,6,9,11-tetraene, 3,6,9,12-tetraene, 3,6,9,13(17)-tetraene, 3,6,9,14-tetraene, 3,6,9,15-tetraene, 3,6,9,16-tetraene, 3,6,9(11),12-tetraene, 3,6,9(11),13(17)-tetraene, 3,6,9(11),14-tetraene, 3,6,9(11),15-tetraene, 3,6,9(11),16-tetraene, 3,6,11,13(17)-tetraene, 3,6,11,14-tetraene, 3,6,11,15-tetraene, 3,6,12,14-tetrane, 3,6,12,15-tetrane, 3,6,12,16-tetrane, 3,6,13(17),14-tetraene, 3,6,13(17),15-tetraene, 3,6,14,16-tetraene, 3,7,9,11-tetraene, 3,7,9,12-tetraene, 3,7,9,13(17)-tetraene, 3,7,9,14-tetraene, 3,7,9,15-tetraene, 3,7,9,16-tetraene, 3,8,11,13(17)-tetraene, 3,8,11,14-tetraene, 3,8,11,15-tetraene, 3,8,11,16-tetraene, 3,8(14),9,11-tetraene, 3,8(14),9,12-tetraene, 3,8(14),9,13(17)-tetraene, 3,8(14),9,15-tetraene, 3,8(14),9,16-tetraene, 3,9,11,13(17)-tetraene, 3,9,11,14-tetraene, 3,9,11,15-tetraene, 3,9,11,16-tetraene, 3,9(11),12,14-tetraene, 3,9(11),12,15-tetraene, 3,9(11),12,16-tetraene, 3,11,13(17),14-tetraene, 3,11,13(17),15-tetraene, 3,11,13(17),16-tetraene, 3,12,14,16-tetraene, 3,8,11,13(17)-tetraene, 3,8,11,14-tetraene, 3,8,11,15-tetraene, 3,9,11,13(17)-tetraene, 3,9,11,14-tetraene, 3,9,11,15-tetraene, 3,9,11,16-tetraene, 3,9(11),12,14-tetraene, 3,9(11),12,15-tetraene, 3,9(11),12,16-tetraene, 3,11,13(17),14-tetraene, 3,11,13(17),15-tetraene, 3,11,13(17),16-tetraene, 3,12,14,16-tetraene, 3,5(10),7,9(11)-tetraene, 3,5(10),7,11-tetraene, 3,5(10),7,12-tetraene, 3,5(10),7,13(17)-tetraene, 3,5(10),7,14-tetraene, 3,5(10),7,15-tetraene, 3,5(10),7,16-tetraene, 3,5(10),8,11-tetraene, 3,5(10),8,12-tetraene, 3,5(10),8,13(17)-tetraene, 3,5(10),8,14-tetraene, 3,5(10),8,15-tetraene, 3,5(10),8,16-tetraene, 3,5(10),8(14),9-tetraene, 3,5(10),8(14),9(11)-tetraene, 3,5(10),8(14),11-tetraene, 3,5(10),8(14),12-tetraene, 3,5(10),8(14), 13(17)-tetraene, 3,5(10),8(14), 15-tetraene, 3,5(10),8(14),16-tetraene, 3,5(10),9,11-tetraene, 3,5(10),9,12-tetraene, 3,5(10),9,13(17)-tetraene, 3,5(10),9,14-tetraene, 3,5(10),9,15-tetraene, 3,5(10),9,16-tetraene, 3,5(10),9(11),12-tetraene, 3,5(10),9(11),13(17)-tetraene, 3,5(10),9(11),14-tetraene, 3,5(10),9(11),15-tetraene, 3,5(10),9(11),16-tetraene, 3,5(10), 11,13(17)-tetraene, 3,5(10),11,14-tetraene, 3,5(10),11,15-tetraene, 3,5(10),11,16-tetraene, 3,5(10),12,14-tetraene, 3,5(10),12,15-tetraene, 3,5(10),12,16-tetraene, 3,5(10),13(17),14-tetraene, 3,5(10),13(17),15-tetraene, 3,5(10),14,16-tetraene, 4,6,8,11-tetraene, 4,6,8,12-tetraene, 4,6,8,13(17)-tetraene, 4,6,8,14-tetraene, 4,6,8,15-tetraene, 4,6,8,16-tetraene, 4,6,8(14),9-tetraene, 4,6,8(14),9(11)-tetraene, 4,6,8(14),11-tetraene, 4,6,8(14),12-tetraene, 4,6,8(14),13(17)-tetraene, 4,6,8(14),15-tetraene, 4,6,8(14),16-tetraene, 4,6,9,11-tetraene, 4,6,9,12-tetraene, 4,6,9,13(17)-tetraene, 4,6,9,14-tetraene, 4,6,9,15-tetraene, 4,6,9,16-tetraene, 4,6,9(11),12-tetraene, 4,6,9(11),13(17)-tetraene, 4,6,9(11),14-tetraene, 4,6,9(11),15-tetraene, 4,6,9(11), 16-tetraene, 4,6,11,13(17)-tetraene, 4,6,11,14-tetraene, 4,6,11,15-tetraene, 4,6,12,14-tetrane, 4,6,12,15-tetrane, 4,6,12,16-tetrane, 4,6,13(17),14-tetraene, 4,6,13(17),15-tetraene, 4,6,14,16-tetraene, 4,7,9,11-tetraene, 4,7,9,12-tetraene, 4,7,9,13(17)-tetraene, 4,7,9,14-tetraene, 4,7,9,15-tetraene, 4,7,9,16-tetraene, 4,8,11,13(17)-tetraene, 4,8,11,14-tetraene, 4,8,11,15-tetraene, 4,8,11,16-tetraene, 4,8(14),9,11-tetraene, 4,8(14),9,12-tetraene, 4,8(14),9,13(17)-tetraene, 4,8(14),9,15-tetraene, 4,8(14),9,16-tetraene, 4,9,11,13(17)-tetraene, 4,9,11,14-tetraene, 4,9,11,15-tetraene, 4,9,11,16-tetraene, 4,9(11),12,14-tetraene, 4,9(11),12,15-tetraene, 4,9(11),12,16-tetraene, 4,11,13(17), 14-tetraene, 4,11,13(17), 15-tetraene, 4,11,13(17),16-tetraene, 4,12,14,16-tetraene, 4,8,11,13(17)-tetraene, 4,8,11,14-tetraene, 4,8,11,15-tetraene, 4,9,11,13(17)-tetraene, 4,9,11,14-tetraene, 4,9,11,15-tetraene, 4,9,11,16-tetraene, 4,9(11),12,14-tetraene, 4,9(11),12,15-tetraene, 4,9(11),12,16-tetraene, 4,11,13(17), 14-tetraene, 4,11,13(17), 15-tetraene, 4,11,13(17),16-tetraene, 4,12,14,16-tetraene, 5,7,9,11-tetraene, 5,7,9,12-tetraene, 5,7,9,13(17)-tetraene, 5,7,9,14-tetraene, 5,7,9,15-tetraene, 5,7,9,16-tetraene, 5,8,11,13(17)-tetraene, 5,8,11,14-tetraene, 5,8,11,15-tetraene, 5,8,11,16-tetraene, 5,8(14),9,11-tetraene, 5,8(14),9,12-tetraene, 5,8(14),9,13(17)-tetraene, 5,8(14),9,15-tetraene, 5,8(14),9,16-tetraene, 5,9,11,13(17)-tetraene, 5,9,11,14-tetraene, 5,9,11,15-tetraene, 5,9,11,16-tetraene, 5,9(11),12,14-tetraene, 5,9(11),12,15-tetraene, 5,9(11),12,16-tetraene, 5,11,13(17),14-tetraene, 5,11,13(17),15-tetraene, 5,11,13(17),16-tetraene, 5,12,14,16-tetraene, 5,8,11,13(17)-tetraene, 5,8,11,14-tetraene, 5,8,11,15-tetraene, 5,9,11,13(17)-tetraene, 5,9,11,14-tetraene, 5,9,11,15-tetraene, 5,9,11,16-tetraene, 5,9(11),12,14-tetraene, 5,9(11),12,15-tetraene, 5,9(11),12,16-tetraene, 5,11,13(17),14-tetraene, 5,11,13(17),15-tetraene, 5,11,13(17),16-tetraene, 5,12,14,16-tetraene, 5(10),8,11,13(17)-tetraene, 5(10),8,11,14-tetraene, 5(10),8,11,15-tetraene, 5(10),8,11,16-tetraene, 5(10),8(14),9,11-tetraene, 5(10),8(14),9,12-tetraene, 5(10),8(14),9,13(17)-tetraene, 5(10),8(14),9,15-tetraene, 5(10),8(14),9,16-tetraene, 5(10),9,11,13(17)-tetraene, 5(10),9,11,14-tetraene, 5(10),9,11,15-tetraene, 5(10),9,11,16-tetraene, 5(10),9(11),12,14-tetraene, 5(10),9(11),12,15-tetraene, 5(10),9(11),12,16-tetraene, 5(10),11,13(17),14-tetraene, 5(10),11,13(17),15-tetraene, 5(10),11,13(17),16-tetraene, 5(10),12,14,16-tetraene, 5(10),8,11,13(17)-tetraene, 5(10),8,11,14-tetraene, 5(10),8,11,15-tetraene, 5(10),9,11,13(17)-tetraene, 5(10),9,11,14-tetraene, 5(10),9,11,15-tetraene, 5(10),9,11,16-tetraene, 5(10),9(11),12,14-tetraene, 5(10),9(11),12,15-tetraene, 5(10),9(11),12,16-tetraene, 5(10),11,13(17),14-tetraene, 5(10),11,13(17),15-tetraene, 5(10),11,13(17),16-tetraene, 5(10),12,14,16-tetraene, 6,8,11,13(17)-tetraene, 6,8,11,14-tetraene, 6,8,11,15-tetraene, 6,8,11,16-tetraene, 6,9,11,13(17)-tetraene, 6,9,11,14-tetraene, 6,9,11,15-tetraene, 6,9,11,16-tetraene, 6,9(11),12,14-tetraene, 6,9(11),12,15-tetraene, 6,9(11),12,16-tetraene, 6,11,13(17),14-tetraene, 6,11,13(17),15-tetraene, 6,12,14,16-tetraene, 7,9,11,13(17)-tetraene, 7,9,11,14-tetraene, 7,9,11,15-tetraene, 7,9,11,16-tetraene, 7,9(11),12,14-tetraene, 7,9(11),12,15-tetraene, 7,9(11),12,16-tetraene, 8,11,13(17),14-tetraene, 8,11,13(17),15-tetraene, 8(14),9,11,13(17)-tetraene, 8(14),9,11,15-tetraene, 8(14),9,11,16-tetraene, 9,11,13(17),14-tetraene, 9,11,13(17),15-tetraene, 9(11),12, 14,16-tetraene, 11,13(17),14,16-tetraene, 1,3,5(10),6,8-pentaene, 1,3,5(10),6,9(11)-pentaene, 1,3,5(10),6,11-pentaene, 1,3,5(10),6,12-pentaene, 1,3,5(10),6,13(17)-pentaene, 1,3,5(10),6,14-pentaene, 1,3,5(10),6,15-pentaene, 1,3,5(10),6,16-pentaene, 1,3,5(10),7,9(11)-pentaene, 1,3,5(10),7,11-pentaene, 1,3,5(10),7,12-pentaene, 1,3,5(10),7,13(17)-pentaene, 1,3,5(10),7,14-pentaene, 1,3,5(10),7,15-pentaene, 1,3,5(10),7,16-pentaene, 1,3,5(10),8,11-pentaene, 1,3,5(10),8,12-pentaene, 1,3,5(10),8,13(17)-pentaene, 1,3,5(10),8,14-pentaene, 1,3,5(10),8,15-pentaene, 1,3,5(10),8,16-pentaene, 1,3,5(10),8(14),9(11)-pentaene, 1,3,5(10),8(14),11-pentaene, 1,3,5(10),8(14),12-pentaene, 1,3,5(10),8(14),13(17)-pentaene, 1,3,5(10),8(14),15-pentaene, 1,3,5(10),8(14),16-pentaene, 1,3,5(10),9(11),12-pentaene, 1,3,5(10),9(11),13(17)-pentaene, 1,3,5(10),9(11),14-pentaene, 1,3,5(10),9(11),15-pentaene, 1,3,5(10),9(11),16-pentaene, 1,3,5(10),11,13(17)-pentaene, 1,3,5(10),11,14-pentaene, 1,3,5(10),11,15-pentaene, 1,3,5(10),11,16-pentaene, 1,3,5(10),12,14-pentaene, 1,3,5(10),12,15-pentaene, 1,3,5(10),12,16-pentaene, 1,3,5(10),13(17),14-pentaene, 1,3,5(10),13(17),15-pentaene or a 1,3,5(10),14,16-pentaene androstene or, when no double bond is present at the 5-position or a 5α-androstene or a 5β-androstene.

4. The method of embodiment 1, 2 or 3 wherein (1) R⁵ and R⁶ respectively are in the α,α, α,β, β,α or β,β configuration and R⁵ and R⁶ are optionally both -CH₃ or are optionally selected from -H, -F, -OH, -CH₃, -C₂H₅, -C≡CH, -C≡CCH₃, and -CH₂OH or (2) R⁵ and R⁶ are both in the β-configuration and R⁵ and R⁶ are optionally both -H, -F, -CH₃ -C≡CH or -CH₂OH.

5. The method of embodiment 1, 2, 3 or 4 wherein R¹⁰ at the 5, 8, 9 and 14-positions (if present) respectively are (1) -H, -H, -H, -H; (2) -H, -H, halogen (-F, -Cl, - Br or -I), -H; (3) -H, -H, -H, -OH; (4) -H, -H, halogen (-F, -Cl, -Br or -I), -OH; (5) - optionally substituted alkyl (e.g., -CH₃, -CH₂OH, -CH₂O-ester, -C₂H₅), -H, -H, -H; (6) - optionally substituted alkyl (e.g., -CH₃, -CH₂OH, -CH₂O-ester, -C₂H₅), -H, halogen (-F, - Cl, -Br or -I), -H; (7) -optionally substituted alkyl (e.g., -CH₃, -CH₂OH, -CH₂O-ester, - C₂H₅), -H, -H, -OH; (8) -acyl (e.g., -C(O)-(CH₂)₀₋₂-CH₃), -H, -H, -H; (9) -ester (e.g., acetoxy or propionoxy), -H, -H, -H; (10) -ether (e.g., -O-(CH₂)₀₋₂-CH₃), -H, -H, -H; (11) - ester (e.g., acetoxy, propionoxy, -O-C(O)-(CH₂)₁₋₆-H), -H, halogen (e.g., -F, -Cl, -Br), -H; (12) -ester (e.g., acetoxy or propionoxy), -H, -H, -OH; (13) -H, -H, -H, -acyl (e.g., -C(O)-(CH₂)₀₋₂CH₃); (14) -H, -H, -H, -ester (e.g., acetoxy or propionoxy); or (15) -H, -H, -H, - ether (e.g., -O-(CH₂)₀₋₂-CH₃, -OCH₃, -OC₂H₅, -OCH₂OH, -OCH₂F, -OCH₂Br, - OCH₂COOH, -OCH₂NH₂, -OCH₂CH₂OH, -OCH₂CH₂F, -OCH₂CH₂Br, -OCH₂CH₂COOH or -OCH₂CH₂NH₂).

6. The method of embodiment 1, 2, 3, 4 or 5 wherein R⁷ is -CH₂-, - CHOH-, -CH(αR¹⁰)-, -CH(βR¹⁰)-, -C(R¹⁰)₂-, -CH(ester)-, -CH(alkoxy)- or -CH(halogen)-where R¹⁰ are independently selected, the hydroxyl, ester or alkoxy group or the halogen atom is present in the α-configuration or the β-configuration and the alkoxy group is optionally selected from -OCH₃, -OC₂H₅ and -OC₃H₇ and the halogen atom is -F, -Cl, -Br or -I.

7. The method of embodiment 1, 2, 3, 4, 5 or 6 wherein R⁸ is -CH₂-, -CF₂-, -CH(α-OH)-, -CH(β-OH)-, -CH(α-R¹⁰)-, -CH(β-R¹⁰)-, -CH(β-ester)-, -CH(α-ester)-, - CH(β-alkoxy)-, -CH(α-alkoxy)-, -CH(β-halogen)- or -CH(α-halogen)- where the alkoxy group is optionally selected from -OCH₃, -OC₂H₅ and -OC₃H₇ and the halogen atom is - F, -CI, -Br or -I.

8. The method of embodiment 1, 2, 3, 4, 5, 6 or 7 wherein the subject has, or is subject or susceptible to developing, neutropenia or thrombocytopenia, opotionally wherein the subject is a human or another primate and optionally wherein the neutropenia is postinfectious neutropenia, autoimmune neutropenia, chronic idiopathic neutropenia or a neutropenia resulting from or potentially resulting result from a cancer chemotherapy, chemotherapy for an autoimmune disease, an antiviral therapy, radiation exposure, tissue or solid organ allograft or xenograft rejection or immune suppression therapy in tissue or solid organ transplantation or aging or immunesenescence.

9. The method of embodiment 1, 2, 3, 4, 5, 6, 7 or 8 wherein (1) the formula 1 compound is a compound in groups 1 through 57, or (2) one R⁴ in the α-configuration is -H or a C-linked moiety such as optionally substituted alkyl moiety and the other R⁴, or both R⁴ together, is in the β-configuration and is an N-linked moiety such as -NH₂, -NHR^{PR}, -N(R^{PR})₂, -NO₂ -N₃, =NOH, =NO-optionally substituted alkyl, =N-optionally substituted alkyl, an N-linked amino acid, a substituted amine, a sulfamate having the structure -NH-S(O)(O)-S-O-optionally substituted alkyl, a carbamate having the structure -NH-C(O)-O-optionally substituted alkyl, a sulfurous diamide having thet structure -NH-S(O)-NH-optionally substituted alkyl, -NH-S(O)-NH₂ or -NH-S(O)-NHR^{PR}, a sulfamide having the structure -NH-S(O)(O)-NH₂, -NH-S(O)(O)-NHR^{PR}, -NH-S(O)(O)-NH-optionally substituted alkyl or-NH-S(O)(O)-N(optionally substituted alkyl)₂, a sulfinamide having the structure -NH-S(O)-optionally substituted alkyl or an amide having the structure -NH-C(O)-optionally substituted alkyl or a salt of any of these moieties, where each optionally substituted alkyl is independently selected and optionally is a C1-12 moiety or a C1-8 moiety, or (3) one R⁴ in the β-configuration is -H or a C-linked moiety such as optionally substituted alkyl and the other R⁴, or both R⁴ together, is in the α-configuration and is an N-linked moiety such as -NH₂, -NHR^{PR}, - N(R^{PR})₂, -NO₂, -N₃, =NOH, =NO-optionally substituted alkyl, =N-optionally substituted alkyl, an N-linked amino acid, a substituted amine, a sulfamate having the structure -NH-S(O)(O)-S-O-optionally substituted alkyl, a carbamate having the structure -NH-C(O)-O-optionally substituted alkyl, a sulfurous diamide having thet structure -NH-S(O)-NH-optionally substituted alkyl, -NH-S(O)-NH₂ or -NH-S(O)-NHR^{PR}, a sulfamide having the structure -NH-S(O)(O)-NH₂, -NH-S(O)(O)-NHR^{PR}, -NH-S(O)(O)-NH-optionally substituted alkyl or -NH-S(O)(O)-N(optionally substituted alkyl)₂, a sulfinamide having the structure - NH-S(O)-optionally substituted alkyl or an amide having the structure -NH-C(O)-optionally substituted alkyl or a salt of any of these moieties, where each optionally substituted alkyl is independently selected and optionally is a C1-16 moiety, a C1-12 moiety or a C1-8 moiety, or (4) R⁴ in the β-configuration is -H or a C-linked moiety such as optionally substituted alkyl and the other R⁴, or both R⁴ together, is in the α-configuration and is an O-linked moiety or a S-linked moiety such as -OH, -SH, =O, =S, - O-S(O)(O), -OR^{PR}, -SR^{PR}, -SCN, -O-optionally substituted alkyl, -S-optionally substituted alkyl, -O-C(O)-optionally substituted alkyl, -S-C(O)-optionally substituted alkyl, -O-C(S)-optionally substituted alkyl, a sulfonate such as -S(O)(O)-O-optionally substituted alkyl or -O-S(O)(O)-optionally substituted alkyl, a sulfate ester such as -O-S(O)(O)-O-optionally substituted alkyl, a sulfite ester such as -O-S(O)-O-optionally substituted alkyl, a sulfamate having the structure -O-S(O)(O)-NH₂, -O-S(O)(O)-NH-optionally substituted alkyl or -O-S(O)(O)-N-(optionally substituted alkyl)₂, an O-linked polymer, an S-linked polymer, an optionally substituted monosaccharide, an optionally substituted disaccharide, an optionally substituted oligosaccharide, a phosphate or thiophosphate such as -O-P(O)(OH)-OH, -O-P(O)(OH)-O-(CH₂)ₙ-CH₃, -O-P(O)[O(CH₂)ₙCH₃]-O-(CH₂)ₙ-CH₃, -O-P(O)(SH)-OH, -O-P(O)(SH)-O-(CH₂)ₙ-CH₃, -O-P(O)(OH)-S-(CH₂)ₙ-CH₃ or a salt of any of these moieties, or (5) R⁴ in the α-configuration is -H or a C-linked moiety such as optionally substituted alkyl and the other R⁴, or both R⁴ together, is in the β-configuration and is an O-linked moiety or a S-linked moiety such as -OH, -SH, =O, =S, - O-S(O)(O), -OR^{PR}, -SR^{PR}, -SCN, -O-optionally substituted alkyl, -S-optionally substituted alkyl, -O-C(O)-optionally substituted alkyl, -S-C(O)-optionally substituted alkyl, -O-C(S)-optionally substituted alkyl, a sulfonate such as -S(O)(O)-O-optionally substituted alkyl or -O-S(O)(O)-optionally substituted alkyl, a sulfate ester such as -O-S(O)(O)-O-optionally substituted alkyl, a sulfite ester such as -O-S(O)-O-optionally substituted alkyl, a sulfamate having the structure -O-S(O)(O)-NH₂, -O-S(O)(O)-NH-optionally substituted alkyl or -O-S(O)(O)-N-(optionally substituted alkyl)₂, an O-linked polymer, an S-linked polymer, an optionally substituted monosaccharide, an optionally substituted disaccharide, an optionally substituted oligosaccharide, a phosphate or thiophosphate such as -O-P(O)(OH)-OH, -O-P(O)(OH)-O-(CH₂)ₙ-CH₃, -O-P(O)[O(CH₂)ₙCH₃]-O-(CH₂)ₙ-CH₃, -O-P(O)(SH)-OH, -O-P(O)(SH)-O-(CH₂)ₙ-CH₃, -O-P(O)(OH)-S-(CH₂)ₙ-CH₃ or a salt of any of these moieties, where for any of the moieties in (2), (3), (4) or (5) each optionally substituted alkyl is independently selected and optionally is a C1-16 moiety, a C1-12 moiety or a C1-8 moiety.

N-Linked R⁴ moieties can optionally be selected from -NHCH₃, -N(CH₃)₂, - NHC₂H₅, -N(C₂H₅)₂, -NHC₃H₇, -N(C₃H₇)₂, -NHC₄H₉, -N(C₄H₉)₂, -NH-C1-8 optionally substituted alkyl, -N(C1-8 optionally substituted alkyl)₂, -NH-C(O)-H, -NH-C(O)-CH₃, - NH-C(O)-OCH₃, -NH-C(O)-OC₂H₅, -NH-C(O)-OC₃H₇, -NH-C(O)-O-C1-8 optionally substituted alkyl, -NH-C(O)-C1-8 optionally substituted alkyl, -NH-(CH₂)₃-CH₃, -NH-(CH₂)₄-CH₃, -NH-(CH₂)₅-CH₃, -NH-(CH₂)₆-CH₃, -NH-(CH₂)₇-CH₃, -NH-CH₂-C₆H₅, -NH-CH₂-C₆H₄OH, -NH-CH₂-C₆H₄F, -NH-CH₂-C₆H₄Cl, -NH-CH₂-C₆H₄OCH₃, -NH-CH₂-C₆H₄CH₃, -NH-CH₂-C₆H₄-O-C(O)-(CH₂)ₙ-CH₃, -NH-CH₂-C₆H₄-C(O)-O-(CH₂)ₙ-CH₃, -NH-CH₂-COOH, -NH-(CH₂)₂-COOH, -NH-(CH₂)₃-COOH, -NH-(CH₂)₄-COOH, -NH-(CH₂)₅-COOH, -NH-CH₂-C(O)-OCH₃, -NH-CH₂-C(O)-OC₂H₅, -NH-CH₂-C(O)-OC₃H₇, -NH-(CH₂)₂-C(O)-OH, -NH-(CH₂)₂-C(O)-OCH₃, -NH-(CH₂)₂-C(O)-OC₂H₅, -NH-(CH₂)₂-C(O)-OC₃H₇, - NH-(CH₂)₃-C(O)-OCH₃, -NH-(CH₂)₃-C(O)-OC₂H₅, -NH-(CH₂)₃-C(O)-OC₃H₇, -NH-(CH₂)₄-C(O)-OCH₃, -NH-(CH₂)₄-C(O)-OC₂H₅, -NH-(CH₂)₄-C(O)-OC₃H₇, -NH-(CH₂)ₙ-C(O)-NH₂, - NH-(CH₂)ₙ-C(O)-NH-(CH₂)ₙ-CH₃, -N[(CH₂)ₙ-C(O)-NH-(CH₂)ₙ-CH₃]₂, -NH-(CH₂)ₙ-OH, -NH-(CH₂)ₙ-F, -NH-(CH₂)ₙ-Cl, -NH-(CH₂)ₙ-CHO, -NH-(CH₂)ₙ-SH, -NH-(CH₂)ₙ-O-CH₃, -NH-(CH₂)ₙ-S-CH₃ or -NH-(CH₂)ₙ-CH₂-NH-CH₃, where each n independently is 0, 1, 2, 3 or 4.

C-Linked R⁴ moieties can optionally be selected from -C(O)-(CH₂)ₙ-CH₃, - C(O)-(CH₂)ₙ-CH₂OH, -C(O)-(CH₂)ₙ-CH₂C(O)OH, -CH₃, -C₂H₅, -C₃H₇, -C₄H₉, -C=CH₂, - C=CH, -C≡CF, -C≡CCl, -C≡CBr, -C≡Cl, -C≡COH, -C=CH₂, -C≡C-(CH₂)ₙ-CH₃, -(CH₂)ₘ-O-CH₃, -(CH₂)ₘ-S-CH₃, -(CH₂)ₘ-NH-CH₃, -(CH₂)ₘ-O-CH₂OH, -(CH₂)ₘ-S-CH₂OH, -(CH₂)ₘ-NH-CH₂OH, -(CH₂)ₘ-O-CH₂SH, -(CH₂)ₘ-S-CH₂SH, -(CH₂)ₘ-NH-CH₂SH, -(CH₂)ₘ-O-CH₂NH₂, -(CH₂)ₘ-S-CH₂NH₂ and -(CH₂)ₘ-NH-CH₂NH₂, where each n independently is 0, 1, 2, 3 or 4 and m is 1, 2, 3 or 4.

10. The method of embodiment 9 wherein the formula 1 compound is (1) 3β-hydroxy-17β-aminoandrost-5(10)-ene, 3α-hydroxy-17β-aminoandrost-5(10)-ene, 3β-hydroxy-3α-methyl-17β-aminoandrost-5(10)-ene, 3α-hydroxy-3β-methyl-17β-aminoandrost-5(10)-ene, 3β-hydroxy-3α-ethynyl-17β-aminoandrost-5(10)-ene, 3α-hydroxy-3β-ethynyl-17β-aminoandrost-5(10)-ene, 3-oxo-17β-aminoandrost-5(10)-ene, 3-thioxo-17β-aminoandrost-5(10)-ene, 3α-mercapto-17β-aminoandrost-5(10)-ene, 3α-mercapto-17β-aminoandrost-5(10)-ene, 3β-amino-17β-hydroxyandrost-5(10)-ene, 3α-amino-17β-hydroxyandrost-5(10)-ene, 3β-amino-17β-hydroxy-17α-ethynylandrost-5(10)-ene, 3α-amino-17β-hydroxy-17α-ethynylandrost-5(10)-ene, 3β,17β-dihydroxy-17α-ethynylandrost-5(10)-ene, 3α,17β-dihydroxy-17α-ethynylandrost-5(10)-ene, 3β,17α-dihydroxy-17β-ethynylandrost-5(10)-ene, 3α,17α-dihydroxy-17β-ethynylandrost-5(10)-ene, 3β-amino-17α-hydroxy-17β-ethynylandrost-5(10)-ene, 3α-amino-17α-hydroxy-17β-ethynylandrost-5(10)-ene, 3β-hydroxy-17β-aminoandrost-9-ene, 3α-hydroxy-17β-aminoandrost-9-ene, 3β-hydroxy-3α-methyl-17β-aminoandrost-9-ene, 3α-hydroxy-3β-methyl-17β-aminoandrost-9-ene or an analog of any of these compounds containing one, two or more of the following substitutions: 2-oxa, 2-thia, 2-aza, 4-oxa, 4-thia, 4-aza, 11-oxa, 11-thia, 11-aza, 2α-hydroxy, 2β-hydroxy, 2-oxo, 2-methylene (=CH₂), 2α-mercapto, 2β-mercapto, 2-thioxo, 2α-fluoro, 2β-fluoro, 2,2-difluoro, 4α-hydroxy, 4β-hydroxy, 4-oxo, 4α-mercapto, 4β-mercapto, 4-thioxo, 4α-fluoro, 4β-fluoro, 4,4-difluoro, 4-methylene, 6α-hydroxy, 6β-hydroxy, 6-oxo, 6α-mercapto, 6β-mercapto, 6-thioxo, 6α-fluoro, 6β-fluoro, 6,6-difluoro, 6-methylene, 7α-hydroxy, 7β-hydroxy, 7-oxo, 7-methylene, 9α-fluoro, 9β-fluoro, 9α-chloro, 9β-chloro, 14α-fluoro, 14β-fluoro, 14α-chloro, 14β-chloro, 16α-hydroxy, 16β-hydroxy, 16-oxo, 16α-mercapto, 16β-mercapto, 16-thioxo, 16α-fluoro, 16β-fluoro, a 3-4 double bond, a 15-16 double bond or a 16-17 double bond, or (2) one R¹ in the α-configuration is -H or a C-linked moiety such as optionally substituted alkyl moiety and the other R¹, or both R¹ together, is in the β-configuration and is an N-linked moiety such as -NH₂, -NHR^{PR}, -N(R^{PR})₂, -NO₂ -N₃, =NOH, =NO-optionally substituted alkyl, =N-optionally substituted alkyl, an N-linked amino acid, a substituted amine, a sulfamate having the structure -NH-S(O)(O)-S-O-optionally substituted alkyl, a carbamate having the structure -NH-C(O)-O-optionally substituted alkyl, a sulfurous diamide having thet structure -NH-S(O)-NH-optionally substituted alkyl, -NH-S(O)-NH₂ or -NH-S(O)-NHR^{PR}, a sulfamide having the structure -NH-S(O)(O)-NH₂, -NH-S(O)(O)-NHR^{PR}, -NH-S(O)(O)-NH-optionally substituted alkyl or-NH-S(O)(O)-N(optionally substituted alkyl)₂, a sulfinamide having the structure -NH-S(O)-optionally substituted alkyl or an amide having the structure -NH-C(O)-optionally substituted alkyl or a salt of any of these moieties, where each optionally substituted alkyl is independently selected and optionally is a C1-12 moiety or a C1-8 moiety, or (3) one R¹ in the β-configuration is -H or a C-linked moiety such as optionally substituted alkyl and the other R¹, or both R¹ together, is in the α-configuration and is an N-linked moiety such as -NH₂, -NHR^{PR}, - N(R^{PR})₂, -NO₂, -N₃, =NOH, =NO-optionally substituted alkyl, =N-optionally substituted alkyl, an N-linked amino acid, a substituted amine, a sulfamate having the structure -NH-S(O)(O)-S-O-optionally substituted alkyl, a carbamate having the structure -NH-C(O)-O-optionally substituted alkyl, a sulfurous diamide having thet structure -NH-S(O)-NH-optionally substituted alkyl, -NH-S(O)-NH₂ or -NH-S(O)-NHR^{PR}, a sulfamide having the structure -NH-S(O)(O)-NH₂, -NH-S(O)(O)-NHR^{PR}, -NH-S(O)(O)-NH-optionally substituted alkyl or -NH-S(O)(O)-N(optionally substituted alkyl)₂, a sulfinamide having the structure - NH-S(O)-optionally substituted alkyl or an amide having the structure -NH-C(O)-optionally substituted alkyl or a salt of any of these moieties, where each optionally substituted alkyl is independently selected and optionally is a C1-16 moiety, a C1-12 moiety or a C1-8 moiety, or (4) R¹ in the β-configuration is -H or a C-linked moiety such as optionally substituted alkyl and the other R¹, or both R¹ together, is in the α-configuration and is an O-linked moiety or a S-linked moiety such as -OH, -SH, =O, =S, - O-S(O)(O), -OR^{PR}, -SR^{PR}, -SCN, -O-optionally substituted alkyl, -S-optionally substituted alkyl, -O-C(O)-optionally substituted alkyl, -S-C(O)-optionally substituted alkyl, -O-C(S)-optionally substituted alkyl, a sulfonate such as -S(O)(O)-O-optionally substituted alkyl or -O-S(O)(O)-optionally substituted alkyl, a sulfate ester such as -O-S(O)(O)-O-optionally substituted alkyl, a sulfite ester such as -O-S(O)-O-optionally substituted alkyl, a sulfamate having the structure -O-S(O)(O)-NH₂, -O-S(O)(O)-NH-optionally substituted alkyl or -O-S(O)(O)-N-(optionally substituted alkyl)₂, an O-linked polymer, an S-linked polymer, an optionally substituted monosaccharide, an optionally substituted disaccharide, an optionally substituted oligosaccharide, a phosphate or thiophosphate such as -O-P(O)(OH)-OH, -O-P(O)(OH)-O-(CH₂)ₙ-CH₃, -O-P(O)[O(CH₂)ₙCH₃]-O-(CH₂)ₙ-CH₃, -O-P(O)(SH)-OH, -O-P(O)(SH)-O-(CH₂)ₙ-CH₃, -O-P(O)(OH)-S-(CH₂)ₙ-CH₃ or a salt of any of these moieties, or (5) R¹ in the α-configuration is -H or a C-linked moiety such as optionally substituted alkyl and the other R¹, or both R¹ together, is in the β-configuration and is an O-linked moiety or a S-linked moiety such as -OH, -SH, =O, =S, - O-S(O)(O), -OR^{PR}, -SR^{PR}, -SCN, -O-optionally substituted alkyl, -S-optionally substituted alkyl, -O-C(O)-optionally substituted alkyl, -S-C(O)-optionally substituted alkyl, -O-C(S)-optionally substituted alkyl, a sulfonate such as -S(O)(O)-O-optionally substituted alkyl or -O-S(O)(O)-optionally substituted alkyl, a sulfate ester such as -O-S(O)(O)-O-optionally substituted alkyl, a sulfite ester such as -O-S(O)-O-optionally substituted alkyl, a sulfamate having the structure -O-S(O)(O)-NH₂, -O-S(O)(O)-NH-optionally substituted alkyl or -O-S(O)(O)-N-(optionally substituted alkyl)₂, an O-linked polymer, an S-linked polymer, an optionally substituted monosaccharide, an optionally substituted disaccharide, an optionally substituted oligosaccharide, a phosphate or thiophosphate such as -O-P(O)(OH)-OH, -O-P(O)(OH)-O-(CH₂)ₙ-CH₃, -O-P(O)[O(CH₂)ₙCH₃]-O-(CH₂)ₙ-CH₃, -O-P(O)(SH)-OH, -O-P(O)(SH)-O-(CH₂)ₙ-CH₃, -O-P(O)(OH)-S-(CH₂)ₙ-CH₃ or a salt of any of these moieties, where for any of the moieties in (2), (3), (4) or (5) each optionally substituted alkyl is independently selected and optionally is a C1-16 moiety, a C1-12 moiety or a C1-8 moiety. N-Linked and C-linked R¹ moieties can optionally be selected from the moieties and genera of moieties described in embodiment 9 or elsewhere herein.

11. The method of embodiment 1, 2, 3, 4, 5, 6, 7, 8, 9 or 10 wherein the subject is a human having cystic fibrosis, optionally wherein one or more symptoms or syndromes associated with cystic fibrosis are ameliorated, or wherein the progression of the disease is detectably slowed or reduced.

12. The method of embodiment 11, wherein the one or more symptoms or syndromes are 1, 2, 3 or more of *Staphylococcus* (e.g., *S. aureus*), *Haemophilus influenzae, Pseudomonas* or *Burkholderia* respiratory tract or lung infection or propensity to develop a detectable infection or colonization, coughing, wheezing, cyanosis, bronchiolitis, bronchospasm, pneumothorax, hemoptysis, pancreatic exocrine insufficiency, bronchiectatic lung disease, atelectasis-consolidation, pulmonary edema, increased lung vascular hydrostatic pressure, increased lung vascular permeability, sinusitis, respiratory insufficiency, bronchial wall or interlobular septa thickening, reduction of forced expiratory volume in 1 second, dyspnea, impaired male fertility, elevated sweat chloride, mucous plugging, tree-in-bud sign, mosaic perfusion pattern, glucose intolerance or abnormal elevation of one or more of IL-4, IL-8, RANTES, neutrophil elastase, eosinophils, macrophages, neutrophils, eosinophil cationic protein or cysteinyl leukotrienes.

13. A method to treat or to reduce the severity of a chronic obstructive pulmonary disease, a respiratory distress syndrome, asthma, a chronic allergy or an atopic disease, or one or more symptoms of the chronic obstructive pulmonary disease, respiratory distress syndrome, asthma, chronic allergy or atopic disease in a subject, comprising administering an effective amount of a F1C, e.g., a F!C described herein such as at embodiment 1, 2, 3, 4, 5, 6, 7, 8, 9 or 10, optionally wherein one R¹ is, or both R¹ together are, -OH, -OR^{PR}, -SR^{PR}, -O-Si-(R¹³)₃, -COOH, -OSO₃H, -OPO₃H, =O, =S, an ester, a thioester, a thionoester, a phosphoester, a phosphothioester, a phosphonoester, a phosphiniester, a sulfite ester, a sulfate ester, an amide, an amino acid, a peptide, an ether, a thioether, a carbonate or a carbamate, and the other R¹ is independently chosen; and optionally wherein one R⁴ is, or both R⁴ together are, -OH, -OR^{PR}, -SR^{PR}, - N(R^{PR})₂, -O-Si-(R¹³)₃, -CHO, -CHS, -CN, -SCN, -NO₂, -NH₂, -COOH, -OSO₃H, -OPO₃H, =O, =S, =N-OH, =N-O-optionally substituted alkyl, an ester, a thioester, a thionoester, a phosphoester, a phosphothioester, a phosphonoester, a phosphiniester, a sulfite ester, a sulfate ester, an amide, an amino acid, a peptide, an ether, a thioether, an acyl group, a thioacyl group, a carbonate or a carbamate, and the other R⁴ is independently chosen.

14. The method of embodiment 13 wherein (1) the level or activity of IgE in the subject is at least transiently detectably reduced and/or (2) the subject is a human who has a sickle cell disease and/or the treatment reduces (a) the severity of pain during vascular or microvascular occlusions, (b) the severity of vascular or microvascular occlusions or (c) the frquency of vascular or microvascular occlusions, and optionally wherein the formula 1 compound is administered by an intermittent administration or dosing protocol.

15. The method of embodiment 13 or 14 wherein one R¹ is, or both R¹ together are, -H, -OH, -OR^{PR}, -SR^{PR}, -O-Si-(R¹³)₃, -COOH, -OSO₃H, -OPO₃H, =O, =S, an ester, a thioester, a thionoester, a phosphoester, a phosphothioester, a phosphonoester, a phosphiniester, a sulfite ester, a sulfate ester, an amide, an amino acid, a peptide, an ether, a thioether, a carbonate or a carbamate, and the other R¹ is independently chosen; and one R⁴ is, or both R⁴ together are, -OH, -OR^{PR}, -SR^{PR}, -N(R^{PR})₂, -O-Si-(R¹³)₃, -CHO, -CHS, -CN, -SCN, -NO₂, -NH₂, -COOH, -OSO₃H, -OPO₃H, =O, =S, =N-OH, =N-O-optionally substituted alkyl, an ester, a thioester, a thionoester, a phosphoester, a phosphothioester, a phosphonoester, a phosphiniester, a sulfite ester, a sulfate ester, an amide, an amino acid, a peptide, an ether, a thioether, an acyl group, a thioacyl group, a carbonate or a carbamate, and the other R⁴ is independently chosen.

18. A method to treat a cardiovascular condition, an autoimmune condition, a trauma, an unwanted inflammation condition or an unwanted immune response to an allograft or rejection of an allogeneic tissue, organ or cell population comprising administering to a subject having or who may be expected to develope the cardiovascular condition, autoimmune condition, unwanted inflammation condition or the unwanted immune response to an allograft or acute or chronic rejection of an allogeneic tissue, organ or cell population an effective amount of a F1C disclosed herein, e.g., an F1 C in embodiment 1, 2, 3, 4, 5, 6, 7, 8, 9 or 10 or the F1 C is a compound or genus of compounds in group 1 through group 57.

19. The method of embodiment 18 wherein (1) the cardiovascular condition is arteriosclerosis, atherosclerosis, hypercholesterolemia, hypertriglyceridemia or a hypertension condition such as pulmonary hypertension, (2) the autoimmune condition or unwanted inflammation is a lupus condition such as systemic lupus erythematosus, rheumatoid arthritis, osteoarthritis or Crohn's disease, inflammatory bowel disease, a scleroderma condition or a vasiculitis such as a giant cell arteritis, polyarteritis nodosa or Kawasaki's disease, and/or (3) the trauma is a bone fracture, a chemical or thermal burn, a hemorrhage, an infarction such as a cerebral infarction or tissue or organ impairment or damage associated with a wound, chemotherapy, toxin or radiation exposure.

20. The method of embodiment 19 wherein the trauma is a skin, central nervous system tissue, blood vessel, heart tissue, lung, liver, pancreas, kidney, thymus, spleen, oral mucosa, intestine, bone marrow, or connective tissue wound, laceration, lesion or trauma.

21. A pharmaceutical formulation comprising one or more excipients and a F1C disclosed herein, e.g., an F1C in embodiment 1, 2, 3, 4, 5, 6, 7, 8, 9 or 10 or the F1C is a compound or genus of compounds in group 1 through group 57.

22. A F1C disclosed herein, e.g., an F1C in embodiment 1, 2, 3, 4, 5, 6, 7, 8, 9 or 10 or a compound or genus of compounds in group 1 through group 57.

23. Use of a F1 C for the preparation of a medicament or for the preparation of a medicament for the treatment of a disease, condition or symptom described herein. The medicament can be for the prophylaxis or treatment of a disease, condition or symptom disclosed herein in a subject having or susceptible to developing the disease, condition or symptom.

24. The method, formulation or use of embodiment 1, 2, 3, 4, 5, 6, 7, 8, 9, 10, 11, 12, 13, 14, 15, 16, 17, 18, 19, 20, 21, 22 or 23 wherein the F1C or the genus of F1Cs has the structure

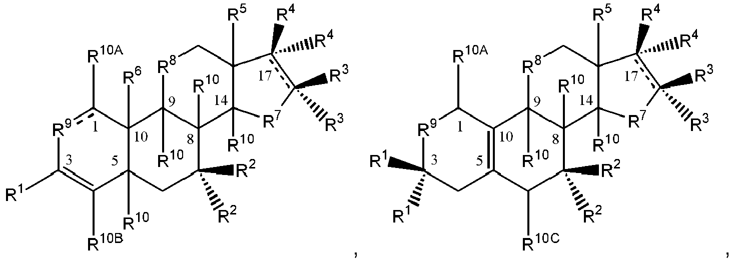

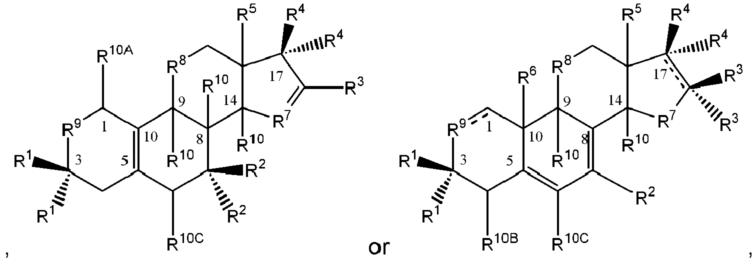

or a metabolic precursor or a metabolite thereof, optionally wherein R^{10A}, R^{10B}, R^{10C}, R^{10D} and R^{10E}, when present are in the α- or β-configuration, unless shown otherwise; R¹⁰ moieties (if present) at the 5, 8, 9 and 14 positions respectively are in the α,α,α,α, α,α,α,β, α,α,β,α, α,β,α,α, β,α,α,α, α,α,β,β, α,β,α,β, β,α,α,β, β,α,β,α, β,β,α,α, α,β,β,α, α,β,β,β, β,α,β,β, β,β,α,β, β,β,β,α or β,β,β,β configurations; wherein R^{10A}, R^{10B}, R^{10C}, R^{10D} and R^{10E} respectively are in the α,α, α,β, β,α or β,β configurations;

25. The method, formulation or use of embodiment 24 wherein, each R¹, R², R³, R⁴, R⁵, R⁶, R¹⁰, R^{10A}, R^{10B}, R^{10C}, R^{10D} and R^{10E} independently or together are -H, - OH, -OR^{PR}, -SR^{PR}, -SH, -N(R^{PR})₂, -NHR^{PR}, -NH₂, -O-Si-(R¹³)₃, -CHO, -CHS, -CN, -SCN, - NO₂, -COOH, -COOR^{PR}, -OSO₃H, -OPO₃H₂, =O, =S, =N-OH, =N-OCH₃, =CH₂, =CH-CH₃, ester, thioester, thionoester, phosphoester, phosphothioester, phosphonate, phosphonate ester, thiophosphonate, thiophosphonate ester, phosphiniester, sulfite ester, sulfate ester, sulfamate, sulfonate, sulfonamide, amide, amino acid, peptide, ether, thioether, acyl, thioacyl, carbonate, carbamate, halogen, optionally substituted alkyl, optionally substituted alkenyl, optionally substituted alkynyl, optionally substituted aryl, optionally substituted heteroaryl, optionally substituted heterocycle, optionally substituted monosaccharide, optionally substituted oligosaccharide, polymer, spiro ring, epoxide, acetal, thioacetal, ketal or a thioketal, =N-O-optionally substituted alkyl, =N-optionally substituted alkyl, -NH-optionally substituted alkyl, -N(optionally substituted alkyl)₂ where each optionally substituted alkyl is independently selected, or, one or more of two adjacent R¹, R², R³, R⁴, R⁵, R⁶ and R¹⁰ comprise an independently selected epoxide or optionally substituted cyclopropyl, cyclobutyl, cyclopentyl or cyclohexyl ring; R⁷ is -C(R¹⁰)₂-, -C(R¹⁰)₂-C(R¹⁰)₂-, -C(R¹⁰)₂-C(R¹⁰)₂-C(R¹⁰)₂-, -C(R¹⁰)₂-O-C(R¹⁰)₂-, -C(R¹⁰)₂-S-C(R¹⁰)₂-, -C(R¹⁰)₂-NR^{PR}-C(R¹⁰)₂-, -O-, -O-C(R¹⁰)₂-, -S-, -S-C(R¹⁰)₂-, -NR^{PR}- or -NR^{PR}-C(R¹⁰)₂-, where each R¹⁰ is independently selected; R⁸ and R⁹ independently are - C(R¹⁰)₂-, -C(R¹⁰)₂-C(R¹⁰)₂-, -O-, -O-C(R¹⁰)₂-, -S-, -S-C(R¹⁰)₂-, -NR^{PR}- or -NR^{PR}-C(R¹⁰)₂-, or one or both of R⁸ or R⁹ independently are absent, leaving a 5-membered ring, where each R¹⁰ is independently selected; R¹³ independently is C₁₋₆ alkyl; and R^{PR} independently orf together are -H or a protecting group, optionally provided that (1) one or two of R^{10A} R^{10B}, R^{10C}, R^{10D} and R^{10E} are not hydrogen or (2) one R⁴ is -NH₂, an opotionally substituted amine, -N(R^{PR})², =NOH, =NO-optionally substituted alkyl, =N-optionally substituted alkyl, an amide or an N-linked amino acid. In these embodiments, the subject may have or be subject to developing the listed condition and the subject can be a human or a primate.

26. The method of embodiment 24 or 25 wherein one each of R¹, R², R³ and R⁴ are -H, and, when no double bond links the second R¹, R², R³ and R⁴ to the ring to which it is bonded and no double bond is present at the 16-17 position, then the second R¹, R², R³ and R⁴ respectively are in the α,α,α,α, α,α,α,β, α,α,β,α, α,β,α,α, β,α,α,α, α,α,β,β, α,β,α,β, β,α,α,β, β,α,β,α, β,β,α,α, α,β,β,α, α,β,β,β, β,α,β,β, β,β,α,β, β,β,β,α o r β,β,β,β configurations and the second R¹, R², R³ and R⁴ are optionally independently selected from -H, -F, -Cl, -Br, -I, -OH, -SH, -NH₂, -COOH, -CH₃, -C₂H₅, -C(CH₃)₃, -OCH₃, -OC₂H₅, -CF₃, -CH₂OH, -C(O)CH₃, -C(O)CH₂OH, -C(O)CH₂F, - C(O)CH₂Cl, -C(O)CH₂Br, -C(O)CH₂I, -C(O)CF₃, -C₂F₅, =O =CH₂, =CHCH₃, amino acid, carbamate, carbonate, optionally substituted C1-C20 alkyl, optionally substituted C1-C20 ether, optionally substituted C1-C20 ester, optionally substituted C1-C20 thioether, optionally substituted C1-C20 thioester, optionally substituted monosaccharide, optionally substituted disaccharide, optionally substituted oligosaccharide.

52. A method to treat a symptom or condition associated with one or more delayed adverse or unwanted effects of radiation exposure or therapy in a subject in need thereof comprising administering to the subject, or delivering to the subject's tissues, an effective amount of a compound of formula 1, wherein the F1C is administered or delivered to the subject's tissues beginning at least 2 weeks after the subject has been exposed to a dose of radiation that will cause or could potentially cause the one or more delayed adverse or unwanted effects of the radiation exposure, or wherein the F1C is administered or delivered to the subject's tissues beginning at least 2 weeks after the subject has been exposed to at least one subdose of a planned course of radiation exposures that will cause or could potentially cause the one or more delayed adverse effects or unwanted effects of the radiation exposure.

53. The method of embodiment 52 wherein the subject has received, or is anticipated to receive, a total radiation dose of at least about 0.5 Gy to about 300 Gy, wherein the subject received the radiation dose in a single dose or in two or more divided doses, e.g., a total radiation dose of at least about Gy 1 to about 12 Gy, or at least about Gy 1 to about 8 Gy.

54. The method of embodiment 52 or 53 wherein the symptom or condition associated with one or more delayed adverse effect of radiation is one or more of encephalopathy, myelopathy, nausea, vomiting, diarrhea, acute inflammation, e.g., of the lung, chronic inflammation, e.g., of the lung, edema, pain, headache, depression, fever, malaise, weakness, hair loss, skin atrophy, skin ulceration, skin lesion, mucositis, a gastrointestinal lesion or ulcer, keratosis, telangiectasia, infection, hypoplasia, atrophy, fibrosis, pneumonitis, bone marrow hypoplasia, hemorrhage, anemia, leucopenia, thrombocytopenia, fever, pain, radiation-induced enteritis or diarrhea, pseudomembranous inflammation, perivascular fibrosis, endothelial cell damage or death, cardiac tissue inflammation or damage or pericardial disease, pulmonary tissue inflammation or damage, hematopoietic or marrow cell inflammation or damage, endocrine or thyroid dysfunction, decreased growth or decreased bone development or density, central nervous system inflammation or damage, connective tissue damage, gastric ulceration or small bowel obstruction or fistula formation.

55. The method of embodiment 52, 53 or 54 wherein the symptom or condition associated with one or more delayed adverse or unwanted effect of the radiation exposure or therapy is caused by or associated with radiation damage to one or more of bone marrow cells, bowel epithelium, bone marrow, testicles, ovaries, brain nerves or tissue, peripheral nerves, spinal cord nerves or tissue or skin epithelium.

56. A method to prevent, treat, ameliorate or slow the progression or development of anemia, neutropenia or thrombocytpoenia in a subject, or to treat a symptom of the anemia, neutropenia or thrombocytopenia, comprising administering to a subject, or delivering to the subject's tissues, an effective amount of a formula 1 compound, e.g., any F1C or genus of F1Cs described herein.

57. The method of embodiment 55 or 56 wherein the subject is a human and wherein the neutropenia is postinfectious neutropenia, autoimmune neutropenia, chronic idiopathic neutropenia or a neutropenia resulting from or potentially resulting result from a cancer chemotherapy, chemotherapy for an autoimmune disease, an antiviral therapy, radiation exposure, tissue or solid organ allograft or xenograft rejection or immune suppression therapy in tissue or solid organ transplantation or aging or immunesenescence.

58. A method to prevent, treat or ameliorate an immune suppression condition or an unwanted inflammation or autoimmune condition in a subject in need thereof comprising administering to the subject, or delivering to the subject's tissues, an effective amount of an F1C, optionally wherein (1) the subject is a human or another primate and/or (2) the immune suppression condition is an innate immune suppression condition or immunosenesence and/or (3) the unwanted inflammation or autoimmune condition is rheumatoid arthritis, osteoarthritis, psoriatic arthritis, polyarthritis, osteoporosis, an allergy, multiple sclerosis, dermatitis, autoimmune glomerulonephritis, systemic lupus erythematosus, autoimmune pulmonary inflammation, asthma, ischemia-reperfusion injury, inflammatory bowel disease, regional enteritis, ulcerative colitis or Crohn's disease and/or (4) the compound is 3β-hydroxy-17β-aminoandrost-5(10)-ene, 3β-hydroxy-3α-methyl-17β-aminoandrost-5(10)-ene, 3α-hydroxy-17β-aminoandrost-5(10)-ene, 3α-hydroxy-3β-methyl-17β-aminoandrost-5(10)-ene or a 2-oxa, 2-aza, 11-oxa, 11-aza, 9-halogen, 16-halogen, 16-hydroxyl, 16-oxo, or 19-nor analog of any of these compounds.

Variations and modifications of these embodiments, the claims and the remaining portions of this disclosure will be apparent to the skilled artisan after a reading thereof. Such variations and modifications are within the scope and spirit of this invention. All citations herein are incorporated herein by reference in their entirety. All citations herein are incorporated herein by reference with specificity.

Examples. The following examples further illustrate the invention and they are not intended to limit it in any way. Variations of these examples that are included in the invention may include, e.g., any of the F1Cs described herein or parts or all of any of the methods, formulations, treatment protocols and/or assays described herein.

**Example 1.** Treatment of cytopenia. Primates treated to induce neutropenia or thrombocytopenia are used to characterize their response to treatment with a F1C. Mitigation of cytopenia, e.g., neutropenia or thrombocytopenia, by the F1C is observed. In an exemplary protocol, Cynomolgus monkeys of about 3.5-8 years of age and weighing about 2.5 to 8 kg are dosed at 35 mg/kg with carboplatin (sterile 10 mg/mL solution in 0.9% sodium chloride) by intravenous infusion over 30 minutes. Beginning at 1 or 2 days after the carboplatin infusion, each animal is dosed once daily or once every other day with the F1 C for 1, 2, 3, 4, 5, 6, 7, 8, 9 or 10 days, e.g., once per day for 5 consecutive days beginning 2 days after carboplatin infusion. The F1C is in a suitable sterile solution or suspension formulation in suitable excipients, e.g., a suspension containing 0.1 % w/v carboxymethyl-cellulose, 0.9% w/v sodium chloride and 0.05% v/v phenol. The suspension contains micronized F1C. Control animals receive the formulation without any F1C. The animals are dosed with the F1C subcutaneously in the interscapular region of the back or intramuscularly in the thigh at a dosage of about 1-45 mg/kg, e.g., 1.25, 2.5, 7.65 or 42.5 mg/kg of the F1C. Blood samples of about 1-1.5 mL are withdrawn at various times, e.g., on days -5 (pre-carboplatin), -2 (pre-carboplatin), 1 (4 hr post-dosing), 3, 7, 10, 12, 14, 16, 18, 20, 22, 24, 26, 28, 32 and 36 days, for analysis such as neutrophil, platelet, reticulocyte, erythrocyte counts. The degree of reduced time and/or severity of cytopenia such as anemia, thrombocytopenia or neutropenia is then observed in F1C treated and control animals.

**Example 2.** Cystic fibrosis treatment. Treatment with a F1C is conducted on human cystic fibrosis ("CF") patients, e.g., 18 years of age or older, who may have two or more of the following characteristics: (1) sweat chloride ≥ 60 mEq/L, e.g., by quantitative pilocarpine iontophoresis test, (2) homozygosity for the F508 genetic mutation, or heterozygosity for 2 well-characterized mutations, e.g., as described herein, associated with CF, (3) FEV₁ ≥ 40% predicted at screening, (4) SaO₂ ≥ 90% at screening, (5) ability to perform pulmonary function tests and (6) clinical stability, with no evidence of acute upper or lower respiratory tract infection or current pulmonary exacerbation. The treatment regimen consists of 1, 2, 3, 4 or 5 consecutive days of once daily dosing of a F1 C or placebo equivalent followed by a 40-day observation period. Daily dosages are about 10-150 mg/day, e.g., about 25 mg/day, 50 mg/day, 75 mg/day or 100 mg/day. The F1C is administered as described herein such as by a parenteral route, e.g., intramuscular or subcutaneous delivery, or by transmucosal delivery, e.g., buccal or sublingual. A follow-up visit will occur 6 weeks after the last treatment course to collect final samples for safety and activity. Patients receive, e.g., 3 treatment courses over a 14-week period, 6 treatment courses over a 28-week period or more courses of treatment over a longer period. The patients are optionally monitored for the status of their condition or in improvement of one or more CF symptoms after dosing, e.g., reduction in the numbers of neutrophils in bronchiolar or alveolar lavage samples, e.g., about a 30%, 40%, 50% or greater reduction, or levels of one or more CF-related inflammation markers, e.g., reduced levels or activity of IL-6, IL-8, IKK-β kinase or neutrophil elastase, or in the reduced occurrence, severity or progression of infections such as a *Burkholderia (Pseudomonas) cepacia* infection.

**Example 3.** Human and primate virus treatment protocol. Humans infected with a virus, e.g., HCV, RSV, HBV or a retrovirus such as HIV1 or HIV2 or primates infected with a virus such as HCV, HIV1, HIV2, SIV or SHIV₂₂₉ are treated with a F1 C formulation. Daily dosages of about 0.05 to about 25 mg/kg are administered daily or on an intermittent basis. The F1C is administered, e.g., orally, by subcutaneous injection, by intramuscular injection or by transmucosal delivery. A typical intermittent dosing protocol for human patients comprises daily dosing of about 0.1-5 mg/kg of the F1 C for 1, 2, 3, 4, 5 or 6 days, followed by no dosing for about 1, 2, 3, 4, 5, 6, 7 or 8 weeks, daily dosing again for 1, 2, 3, 4, 5 or 6 days, no dosing for about 1, 2, 3, 4, 5, 6, 7 or 8 weeks and optionally repeating this dosing schedule as desired, e.g., for 3, 4, 5, 10, 15 or more rounds of dosing. A related dosing protocol involves dosing on every 2^{nd} or 3^{rd} day to deliver 2, 3, 4, 5 or 6 doses of the F1 C, no dosing for about 2, 3, 4, 5, 6, 7 or 8 weeks and optionally repeating this dosing schedule as desired, e.g., for 3, 4, 5, 10, 15 or more rounds of dosing. Typical daily F1 C doses in human treatment protocols is about 5 mg to about 1000 mg, usually about 10-150 mg. Daily doses can vary depending on the route of F1C administration and on the patient's weight and clinical condition, with oral administration usually requiring higher daily doses than parenteral or transmucosal administration.

In treating a viral infection such as a human HIV1 or HIV2 infection, one can optionally monitor one or more aspects the patient's response, e.g., periodic assay for viral load or for the level or activity of a given immune cell type or a biomolecule described herein such as CD4⁺ T cells, CD123⁺ dendritic cells, IL-6, IL-10 or TNFα. Changes in cell types, viral load or biomolecules can be transient, e.g., detectably changed over a period of about 2-48 hours, or sustained, e.g., detectably changed for about 3-6 days or about 1-8 weeks. Other aspects of the patient's response may also be monitored such as the incidence, severity or rate of progression of symptoms or associated conditions such as coinfection, fatigue, weight loss or side effects of other suitable therapies. In retrovirus-infected patients that are treated with the F1C, the rate or progression of a clinically significant coinfection by *Mycobacteria* or *Pneumocystis* is generally reduced, including for patients with a CD4+ T cell count of less than about 100 cells/mm³ or less than about 75 cells/mm³.

**Example 4.** Stimulation of phagocytosis. The capacity of F1Cs to influence phagocytosis of *Plasmodium* parasite-infected RBC is examined using adherent human monocytes. The parasitemia level is about 8-10% and human monocytes are obtained from buffy coats from blood as follows. Peripheral blood mononuclear cells are separated from freshly collected platelet-poor buffy coats discarded from blood samples of healthy adult donors of both sexes. Separated cells are washed once with luke-warm PBS supplemented with 10 mM glucose (PBS-G) and resuspended at 5 x 10⁶ cells/mL in ice-cold RPMI 1640 medium supplemented with 23 mM NaHCO₃ and 25 mM Hepes, pH 7.4 (RMBH). Dynabeads M450 Pan B and Pan T (Dynal) are added to cells in a 4:1 ratio for 20 min at 4°C. B-lymphocytes and T-lymphocytes are removed as specified by the manufacturer. The remaining monocytes are washed 2 times in RMBH, resuspended in AIM V cell culture medium (Gibco) at 1 x 10⁶ cell/mL. The monocyte layer is collected, washed with PBS-G at 37°C and resuspended in AIM V medium at 1 x 10⁶ cells/mL. Purified cells are >90% monocytes as assessed by CD14 expression.

Phagocytosis of opsonized parasitized RBC (PE) is determined as follows. Phagocytosis of fresh-serum opsonized PE is initiated by mixing 10 PE/monocyte. Suspensions are briefly centrifuged (150 x g for 5 sec at room temperature) to improve contact between PE and monocytes. To avoid attachment of monocytes after centrifugation and during the whole incubation period, cells are kept in suspension at 5 x 10⁶ cells/5 mL AIM V medium in 6 cm diameter Teflon bottom dishes (Heraeus) in a humidified incubator (95% air, 5% CO₂) at 37°C. On average, at least 90% of the monocytes phagocytose PE, as assessed by microscopic inspection. Control cells are kept under similar conditions without phagocytosis. Quantitative assessment of phagocytosis is performed by a previously described bioluminescence method (E. Schwarzer, et al., Br. J. Haematol. 1994 88: 740-745).

Erythrocyte treatments and parasite cultures are as follows. Fresh blood (Rh+) is used to isolate erythrocytes (RBC). Washed RBC are infected with schizont/trophozoite parasite stages (Palo Alto strain, mycoplasma-free). Stage specific parasites are isolated by the Percoll-mannitol method. Briefly, normal schizont-stage parasitized RBC (SPE) separated on Percoll-mannitol gradient (parasitemia > 95% SPE) are mixed with RBC suspended in growth medium (RPMI 1640 medium containing 25 mmol/L Hepes, 20 mmol/L glucose, 2 mmol/L glutamine, 24 mmol/L NaHCO₃, 32 mg/L gentamicin and 10% AB or A human serum, pH 7.30) to start synchronous cultures at selected hematocrit values. The inoculum parasitemia is adjusted to 20% normal SPE for isolation of ring parasitized RBC (RPE) and to 5% normal SPE for isolation of trophozoite-stage parasitized RBC (TPE). At 14-18 hours after inoculum parasites are at ring-stage in the first cycle; at 34-33 hours, parasites are at trophozoite-stage in the first cycle; and at 40-44 hours after inoculum parasites are at schizont-stage in the first cycle. RPE, TPE and SPE are separated on Percoll-mannitol gradients. The parasitemia is usually 8-10% RPE, and >95% TPE. Nonparasitized and parasitized RBC are counted electronically. To assess total parasitemia and relative contribution of RPE, TPE and SPE, slides are prepared from cultures at indicated times, stained with Diff-Quik^{™} parasite stain and about 400-1000 cells are examined microscopically.

The effect of a formula 1 compound such as F1C in parasitized RBC is examined using various concentrations of the compound, e.g., F1C, e.g., 0.001 µM, 0.01 µM, 0.05 µM, 0.1 µM, 0.5 µM, 1 µM, 10 µM, 25 µM and 50 µM. Trophozoite-parasitized RBC, schizont-parasitized RBC or ring-parasitized RBC are examined as described.

**Example 5.** Cyclodextrin formulation. A cyclodextrin formulation containing a F1C is prepared by mixing a sulfobutyl β-cyclodextrin and the F1C in one or more liquids such as ethanol, DMSO, N-methylpyrrolidine, pyridine or water. The sulfobutyl β-cyclodextrin contains one or more butyl sulfonate or -O-(CH₂)₄-SO₃⁻Na⁺ moieties, typically about 5, 6 or 7 per cyclodextrin molecule. F1 Cs that contain a positive charge are especially helpful in forming complexes with the multiple negative charges of sulfobutyl cyclodextrin. For parenteral formulations, the maximum concentrations could be achieved at about the maximum cyclodextrin concentration that is still syringable, about 50% w:v. The F1 C can be added to a solution of sulfobutyl β-cyclodextrin at a molar ratio of about 1:1, e.g., 0.5:1 to about 2:1, and stirred with or without heat for up to about 1 week to form the complex. The solution is optionally filtered or sterilized before filling in vials or injection delivery by any route. The vials can be sterilized by radiation or by sterilie filtration. An exemplary preparation is made using 500 grams of sulfobutyl β-cyclodextrin (about 230 mmoles) combined with about 230 mmoles of the F1C. Solutions containing about 20-80 mg/mL of the F1C are typically obtained. For pharmaceutical formulations, the complex is prepared under GMP compliance conditions. The dried complex is prepared by lyophilization and can be reconstituted, e.g., using sterile 0.9% NaCl. The cyclodextrin complex can also be dried for preparation of formulations for oral or transmucosal administration or reconstituted with water for parenteral delivery, e.g., by subcutaneous or intramuscular injection.

**Example 6.** Inhibition of inflammation. The capacity of formula 1 compounds to limit or inhibit inflammation or symptoms of inflammation is shown using animal models for inflammatory bowel disease. In an exemplary protocol, groups of 3 male Wistar rats (180 ± 20 grams) fasted for 24 hours before 2,4-dinitrobenzene sulfonic acid (DNBS) or saline challenge are used. Distal colitis is induced by intra-colonic instillation of 0.5 mL of an ethanolic solution of DNBS (30 mg in 0.5 mL of a 30% ethanol in saline solution) after which 2 mL of air is injected through the cannula to ensure that the solution remained in the colon. The volume used is 0.1 mL per injection of 2 and 20 mg/mL of a F1C in a liquid formulation, which is administered by subcutaneous injection once a day for 6 days. The formulation contained 100 mg/mL of the F1C in a non-aqueous suspension, e.g., 2% benzyl alcohol w/v, 0.1 % Brij 96 w/v and equal volumes of PEG 300 and propylene glycol. Concentrations of 2 mg/mL and 20 mg/mL are obtained by diluting the 20 mg/mL formulation with vehicle that lacked the F1C.

The first F1C dose is given 30 minutes after DNBS challenge. Sulfasalazine (30 mg/mL in 2% Tween 80 in distilled water) is administered orally (PO) once a day (10 mUkg/day) for 7 days, the first two doses beginning 24 hours and 2 hours before DNBS challenge. The presence of diarrhea is recorded daily by examining the anal area. Animals are fasted for 24 hours prior to being sacrificed. Animals are sacrificed on day 7 or day 8 and their colons are removed and weighed. Before removal of the colon, signs of adhesion between the colon and other organs are recorded. Also, the presence of ulcerations is noted after weighing of each colon. The "net" change of colon-to-body weight (BW) ratio is normalized relative to saline-challenged baseline group. A 30% decrease in "net" colon-to-body weight ratio is considered significant.

**Example 7.** Modulation of delayed type hypersensitivity. The capacity of F1Cs to modulate delayed type hypersensitivity (DTH) is determined in mice. Groups of five female BALB/cByJ mice (20-25 grams) are anesthetized and 100 µL of a 3% solution of oxazolone is applied on day 0 to the shaved abdomen and dried. Seven days later, on day 7, the mice are challenged by applying 5 µL of oxazolone topically to each side of the right ear. The F1C (at about 20-100 mg/mL) in vehicle is administered by subcutaneous injection (2 mg/day) one time on day 6, 24 hours before the oxazolone challenge. The vehicle as a non-aqueous suspension of the F1C in, e.g., 2% benzyl alcohol w/v, 0.1 % Brij 96 w/v and equal volumes of PEG 300 and propylene glycol.

Dexamethasone in saline (0.2 mg/mL) is administered daily for 9 days (day -1 to 7), first dose 24 hours before sensitization, last dose at challenge by subcutaneous injection (0.01 mg/dose, 50 µL/injection). On Day 8, 24 hours following the oxazolone challenge, both the right and left ear thicknesses are measured using a micrometer caliper and the differences are determined. The differential ear thickness is measured as an indicator of the DTH response to topical oxazolone challenge. The DTH response is expressed as the difference in the thickness (mm) between the right and the left ears for each animal.

The differential ear thickness in animals receiving vehicle alone is 0.225 mm and treatment with dexamethasone (high dose) or cyclophosphamide reduced the DTH response (0.144 mm and 0.092 mm, respectively).

**Example 8.** Reversal of immunosenescence. Healthy aged (20-month) or immunologically-mature (3-month) BALB/c mice are vaccinated with hepatitis B surface antigen (HbsAg) (2 µg; Recombivax-HB; Merck) and Alum (2.75 µg). The aged mice are vaccinated with the antigen and also received a single subcutaneous injection of either 0.3 mg or 3.0 mg of selected F1 Cs or the vehicle (placebo control).

Blood samples are collected 14, 21 and 34 days after treatment and the sera are analyzed by ELISA to determine the concentration of HbsAg-specific IgG (total IgG). In addition, samples obtained on day 21 are analyzed to determine the concentration of HbsAg-specific IgG1 and IgG2a subclasses. The results can be summarized as average values obtained with blood samples collected 21 days after vaccination of groups of 8 mice. Subcutaneous injection is performed after shaving the hair from the thighs of each mouse. The injected volume is 50 µL containing 3.0 mg or 0.3 mg of compound or placebo, and for vaccine preparation. The vehicle control consists of carboxymethyl-cellulose (0.5%) in saline (0.9%). Antibody titers are determined by ELISA.

Treatment of aged, vaccinated animals with the F1Cs, can result in higher anti-HbsAg IgG titers than aged animals receiving the vaccination only. Such results would show that the F1Cs can enhance immune response to antigen challenge in immune senescent animals.

The serum samples are also analyzed for the titers of HbsAg-specific, IgG1 or IgG2a immunoglobulin subclasses. A bias to IgG1 is seen in aged mice and this is considered symptomatic of immune senescence or a suboptimal immune response associated with immune senescence. The IgG1/IgG2a ratio is an indicator of immune status. Th2 cells predominantly assist in the generation of humoral immunity, while Th1 cells enhance, e.g., cellular immunity. Humoral immunity (Th2) becomes predominant with age, while the decreasing cellular (Th1) immunity leads to increased susceptibility to, e.g., infectious diseases.

To examine the secondary antibody response, 42 days after the initial exposure to HbsAg, serum samples are taken from the mice and these are tested for anti-HbsAg IgG. At this time-point, vaccine-specific IgG titers are either low or undetectable. Three days later (45 days after first vaccination), the mice are injected again with HbsAg in alum, but this time, none of the mice receive any F1C (secondary vaccination). Serum samples collected 7 days and 14 days after the second exposure to HbsAg vaccine are assayed for anti-HbsAg antibody. In the young mice, a marked increase in specific antibody is seen in response to the second vaccination. In aged mice that had receive no F1C with the first HbsAg injection, levels of anti-HbsAg are measured. The data is analyzed for increases in anti-HbsAg titers following secondary vaccination in aged animals that had been treated with a F1C in conjunction with the first HbsAg exposure.

**Example 9.** Supression of TNF-α induced adhesion molecule expression. The recruitment of lymphocytes to areas of inflammation and angiogenesis involves specific receptor-ligand interactions between cell surface adhesion molecules (CAMs) on lymphocytes and the vascular endothelium. The adhesion process, in both normal and pathological settings, follows a multi-step cascade that involves intercellular adhesion molecule-1 (ICAM-1), vascular cell adhesion molecule-1 (VCAM-1), and endothelial leukocyte adhesion molecule-1 (E-selectin) expression on endothelial cells (EC). The expression of these molecules and others on the vascular endothelium determines the efficiency with which leukocytes may adhere to the local vasculature and extravasate into the local tissue during the development of an inflammatory response. The local concentration of cytokines and growth factor participate in the modulation of the expression of these CAMS.

Tumor necrosis factor alpha (TNF-α), is a proinflammatory cytokine and stimulates all three CAMs on endothelial cells. It may be involved in a wide variety of inflammatory responses, often resulting in a pathological outcome. The capacity of a formula 1 compound to mediate a suppression of TNF-α induced CAM expression can be examined. A modified ELISA assay which uses Ecs as a solid phase absorbent is employed to measure the amount of CAM expression on TNF-a treated Ecs when co-stimulated with a member of the FGF family of proteins. To perform the experiment, human umbilical vein endothelial cell (HUVEC) cultures are obtained from pooled cord harvests and maintained in growth medium (EGM-2, Clonetics, San Diego, CA) supplemented with 10% FCS and 1% penicillin/streptomycin in a 37°C humidified incubator containing 5% CO₂. HUVECs are seeded in 96-well plates at concentrations of about 1 x 10⁴ cells/well in EGM medium at 37°C for 18-24 hrs or until confluent. The monolayers are subsequently washed 3 times with a serum-free solution of RPMI-1640 optionally supplemented with 100 U/mL penicillin and 100 mg/mL streptomycin, and treated with a given cytokine and/or growth 5 factor(s) for 24 h at 37°C. Following incubation, the cells are then evaluated for CAM expression.

HUVECs are grown in a standard 96 well plate to confluence. Growth medium is removed from the cells and replaced with 90 µL of 199 Medium (10% FBS). Samples for testing and positive or negative controls are added to the plate in triplicate (in 10 µL volumes). Plates are incubated at 37°C for either 5 h (selectin and integrin expression) or 24 h (integrin expression). Plates are aspirated to remove medium and 100 pL of 0.1 % paraformaldehyde-PBS (with Ca⁺⁺ and Mg⁺⁺) is added to each well. Plates are held at 4°C for 30 min. Fixative is then removed from the wells and wells are washed 1X with PBS(+Ca,Mg)+0.5% BSA and drained. Do not allow the wells to dry. 10 pL of diluted primary antibody is added to the test and control wells. Anti-ICAM-1-Biotin, Anti-VCAM1-Biotin and Anti-E-selectin-Biotin are used at a concentration of 10 pg/ml (1:10 dilution of 0.1 mg/ml stock antibody). Cells are incubated at 37°C for 30 min. in a humidified environment. Wells are washed X3 with PBS (with Ca, Mg) and 0.5% BSA. Then add 20 pL of diluted ExtrAvidin- Alkaline Phosphotase (1:5,000 dilution) to each well and incubate at 37°C for 30 min. Wells are washed X3 with PBS (with Ca, Mg) and 0.5% BSA. 1 tablet of p-Nitrophenol Phosphate pNPP is dissolved in 5 mL of glycine buffer (pH 10.4). 100 pl of pNPP substrate in glycine buffer is added to each test well. Standard wells in triplicate are prepared from the working dilution of the ExtrAvidin-Alkaline Phosphotase in glycine buffer: 5 pL of each dilution is added to triplicate wells and the resulting AP content in each well is 5.50 ng, 1.74 ng, 0.55 ng, 0.18 ng. 100 pl of pNNP reagent is then be added to each of the standard wells. The plate is incubated at 37°C for 4h. A volume of 50 pL of 3M NaOH is added to all wells. The results are quantified on a plate reader at 405 nm. The background subtraction option is used on blank wells filled with glycine buffer only. The template is set up to indicate the concentration of AP-conjugate in each standard well. Results are indicated as amount of bound APconjugate in each sample.

**Example 10.** Effects on the CNS. The effects of the formula 1 compounds on memory, learning, motor function or the status of a neurological condition or neurodegeneration condition are assayed using standard methods. For example, aged, two year old mice are tested in the Morris water maze procedure by training the mice to locate a pedestal in less than 15 seconds in three consecutive trials. Immediately upon completion of training one group of mice is treated with a formula 1 compound (5-30 mg/kg) and a second group is treated with a placebo. The treatment comprises one, two or three intraperitoneal, subcutaneous, intramuscular or intravenous injections of the formula 1 compound and the vehicle placebo. The injections are given once per day. Two weeks after treatment, the time to rescue is timed in the Morris water maze procedure and the control result is compared to the placebo control. The use of Morris water maze and other procedures to measure the effect of various conditions or compounds on learning, memory or neurological conditions have been described, see e.g., R. Gerlai Trends Neurosci. 1996, 19:177-181, J.L.W. Lau et al., Proc. Nat'l. Acad. Sci. 2001, 98:4716-4721, U.S. patent Nos. 6348489, 6251942 and 6277886.

Scopolamine induced amnesia is examined essentially as follows. Groups of 13 to 16 C57BL76 mice (about 35 gm) are trained in the Morris water maze procedure to locate a pedestal in less than 15 seconds in three consecutive trials. Immediately upon completion of training the mice in each of three groups are treated with scopolamine (1 mg/kg), scopolamine plus a formula 1 compound at one or more dosages (e.g., about 5-50 mg/kg), and scopolamine plus a placebo. The treatment comprises one, two or three intraperitoneal, subcutaneous, intramuscular or intravenous injections of the formula 1 compound and the vehicle placebo. The injections are given once per day. Six days after treatment the average time (sec) to rescue is timed using the Morris water maze procedure and the results from each group are compared. Results for a F1 C are optionally compared to the results that are obtained in these protocols using another control compound, e.g., (S)-(-)-N-propargyl-1-aminoindan or nefiracetam, or another F1 C.

For subjects having a neurological trauma, e.g., an experimental a brain or spine injury, administration of a F1 C can begin at various times relative to the trauma. Administration can begin about 1, 2 or 3 days before the trauma or at times thereafter, e.g., commencing at about 1, 1.5, 2, 2.5, 3, 3.5, 4, 4.5, 5, 5.5, 6, 7, 8, 9, 10, 12, 14, 16, 18, 20, 24, 28, 36, 48 or more hours after the trauma. Administration of the F1C can be daily dosing or intermittent dosing.

**Example 11.** Ischemia treatment. The capacity of F1Cs to limit injury associated with ischemia and reperfusion is determined in an animal model essentially as follows. Male Sprague-Dawley rats weighing 130-170 g are randomly assigned to no pre-treatment, vehicle pre-treatment or formula 1 compound pre-treatment using one or more dosages, e.g., about 1-10 mg/kg. Animals are treated with vehicle or F1C the day before and the day of surgery. Anesthesia is induced with intraperitoneal pentobarbital (60-70 mg/kg). The rats are placed on a heating pad, and body temperature is maintained at about 36°C. Detection of the cremaster muscle on its neurovascular pedicle is performed essentially according to conventional techniques, e.g., Anderson, G. L. et al., Microvascular Res. 1988 36:56-63, Siemionow, M. et al., Microcirc. Endoth. Lymphatics 1991 7:183-197, Siemionow, M. et al., J. Hand Surgery 1993 18A:963-971.

Briefly, a skin incision is made from the anterior iliac spine to the tip of the scrotum. The testis with cremaster muscle intact is then dissected away from the scrotum. An opening of 1 cm is made on the ventral surface of the cremaster, and the testis and spermatic cord are removed. Under a microscope, the neurovascular pedicle, consisting of the pubic-epigastric arteries, vein, and genitofemoral nerve, is then completely isolated by dissecting to the origin of the vessels from the external iliac artery and vein. The front wall of the cremaster muscle sac is opened and the island cremaster muscle flap is prepared for intravital videomicroscopy. The rat is secured on a tissue bath, and the cremaster muscle flap is spread over the coverglass in the opening at the bottom of the bath and fixed with 5-0 silk sutures. It is then transilluminated from below, using a fiber optic tungsten lamp. The muscle is kept moist and covered with impermeable plastic film. The tissue bath, designed specifically for temperature control, is filled with 0.9% saline and the temperature maintained at between 35-36°C. The microscope is equipped with a color video camera. The video image of the microcirculation is displayed on a 19" monitor, where the final magnification is 1800X. Measurement of microvascular activity is recorded after isolation of the muscle to establish the pre-ischemia baseline. After proper positioning of clamps to completely shut down blood flow to the muscle flap, the duration of the ischemic period is six hours. Following removal of clamps to induce reperfusion injury, activity in the microvasculature is measured at e.g., 30, 60 and 90 minutes post-reperfusion. In all experimental subjects, ischemia is followed by reflow and then by an initial period of flow of blood through the microcirculation. This burst of circulatory activity is followed by marked reperfusion injury that induces loss of flow.

One or more of the following parameters are used to evaluate the state of the cremaster muscle microvasculatory system prior to ischemia and after reperfusion. The density of perfused capillaries in each of three flap regions is measured by counting the number of flowing capillaries in proximity to the preselected post-capillary venule. Nine visual fields of capillaries are counted at each postcapillary venule site, for a total of 27 fields per cremaster muscle flap.

A leukocyte count in postcapillary venules is taken using video scans of three pre-selected post-capillary venules in proximal, middle and distal flap regions. For each venule, the number of leukocytes rolling through the lumen, the number adhering to the endothelium and the number migrating across the endothelium over a two-minute period are recorded. Results are optionally obtained for rollers, strikers and diapedesis.

Red blood cell velocities in first order and second order arterioles are measured. Red blood cell velocities are recorded in the main arterioles of the cremaster flap using an optical Doppler velocimeter. Results are obtained for velocity of venous and arterial blood.

In an exemplary protocol, six rats are untreated and six rats are pre-treated with vehicle. Under conditions of six hours of ischemia and 90 minutes of reperfusion, the absolute number of rolling, sticking and transmigrated leukocytes is determined within 60 minutes of reperfusion and at 90 minutes. Rats are pre-treated with a formula 1 compound by subcutaneous injection the day before and the day of surgery to measure any protective effect of the therapy. One or more of the three parameters are determined and are compared to normal values. The endothelial-adherent properties compared to baseline values are optionally determined, using numbers of rolling, sticking and transmigrating leukocytes. Red cell velocities in second order arterioles are compared to normal rates of flow at, e.g., 90 minutes post-reperfusion.

**Example 12.** Pulmonary vasoconstriction. The capacity of F1Cs to limit hypoxia induced pulmonary vasoconstriction is demonstrated using an animal model essentially as follows. Isolated perfused ferret lungs are an established animal model to study secondary pulmonary hypertension. In brief, male ferrets are anesthetized with intraperitoneal pentobarbital sodium and the chest is opened. Stainless steel cannulae are secured in the left atrium and pulmonary artery, and the pulmonary artery and the aorta are ligated. The lungs are perfused with a mixture of autologous blood and Krebs-Henseleit buffer in a circulating manner at a constant rate of about 85 mL/min. The perfusion circuit includes a perfusate reservoir, a roller perfusion pump, filter, and a heat exchanger. The perfusion system is made of, e.g., tygon tubing, which is used for connections and for passage through the perfusion pump. The temperature of the perfusate is kept about 37-38°C. and the pH is maintained at 7.35 to 7.40 by adding sodium bicarbonate to the reservoir as needed. The venous reservoir is placed below the lowermost portion of the lung.

The lungs are ventilated with a hypoxic gas mixture of 5% CO₂, 4% O₂, and 91 % N₂ by a tracheotomy with a Harvard animal respirator for 30 minutes. The animals are ventilated with a tidal volume of 30 mL, at a rate of 18 breaths/min. and with 2 cm of H₂O positive end-expiatory pressure. For measurements, pulmonary arterial, left atrial and tracheal pressures are monitored using Gould Statha P231 D pressure transducers or an equivalent connected to the inflow circulation and recorded on, e.g., a Grass polygraph. After 30 minutes of ventilation with hypoxic gas mixture, a formula 1 compound in a dose between about 5-25 mg/kg body weight is added to the reservoir, and perfusate is allowed to perfuse the ferret lungs for 1.5 hours. Pulmonary artery pressure is measured until the end of the experiment, i.e., a total of two hours. Pressure that remains at or near basal level indicates the vasodilatory effect of the F1C in pulmonary circulation that is otherwise constricted in response to hypoxia. The effects of the F1Cs can be compared to the effects and duration of nitric oxide, a therapeutic agent that is optionally used in this model as a control.

**Example 13.** Hematopoiesis modulaton. Modulation of hematopoiesis is observed in mammals with injury from, e.g., radiation exposure or from an immunosuppressive chemotherapy to characterize the biological activity of the F1Cs. In an example, animals are used to demonstrate the effect of F1 Cs on hematopoiesis after immune system injury due to radiation. Hematopoiesis in the murine or non-human primate immune system after radiation is optionally used because of the similar responses of murine and human hematopoiesis to drugs and toxic insults (see, e.g., J.H. Hendry and B.I. Lord, editors, Radiation toxicology: Bone marrow and leukaemia 1995 Taylor & Francis Inc., London).

In an exemplary protocol, B6D2F1/J female mice (Jackson Laboratory, Bar Harbor, ME), 18-24 weeks of age, 22-30 g body weight, are obtained and held in quarantine for two weeks. Up to 10 mice are housed in sanitized 46 x 24 x 15 cm polycarbonate boxes with filter covers (Microlsolator; Lab Products, Inc, Maywood, NJ) on autoclaved hardwood chip bedding. Mice are given feed and acidified (pH 2.5) water freely. The animal holding room is maintained with conditioned fresh air at approximately 21 °C and 50° (±10%) relative humidity and with a 12-h light/dark full spectrum lighting cycle.

Mice are placed in ventilated Plexiglas containers and exposed bilaterally to gamma-radiation from a ⁶⁰Co source. Exposure time is adjusted so that each animal received a midline tissue-absorbed dose of 1-12 Gy at a nominal dose rate of 0.4 Gy/min at ambient temperature. Using a standardized technique, the midline dose rate is measured by placing a 0.5 cc tissue-equivalent ionization chamber at the center of a 2.5-cm diameter cylindrical acrylic mouse phantom. The tissue-air ratio, defined as the ratio of the dose rate measured in the phantom to the dose rate in free air, for this array is about 0.96. Variation within the exposure field is less than about 4%. Dosimetric measurements are made in accordance with the American Association of Physicists in Medicine protocol for the determination of absorbed dose from high-energy photon and electron beams (Med. Phys. 1983 10:741-771). Sham-irradiated mice are treated in the same way as the irradiated animals, except that the animals are not irridiated.

Various formula 1 compounds are formulated with a suitable vehicle (e.g., PEG-400) or sterile 0.9% NaCl (saline) optionally containing other excipients such as a cyclodextrin. The compounds are injected subcutaneously in a volume of about 0.1 mL or they are delivered orally or they are administered by another route. Doses typically range from about 1 mg/kg to about 350 mg/kg, e.g., about 1, 10, 20, 40, 80, 160 or 320 mg/ kg.

Blood (0.6-1.0 mL) is obtained from halothane-anesthetized mice by cardiac puncture using a heparinized syringe attached to a 21-gauge needle. Blood is collected in EDTA-containing sample tubes. Mice are euthanized by cervical dislocation after blood collection. White blood cell (WBC), red blood cell (RBC) and platelet (PLT) counts are performed using, e.g., a Hematology System 9000 (Biochem Immunosys-tems). Wright-stained blood smears from the same samples are made for differential counts of neutrophils and lymphocytes by light microscopy.

Hemopoietic progenitor cells committed to granulocyte-macrophage differentiation (GM-CFC) are assayed by a single-layer modification of a double-layer semisolid agar technique essentially as described (Patchen et al. Adv. Space Res. 1992 12:223-248). For example, femoral marrow is extracted and cell suspensions are prepared by flushing with 3 mL of McCoy's 5A medium containing 10% heat-inactivated fetal bovine serum (HIFBS; Hyclone, Logan, UT). Each cell suspension represented a pool of marrow from four femurs, i.e., both femurs from each of two mice. The total number of nucleated cells in each suspension is determined with, e.g., a Coulter counter. The agar-medium mixture consisted of equal volumes of 0.66% agar and double-strength supplemented CMRL 1066 medium (Gibco, Grand Island, NY). The medium is supplemented with final concentrations of 10% HIFBS, 5% tryptic soy broth, 5% heat-inactivated horse serum, antibiotics, and L-serine. One milliliter of the agar-medium mixture is added to each 35-mm plastic Petri dish (two dishes per suspension) and mixed with 50 µL of 0.1 ng/µL recombinant mouse GM-CSF (Genzyme, Cambridge, MA). Cell suspensions are then mixed into the agar-medium mixture to a final concentration of 0.5 x 10⁵ cells/mL for unirradiated animals, and 1.0 x 10⁵ or 1.5 x 10⁵ cells/mL for irradiated animals to ensure sufficient colonies per plate for quantitation. Control experiments are done to confirm linearity of colonies at cell concentrations of 0.5-1.5 x 10⁵ cells/ mL. Colonies (> 50 cells) are counted after seven days incubation in a 37 °C humidified environment containing 5% CO₂. The average of the counts for the two dishes is taken as the value for each pool. About six animals are used per group in each of two experiments.

For histological examination of myeloid hyperplasia in bone marrow after administration of the formula 1 compound, mice are euthanized with halothane, tissues are immersed in formalin, bones are decalcified and routine H&E-stained 6-µm paraffin sections are prepared.

For induced-infection studies, a clinical isolate of K. *pneumoniae,* capsule type 5 (strain AFRRI 7), that is kept frozen at 70 °C in skim milk, is grown overnight at 35 °C on Trypticase Soy Agar (Becton-Dickinson, Sparks, MD). Five typical colonies are inoculated into 8 mL of brain heart infusion broth (Becton-Dickinson) and incubated overnight at 35 °C. Two milliliters of this overnight suspension is inoculated into 95 mL of prewarmed brain heart infusion broth. The culture is incubated at 35 °C with shaking for approximately 2.5 h. The optical density of bacterial growth is monitored with a spectrophotometer at a wavelength of 550 nm. Late log-phase cells are ished and suspended in cold saline to yield 10⁹ viable bacteria per mL. Appropriate dilutions for inoculations are made in cold saline.

To induce a bacterial infection, all mice are injected sc with *K*. *pneumoniae* four days after sham-irradiation or irradiation when circulating leukocytes are depressed. Mice are inoculated sc rather than iv or ip, to establish infection leading to sepsis, but not rapid septic shock. After sc inoculations of *K. pneumoniae* in the mice, the infection remains largely localized to the injection site. *K. pneumoniae* are not detectable in blood of inoculated mice until a few hours before death.

Different doses of the bacteria are inoculated for each of three radiation dose levels (0, 1 or 3 Gy) to approximate the LD_{95/30} (radiation dose that is lethal for 30-95% of animals), because the effects of radiation on hematopoiesis and susceptibility to infection are dependent on the dose of radiation. The LD_{95/30} for bacteria at each radiation dose is calculated from probit analysis. The actual doses are estimated by dilution plating of inocula onto Trypticase Soy Agar and incubating overnight at 35 °C. Since different bacterial doses are expected to be needed for different radiation doses, the LD_{95/30} is estimated for each group and different mortality rates are observed in the vehicle-injected control groups. Bacterial doses for induced-infection experiments are prepared and calculated in the same manner.

Animals are checked frequently, e.g., once or twice daily, six or seven days per week, to monitor survival and to euthanize mice that are in a moribund state. To verify that mortality in the induced-infection experiments is associated with *K*. *pneumoniae* injection, heart blood from recently deceased animals (or moribund animals euthanized by cervical dislocation) is cultured overnight at 35 °C on Columbia sheep blood agar plates (Becton-Dickinson, Sparks, MD). Colonies are identified as *K*. *pneumoniae* by a suitable means, e.g., Biolog analysis.

For histological analysis of bone marrow, coded slides are scored blind using a five-level semiquantitative scale and the results analyzed with a randomization t-test to obtain exact P-values. Thirty-day survival values are compared using the generalized Savage (Mantel-Cox) procedure (BMDP Statistical Software, Inc, Los Angeles, CA). To calculate dose reduction factors (DRFs), probit analysis is performed on mortality data.

To characterize the potency of formula 1 compounds to ameliorate radiation-induced defects in hematopoiesis, mice are exposed to bilateral whole-body gamma-radiation and receive a dose of 3 Gy (or are sham-irradiated). One hour after irradiation or sham-irradiation, mice are injected with 320 mg/kg 3β,17β-dihydroxyandrost-5-ene ("AED") or PEG-400 vehicle. Between-group differences in blood cell elements, e.g., neutrophils, GM-CFC and platelets are generally determined. Irradiation results in a decrease in neutrophils at about four days after radiation compared to sham-irradiated animals.

**Example 15.** Treatment of neurodegenerative conditions. Experimental allergic encephalomyelitis (EAE), is demyelinating disease of the central nervous system with many pathological and clinical similarities with multiple sclerosis (MS). EAE is thus a recognized animal model for human autoimmune conditions such as MS. F1Cs are tested for their capacity to delay the onset of EAE or to reduce its symptoms. Female SJL mice (5 animals per group) are immunized with 150 to 200 µg of PLP-139-151 peptide/CFA to induce EAE. Starting 7 days before injection of the peptide, the animals are given daily injections (s.c.) of the compounds (3.0 mg) in 0.1 mL vehicle, or vehicle alone for 33 days. The vehicle consisted of a suspension of the formula 1 compound in saline and carboxymethylcellulose. Delayed onset, reduced peak clinical score (from 5.2 ± 0.6 to 2.8 ± 1.8) and cumulative disease index (>60%) of EAE, and prevention of or significant attenuation of relapses are measured. Reduced numbers of PLP-139-151 specific T cell responses and reduced numbers of TNF-α producing cells in the CNS indicate reduced disease progression or severity. Reduced production of autoimmune Th-1 associated cytokines, is consistent with restoration of a more normal Th-1/Th-2 immune balance and/or with reduction of inflammation in this model.

The efficacy of the formula 1 compounds to treat other autoimmune conditions can be determined by incorporating their use with suitable animal models or assay methods, e.g., collagen-induced arthritis as a model for human autoimmune polyarthritis (e.g., L.K. Myers et al., Clin. Immunol. 2002, 102:185-191, A. Matejuk et al., Endocrinology 2002, 143:313-319, S. Thornton et al., J. Immunol. 165:1557-1563). The effect of the compounds on markers of inflammation such as TNFα, MIP-1β, IL-13, IL-15, IL-17 or IL-18, e.g., reduced expression or activity, is optionally observed in any autoimmune or inflammatory condition.

**Example 17**. The effect of F1Cs on transcript or gene product levels in cells *in vitro* is studied *in vitro* using a cell type of interest, e.g., the murine macrophage cell line designated RAW264.7 ("RAW" cells). For the RAW cells, the cells are maintained in a suitable medium, e.g., RPMI 1640 supplemented with 10% FBS, standard Penn/Strep antibiotic solution and 2mM glutamine. The F1C is dissolved in a suitable solvent, e.g., DMSO or pyrrolidone, to generate a 10 mM stock solution. For DMSO solutions, appropriate dilutions are made to give a F1C final concentration in culture media of about 1 nM to about 10 µM, with a final DMSO content of no more than 0.1 % v/v. The cells are induced with LPS at 100 ng/ml (stock solution in water, diluted in serum-free culture media).

In a typical protocol, on day 0 the cells are plated at a density to reach a sub-confluent state of greater than about 75% confluency on the following day. For 6- well plates, about 500,000 to 700,000 cells/well are plated. On the following day, day 1, the cells are treated with the F1 C or vehicle, e.g., DMSO, with or without LPS, for selected times, e.g., 0.5, 1, 2, 4, 6, 8, 10, 12, 16, 24, 36, 48 hours. After incubation, cells are harvested with a cell scraper and total RNA is extracted to generate samples for PCR analysis. 1 mL of culture media is optionally saved at -20°C for future ELISA analysis to determine gene transcript levels. On day 2, cells are harvested after, e.g., 24 hr of F1C in DMSO treatment. LPS induction is started in cells pre-treated with F1C in, e.g., DMSO. Exemplary treatment conditions and time points for cell harvesting are as follows:

| | | | | | |
|---|---|---|---|---|---|
| No treatment | 0 hr | | | | 24 hr |
| DMSO + LPS | | 1 hr | 4 hr | 8 hr | 24 hr |
| F1C 10 µM + LPS | | 1 hr | 4 hr | 8 hr | 24 hr |
| F1C 1 µM + LPS | | 1 hr | 4 hr | 8 hr | 24 hr |
| F1C 10 nM + LPS | | 1 hr | 4 hr | 8 hr | 24 hr |
| DMSO | 24 hr + LPS | 1 hr | 4 hr | 8 hr | 24 hr |
| F1C 10 µM | 24 hr + LPS | 1 hr | 4 hr | 8 hr | 24 hr |
| F1C 1 µM | 24 hr + LPS | 1 hr | 4 hr | 8 hr | 24 hr |
| F1C 10 nM | 24 hr + LPS | 1 hr | 4 hr | 8 hr | 24 hr |

Exemplary genes of interest that can be analyzed by this or a similar protocol include 1, 2, 3, 4, 5, 6 or more of iNOS (inducible nitric oxide synthase), eNOS (constitutive nitric oxide synthase), COX-2 (cycloxygenase-2, PGE2 synthase), IκBβ, TNFα, IL-1β, IL-1 Ra (interleukin 1 receptor antagonist), NFκB1 (p105), NFκB2 (p49/p100), IL-6, MCP-1 (monocyte chemoattractant prtein-1 or CCL2), MIP-2 (macrophage inflammatory protein-2), MMP9 (matrix metalloproteinase 9), gelatinase B, HO-1 (heme oxygenase 1), HIF1α (hypoxia inducible factor 1, alpha subunit), GCLC (gamma glutamylcycteine synthetase catalytic (heavy) subunit or γGCS-hs), GCLM (gamma glutamylcycteine synthetase modifier (light) subunit or γGCS-Is), xCT (cystine/glutamate exchange transporter), NQO1 (NAD(P)H: quinone oxidoreductase 1), TXNRD1 (thioredoxin reduatase 1), EBBP (estrogen responsive B-box protein), CYP1A1 (cytochrome P450), CD36 (SR-B), SR-A (scavenger receptor A or Msr1), ABCA1 (ATP-binding cassette transporter A1), ABCG1 (ATP-binding cassette transporter G1), LDLR (low-density lipoprotein receptor), NR1H3 (nuclear receptor 1 H3 or LXRα), NR1C3 (nuclear receptor 1C3 or PPARγ), SCD-1 (stearoyl-CoA desaturase 1) and NR4A1 (nuclear receptor 4A1 or Nur77).

**Example 19.** Exposure of rodents to ionizing radiation exposure. The effect of selected F1Cs on survival of lethally-irradiated female B6D2F1 mice were compared to control animals treated with vehicle alone. The animals were exposed to 10 Gy of total body irradiation at 2.5 Gy/min using a ¹³⁷Cs source. Groups of 12 animals were used in a total of 5 groups. For Groups 1, 2, 3, and 5, test article was administered as a 100 µL volume, by subcutaneous injection, for three consecutive days, with the first dose administered 2 to 4 hours following exposure to radiation. For Group 4, test article was administered as a 50 µL volume, by intramuscular injection for three consecutive days. The formulation was a a suspension containing 0.1 % w/v carboxymethyl-cellulose, 0.9% w/v sodium chloride and 0.05% v/v phenol. The formulations were agitated to uniformly resuspend the F1C before syringing, and injected into animals within a few minutes of drawing into the syringe to prevent settling in the syringe.

The groups of animals were treated as follows. Group 1 received vehicle only by daily subcutaneous injection for 3 consecutive days. Group 2 received 0.6 mg in 100 µL of a suspension of 3β,17β-dihydroxyandrost-5-ene by daily subcutaneous injection for 3 consecutive days. Group 3 received 3.0 mg in 100 µL of a suspension of 3β,17β-dihydroxyandrost-5-ene by daily subcutaneous injection for 3 consecutive days. Group 4 received 0.6 mg in 50 µL of a suspension of 3β,17β-dihydroxyandrost-5-ene by daily intramuscular injection for 3 consecutive days. Group 5 received 0.6 mg in 100 µL of a suspension of 3β-hydroxy-17β-aminoandrost-5-ene by daily subcutaneous injection for 3 consecutive days.

Survival of the animals was monitored for 21 days after irradiation and the following results were obtained. The number of surviving animals is shown for day 6, 7, 12 and 21. The results show that the F1Cs increased the rate of survival of subjects that were exposed to an otherwise lethal dose of ionizing radiation.

| | | Day | | | |
|---|---|---|---|---|---|
| Group | | 6 | 7 | 12 | 21 |
| 1 | vehicle control | 12 | 11 | 4 | 1 |
| 2 | 0.6 mg s.c. | 12 | 11 | 10 | 7 |
| 3 | 3.0 mg s.c. | 12 | 12 | 9 | 7 |
| 4 | 0.6 mg i.m. | 12 | 12 | 11 | 9 |
| 5 | 0.6 mg s.c. | 12 | 12 | 12 | 11 |

**Example 21.** Measurement of biological parameters in non-human primates after biological insult. A study was conducted to characterize a biological insult of 600cGy of whole body irradiation to male Rhesus (*Macaca mulatta*) primates weighing 2.5 to 4.5 kg at an age range of about 1.75 to 3.5 years. Core body temperature was monitored by telemetry in the monkeys for a period of 40 consecutive days. Two groups of 10 animals each were used in the study. Core body temperature transmitters were surgically implanted in the abdomen prior to initiation of the radiation protocol. Core body temperature was continuously recorded from day -7 to day 41 for correlation with survival, hematology results, and other clinical parameters.

Temperature transmitters. Before initiation of the temperature transmitter implantation protocol, all animals were subject to a detailed physical examination and body weight measurement under the direction of a clinical veterinarian. Blood was collected from all animals, which were not food and water deprived, and assessed for basic blood chemistry and hematology. The results of the evaluation was reviewed by the clinical veterinarian to ensure satisfactory health status.

Implantation of the temperature transmitters was accomplished using animals that were fasted overnight prior to surgery and then anesthetized by an intra-muscular (IM) injection of acepromazine (10 mg/mL, 0.14 mg/kg) and ketamine (100 mg/mL, 13.6 mg/kg) and intubated. Where needed, lidocain spray (10% w/w) was administered onto the glottis prior to intubation. An ophthalmic ointment was applied to both eyes to prevent drying of the cornea. Animals were placed on a heating pad and administered isoflurane by inhalation, with an oxygen flow of approximately 200 mg/kg/min. A ventilator was used to maintain the respiratory rate between 8 and 20 breaths/min with a ventilation pressure of 18-25 cm H₂O. Monitoring during anesthesia included heart rate and oxygen saturation of the blood using a pulse oximeter. Prophylactic antibiotics (cefazolin 25 mg/kg) were administered by intramuscular injection at least 1-hour prior to surgery, and every 6 to 8 hours post injection for at least 24-hours post surgery. Analgesia (buprenorphine 0.05 mg/kg) was administered by intramuscular injection every 6 to 12 hours for at least 24-hours post surgery. Intravenous fluid therapy were given throughout the anesthesia using sterile Lactate Ringer's solution at a rate of 10 mL/kg/hr.

The surgical site was shaved and aseptically prepared using chlorhexidine gluconate 4% and isopropyl alcohol 70%. A longitudinal incision was performed lateral but close to the linea alba. The internal abdominal oblique muscle was separated from the aponeurosis of the transversus abdominis by blunt dissection. A sterile core body temperature transmitter (Data Science International, TA10TAD70) was inserted between the internal abdominal oblique muscle and the aponeurosis of the transversus abdominis. Hemostasis was maintained using appropriate suture material. Sterile saline was used to allow ease of placement of the transmitters. The incision was closed with absorbable suture material using simple continuous sutures. The skin was closed with discontinuous buried sutures using absorbable suture material. Additional post-operative cares (analgesia and antibiotics) was provided to the animals when needed. Rectal body temperature was monitored in the post-operative period. Once the body temperature was within an acceptable range and the animal was alert, each animal was returned to their cages. A postoperative period of at least 2 weeks was allowed prior to initiation of radiation.

Acclimation and whole-body irradiation. Before transportation to the radiation facility, the animals were acclimated to the radiotherapy chair and to transportation. During the acclimation period, animals were assigned to their respective dose groups by block randomization based on the absolute neutrophil count. Any animal with unacceptable pretreatment data was replaced by an animal kept under identical environmental conditions. Animals with pretreatment data considered acceptable but marginally different from normal values were assigned to the sham group to allow longer post-operative recovery.

Animals were fasted overnight prior to whole-body irradiation and fed upon return to the holding facility. Animals were transferred to the irradiation facility in a transport vehicle with controlled environment. During transportation, each animal was individually housed in a stainless steel squeeze back cage. The animal's clinical signs were monitored immediately before and after transportation. Group 1 animals, sham irradiated, were subject to the same irradiation procedure as Group 2 animals, however, these animals did receive radiation. The 10 control animals, Group 1, were sham irradiated by placing each animal in the restraint for 10 minutes. The 10 treated animals, Group 2, received a midline ⁶⁰Co γ-radiation dose of 6 Gy at a dose rate of about 60 cGy/minute (day 1). The animals receiving this 6 Gy radiation insult were restrained during the radiation exposure by placing each animal in a chair allowing appropriate restraining in a symmetric position. An insulated cover was placed on the radiotherapy chair during transportation between the transport vehicle and the treatment site. Music was provided inside the treatment room to reduce stress to the animals. Animal positioning was confirmed with linear markers installed in the treatment room. To produce a homogenous dose distribution, treatment was divided in two parts. First, the animal received half of the dose by anteroposterior (AP) irradiation. The second half of the dose was delivered by posteroanterior (PA) irradiation. Group 1 animals were placed in an identical restraining chair in the sham treatment site for approximately the same period of time without exposure to radiation. Once the treatment was completed, animals were returned to the transport vehicle and were transported to their housing facility. The radiation dose was calibrated using an acrylic phantom placed in the same experimental set up that was used for animal irradiation.

Animal maintenance. Animals were housed individually in stainless steel squeeze back cages equipped with an automatic watering system except during transportation where water bottles were provided. The cages were labeled with a color-coded cage card indicating study number, group, animal number, species, sex and dose level. The animal room environment was controlled (temperature 21 ± 3°C, humidity 30-70%, 10-15 air changes per hour, 12 hours light, 12 hours dark). Temperature and humidity were monitored continuously except during animal transportation and inside the radiation facility where only temperature was recorded. A standard certified commercial primate chow (Teklad Certified Global 25% Primate Diet # 2055C) was made available to each monkey daily. Food was withdrawn overnight prior to radiation and necropsy. Maximum allowable concentrations of contaminants in the diet (e.g., heavy metals, aflatoxin, organophosphates, chlorinated hydrocarbons and PCBs) were controlled and routinely analyzed by the manufacturers. If an animal stopped eating during the study, the diet was supplemented at the discretion of the study director. Tap water was purified by reverse osmosis and provided to the animals ad libitum throughout the study. Periodic analyses of the tap water and reverse osmosis water were performed. It was considered that there were no known contaminants in the diet or water. During the pre-treatment period cage side observations of clinical signs were generally performed once daily.

Observations. Mortality checks were performed twice a day during all phases of the study. Moribund animals were euthanized for humane reasons based on the clinical judgments. Sacrificed animals were subject to a clinical examination. When the core body temperature was 33° C (91.4° F) or lower or when an animal experienced a weight loss of more than 20% over a 4 day period, the animal was euthanized. Animals were also euthanized when they displayed complete anorexia for 3 days with deteriorating conditions based on the clinical examination or when they displayed an absence of response to stimuli.

Results obtained from the study were used to correlate the changes in biological parameters such as core body temperature and hematology with clinical signs following whole body irradiation. These results were used to obtain a status profile or surrogate endpoint such as incidence or duration of fever. During the pre-treatment period cage side observations of clinical signs were performed once daily. During the treatment period, clinical signs were recorded at cage-side twice daily for all animals or as often as deemed necessary. A detailed clinical examination was performed on all animals, once prior to irradiation on day 1, weekly thereafter, including on day 41 prior to necropsy.

The core body temperature and activity was recorded at 1 minute intervals for all animals from day -7 to day 41 using the implanted transmitter. Each animal cage was equipped with a telemetry receiver. The values of calibration of the transmitter implanted in each animal were entered in a telemetry computer system to ensure accurate temperature monitoring. Core body temperature was not recorded when animals were handled or during transport, but core temperature was generally monitored continuously at other times. Body weights were recorded for all animals once prior to randomization, prior to treatment on day 1 and weekly thereafter, including on day 40 (non-fasted) and on day 41 before necropsy. Hematology measurements were performed on all animals three times during the pre-treatment period and during the treatment period on days 2, daily from day 5 to day 27 and once on days 30, 33, 36 and 40. Blood samples of 0.5 mL were collected from the femoral vein or artery or from any appropriate vessel by venipuncture for hematological analysis. Food and water was available to the animals before blood collections.

Hematology parameters that were examined at most time points included red blood cell count, hematocrit, hemoglobin, white blood cell count, absolute differential WBC count, relative differential WBC count, relative reticulocyte count, mean corpuscular hemoglobin, platelet count, platelet volume, immature granulocyte count and red cell distribution width. EDTA was used as an anticoagulant and blood smears were prepared for each time point, stained with Modified Wright's stain and evaluated.

On day 41, the irradiated group 2 animals were sedated using ketamine and acepromazine and then euthanized by an overdose of barbiturate (e.g. sodium pentobarbital), which was administered intravenously, followed by exsanguination. For euthanized animals, gross pathology consisted of an external examination, identification of clinically recorded lesions and a detailed internal examination. To avoid autolytic changes, the necropsy examination was conducted as soon as possible on all animals that died while on study or that were euthanized during the study or at termination of the study at day 41. The animals were stored at 2-8 °C before examination. For all animals that were euthanized, the following organs were dissected, trimmed free of fat and weighed: Brain, testes, heart, prostate, kidneys, seminal vesicles, large intestine, small intestine, liver, spleen, lungs with trachea and thymus. The large intestine and small intestine were examined by making a longitudinal incision to open the lumen and removal of contents. The intestinal mucosa was washed with saline and excess saline was removed and the organs weighed. Paired organs were weighed together. Absolute and relative (to body weight) organ weights were calculated. On completion of the gross pathology examination, abnormal tissues brain (right part), femur and marrow, heart (both ventricles and atria, septum with papillary muscle), sternum and marrow, thymus were retained. Neutral buffered 10% formalin was usually used for fixation and preservation. Three femoral bone marrow smears were prepared from each euthanized animal (right femur), stained with Modified Wright's stain and evaluated.

Tissue samples from liver, lungs (right and left separately), kidneys, brain (left) and spleen were collected at necropsy from all euthanized animals for bacteriological culture. Tissue samples were stored refrigerated 2-8°C pending analysis. A selected area at the surface of the tissue sample was burned to eliminate possible surface contaminant. A sterile culture swab was inserted in the tissue sample through the burned surface for isolation and identification of aerobic and anaerobic bacteria. Histopathological examination was performed on the tissues from euthanized animals. Tissues were prepared for histological examination by embedding in paraffin wax, sectioning and staining with hematoxylin and eosinphloxin.

**Example 22.** Results and calculation of status profiles for non-human primates using biological parameter measurements. Numerical data obtained from the protocol described in example 21 was subjected to calculation of group means, standard deviations and other statistical analyses.

Statistically significant status profiles were obtained based on five biological parameters, i.e., anemia (based on hematocrit), thrombocytopenia (platelets), neutropenia (neutrophils), elevated temperature and circadian rhythm disruption. Each parameter alone gave statistically significant P_{lethality} and Pₛᵤᵣᵥᵢᵥₐₗ status profiles. When hematocrit nadirs for individual animals fell below 20% of normal, 4 of 4 animals died, while 5 of 6 animals survived when individual hematocrits remained above 20%. Calculation by an unpaired t-test analysis gave P_{lethality} and Pₛᵤᵣᵥᵢᵥₐₗ status profiles of 0.02 for a mean hematocrit nadir of 16.4% and 25.6% respectively.

When platelets for individual animals fell to less than 7,000 per µL, 5 of 6 animals died, while 4 of 4 animals survived when the platelet count nadir remained above about 7,000 per µL. Calculation by an unpaired t-test analysis of P_{lethality} and Pₛᵤᵣᵥᵢᵥₐₗ status profiles of 0.01 for a mean platelet nadir of 4,800 platelets per µL blood and 12,800 platelets per µL blood, respectively.

When the neutrophil nadir for individual animals fell to less than 50 per µL, 5 of 6 animals died, while 4 of 4 animals survived when the neutrophil count nadir remained above 50 per µL. Calculation by an unpaired t-test analysis of P_{lethality} and Pₛᵤᵣᵥᵢᵥₐₗ were 0.02 for a mean neutrophil nadir of 28 neutrophils per µL blood and 58 neutrophils per µL blood respectively.

For fever, P_{lethality} was less than 0.05 when the animals experienced fever or Pₛᵤᵣᵥᵢᵥₐₗ was greater than 0.95 when the animals did not have an elevated temperature or a fever. For this biological response, fever or elevated temperature was defined as a temperature of at least about 39.0°C for at least about 15 minutes within 12 hours after the animals were irradiated on day 1. The baseline temperature for the animals was considered to be 37.3°C, although temperatures for the 10 control (non-irradiated) animals in example 1 varied with the animal's circadian rhythm between about 36.8°C and 37.9°C. The control animal's circadian core body temperature rhythm was quite regular, while irradiated animals that survived the radiation was relatively regular and was indistinguishable from non-irradiated controls by about 5-8 days after irradiation. However, circadian core body temperature rhythm from irradiated animals that did not survive the radiation was destroyed and did not recover at any time after its disruption. P_{lethality} was less than 0.05 when circadian rhythm was disrupted, and Pₛᵤᵣᵥᵢᵥₐₗ was greater than about 0.95 when circadian rhythm was not disrupted. The loss of circadian rhythm was detectable within 24 to 48 hours after the animals were exposed to the 6 Gy dose of γ-radiation.

The P_{lethality} and Pₛᵤᵣᵥᵢᵥₐₗ status profiles for platelets, hematocrit and neutrophils given above was obtained using an unpaired T-test analysis based on the animals described in example 1. Five of the irradiated animals in example 1 survived the 6 Gy radiation exposure and the hematocrit, platelet and neutrophil nadir from irradiated surviving animals (variable 1) was compared to the hematocrit, platelet and neutrophil nadir from the 5 irradiated non-survivors (variable 2).

| Hematocrit t-Test: Two-Sample Assuming Unequal Variances | | |
|---|---|---|
| | variable 1 | variable 2 |
| Mean | 25.6 | 16.4 |
| Variance | 17.3 | 30.8 |
| Observations | 5 | 5 |
| Hypothesized Mean Difference | 0 | |
| df | 7 | |
| t Stat | 2.9662 | |
| P(T<=t) one-tail | 0.0105 | |
| t Critical one-tail | 1.8946 | |
| P(T<=t) two-tail | 0.0209 | |
| t Critical two-tail | 2.3646 | |

| Platelet t-Test: Two-Sample Assuming Unequal Variances | | |
|---|---|---|
| | variable 1 | variable 2 |
| Mean | 12.8 | 4.8 |
| Variance | 21.7 | 1.7 |
| Observations | 5 | 5 |
| Hypothesized Mean Difference | 0 | |
| df | 5 | |
| t Stat | 3.698001 | |
| P(T<=t) one-tail | 0.007014 | |
| t Critical one-tail | 2.015049 | |
| P(T<=t) two-tail | 0.014028 | |
| t Critical two-tail | 2.570578 | |

| Neutrophil t-Test: Two-Sample Assuming Unequal Variances | | |
|---|---|---|
| | variable 1 | variable 2 |
| Mean | 0.058 | 0.028 |
| Variance | 0.00037 | 7E-05 |
| Observations | 5 | 5 |
| Hypothesized Mean Difference | 0 | |
| df | 5 | |
| t Stat | 3.19801 | |
| P(T<=t) one-tail | 0.01202 | |
| t Critical one-tail | 2.01505 | |
| P(T<=t) two-tail | 0.02405 | |
| t Critical two-tail | 2.57058 | |

For the 5 surviving animals, the hematocrit nadirs were 28, 31, 24, 25 and 20, while hematocrit nadirs for the non-surviving animals were 14, 16, 12, 14 and 26. For the 5 surviving animals, the platelet nadirs were 10 x 10³ per µL, 18 x 10³ per µL, 12 x 10³ per µL, 17 x 10³ per µL and 7 x 10³ per µL, while platelet nadirs for the non-surviving animals were 5 x 10³ per µL, 4 x 10³ per µL, 4 x 10³ per µL, 4 x 10³ per µL and 7 x 10³ per µL. For the 5 surviving animals, the neutrophil nadirs were 80 per mm³, 70 per mm³, 50 per mm³, 60 per mm³ and 30 per mm³, while neutrophil nadirs for the non-surviving animals were 20 per mm³, 30 per mm³, 20 per mm³, 40 per mm³ and 30 per mm³. The raw data for hematocrit, platelets and neutrophils from day -6 through day 26 are shown below and this data were used for the unpaired t-test P_{lethality} and Pₛᵤᵣᵥᵢᵥₐₗ calculations above.

| Hematocrits (% or UL) for irradiated animals at day -6 to day 10 | | | | | | | | |
|---|---|---|---|---|---|---|---|---|
| day | -6 | 2 | 5 | 6 | 7 | 8 | 9 | 10 |
| animal | | | | | | | | |
| 1 | 0.38 | 0.40 | 0.39 | 0.40 | 0.39 | 0.38 | 0.35 | 0.36 |
| 2 | 0.38 | 0.40 | 0.40 | 0.43 | 0.41 | 0.38 | 0.37 | 0.38 |
| 3 | 0.37 | 0.38 | 0.39 | 0.38 | 0.35 | 0.36 | 0.33 | 0.34 |
| 4 | 0.34 | 0.36 | 0.34 | 0.34 | 0.34 | 0.34 | 0.30 | 0.29 |
| 5 | 0.38 | 0.37 | 0.36 | 0.35 | 0.39 | 0.35 | 0.29 | 0.29 |
| 6 | 0.38 | 0.39 | 0.40 | 0.38 | 0.37 | 0.37 | 0.35 | 0.34 |
| 7 | 0.39 | 0.39 | 0.38 | 0.39 | 0.40 | 0.38 | 0.39 | 0.35 |
| 8 | 0.40 | 0.39 | 0.39 | 0.37 | 0.37 | 0.37 | 0.34 | 0.33 |
| 9 | 0.38 | 0.36 | 0.37 | 0.36 | 0.36 | 0.37 | 0.33 | 0.32 |
| 10 | 0.40 | 0.43 | 0.42 | 0.39 | 0.37 | 0.38 | 0.36 | 0.33 |
| mean | 0.38 | 0.39 | 0.38 | 0.38 | 0.38 | 0.37 | 0.34 | 0.33 |

| Hematocrits (% or UL) for irradiated animals at day 11 to day 18 | | | | | | | | |
|---|---|---|---|---|---|---|---|---|
| day | 11 | 12 | 13 | 14 | 15 | 16 | 17 | 18 |
| animal | | | | | | | | |
| 1 | 0.35 | 0.35 | 0.36 | 0.36 | 0.34 | 0.36 | 0.32 | 0.33 |
| 2 | 0.36 | 0.37 | 0.36 | 0.38 | 0.35 | 0.32 | 0.32 | 0.31 |
| 3 | 0.31 | 0.26 | 0.28 | 0.28 | 0.21 | 0.19 | 0.16 | 0.14 |
| 4 | 0.33 | 0.27 | 0.25 | 0.21 | 0.16 | * | | |
| 5 | 0.29 | 0.26 | 0.25 | 0.25 | 0.20 | 0.20 | 0.17 | 0.15 |
| 6 | 0.35 | 0.34 | 0.32 | 0.33 | 0.31 | 0.28 | 0.29 | 0.27 |
| 7 | 0.34 | 0.35 | 0.33 | 0.32 | 0.29 | 0.28 | 0.28 | 0.27 |
| 8 | 0.32 | 0.33 | 0.31 | 0.27 | 0.26 | 0.24 | 0.24 | 0.20 |
| 9 | 0.33 | 0.30 | 0.30 | 0.27 | 0.21 | 0.18 | 0.16 | 0.14 |
| 10 | 0.34 | 0.35 | 0.31 | 0.30 | 0.29 | 0.27 | 0.26 | 0.26 |
| mean | 0.33 | 0.32 | 0.31 | 0.30 | 0.26 | 0.26 | 0.24 | 0.23 |

| | | | | | | | | |
|---|---|---|---|---|---|---|---|---|
| * animal euthanized | | | | | | | | |

| Hematocrits (% or UL) for irradiated animals at day 19 to day 26 | | | | | | | | |
|---|---|---|---|---|---|---|---|---|
| day | 19 | 20 | 21 | 22 | 23 | 24 | 25 | 26 |
| animal | | | | | | | | |
| 1 | 0.31 | 0.30 | 0.29 | 0.28 | 0.33 | 0.30 | 0.31 | 0.32 |
| 2 | 0.32 | 0.32 | 0.32 | 0.31 | 0.34 | 0.33 | 0.34 | 0.34 |
| 3 | * | | | | | | | |
| 4 | * | | | | | | | |
| 5 | ** | ** | 0.14 | 0.14 | 0.12 | ** | 0.12 | * |
| 6 | ** | 0.26 | 0.25 | 0.25 | 0.24 | 0.25 | 0.26 | 0.28 |
| 7 | 0.28 | 0.26 | 0.25 | 0.25 | 0.25 | 0.26 | 0.27 | 0.29 |
| 8 | 0.20 | 0.20 | 0.20 | 0.20 | 0.22 | 0.23 | 0.24 | 0.26 |
| 9 | * | | | | | | | |
| 10 | 0.29 | * | | | | | | |
| mean | 0.28 | 0.27 | 0.24 | 0.23 | 0.25 | 0.27 | 0.26 | 0.30 |

| | | | | | | | | |
|---|---|---|---|---|---|---|---|---|
| * animal euthanized ** measurement not obtained | | | | | | | | |

| Platelets (x10⁻³/uL) for irradiated animals at day -6 to day 10 | | | | | | | | |
|---|---|---|---|---|---|---|---|---|
| day | -6 | 2 | 5 | 6 | 7 | 8 | 9 | 10 |
| animal | | | | | | | | |
| 1 | 608 | 525 | 580 | 538 | 433 | 324 | 232 | 111 |
| 2 | 547 | 397 | 406 | 401 | 324 | 255 | 174 | 113 |
| 3 | 363 | 313 | 356 | 315 | 221 | 169 | 101 | 45 |
| 4 | 295 | 266 | 267 | 253 | 180 | 141 | 71 | 28 |
| 5 | 472 | 325 | 336 | 316 | 273 | 203 | 117 | 22 |
| 6 | 400 | 410 | 443 | 386 | 290 | 193 | 103 | 26 |
| 7 | 485 | 438 | 385 | 489 | 409 | 353 | 275 | 175 |
| 8 | 472 | 380 | 401 | 342 | 305 | 235 | 145 | 59 |
| 9 | 510 | 363 | 307 | 370 | 261 | 109 | 46 | 20 |
| 10 | 419 | 381 | 478 | 409 | 327 | 185 | 79 | 36 |
| mean | 457 | 380 | 396 | 382 | 302 | 217 | 134 | 64 |

| Platelets (x10⁻³/uL) for irradiated animals at day 11 to day 18 | | | | | | | | |
|---|---|---|---|---|---|---|---|---|
| day | 11 | 12 | 13 | 14 | 15 | 16 | 17 | 18 |
| animal | | | | | | | | |
| 1 | 57 | 42 | 25 | 23 | 22 | 10 | 23 | 45 |
| 2 | 61 | 32 | 25 | 18 | 28 | 55 | 107 | 177 |
| 3 | 30 | 13 | 10 | 6 | 5 | 8 | 7 | 7 |
| 4 | 20 | 6 | 5 | 4 | 5 | * | | |
| 5 | 17 | 11 | 7 | 4 | 6 | 10 | 12 | 16 |
| 6 | 33 | 17 | 19 | 18 | 12 | 12 | 12 | 16 |
| 7 | 88 | 30 | 27 | 20 | 17 | 17 | 27 | 44 |
| 8 | 39 | 12 | 13 | 8 | 7 | 15 | 24 | 48 |
| 9 | 23 | 7 | 8 | 8 | 4 | 7 | 6 | 9 |
| 10 | 24 | 16 | 12 | 11 | 7 | 12 | 20 | 19 |
| mean | 39 | 19 | 15 | 12 | 11 | 16 | 26 | 42 |

| | | | | | | | | |
|---|---|---|---|---|---|---|---|---|
| * animal euthanized | | | | | | | | |

| Platelets (x10⁻³/uL) for irradiated animals at day 19 to day 26 | | | | | | | | |
|---|---|---|---|---|---|---|---|---|
| day | 19 | 20 | 21 | 22 | 23 | 24 | 25 | 26 |
| animal | | | | | | | | |
| 1 | 64 | 91 | 118 | 139 | 134 | 156 | 200 | 222 |
| 2 | 261 | 300 | 349 | 343 | 358 | 330 | 327 | 303 |
| 3 | * | | | | | | | |
| 4 | | | | | | | | |
| 5 | ** | ** | 44 | 53 | 74 | * | | |
| 6 | ** | 44 | 104 | 142 | 217 | 259 | 305 | 318 |
| 7 | 89 | 144 | 278 | 353 | 448 | 523 | 519 | 514 |
| 8 | 90 | 92 | 158 | 194 | 246 | 285 | 341 | 406 |
| 9 | * | | | | | | | |
| 10 | 12 | * | | | | | | |
| mean | 103 | 134 | 175.17 | 217.00 | 246.17 | 310.60 | 313.00 | 352.60 |

| | | | | | | | | |
|---|---|---|---|---|---|---|---|---|
| * animal euthanized ** measurement not obtained | | | | | | | | |

| Neutrophils (x10⁻³/mm³) for irradiated animals at day -6 to day 10 | | | | | | | | |
|---|---|---|---|---|---|---|---|---|
| day | -6 | 2 | 5 | 6 | 7 | 8 | 9 | 10 |
| animal | | | | | | | | |
| 1 | 4.30 | 2.59 | 1.07 | 0.58 | 0.34 | 0.39 | 0.40 | 0.39 |
| 2 | 5.10 | 6.08 | 2.10 | 1.41 | 0.28 | 0.27 | 0.34 | 0.36 |
| 3 | 8.17 | 5.09 | 2.05 | 1.02 | 0.76 | 0.68 | 0.60 | 0.48 |
| 4 | 9.46 | 6.98 | 0.68 | 0.30 | 0.25 | 0.32 | 0.30 | 0.22 |
| 5 | 3.01 | 4.45 | 2.53 | 0.76 | 0.29 | 0.28 | 0.35 | 0.13 |
| 6 | 2.07 | 4.55 | 2.39 | 0.80 | 0.33 | 0.30 | 0.38 | 0.27 |
| 7 | 5.94 | 6.01 | 1.19 | 0.79 | 0.34 | 0.35 | 0.54 | 0.36 |
| 8 | 2.59 | 2.50 | 1.13 | 0.28 | 0.15 | 0.26 | 0.28 | 0.14 |
| 9 | 3.62 | 6.36 | 0.46 | 0.25 | 0.31 | 0.43 | 0.57 | 0.21 |
| 10 | 3.22 | 5.34 | 1.30 | 0.46 | 0.37 | 0.34 | 0.31 | 0.13 |
| mean | 4.75 | 5.00 | 1.49 | 0.67 | 0.34 | 0.36 | 0.41 | 0.27 |

| Neutrophils (x10⁻³/mm³) for irradiated animals at day 11 to day 18 | | | | | | | | |
|---|---|---|---|---|---|---|---|---|
| day | 11 | 12 | 13 | 14 | 15 | 16 | 17 | 18 |
| animal | | | | | | | | |
| 1 | 0.17 | 0.10 | 0.08 | 0.14 | 0.17 | 0.09 | 0.10 | 0.08 |
| 2 | 0.30 | 0.15 | 0.10 | 0.10 | 0.07 | 0.07 | 0.19 | 0.46 |
| 3 | 0.13 | 0.09 | 0.12 | 0.09 | 0.04 | 0.05 | 0.07 | 0.02 |
| 4 | 0.16 | 0.11 | 0.04 | 0.06 | 0.03 | * | | |
| 5 | 0.07 | 0.09 | 0.06 | 0.07 | 0.02 | 0.04 | 0.10 | 0.24 |
| 6 | 0.13 | 0.10 | 0.13 | 0.09 | 0.06 | 0.06 | 0.05 | 0.05 |
| 7 | 0.24 | 0.19 | 0.10 | 0.06 | 0.12 | 0.20 | 0.12 | 0.15 |
| 8 | 0.11 | 0.06 | 0.05 | 0.05 | 0.03 | 0.05 | 0.18 | 0.69 |
| 9 | 0.13 | 0.16 | 0.11 | 0.06 | 0.08 | 0.05 | 0.06 | 0.04 |
| 10 | 0.09 | 0.09 | 0.05 | 0.07 | 0.03 | 0.04 | 0.07 | 0.05 |
| mean | 0.15 | 0.11 | 0.08 | 0.08 | 0.07 | 0.07 | 0.10 | 0.20 |

| | | | | | | | | |
|---|---|---|---|---|---|---|---|---|
| * animal euthanized | | | | | | | | |

| Neutrophils (x10⁻³/mm³) for irradiated animals at day 19 to day 26 | | | | | | | | |
|---|---|---|---|---|---|---|---|---|
| day | 19 | 20 | 21 | 22 | 23 | 24 | 25 | 26 |
| animal | | | | | | | | |
| 1 | 0.30 | 1.37 | 1.21 | 1.72 | 2.00 | 2.51 | 3.62 | 4.28 |
| 2 | 0.70 | 1.23 | 2.38 | 3.63 | 5.67 | 6.47 | 6.63 | 5.53 |
| 3 | * | | | | | | | |
| 4 | | | | | | | | |
| 5 | ** | ** | 2.57 | 4.51 | 4.60 | * | | |
| 6 | ** | 0.18 | 0.30 | 1.57 | 1.32 | 2.12 | 5.52 | 6.14 |
| 7 | 0.14 | 0.08 | 0.27 | 0.83 | 1.66 | 3.38 | 5.54 | 11.53 |
| 8 | 1.80 | 0.84 | 1.86 | 4.07 | 2.82 | 3.6 | 3.59 | 6.77 |
| 9 | * | | | | | | | |
| 10 | 0.04 | * | | | | | | |
| mean | 0.60 | 0.74 | 1.43 | 2.92 | 3.01 | 3.62 | 5.16 | 6.85 |

| | | | | | | | | |
|---|---|---|---|---|---|---|---|---|
| * animal euthanized ** measurement not obtained | | | | | | | | |

**Example 25.** Measurement of biological parameters in non-human primates after biological insult. A study was conducted to characterize a biological insult of 600cGy of whole body irradiation to male Rhesus (*Macaca mulatta*) primates weighing 2.5 to 4.5 kg at an age range of about 1.75 to 3.5 years. Core body temperature was monitored by telemetry in the monkeys for a period of 40 consecutive days. Two groups of 10 animals each were used in the study. Core body temperature transmitters were surgically implanted in the abdomen prior to initiation of the radiation protocol. Core body temperature was continuously recorded from day -7 to day 41 for correlation with survival, hematology results, and other clinical parameters.

Temperature transmitters. Before initiation of the temperature transmitter implantation protocol, all animals were subject to a detailed physical examination and body weight measurement under the direction of a clinical veterinarian. Blood was collected from all animals, which were not food and water deprived, and assessed for basic blood chemistry and hematology. The results of the evaluation was reviewed by the clinical veterinarian to ensure satisfactory health status. Implantation of the temperature transmitters was accomplished using animals that were fasted overnight prior to surgery and then anesthetized by an intra-muscular (IM) injection of acepromazine (10 mg/mL, 0.14 mg/kg) and ketamine (100 mg/mL, 13.6 mg/kg) and intubated. Where needed, lidocain spray (10% w/w) was administered onto the glottis prior to intubation. An ophthalmic ointment was applied to both eyes to prevent drying of the cornea. Animals were placed on a heating pad and administered isoflurane by inhalation, with an oxygen flow of approximately 200 mg/kg/min. A ventilator was used to maintain the respiratory rate between 8 and 20 breaths/min with a ventilation pressure of 18-25 cm H₂O. Monitoring during anesthesia included heart rate and oxygen saturation of the blood using a pulse oximeter. Prophylactic antibiotics (cefazolin 25 mg/kg) were administered by intramuscular injection at least 1-hour prior to surgery, and every 6 to 8 hours post injection for at least 24-hours post surgery. Analgesia (buprenorphine 0.05 mg/kg) was administered by intramuscular injection every 6 to 12 hours for at least 24-hours post surgery. Intravenous fluid therapy were given throughout the anesthesia using sterile Lactate Ringer's solution at a rate of 10 mL/kg/hr.

The surgical site was shaved and aseptically prepared using chlorhexidine gluconate 4% and isopropyl alcohol 70%. A longitudinal incision was performed lateral but close to the linea alba. The internal abdominal oblique muscle was separated from the aponeurosis of the transversus abdominis by blunt dissection. A sterile core body temperature transmitter (Data Science International, TA10TAD70) was inserted between the internal abdominal oblique muscle and the aponeurosis of the transversus abdominis. Hemostasis was maintained using appropriate suture material. Sterile saline was used to allow ease of placement of the transmitters. The incision was closed with absorbable suture material using simple continuous sutures. The skin was closed with discontinuous buried sutures using absorbable suture material. Additional post-operative cares (analgesia and antibiotics) was provided to the animals when needed. Rectal body temperature was monitored in the post-operative period. Once the body temperature was within an acceptable range and the animal was alert, each animal was returned to their cages. A postoperative period of at least 2 weeks was allowed prior to initiation of radiation.

Acclimation and whole-body irradiation. Before transportation to the radiation facility, the animals were acclimated to the radiotherapy chair and to transportation. During the acclimation period, animals were assigned to their respective dose groups by block randomization based on the absolute neutrophil count. Any animal with unacceptable pretreatment data was replaced by an animal kept under identical environmental conditions. Animals with pretreatment data considered acceptable but marginally different from normal values were assigned to the sham group to allow longer post-operative recovery.

Animals were fasted overnight prior to whole-body irradiation and fed upon return to the holding facility. Animals were transferred to the irradiation facility in a transport vehicle with controlled environment. During transportation, each animal was individually housed in a stainless steel squeeze back cage. The animal's clinical signs were monitored immediately before and after transportation. Group 1 animals, sham irradiated, were subject to the same irradiation procedure as Group 2 animals, however, these animals did receive radiation. The 10 control animals, Group 1, were sham irradiated by placing each animal in the restraint for 10 minutes. The 10 treated animals, Group 2, received a midline ⁶⁰Co γ-radiation dose of 6 Gy at a dose rate of about 60 cGy/minute (day 1). The animals receiving this 6 Gy radiation insult were restrained during the radiation exposure by placing each animal in a chair allowing appropriate restraining in a symmetric position. An insulated cover was placed on the radiotherapy chair during transportation between the transport vehicle and the treatment site. Music was provided inside the treatment room to reduce stress to the animals. Animal positioning was confirmed with linear markers installed in the treatment room. To produce a homogenous dose distribution, treatment was divided in two parts. First, the animal received half of the dose by anteroposterior (AP) irradiation. The second half of the dose was delivered by posteroanterior (PA) irradiation. Group 1 animals were placed in an identical restraining chair in the sham treatment site for approximately the same period of time without exposure to radiation. Once the treatment was completed, animals were returned to the transport vehicle and were transported to their housing facility. The radiation dose was calibrated using an acrylic phantom placed in the same experimental set up that was used for animal irradiation.

Animal maintenance. Animals were housed individually in stainless steel squeeze back cages equipped with an automatic watering system except during transportation where water bottles were provided. The cages were labeled with a color-coded cage card indicating study number, group, animal number, species, sex and dose level. The animal room environment was controlled (temperature 21 ± 3°C, humidity 30-70%, 10-15 air changes per hour, 12 hours light, 12 hours dark). Temperature and humidity were monitored continuously except during animal transportation and inside the radiation facility where only temperature was recorded. A standard certified commercial primate chow (Teklad Certified Global 25% Primate Diet # 2055C) was made available to each monkey daily. Food was withdrawn overnight prior to radiation and necropsy. Maximum allowable concentrations of contaminants in the diet (e.g., heavy metals, aflatoxin, organophosphates, chlorinated hydrocarbons and PCBs) were controlled and routinely analyzed by the manufacturers. If an animal stopped eating during the study, the diet was supplemented at the discretion of the study director. Tap water was purified by reverse osmosis and provided to the animals ad libitum throughout the study. Periodic analyses of the tap water and reverse osmosis water were performed. It was considered that there were no known contaminants in the diet or water. During the pre-treatment period cage side observations of clinical signs were generally performed once daily.

Observations. Mortality checks were performed twice a day during all phases of the study. Moribund animals were euthanized for humane reasons based on the clinical judgments. Sacrificed animals were subject to a clinical examination. When the core body temperature was 33° C (91.4° F) or lower or when an animal experienced a weight loss of more than 20% over a 4 day period, the animal was euthanized. Animals were also euthanized when they displayed complete anorexia for 3 days with deteriorating conditions based on the clinical examination or when they displayed an absence of response to stimuli.

Results obtained from the study were used to correlate the changes in biological parameters such as core body temperature and hematology with clinical signs following whole body irradiation. These results were used to obtain a status profile or surrogate endpoint such as incidence or duration of fever, followed by salvage with clinical support (antibiotics and blood transfusion), to assess the probability of survival or death of the treated individuals or similarly situated individuals that may have been subject to similar biological insults. During the pre-treatment period cage side observations of clinical signs were performed once daily. During the treatment period, clinical signs were recorded at cage-side twice daily for all animals or as often as deemed necessary. A detailed clinical examination was performed on all animals, once prior to irradiation on day 1, weekly thereafter, including on day 41 prior to necropsy.

The core body temperature and activity was recorded at 1 minute intervals for all animals from day -7 to day 41 using the implanted transmitter. Each animal cage was equipped with a telemetry receiver. The values of calibration of the transmitter implanted in each animal were entered in a telemetry computer system to ensure accurate temperature monitoring. Core body temperature was not recorded when animals were handled or during transport, but core temperature was generally monitored continuously at other times. Body weights were recorded for all animals once prior to randomization, prior to treatment on day 1 and weekly thereafter, including on day 40 (non-fasted) and on day 41 before necropsy. Hematology measurements were performed on all animals three times during the pre-treatment period and during the treatment period on days 2, daily from day 5 to day 27 and once on days 30, 33, 36 and 40. Blood samples of 0.5 mL were collected from the femoral vein or artery or from any appropriate vessel by venipuncture for hematological analysis. Food and water was available to the animals before blood collections.

Hematology parameters that were examined at most time points included red blood cell count, hematocrit, hemoglobin, white blood cell count, absolute differential WBC count, relative differential WBC count, relative reticulocyte count, mean corpuscular hemoglobin, platelet count, platelet volume, immature granulocyte count and red cell distribution width. EDTA was used as an anticoagulant and blood smears were prepared for each time point, stained with Modified Wright's stain and evaluated.

On day 41, the irradiated group 2 animals were sedated using ketamine and acepromazine and then euthanized by an overdose of barbiturate (e.g. sodium pentobarbital), which was administered intravenously, followed by exsanguination. For euthanized animals, gross pathology consisted of an external examination, identification of clinically recorded lesions and a detailed internal examination. To avoid autolytic changes, the necropsy examination was conducted as soon as possible on all animals that died while on study or that were euthanized during the study or at termination of the study at day 41. The animals were stored at 2-8 °C before examination. For all animals that were euthanized, the following organs were dissected, trimmed free of fat and weighed: Brain, testes, heart, prostate, kidneys, seminal vesicles, large intestine, small intestine, liver, spleen, lungs with trachea and thymus. The large intestine and small intestine were examined by making a longitudinal incision to open the lumen and removal of contents. The intestinal mucosa was washed with saline and excess saline was removed and the organs weighed. Paired organs were weighed together. Absolute and relative (to body weight) organ weights were calculated. On completion of the gross pathology examination, abnormal tissues brain (right part), femur and marrow, heart (both ventricles and atria, septum with papillary muscle), sternum and marrow, thymus were retained. Neutral buffered 10% formalin was usually used for fixation and preservation. Three femoral bone marrow smears were prepared from each euthanized animal (right femur), stained with Modified Wright's stain and evaluated.

Tissue samples from liver, lungs (right and left separately), kidneys, brain (left) and spleen were collected at necropsy from all euthanized animals for bacteriological culture. Tissue samples were stored refrigerated 2-8°C pending analysis. A selected area at the surface of the tissue sample was burned to eliminate possible surface contaminant. A sterile culture swab was inserted in the tissue sample through the burned surface for isolation and identification of aerobic and anaerobic bacteria. Histopathological examination was performed on the tissues from euthanized animals. Tissues were prepared for histological examination by embedding in paraffin wax, sectioning and staining with hematoxylin and eosinphloxin.

**Example 26.** Results and calculation of clinical status profiles for non-human primates using biological parameter measurements. Numerical data obtained from the protocol described in example 1 was subjected to calculation of group means, standard deviations and other statistical analyses.

Statistically significant status profiles were obtained based on five biological parameters, i.e., anemia (based on hematocrit), thrombocytopenia (platelets), neutropenia (neutrophils), elevated temperature and circadian rhythm disruption. Each parameter alone gave statistically significant P_{lethality} and Pₛᵤᵣᵥᵢᵥₐₗ status profiles. When hematocrit nadirs for individual animals fell below 20% of normal, 4 of 4 animals died, while 5 of 6 animals survived when individual hematocrits remained above 20%. Calculation by an unpaired t-test analysis gave P_{lethality} and Pₛᵤᵣᵥᵢᵥₐₗ status profiles of 0.02 for a mean hematocrit nadir of 16.4% and 25.6% respectively.

When platelets for individual animals fell to less than 7,000 per µL, 5 of 6 animals died, while 4 of 4 animals survived when the platelet count nadir remained above about 7,000 per µL. Calculation by an unpaired t-test analysis of P_{lethality} and Pₛᵤᵣᵥᵢᵥₐₗ status profiles of 0.01 for a mean platelet nadir of 4,800 platelets per µL blood and 12,800 platelets per µL blood, respectively.

When the neutrophil nadir for individual animals fell to less than 50 per µL, 5 of 6 animals died, while 4 of 4 animals survived when the neutrophil count nadir remained above 50 per µL. Calculation by an unpaired t-test analysis of P_{lethality} and Pₛᵤᵥᵢᵥₐₗ were 0.02 for a mean neutrophil nadir of 28 neutrophils per µL blood and 58 neutrophils per µL blood respectively.

For fever, P_{lethality} was less than 0.05 when the animals experienced fever or Pₛᵤᵣᵥᵢᵥₐₗ was greater than 0.95 when the animals did not have an elevated temperature or a fever. For this biological response, fever or elevated temperature was defined as a temperature of at least about 39.0°C for at least about 15 minutes within 12 hours after the animals were irradiated on day 1. The baseline temperature for the animals was considered to be 37.3°C, although temperatures for the 10 control (non-irradiated) animals in example 1 varied with the animal's circadian rhythm between about 36.8°C and 37.9 °C. The control animal's circadian core body temperature rhythm was quite regular, while irradiated animals that survived the radiation was relatively regular and was indistinguishable from non-irradiated controls by about 5-8 days after irradiation. However, circadian core body temperature rhythm from irradiated animals that did not survive the radiation was destroyed and did not recover at any time after its disruption. P_{lethality} was less than 0.05 when circadian rhythm was disrupted, and Pₛᵤᵣᵥᵢᵥₐₗ was greater than about 0.95 when circadian rhythm was not disrupted. The loss of circadian rhythm was detectable within 24 to 48 hours after the animals were exposed to the 6 Gy dose of γ-radiation.

The P_{lethality} and Pₛᵤᵣᵥᵢᵥₐₗ status profiles for platelets, hematocrit and neutrophils given above was obtained using an unpaired T-test analysis based on the animals described in example 1. Five of the irradiated animals in example 1 survived the 6 Gy radiation exposure and the hematocrit, platelet and neutrophil nadir from irradiated surviving animals (variable 1) was compared to the hematocrit, platelet and neutrophil nadir from the 5 irradiated non-survivors (variable 2).

| Hematocrit t-Test: Two-Sample Assuming Unequal Variances | | |
|---|---|---|
| | variable 1 | variable 2 |
| Mean | 25.6 | 16.4 |
| Variance | 17.3 | 30.8 |
| Observations | 5 | 5 |
| Hypothesized Mean Difference | 0 | |
| df | 7 | |
| t Stat | 2.9662 | |
| P(T<=t) one-tail | 0.0105 | |
| t Critical one-tail | 1.8946 | |
| P(T<=t) two-tail | 0.0209 | |
| t Critical two-tail | 2.3646 | |

| Platelet t-Test: Two-Sample Assuming Unequal Variances | | |
|---|---|---|
| | variable 1 | variable 2 |
| Mean | 12.8 | 4.8 |
| Variance | 21.7 | 1.7 |
| Observations | 5 | 5 |
| Hypothesized Mean Difference | 0 | |
| df | 5 | |
| t Stat | 3.698001 | |
| P(T<=t) one-tail | 0.007014 | |
| t Critical one-tail | 2.015049 | |
| P(T<=t) two-tail | 0.014028 | |
| t Critical two-tail | 2.570578 | |

| Neutrophil t-Test: Two-Sample Assuming Unequal Variances | | |
|---|---|---|
| | variable 1 | variable 2 |
| Mean | 0.058 | 0.028 |
| Variance | 0.00037 | 7E-05 |
| Observations | 5 | 5 |
| Hypothesized Mean Difference | 0 | |
| df | 5 | |
| t Stat | 3.19801 | |
| P(T<=t) one-tail | 0.01202 | |
| t Critical one-tail | 2.01505 | |
| P(T<=t) two-tail | 0.02405 | |
| t Critical two-tail | 2.57058 | |

For the 5 surviving animals, the hematocrit nadirs were 28, 31, 24, 25 and 20, while hematocrit nadirs for the non-surviving animals were 14, 16, 12, 14 and 26. For the 5 surviving animals, the platelet nadirs were 10 x 10³ per µL, 18 x 10³ per µL, 12 x 10³ per µL, 17 x 10³ per µL and 7 x 10³ per µL, while platelet nadirs for the non-surviving animals were 5 x 10³ per µL, 4 x 10³ per µL, 4 x 10³ per µL, 4 x 10³ per µL and 7 x 10³ per µL. For the 5 surviving animals, the neutrophil nadirs were 80 per mm³, 70 per mm³, 50 per mm³, 60 per mm³ and 30 per mm³, while neutrophil nadirs for the non-surviving animals were 20 per mm³, 30 per mm³, 20 per mm³, 40 per mm³ and 30 per mm³. The raw data for hematocrit, platelets and neutrophils from day -6 through day 26 are shown below and this data were used for the unpaired t-test P_{lethality} and Pₛᵤᵣᵥᵢᵥₐₗ calculations above.

| Hematocrits (% or UL) for irradiated animals at day -6 to day 10 | | | | | | | | |
|---|---|---|---|---|---|---|---|---|
| day | -6 | 2 | 5 | 6 | 7 | 8 | 9 | 10 |
| animal | | | | | | | | |
| 1 | 0.38 | 0.40 | 0.39 | 0.40 | 0.39 | 0.38 | 0.35 | 0.36 |
| 2 | 0.38 | 0.40 | 0.40 | 0.43 | 0.41 | 0.38 | 0.37 | 0.38 |
| 3 | 0.37 | 0.38 | 0.39 | 0.38 | 0.35 | 0.36 | 0.33 | 0.34 |
| 4 | 0.34 | 0.36 | 0.34 | 0.34 | 0.34 | 0.34 | 0.30 | 0.29 |
| 5 | 0.38 | 0.37 | 0.36 | 0.35 | 0.39 | 0.35 | 0.29 | 0.29 |
| 6 | 0.38 | 0.39 | 0.40 | 0.38 | 0.37 | 0.37 | 0.35 | 0.34 |
| 7 | 0.39 | 0.39 | 0.38 | 0.39 | 0.40 | 0.38 | 0.39 | 0.35 |
| 8 | 0.40 | 0.39 | 0.39 | 0.37 | 0.37 | 0.37 | 0.34 | 0.33 |
| 9 | 0.38 | 0.36 | 0.37 | 0.36 | 0.36 | 0.37 | 0.33 | 0.32 |
| 10 | 0.40 | 0.43 | 0.42 | 0.39 | 0.37 | 0.38 | 0.36 | 0.33 |
| mean | 0.38 | 0.39 | 0.38 | 0.38 | 0.38 | 0.37 | 0.34 | 0.33 |

| Hematocrits (% or UL) for irradiated animals at day 11 to day 18 | | | | | | | | |
|---|---|---|---|---|---|---|---|---|
| day | 11 | 12 | 13 | 14 | 15 | 16 | 17 | 18 |
| animal | | | | | | | | |
| 1 | 0.35 | 0.35 | 0.36 | 0.36 | 0.34 | 0.36 | 0.32 | 0.33 |
| 2 | 0.36 | 0.37 | 0.36 | 0.38 | 0.35 | 0.32 | 0.32 | 0.31 |
| 3 | 0.31 | 0.26 | 0.28 | 0.28 | 0.21 | 0.19 | 0.16 | 0.14 |
| 4 | 0.33 | 0.27 | 0.25 | 0.21 | 0.16 | * | | |
| 5 | 0.29 | 0.26 | 0.25 | 0.25 | 0.20 | 0.20 | 0.17 | 0.15 |
| 6 | 0.35 | 0.34 | 0.32 | 0.33 | 0.31 | 0.28 | 0.29 | 0.27 |
| 7 | 0.34 | 0.35 | 0.33 | 0.32 | 0.29 | 0.28 | 0.28 | 0.27 |
| 8 | 0.32 | 0.33 | 0.31 | 0.27 | 0.26 | 0.24 | 0.24 | 0.20 |
| 9 | 0.33 | 0.30 | 0.30 | 0.27 | 0.21 | 0.18 | 0.16 | 0.14 |
| 10 | 0.34 | 0.35 | 0.31 | 0.30 | 0.29 | 0.27 | 0.26 | 0.26 |
| mean | 0.33 | 0.32 | 0.31 | 0.30 | 0.26 | 0.26 | 0.24 | 0.23 |

| | | | | | | | | |
|---|---|---|---|---|---|---|---|---|
| * animal euthanized | | | | | | | | |

| Hematocrits (% or UL) for irradiated animals at day 19 to day 26 | | | | | | | | |
|---|---|---|---|---|---|---|---|---|
| day | 19 | 20 | 21 | 22 | 23 | 24 | 25 | 26 |
| animal | | | | | | | | |
| 1 | 0.31 | 0.30 | 0.29 | 0.28 | 0.33 | 0.30 | 0.31 | 0.32 |
| 2 | 0.32 | 0.32 | 0.32 | 0.31 | 0.34 | 0.33 | 0.34 | 0.34 |
| 3 | * | | | | | | | |
| 4 | * | | | | | | | |
| 5 | ** | ** | 0.14 | 0.14 | 0.12 | ** | 0.12 | * |
| 6 | ** | 0.26 | 0.25 | 0.25 | 0.24 | 0.25 | 0.26 | 0.28 |
| 7 | 0.28 | 0.26 | 0.25 | 0.25 | 0.25 | 0.26 | 0.27 | 0.29 |
| 8 | 0.20 | 0.20 | 0.20 | 0.20 | 0.22 | 0.23 | 0.24 | 0.26 |
| 9 | * | | | | | | | |
| 10 | 0.29 | * | | | | | | |
| mean | 0.28 | 0.27 | 0.24 | 0.23 | 0.25 | 0.27 | 0.26 | 0.30 |

| | | | | | | | | |
|---|---|---|---|---|---|---|---|---|
| * animal euthanized ** measurement not obtained | | | | | | | | |

| Platelets (x10⁻³/µL) for irradiated animals at day -6 to day 10 | | | | | | | | |
|---|---|---|---|---|---|---|---|---|
| day | -6 | 2 | 5 | 6 | 7 | 8 | 9 | 10 |
| animal | | | | | | | | |
| 1 | 608 | 525 | 580 | 538 | 433 | 324 | 232 | 111 |
| 2 | 547 | 397 | 406 | 401 | 324 | 255 | 174 | 113 |
| 3 | 363 | 313 | 356 | 315 | 221 | 169 | 101 | 45 |
| 4 | 295 | 266 | 267 | 253 | 180 | 141 | 71 | 28 |
| 5 | 472 | 325 | 336 | 316 | 273 | 203 | 117 | 22 |
| 6 | 400 | 410 | 443 | 386 | 290 | 193 | 103 | 26 |
| 7 | 485 | 438 | 385 | 489 | 409 | 353 | 275 | 175 |
| 8 | 472 | 380 | 401 | 342 | 305 | 235 | 145 | 59 |
| 9 | 510 | 363 | 307 | 370 | 261 | 109 | 46 | 20 |
| 10 | 419 | 381 | 478 | 409 | 327 | 185 | 79 | 36 |
| mean | 457 | 380 | 396 | 382 | 302 | 217 | 134 | 64 |

| Platelets (x10⁻³/µL) for irradiated animals at day 11 to day 18 | | | | | | | | |
|---|---|---|---|---|---|---|---|---|
| day | 11 | 12 | 13 | 14 | 15 | 16 | 17 | 18 |
| animal | | | | | | | | |
| 1 | 57 | 42 | 25 | 23 | 22 | 10 | 23 | 45 |
| 2 | 61 | 32 | 25 | 18 | 28 | 55 | 107 | 177 |
| 3 | 30 | 13 | 10 | 6 | 5 | 8 | 7 | 7 |
| 4 | 20 | 6 | 5 | 4 | 5 | * | | |
| 5 | 17 | 11 | 7 | 4 | 6 | 10 | 12 | 16 |
| 6 | 33 | 17 | 19 | 18 | 12 | 12 | 12 | 16 |
| 7 | 88 | 30 | 27 | 20 | 17 | 17 | 27 | 44 |
| 8 | 39 | 12 | 13 | 8 | 7 | 15 | 24 | 48 |
| 9 | 23 | 7 | 8 | 8 | 4 | 7 | 6 | 9 |
| 10 | 24 | 16 | 12 | 11 | 7 | 12 | 20 | 19 |
| mean | 39 | 19 | 15 | 12 | 11 | 16 | 26 | 42 |

| | | | | | | | | |
|---|---|---|---|---|---|---|---|---|
| * animal euthanized | | | | | | | | |

| Platelets (x10⁻³/µL) for irradiated animals at day 19 to day 26 | | | | | | | | |
|---|---|---|---|---|---|---|---|---|
| day | 19 | 20 | 21 | 22 | 23 | 24 | 25 | 26 |
| animal | | | | | | | | |
| 1 | 64 | 91 | 118 | 139 | 134 | 156 | 200 | 222 |
| 2 | 261 | 300 | 349 | 343 | 358 | 330 | 327 | 303 |
| 3 | * | | | | | | | |
| 4 | | | | | | | | |
| 5 | ** | ** | 44 | 53 | 74 | * | 186 | |
| 6 | ** | 44 | 104 | 142 | 217 | 259 | 305 | 318 |
| 7 | 89 | 144 | 278 | 353 | 448 | 523 | 519 | 514 |
| 8 | 90 | 92 | 158 | 194 | 246 | 285 | 341 | 406 |
| 9 | * | | | | | | | |
| 10 | 12 | * | | | | | | |
| mean | 103 | 134 | 175.17 | 217.00 | 246.17 | 310.60 | 313.00 | 352.60 |

| | | | | | | | | |
|---|---|---|---|---|---|---|---|---|
| * animal euthanized ** measurement not obtained | | | | | | | | |

| Neutrophils (x10⁻³/mm³) for irradiated animals at day -6 to day 10 | | | | | | | | |
|---|---|---|---|---|---|---|---|---|
| day | -6 | 2 | 5 | 6 | 7 | 8 | 9 | 10 |
| animal | | | | | | | | |
| 1 | 4.30 | 2.59 | 1.07 | 0.58 | 0.34 | 0.39 | 0.40 | 0.39 |
| 2 | 5.10 | 6.08 | 2.10 | 1.41 | 0.28 | 0.27 | 0.34 | 0.36 |
| 3 | 8.17 | 5.09 | 2.05 | 1.02 | 0.76 | 0.68 | 0.60 | 0.48 |
| 4 | 9.46 | 6.98 | 0.68 | 0.30 | 0.25 | 0.32 | 0.30 | 0.22 |
| 5 | 3.01 | 4.45 | 2.53 | 0.76 | 0.29 | 0.28 | 0.35 | 0.13 |
| 6 | 2.07 | 4.55 | 2.39 | 0.80 | 0.33 | 0.30 | 0.38 | 0.27 |
| 7 | 5.94 | 6.01 | 1.19 | 0.79 | 0.34 | 0.35 | 0.54 | 0.36 |
| 8 | 2.59 | 2.50 | 1.13 | 0.28 | 0.15 | 0.26 | 0.28 | 0.14 |
| 9 | 3.62 | 6.36 | 0.46 | 0.25 | 0.31 | 0.43 | 0.57 | 0.21 |
| 10 | 3.22 | 5.34 | 1.30 | 0.46 | 0.37 | 0.34 | 0.31 | 0.13 |
| mean | 4.75 | 5.00 | 1.49 | 0.67 | 0.34 | 0.36 | 0.41 | 0.27 |

| Neutrophils (x10⁻³/mm³) for irradiated animals at day 11 to day 18 | | | | | | | | |
|---|---|---|---|---|---|---|---|---|
| day | 11 | 12 | 13 | 14 | 15 | 16 | 17 | 18 |
| animal | | | | | | | | |
| 1 | 0.17 | 0.10 | 0.08 | 0.14 | 0.17 | 0.09 | 0.10 | 0.08 |
| 2 | 0.30 | 0.15 | 0.10 | 0.10 | 0.07 | 0.07 | 0.19 | 0.46 |
| 3 | 0.13 | 0.09 | 0.12 | 0.09 | 0.04 | 0.05 | 0.07 | 0.02 |
| 4 | 0.16 | 0.11 | 0.04 | 0.06 | 0.03 | * | | |
| 5 | 0.07 | 0.09 | 0.06 | 0.07 | 0.02 | 0.04 | 0.10 | 0.24 |
| 6 | 0.13 | 0.10 | 0.13 | 0.09 | 0.06 | 0.06 | 0.05 | 0.05 |
| 7 | 0.24 | 0.19 | 0.10 | 0.06 | 0.12 | 0.20 | 0.12 | 0.15 |
| 8 | 0.11 | 0.06 | 0.05 | 0.05 | 0.03 | 0.05 | 0.18 | 0.69 |
| 9 | 0.13 | 0.16 | 0.11 | 0.06 | 0.08 | 0.05 | 0.06 | 0.04 |
| 10 | 0.09 | 0.09 | 0.05 | 0.07 | 0.03 | 0.04 | 0.07 | 0.05 |
| mean | 0.15 | 0.11 | 0.08 | 0.08 | 0.07 | 0.07 | 0.10 | 0.20 |

| | | | | | | | | |
|---|---|---|---|---|---|---|---|---|
| * animal euthanized | | | | | | | | |

| Neutrophils (x10⁻³/mm³) for irradiated animals at day 19 to day 26 | | | | | | | | |
|---|---|---|---|---|---|---|---|---|
| day | 19 | 20 | 21 | 22 | 23 | 24 | 25 | 26 |
| animal | | | | | | | | |
| 1 | 0.30 | 1.37 | 1.21 | 1.72 | 2.00 | 2.51 | 3.62 | 4.28 |
| 2 | 0.70 | 1.23 | 2.38 | 3.63 | 5.67 | 6.47 | 6.63 | 5.53 |
| 3 | * | | | | | | | |
| 4 | | | | | | | | |
| 5 | ** | ** | 2.57 | 4.51 | 4.60 | * | 6.04 | |
| 6 | ** | 0.18 | 0.30 | 1.57 | 1.32 | 2.12 | 5.52 | 6.14 |
| 7 | 0.14 | 0.08 | 0.27 | 0.83 | 1.66 | 3.38 | 5.54 | 11.53 |
| 8 | 1.80 | 0.84 | 1.86 | 4.07 | 2.82 | 3.6 | 3.59 | 6.77 |
| 9 | * | | | | | | | |
| 10 | 0.04 | * | | | | | | |
| mean | 0.60 | 0.74 | 1.43 | 2.92 | 3.01 | 3.62 | 5.16 | 6.85 |

| | | | | | | | | |
|---|---|---|---|---|---|---|---|---|
| * animal euthanized ** measurement not obtained | | | | | | | | |

**Example 3.** Treatment of whole body lethal radiation and characterization of mortality surrogate markers. Two groups of 10 *Macaca mulatta* (Rhesus monkey) were exposed to a 6 Gy dose of γ-radiation from a ⁶⁰Co source. This dose is an LD_{50/30} dose for this species. After irradiation, one group of animals was treated with test article, 15 mg/kg of 3β,17β-dihydroxyandrost-5-ene ("AED") in vehicle, and the other 10-animal group was treated with the vehicle alone. The animals in each group were treated once per day for 5 consecutive days beginning on the day the animals were exposed to radiation. The animals consisted of 12 males and 8 females with a body weight range of about 2.5-5.5 kg at the onset of treatment. The age range was 1.75-5.0 years at the onset of treatment. Procedures involving the care and use of animals in this protocol was reviewed and approved by the Institutional Animal Care and Use Committee before conduct. During the study, the care and use of animals were conducted in accordance with the applicable rules and codes.

The animals were housed individually in stainless steel squeeze back cages equipped with an automatic watering system except during transportation where water bottles were provided. The cages were clearly labeled with a color-coded cage card indicating study number, group, animal number, species, sex and dose level. The animal room environment was controlled (temperature 21 ± 3°C, humidity 30-70%, 10-15 air changes per hour, 12 hours light, 12 hours dark). Temperature and humidity was monitored continuously except during animal transportation and inside the radiation facility where only temperature was recorded. During transportation, only temperature was controlled. Air was not filtered during animal transportation and inside the radiation facility. A standard certified commercial primate chow (Teklad Certified Global 25% Primate Diet # 2055C) was made available to each monkey daily. Food was withdrawn overnight prior to radiation and necropsy. Maximum allowable concentrations of contaminants in the diet (e.g., heavy metals, aflatoxin, organophosphates, chlorinated hydrocarbons and PCBs) were controlled and routinely analyzed by the manufacturer. When an animal became inappetent during the study, the diet could be supplemented.

Tap water purified by reverse osmosis was provided to the animals *ad libitum* throughout the study. There were no known contaminants in the diet or water. Before transportation to the radiation facility, animals were acclimated to the radiotherapy chair and to transportation. Positive reinforcement was used to facilitate acclimation. Certified non human primate treats were given after acclimation periods. Twelve male and eight female rhesus monkeys were assigned to the study. Each group comprised of seven male and three female animals. During the acclimation period, animals were assigned to their respective dose groups by randomization based on the absolute neutrophil count. The average of 3 pretreatment absolute neutrophil counts were used for each animal.

The test article (100 mg/mL 3β, 17β-dihydroxyandrost-5-ene) and vehicle or control article in aliquots of 10 mL. Test article was an aqueous suspension in vehicle. The vehicle article consists of a solution of sodium chloride (0.9% w/v), carboxymethylcellulose (0.5% w/v), polysorbate 80 (2 % v/v), benzalkonium chloride (0.02 % v/v) and sodium phosphate (10 mM, pH 6.5). Immediately prior to drawing into a syringe, the test article formulation was briefly vortexed to uniformly distribute sedimented test article. Once drawn into a syringe, the test article was administered within 10 minutes. Just prior to injection, the syringe containing the test article was rotated end-over-end to uniformly disperse the compound.

During the pre-treatment period, body temperature transmitters were surgically implanted to allow core body temperature and physical activity monitoring. The animals were fasted overnight before the implant surgery. The animals were anesthetized by an intra-muscular injection of acepromazine (10 mg/mL, 0.14 mg/kg) and ketamine (100 mg/mL, 13.6 mg/kg) and intubated. Where needed, lidocain spray (10% w/w) was administered onto the glottis prior to intubation. An ophthalmic ointment was applied to both eyes to prevent drying of the cornea. Animals were then placed on a heating pad and administered isoflurane by inhalation, with an oxygen flow of approximately 200 mL/kg/min or as needed. A ventilator was used to maintain the respiratory rate between 8 and 20 breaths/min with a ventilation pressure of 18-25 cm H₂O. Monitoring during anesthesia included heart rate and oxygen saturation of the blood using a pulse oximeter.

Prophylactic antibiotics (cefazolin 25 mg/kg) were administered by intramuscular injection at least 1-hour prior to surgery, and every 4 to 8 hours post injection for at least 24-hours post surgery. Analgesia (buprenorphine 0.05 mg/kg) was administered by intramuscular injection every 6 to 12 hours for at least 24-hours post surgery. Intravenous fluid therapy was given throughout the anesthesia using sterile Lactate Ringer's solution at a rate of 10ml/kg/hr. The surgical site was shaved and was aseptically prepared using chlorhexidine gluconate 4% and isopropyl alcohol 70%. A longitudinal incision was performed lateral but close to the linea alba. The internal abdominal oblique muscle was separated from the aponeurosis of the transversus abdominis by blunt dissection. A sterile core body temperature transmitter (Data Science International, TA10TAD70) was inserted between the internal abdominal oblique muscle and the aponeurosis of the transversus abdominis. The serial number of the transmitter was recorded. Hemostasis was maintained using appropriate suture material. Sterile saline was used to allow ease of placement of the transmitters. The incision was closed with absorbable suture material using simple continuous sutures. The skin was closed with discontinuous buried sutures using absorbable suture material. Additional post-operative care (analgesia and antibiotics) were given to all animals where required. Rectal body temperature was monitored in the post-operative period. Once the body temperature was within an acceptable range and the animal was alert, each animal was returned to its cage. A post-operative period of at least 2 weeks was allowed prior to initiation of treatment. Core body temperature was monitored at 1 minute intervals beginning 6 days before radiation exposure and continued until 40 days after exposure.

Whole body radiation. The animals were exposed to ionizing as follows. Dosimetry measurements using phantoms, the dose rate and duration of irradiation and the actual time of irradiation for each individual animal was recorded. Animals were fasted overnight prior to whole-body irradiation and fed upon return to the housing facility. Animals were transferred to the treatment facility in a transport vehicle with controlled environment. During transportation, each animal was individually housed in a stainless steel squeeze back cage. Temperature in the transport vehicle was automatically recorded every 5 minutes during transportation. Clinical signs were monitored immediately before and after transportation.

Upon arrival to the site of irradiation, each animal was placed in a chair allowing appropriate restraining in a symmetric position. An insulated cover was placed on the radiotherapy chair during transportation between the truck and the treatment room. Each animal was brought in the treatment room. Music was provided inside the treatment room to reduce stress to the animals. Animal positioning was confirmed with linear markers installed in the treatment room.

The animals received a midline treatment dose of 600 cGy. The dose rate of the ⁶⁰Co gamma source was about 60 cGy per minute and the actual rate was recorded for each animal. To obtain a homogenous dose distribution, the radiation treatment was divided in two parts. First, the animal received half of the dose by anteroposterior irradiation. The second half of the dose was delivered by posteroanterior irradiation. Once the treatment was completed, animals were returned to the transport vehicle and transported to the housing facility. The radiation dose was calibrated using an acrylic phantom placed in the same experimental set up that was used for animal irradiation.

Administration of 3β,17β-dihydroxyandrost-5-ene and vehicle control. The animals received the vehicle control once daily for five consecutive days by intramuscular injections. The first injection on day 1 was administered at 2-3 hours after irradiation. The dose volume was 0.15 mL/kg for all animals. The dose volume was evenly divided between two distinct sites (approximately 0.075 mL/kg per site). The actual volume delivered was calculated and adjusted based on each animal's body weight. To verify the concentration and homogeneity of the test and control articles in the dosing formulation, duplicate samples (1 mL/sample) from the bottom of each dosing formulation was taken prior to dosing on days 1, 2, 3, 4 and 5 and stored frozen (-70 ± 10°C) pending analysis.

During the pre-treatment period cage side observations of clinical signs were performed once daily. A detailed clinical examination was performed on all animals once prior to irradiation on day 1, day 9, weekly thereafter and at day 40 and 41. After radiation, the animals and clinical signs were observed twice a day during the protocol or as often as deemed necessary. Moribund animals were euthanized for humane reasons. Euthanasia criteria consisted of (i) a core body temperature of 35.9° C after a period of febrile neutropenia, (ii) more than a 20% weight loss over a 3 day period, (iii) complete anorexia for 3 days with deteriorating conditions based on clinical examination or (iv) absence of response to stimuli.

Core body temperature and activity was recorded every minute for all animals from Day -10 to sacrifice using the implanted transmitter. Core body temperature and activity was recorded when animals were housed in their designated cage. Each designated cage was equipped with a telemetry receiver. Core body temperature was not recorded when animals were handled or during transport to the radiation facility. Body weights were recorded for all animals on the day following transfer, once before randomization, prior to treatment on day 1, day 9, weekly thereafter, at on the day the protocol ended. Laboratory hematology investigations were performed on all animals three times during the pre-treatment period and during the treatment period on day 2, daily from day 5 to day 27 and once on days 30, 33, 36 and 40.

For hematology analyses, blood samples of 0.5 mL were collected from the femoral vein or artery or from any appropriate vessel by venipuncture. EDTA was used as an anticoagulant. Animals were not deprived of food or water prior to blood collections. Parameters such as red blood cell count, hematocrit, hemoglobin, mean corpuscular volume, red blood cell count, mean corpuscular hemoglobin, white blood cell count, WBC differential (absolute), platelet count, WBC differential (relative), red cell distribution width, reticulocyte count and immature granulocyte count were measured. Blood smears were prepared for each time point, stained with Modified Wright's stain and evaluated.

For pharmacokinetic evaluation, blood samples (approximately 1.0 mL) were collected from all animals at about 22.0 to 23.5 hours following the first compound and control vehicle article administration on day 2. Each blood sample was collected into an EDTA potassium tube and kept on wet ice, for a maximum of 30 minutes, until centrifugation. The samples were centrifuged under refrigeration (2 to 8°C) for approximately 10 minutes at 1500 g (RCF). The harvested plasma was transferred in one aliquot per sample. Blood samples (approximately 2.0 mL) were collected from all animals prior to sacrifice on days 40 and 41. Each blood sample was collected into an EDTA potassium tube and kept on wet ice, for a maximum of 30 minutes, until centrifugation. The samples were centrifuged under refrigeration (2 to 8°C) for approximately 10 minutes at 1500 g (RCF). The harvested plasma was transferred in two separate aliquots per sample.

Liver, lung (right and left separately), kidney, brain (left) and spleen tissues were collected at necropsy from all euthanized animals for bacteriological culture. The tissue samples were stored refrigerated (2-10 °C) pending analysis. A selected area at the surface of the tissue sample was burned to eliminate possible surface contaminants. A sterile culture swab was inserted in the tissue sample through the burned surface for isolation and identification of aerobic and anaerobic bacteria.

Numerical data obtained during the conduct of the study was subjected to calculation of group means and standard deviations. Data was analyzed using the Analysis of Variance (ANOVA) and the significance of inter-group differences were analyzed by Dunnett's "t" test or other appropriate tests using the SPSS for Windows, version 12.0, SPSS, Inc.

In the vehicle-treated control animal group, 4 of 10 animals survived, with 3 of the four non-survivors having febrile severe neutropenia, which was defined as a core body temperature of > 40.4°C, i.e., ≥ 40.5°C, and an absolute neutrophil count of less than 500 cells/µL. In the 3β,17β-dihydroxyandrost-5-ene treated animal group, 9 of 10 animals survived, with the non-survivor not having febrile severe neutropenia and 2 survivors having the condition at some time during the protocol. Mortality in the untreated control group thus was 40% and 10% in the treated group. When the two control groups from this protocol and from the protocol described in examples 1 and 2 were combined, the total combined mortality of the 20 irradiated untreated animals was 45%. A reduction of mortality was observed (Fisher's exact test mid p = 0.073) in the treated group compared to these two control groups. In the control group from this example only (vehicle treated) the animals experienced a median of 5 days of febrile severe neutropenia (95% CI 0,8) while the animals treated with 3β, 17β-dihydroxyandrost-5-ene experienced a median of 0 days of febrile severe neutropenia (95% CI 0,2), giving a p = 0.037 by the exact log rank test.

In the vehicle treated control animals from this example the animals collectively experienced 51 days of severe thrombocytopenia, less than 20,000 platelets/µL, while the animals treated with 3β,17β-dihydroxyandrost-5-ene collectively experienced 32 days of severe thrombocytopenia. This difference was p = 0.009 by the exact test of homogeneity.

To the extent not already indicated, it will be understood by those of ordinary skill in the art that any of the various specific embodiments, compounds or compositions described herein may be modified to incorporate other appropriate features, e.g., as shown in any other of the specific embodiments disclosed herein or in the cited references.

The following embodiments of the invention are numbered as embodiments 1 to 11 and relate to:
1. A method to characterize the capacity of a formula 1 compound to increase survival of a nonhuman primate that has been exposed to radiation comprising;
   (1) exposing a group of nonhuman primates to a radiation dose of about an LD_{50/30} or about an LD_{60/30} to obtain exposed subjects and administering a formula 1 compound treatment to the exposed subjects to obtain exposed treated subjects, wherein the exposed treated subjects are not provided with another treatment selected from a transfusion such as a whole blood transfusion(s), a platelet transfusion(s), or an immunoglobulin transfusion, an antimicrobial treatment(s) to treat or prevent an infection and assisted feeding such as feeding by parenteral feeding or catheter or by tube feeding to the stomach;
   (2) determining the survival rate of the exposed treated subjects; and
   (3) comparing the effect of the F1C on the survival rate of the exposed treated subjects with the survival rate of control subjects of the same or a closely related species that had been exposed to the same or a similar or a comparable biological insult to obtain comparison controls, where the comparison controls were treated with a comparison compound in a comparison treatment protocol whereby the comparison treatment protocol with the comparison compound detectably increased the survival rate of the subjects that had been exposed to the same or similar or comparable biological insult, wherein the formula 1 compound has the structure 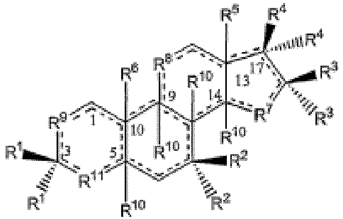
      wherein the dotted lines are optional double bonds and 0, 1 , 2, 3, 4 or 5 double bonds are present;
      each R¹, R², R³, R⁴, R⁵, R⁶ and R¹⁰ independently or together are -H, -OH, -OR^{PR}, -SR^{PR}, -SH, -N(R^{PR})₂, -NHR^{PR}, -NH₂, -O-Si-(R¹³)₃, -CHO, -CHS, -CN, -SCN, -NO₂, -N₃, -COOH, - COOR^{PR}, -OSO₃H, -OSO₂H, -OPO₃H₂, =O, =S, =N-OH, =N-OCH₃, =CH₂, =CH-CH₃, =CH-optionally substituted alkyl, =N-optionally substituted alkyl, =N-O-optionally substituted alkyl, -NH-S(O)(O)-optionally substituted alkyl, -S-S-optionally substituted alkyl, ester, thioester, thionoester, phosphoester, phosphothioester, phosphonate, phosphonate ester, thiophosphonate, thiophosphonate ester, phosphiniester, sulfite ester, sulfate ester, sulfamate, sulfonate, sulfonamide, amide, amino acid, peptide, ether, thioether, acyl, thioacyl, carbonate, carbamate, halogen, optionally substituted alkyl, optionally substituted alkenyl, optionally substituted alkynyl, optionally substituted aryl, optionally substituted heteroaryl, optionally substituted heterocycle, optionally substituted monosaccharide, optionally substituted oligosaccharide, polymer, spiro ring, epoxide, acetal, thioacetal, ketal or a thioketal, =N-O-optionally substituted alkyl, =N-optionally substituted alkyl, -NH-optionally substituted alkyl, -N(optionally substituted alkyl)₂ where each optionally substituted alkyl is independently selected, or, one or more of two adjacent R¹, R², R³, R⁴, R⁵, R⁶ and R¹⁰ comprise an independently selected epoxide or optionally substituted, saturated or unsaturated cyclopropyl, cyclobutyl, cyclopentyl or cyclohexyl ring any of which rings optionally contain one or two independently selected -O-, -S-, -S(O)(O)-, -NH- -N(optionally substituted alkyl)- or =N-heteroatoms;
      R⁷ is -O-, -S-, -NR^{PR}-, -C(R¹⁰)₂, -C(R¹⁰)₂-C(R¹⁰)₂-, -C(R¹⁰)₂-C(R¹⁰)₂-C(R¹⁰)₂, -C(R¹⁰)₂-O-C(R¹⁰)₂,₋-C(R¹⁰)₂-S-C(R¹⁰)₂, -C(R¹⁰)₂-NR^{PR}-C(R¹⁰)₂, O-C(R¹⁰)₂, -S-C(R¹⁰)₂ or -NR^{PR}-C(R¹⁰)₂, where each R¹⁰ is independently selected;
      R⁸ and R⁹ independently are -C(R¹⁰)₂, -C(R¹⁰)₂-C(R¹⁰)₂, -O-, -O-C(R¹⁰)₂, -S-, -S-C(R¹⁰)₂, -NR^{PR}- or -NR^{PR}-C(R¹⁰)₂ or one or both of R⁸ or R⁹ independently are absent, leaving a 5-membered ring, where each R¹⁰ is independently selected;
      R¹¹ is -O-, -S-, -S(O)(O)-, -NR^{PR}-, -CH₂-, -CHR¹⁰-, -C(R¹⁰)₂, -C(R¹⁰)₂-O-C(R¹⁰)₂, -C(R¹⁰)₂-S-C(R¹⁰)₂, -C(R¹⁰)₂-S(O)(O)-C(R¹⁰)₂, -C(R¹⁰)₂-NR^{PR}-C(R¹⁰)₂, O-C(R¹⁰)₂,-S-C(R¹⁰)₂,-S(O)(O)-C(R¹⁰)₂ or -NR^{PR}-C(R¹⁰)₂ where each R¹⁰ is independently selected;
      R¹³ independently is C₁₋₆ alkyl; and
      R^{PR} independently are -H or a protecting group, wherein one or two independently selected R¹⁰ moieties are present at the 1-, 6- and 12-positions.
2. The method of embodiment 1 wherein the formula 1 compound is not 3β,17β-dihydroxyandrost-5-ene, the comparison compound is 3β,17β-dihydroxyandrost-5-ene and the comparison treatment protocol is administering once per day to the nonhuman primates about 8 mg/kg/day to about 50 mg/kg/day of the 3β,17β-dihydroxyandrost-5-ene for 1, 2, 3, 4, 5, 6, 7, 8, 9 or 10 consecutive days wherein the administration of the 3β,17β-dihydroxyandrost-5-ene begins immediately after the radiation exposure or within about 15 minutes to about 6 hours after the radiation exposure.
3. The method of embodiment 2 wherein the radiation dose is about 600 cGy to about 640 cGy, the nonhuman primate is a rhesus monkey and the comparison treatment protocol increased survival of the control subjects by about 5%, about 10%, about 15%, about 20% or more.
4. The method of embodiment 3 wherein the formula 1 compound treatment comprises administering about 0.1 mg/kg/day to about 80 mg/kg/day of the formula 1 compound for 1 , 2, 3, 4, 5, 6, 7, 8, 9 or 10 consecutive days wherein the administration begins immdeiately after the radiation exposure or within about 15 minutes to about 6 hours after the radiation exposure.
5. The method of embodiment 4 wherein the radiation is whole body radiation exposure to γ-radiation, X-radiation, β-radiation, fast neutrons or slow neutrons.
6. The method of embodiment 4 wherein the formula 1 compound is a 1,5,9(11 )-triene, 1,5,11-triene, 1,5,14-triene, 1,5,15-triene, 1,3,14-triene, 1,3,8(9)-triene, 1,3,8(14)-triene, 1,3,9(11)-triene, 1,3,11-triene or a 1,3,14-triene, optionally wherein one R¹ is an O-linked moiety or an N-linked moiety, the other R¹ is -H or a C-linked moiety, or, if a double bond is present at the 3-position, R¹ is O-linked moiety or an N-linked moiety, R⁴ in the β-configuration is an O-linked moiety, R⁴ in the α-configuration is -H or a C-linked moiety and one or both R³ independently or together are -H, -OH, =O, an O-linked moiety, halogen or a C-linked moiety .
7. The method of embodiment 1 wherein the group of nonhuman primates of step (a) contains 2, 3, 4, 5, 6, 7, 8, 9, 10, 11 or 12 or more nonhuman primates.
8. The method of embodiment 1 further comprising comparing the survival rate of the exposed treated subjects with the survival rate of nonhuman primates of the same or a closely related species that had been exposed to the same or comparable radiation dose, where such untreated subjects had not been treated with the formula 1 compound to obtain untreated control subjects.
9. The method of embodiment 1 wherein the formula 1 compound is administered as a formulation comprising the formula 1 compound and one or more excipients, optionally wherein the formulation is an oral formulation or a sterile parenteral formulation.
10. The method of embodiment 1 wherein step (3) is comparing the effect of the F1 C on the survival rate of the exposed treated subjects with the survival rate of control subjects of the same or a closely related species that had been exposed to the same or a similar or a comparable biological insult to obtain comparison controls, where the comparison controls were not treated with any comparison compound or treatment protocol.
11. A formulation comprising one or more excipients and a compound having the structure 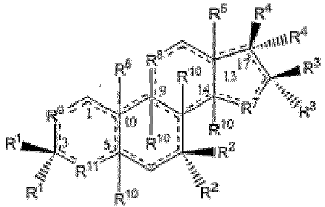
   wherein the dotted lines are optional double bonds;
   each R¹, R², R³, R⁴, R⁵, R⁶ and R¹⁰ independently or together are -H, -OH, -OR^{PR}, -SR^{PR}, -SH, -N(R^{PR})₂, -NHR^{PR}, -NH₂, -O-Si-(R¹³)₃, -CHO, -CHS, -CN, -SCN, -NO₂, -N₃, -COOH, - COOR^{PR}, -OSO₃H, -OSO₂H, -OPO₃H₂, =O, =S, =N-OH, =N-OCH₃, =CH₂, =CH-CH₃, =CH-optionally substituted alkyl, =N-optionally substituted alkyl, =N-O-optionally substituted alkyl, -NH-S(O)(O)-optionally substituted alkyl, -S-S-optionally substituted alkyl, ester, thioester, thionoester, phosphoester, phosphothioester, phosphonate, phosphonate ester, thiophosphonate, thiophosphonate ester, phosphiniester, sulfite ester, sulfate ester, sulfamate, sulfonate, sulfonamide, amide, amino acid, peptide, ether, thioether, acyl, thioacyl, carbonate, carbamate, halogen, optionally substituted alkyl, optionally substituted alkenyl, optionally substituted alkynyl, optionally substituted aryl, optionally substituted heteroaryl, optionally substituted heterocycle, optionally substituted monosaccharide, optionally substituted oligosaccharide, polymer, spiro ring, epoxide, acetal, thioacetal, ketal or a thioketal, =N-O-optionally substituted alkyl, =N-optionally substituted alkyl, -NH-optionally substituted alkyl, -N(optionally substituted alkyl)₂ where each optionally substituted alkyl is independently selected, or, one or more of two adjacent R¹, R², R³, R⁴, R⁵, R⁶ and R¹⁰ comprise an independently selected epoxide or optionally substituted, saturated or unsaturated cyclopropyl, cyclobutyl, cyclopentyl or cyclohexyl ring any of which rings optionally contain one or two independently selected -O-, -S-, -S(O)(O)-, -NH- -N(optionally substituted alkyl)- or =N-heteroatoms;
   R⁷ is -O-, -S-, -NR^{PR}-, -C(R¹⁰)₂, -C(R¹⁰)₂-C(R¹⁰)₂-, -C(R¹⁰)₂-C(R¹⁰)₂-C(R¹⁰)₂,-C(R'⁰)₂-0-C(R¹⁰)₂,₋-C(R¹⁰)₂-S-C(R¹⁰)₂, -C(R¹⁰)₂-NR^{PR}-C(R¹⁰)₂, O-C(R¹⁰)₂, -S-C(R¹⁰)₂ or-NR^{PR}-C(R¹⁰)₂, where each R¹⁰ is independently selected;
   R⁸ and R⁹ independently are -C(R¹⁰)₂, -C(R¹⁰)₂-C(R¹⁰)₂, -O-, -O-C(R¹⁰)₂, -S-, -S-C(R¹⁰)₂, - NR^{PR}- or -NR^{PR}-C(R¹⁰)₂ or one or both of R⁸ or R⁹ independently are absent, leaving a 5-membered ring, where each R¹⁰ is independently selected;
   R¹¹ is -O-, -S-, -S(O)(O)-, -NR^{PR}-, -CH₂-, -CHR¹⁰-, -C(R¹⁰)₂, -C(R¹⁰)₂-O-C(R¹⁰)₂, -C(R¹⁰)₂-S-C(R¹⁰)₂, -C(R¹⁰)₂-S(O)(O)-C(R¹⁰)₂, -C(R¹⁰)₂-N^{PR}-C(R¹⁰)₂, O-C(R¹⁰)₂, -S-C(R¹⁰)₂,-S(O)(O)-C(R¹⁰)₂ or -NR^{PR}-C(R¹⁰)₂ where each R¹⁰ is independently selected;
   R¹³ independently is C₁₋₆ alkyl; and
   R^{PR} independently are -H or a protecting group, wherein one or two independently selected R¹⁰ moieties are present at the 1-, 6- and 12-positions and wherein the compound is a 1 ,5,11-triene.

## Claims

1. A compound having the structure 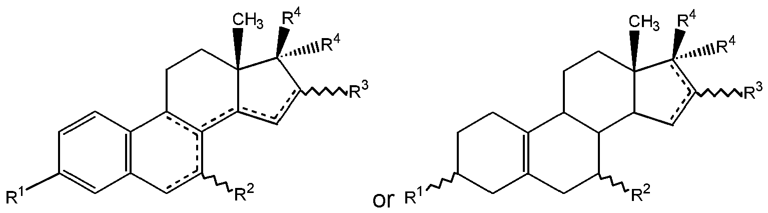 wherein
R¹ is -OH, ester or ether;
R² is -OH, ester or ether;
R³ is -OH, ester, ether or halogen;
one R⁴ is -OH, ester or ether and the other R⁴ is -H or a C-linked moiety.

2. A compound according to claim 1 wherein R³ is -OH, ester or ether and one R⁴ is -H.

3. A compound according to claim 1 or 2 wherein the compound has the structure

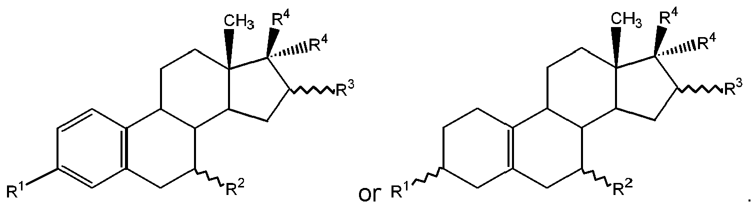

4. A compound according to claim 3 wherein R¹ R², R³ and R⁴ are -OH.

5. A compound according to claim 2 wherein R¹ is -OH, -OCH₃, - OC(O)CH₃, or -OC(O)CH₂CH₃.

6. A compound according to claim 5 wherein the compound has the structure 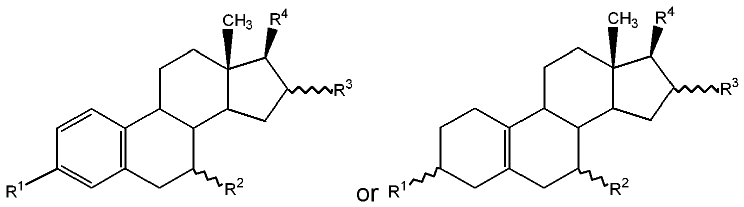 and R² is -OH, -OCH₃, -OC₂H₅, -OC(O)CH₃, or -OC(O)CH₂CH₃, R³ is -OH, -OC(O)CH₃, -F, -Cl, -Br or -I and R⁴ is -OH, -OCH₃, -OC(O)CH₃, or - OC(O)CH₂CH₃.

7. A compound according to claim 6 wherein the compound has the structure

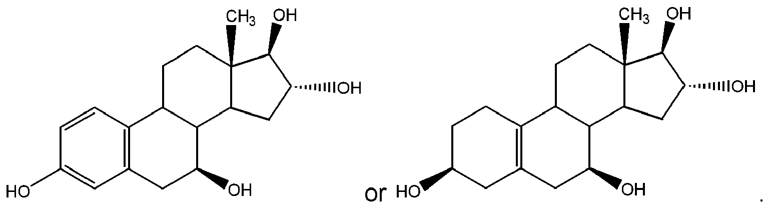

8. A pharmaceutical formulation comprising one or more excipients and a compound according to claim 1.

9. A pharmaceutical formulation according to claim 8 wherein the compound has the structure

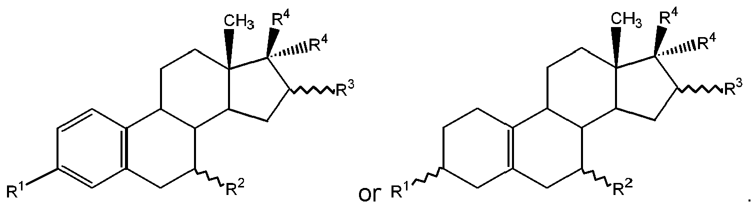

10. A pharmaceutical formulation according to claim 9 wherein R¹ is -OH, - OCH₃, -OC(O)CH₃, or -OC(O)CH₂CH₃, R² is -OH, -OCH₃, -OC₂H₅, -OC(O)CH₃, or -OC(O)CH₂CH₃, R³ is -OH, -OC(O)CH₃, -F, -Cl, -Br or -I and R⁴ is -OH, -OCH₃, -OC(O)CH₃, or -OC(O)CH₂CH₃.

11. A pharmaceutical formulation according to claim 10 wherein the compound has the structure

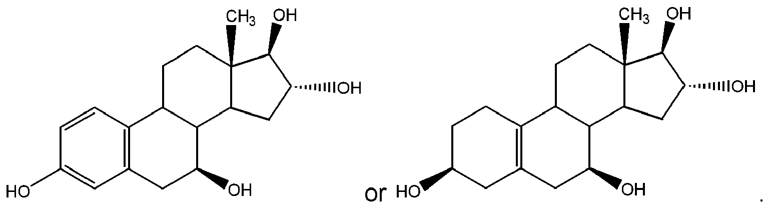

12. A compound according to claim 1 for use as a medicament.

13. A compound for use according to claim 12 wherein the compound has the structure

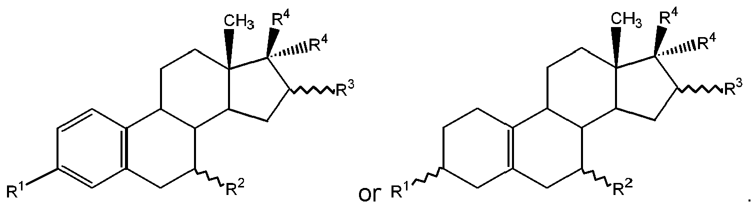

14. A compound for use according to claim 13 wherein R¹ is -OH, -OCH₃, - OC(O)CH₃, or -OC(O)CH₂CH₃, R² is -OH, -OCH₃, -OC₂H₅, -OC(O)CH₃, or - OC(O)CH₂CH₃, R³ is -OH, -OC(O)CH₃, -F, -Cl, -Br or -I and R⁴ is -OH, -OCH₃, - OC(O)CH₃, or -OC(O)CH₂CH₃.

15. A compound for use according to claim 14 wherein the compound has the structure
